# EUROPEAN PATENT SPECIFICATION

(11) **EP 4 073 054 B1**
(45) Date of publication and mention of the grant of the patent: **21.10.2026**
(21) Application number: 20823960.8
(22) Date of filing: 11.12.2020
(51) Int. Cl.: C07D 401/04, C07D 401/14, C07D 405/14, A61K 31/4709, A61P 35/00

(54) **COMPOUNDS AND COMPOSITIONS FOR THE TREATMENT OF TUMORS**
VERBINDUNGEN UND ZUSAMMENSETZUNGEN ZUR BEHANDLUNG VON TUMOREN
COMPOSÉS ET COMPOSITIONS UTILES POUR LE TRAITEMENT DES TUMEURS

(30) Priority: 11.12.2019 IT 201900023700
(43) Date of publication of application: 19.10.2022
(73) Proprietor: Fondazione Istituto Italiano di Tecnologia, 16163 Genova (IT); Tes Pharma S.r.l., 06073 Corciano (PG) (IT); Alma Mater Studiorum - Universita' Di Bologna, 40126 Bologna (IT); Istituto Europeo di Oncologia S.r.l., 20121 Milano (IT)
(72) Inventor: CAVALLI, Andrea, 16163 Genova (IT); MILANO, Domenico, 16163 Genova (IT); MANERBA, Marcella, 16163 Genova (IT); BAGNOLINI, Greta, 16163 Genova (IT); ORTEGA MARTINEZ, Jose Antonio, 16163 Genova (IT); ROBERTI, Marinella, 40126 Bologna (IT); DI STEFANO, Giuseppina, 40126 Bologna (IT); PELLICCIARI, Roberto, 06121 Perugia (IT); DE FRANCO, Francesca, 06121 Perugia (IT); MINUCCI, Saverio, 20121 Milano (IT); PELICCI, Pier Giuseppe, 20121 Milano (IT); PREVITALI, Viola, 16163 Genova (IT)
(74) Representative: Studio Torta S.p.A.
(86) International application number: PCT/IB2020/061825
(87) International publication number: WO 2021/116999

(56) References cited:
- WO-A1-2014/210456
- WO-A1-2019/200224
- WO-A2-2010/088408
- OYEWUMI MOSES O ET AL: "Screening and identification of novel compounds with potential anti-proliferative effects on gallium-resistant lung cancer through an AXL kinase pathway", BIOORGANIC & MEDICINAL CHEMISTRY LETTERS, PERGAMON, AMSTERDAM, NL, vol. 24, no. 18, 2 August 2014 (2014-08-02), pages 4553 - 4556, XP029053311, ISSN: 0960-894X, DOI: 10.1016/J.BMCL.2014.07.072
- WANG YANXING ET AL: "Discovery of novel glycogen synthase kinase-3[alpha] inhibitors: Structure-based virtual screening, preliminary SAR and biological evaluation for treatment of acute myeloid leukemia", EUROPEAN JOURNAL OF MEDICINAL CHEMISTRY, vol. 171, 20 March 2019 (2019-03-20), pages 221 - 234, XP085661309, ISSN: 0223-5234, DOI: 10.1016/J.EJMECH.2019.03.039
- T. M. ACKER ET AL: "Mechanism for Noncompetitive Inhibition by Novel GluN2C/D N-Methyl-D-aspartate Receptor Subunit-Selective Modulators", MOLECULAR PHARMACOLOGY, vol. 80, no. 5, 1 August 2011 (2011-08-01), pages 782 - 795, XP055121576, DOI: 10.1124/mol.111.073239
- DATABASE REGISTRY [online] CHEMICAL ABSTRACTS SERVICE, COLUMBUS, OHIO, US; 29 September 2015 (2015-09-29), XP002799985, retrieved from STN Database accession no. 1808772-20-4
- DATABASE REGISTRY [online] CHEMICAL ABSTRACTS SERVICE, COLUMBUS, OHIO, US; 6 July 2015 (2015-07-06), XP002799986, retrieved from STN Database accession no. 1795520-18-1
- DATABASE REGISTRY [online] CHEMICAL ABSTRACTS SERVICE, COLUMBUS, OHIO, US; 31 December 2008 (2008-12-31), XP002799987, retrieved from STN Database accession no. 1092329-52-6
- DATABASE REGISTRY [online] CHEMICAL ABSTRACTS SERVICE, COLUMBUS, OHIO, US; 24 June 2015 (2015-06-24), XP002799988, retrieved from STN Database accession no. 1787857-52-6
- DATABASE REGISTRY [online] CHEMICAL ABSTRACTS SERVICE, COLUMBUS, OHIO, US; 20 February 2002 (2002-02-20), XP002799989, retrieved from STN Database accession no. 393837-93-9
- DATABASE REGISTRY [online] CHEMICAL ABSTRACTS SERVICE, COLUMBUS, OHIO, US; 11 October 2001 (2001-10-11), XP002799990, retrieved from STN Database accession no. 361479-89-2
- DATABASE REGISTRY [online] CHEMICAL ABSTRACTS SERVICE, COLUMBUS, OHIO, US; 13 April 2001 (2001-04-13), XP002799991, retrieved from STN Database accession no. 331275-17-3
- DATABASE REGISTRY [online] CHEMICAL ABSTRACTS SERVICE, COLUMBUS, OHIO, US; 3 January 2001 (2001-01-03), XP002799992, retrieved from STN Database accession no. 312623-46-4
- DATABASE REGISTRY [online] CHEMICAL ABSTRACTS SERVICE, COLUMBUS, OHIO, US; 29 September 2015 (2015-09-29), XP002799993, retrieved from STN Database accession no. 1808401-78-6
- DATABASE REGISTRY [online] CHEMICAL ABSTRACTS SERVICE, COLUMBUS, OHIO, US; 10 July 2015 (2015-07-10), XP002799994, retrieved from STN Database accession no. 1798716-46-7
- DATABASE REGISTRY [online] CHEMICAL ABSTRACTS SERVICE, COLUMBUS, OHIO, US; 24 June 2015 (2015-06-24), XP002799995, retrieved from STN Database accession no. 1787857-52-6
- DATABASE REGISTRY [online] CHEMICAL ABSTRACTS SERVICE, COLUMBUS, OHIO, US; 22 July 2004 (2004-07-22), XP002799996, retrieved from STN Database accession no. 714239-58-4
- DATABASE REGISTRY [online] CHEMICAL ABSTRACTS SERVICE, COLUMBUS, OHIO, US; 9 April 2004 (2004-04-09), XP002799997, retrieved from STN Database accession no. 673445-92-6
- DATABASE REGISTRY [online] CHEMICAL ABSTRACTS SERVICE, COLUMBUS, OHIO, US; 23 April 2002 (2002-04-23), XP002799998, retrieved from STN Database accession no. 406724-28-5
- DATABASE REGISTRY [online] CHEMICAL ABSTRACTS SERVICE, COLUMBUS, OHIO, US; 21 February 2002 (2002-02-21), XP002799999, retrieved from STN Database accession no. 394225-81-1

## Description

### CROSS-REFERENCE TO RELATED APPLICATIONS

This Patent Application claims priority from Italian Patent Application No. 102019000023700 filed on December 11, 2019.

### TECHNICAL FIELD

Synthetic lethality is a new concept with great potential for discovering new anticancer molecules within the framework of precision medicine. Synthetic lethality arose from genetic studies in model organisms. Two genes are synthetically lethal if the perturbation of either gene alone is compatible with cell viability, but simultaneous perturbation of both genes leads to cell death (1).

### BACKGROUND ART

In recent years, synthetic lethality explained the sensitivity of some cancer cells to certain drugs and provided a new perspective for anticancer therapy. Indeed, synthetic lethality shows that is possible to kill selectively cancer cells, while sparing healthy cells, by using a small organic molecule to target the synthetically lethal partner of a mutant gene (2). While one DNA repair mechanism is genetically inactivated, the other is inhibited by the small organic molecule. Selectivity comes from the much higher genetic instability of cancer cells, compared to normal cells, which are therefore much more sensitive to the inhibition of DNA repair mechanisms.

One example of synthetic lethality is the use of poly [ADPribose] polymerase (PARP) inhibitors in oncology patients with BRCA1/2 mutations. BRCA2 is important for repairing DNA double-strands breaks (DSBs) by homologous recombination (HR), whereas PARP is important for repairing single-strand breaks. Synthetic lethality arises from the simultaneous impairment of the repair mechanisms for single-strand and double-strand breaks. Olaparib (Lynparza^{™}), developed by AstraZeneca, is an example of PARP inhibitor (PARPi) which exploits synthetic lethality mechanism. In 2014, it was the first PARPi to treat advanced ovarian cancer associated with defective BRCA genes (3) and, nowadays, is in clinical trials to treat breast and pancreatic tumors with defective BRCA genes.

Synthetic lethality concept can be broader than its genetic origin; the inhibition of two independent DNA repair processes can mimic synthetic lethality in the absence of gene mutations (4). In the case of BRCA2, its key mechanism point is the recruitment of Rad51, an evolutionarily conserved recombinase that uses homologous recombination to repair double-strand breaks. Rad51 and BRCA2 interact through eight well-conserved motifs, namely the BRC repeats (5).In a previous study, the same authors proved that the disruption of the Rad51-BRCA2 interaction with small organic molecules results in a pharmacologically induced synthetic lethality when combined with PARP inhibitors, like Olaparib, or a DNA damage agent, like Cisplatin (6, 7).

This result was explained by the chemical inhibition of both single and double-strand DNA repair mechanisms simultaneously. Rad51 is over expressed in a wide variety of cancers (e.g. ovarian, breast and pancreatic), where an enhanced homologous recombination activity has been observed (8).

This can be explained by the increased genetic instability of cancer cells, which experience DNA damage more frequently than healthy ones. Therefore, the disruption Rad51-BRCA2 interaction could make cancer cells more sensitive to PARPi, DNA-damaging agents, and radiotherapy in individuals without BRCA mutations because a sensitivity to PARP inhibitors will be induced, as observed in genetically BRCA defective oncology patients.

Accordingly, there is a need for novel compounds to be used for the disruption Rad51-BRCA2 interaction, for example for the treatment of cancer.

WO2014210456A1 discloses pyrazoline dihydroquinolone derivatives for the treatment of brain tumors. These compounds are developed as selective NMDA receptor inhibitors and their use in therapeutic methods related thereto.

### DISCLOSURE OF INVENTION

The aim of the present invention is to provide novel compounds acting as Rad51-BRCA2 interaction disruptors.

The aforementioned objective has been met according to compounds of claim 1, to a pharmaceutical composition of claim 6, to the uses of claims 7 and 8. Preferred embodiments are set out within the dependent claims.

### BRIEF DESCRIPTION OF THE DRAWINGS

The present invention will be described in detail with reference to the figures in the annexed drawings, which show purely illustrative and non-exhaustive examples in which:
- Figure 1 illustrates the pharmacokinetic profile of compound d.12 administered intravenously;
- Figure 2 illustrates the pharmacokinetic profile of compound d.12 administered orally;
- Figure 3 illustrates the pharmacokinetic profile of compound d.12 administered intraperitoneally.

### BEST MODE FOR CARRYING OUT THE INVENTION

The following paragraphs provide definitions of the various chemical moieties of the compounds according to the invention and are intended to apply uniformly throughout the specification and claims unless an otherwise expressly set out definition provides a broader definition.

The term "alkyl", as used herein, refers to saturated aliphatic hydrocarbon groups. Such term includes straight (unbranched) chains or branched chains.

Non-limiting examples of alkyl groups according to the invention are, for example, methyl, ethyl, propyl, isopropyl, n-butyl, iso-butyl, tert-butyl, n-pentyl, *iso*pentyl, n-hexyl and the like.

The terms "alkenyl" and "alkynyl", as used herein, refer to partially unsaturated aliphatic hydrocarbon groups. Such term includes straight (unbranched) chains or branched chains.

Non-limiting examples of alkyl groups according to the invention are, for example, ethenyl, 1-propenyl, 2-propenyl, 1- or 2-butenyl, ethynyl, 1-propynyl, 2-propynyl, 1- or 2-butynyl, and the like.

The term "cycloalkyl", as used herein, refers to a saturated or partially unsaturated carbocyclic group having a single ring. It includes cycloalkenyl groups.

Non-limiting examples of cycloalkyl groups according to the invention are, for example, cyclopropane, cyclobutane, cyclopentane, cyclohexane, cycloheptane, cyclopentene, cyclohexene, cyclohexadiene and the like.

The term "heterocycloalkyl" group, ("non-aromatic heterocycle" group), refers to a cycloalkyl group (non aromatic group) wherein at least one of the carbon atoms has been replaced by a heteroatom selected from nitrogen, oxygen and sulfur. Heterocycloalkyl groups can be unsubstituted or substituted by one or more substituents as defined below.

Examples of heterocycloalkyls include, but are not limited to lactams, lactones, cyclic imides, cyclic thioimides, cyclic carbamates, 1-(1,2,5,6-tetrahydropyridyl), tetrahydrothiopyran, 4H-pyran, tetrahydropyran, piperidine (1-piperidinyl, 2-piperidinyl, 3-piperidinyl), 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, morpholine (4-morpholinyl, 3-morpholinyl, 2-morpholinyl) trioxane, hexahydro-1,3,5-triazine, tetrahydrothiophene, tetrahydrofuran (tetrahydrofuran-2-yl, tetrahydrofuran-3-yl), pyrroline, pyrrolidine, pyrrolidone, pyrrolidindione, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane.

The term "halogen", as used herein, refers to fluorine, chlorine, bromine and iodine.

The term "aryl", as used herein, refers to a hydrocarbon consisting of an unsubstituted or substituted mono-, bi- or tricarbocyclic ring system, wherein the rings are fused together and at least one of the carbocyclic ring is aromatic. The term "aryl" means for example a cyclic aromatic such as a 6-membered hydrocarbon ring, a two six-membered fused hydrocarbon rings. Non-limiting examples of aryl groups are, for example, phenyl, alpha- or beta-naphthyl, 9,10-dihydroanthracenyl, indanyl, fluorenyl and the like. Aryl groups according to the present invention may be unsubstituted or substituted by one or more substituents as defined below.

The term "heteroaryl", as used herein, refers to an aryl as defined above wherein one to four carbon atoms are independently replaced by heteroatoms chosen from the group consisting of nitrogen, oxygen and sulphur. Non-limiting examples of heteroaryl groups are, for example, pyrrolyl, furyl, thiophenyl, imidazolyl, pyrazolyl, oxazolyl, isoxazolyl, thiazolyl, isothiazolyl, indolyl, benzofuranyl, benzothiophenyl, benzimidazolyl, benzopyrazolyl, benzoxazolyl, benzoisoxazolyl, benzothiazolyl, benzoisothiazolyl, triazolyl, oxadiazolyl, tetrazolyl, pyridyl, pyrazinyl, pyrimidinyl, pyridazinyl, quinolinyl, isoquinolinyl, quinazolinyl, quinoxalinyl. Heteroaryl groups according to the present invention may be unsubstituted or substituted by one or more substituents as defined below.

The term "pharmaceutically acceptable salts" refers to salts of the below identified compounds of Formula (I) that retain the desired biological activity and are accepted by regulatory authorities.

As used herein, the term "salt" refers to any salt of a compound according to the present invention prepared from an inorganic or organic acid or base and internally formed salts. Typically, such salts have a physiologically acceptable anion or cation.

Furthermore, the compounds of Formula (Ia) and (Ib) may form an acid addition salt or a salt with a base, depending on the kind of the substituents, and these salts are included in the present invention, as long as they are pharmaceutically acceptable salts.

Examples of such salts include, but are not restricted to acid addition salts formed with inorganic acids (e. g. hydrochloric acid, hydrobromic acid, sulfuric acid, phosphoric acid, nitric acid, and the like), and salts formed with organic acids such as acetic acid, trifluoroacetic acid, oxalic acid, tartaric acid, succinic acid, malic acid, fumaric acid, maleic acid, ascorbic acid, benzoic acid, alginic acid, polyglutamic acid, methanesulfonic acid, p-toluene sulfonic acid, and naphthalene sulfonic acid.

The compounds of Formula (Ia) and (Ib) containing acidic protons may be converted into their therapeutically active, non-toxic base addition salt forms, e.g. metal or amine salts, by treatment with appropriate organic and inorganic bases. Appropriate base salt forms include, for example, ammonium salts, alkali and earth alkaline metal salts, e.g. lithium, sodium, potassium, magnesium, calcium salts and the like, salts with organic bases, e.g. N-methyl-D-glucamine, hydrabamine salts, and salts with amino acids such as, for example, arginine, lysine and the like.

Physiologically or pharmaceutically acceptable salts are particularly suitable for medical applications because of their greater aqueous solubility relative to the parent compound.

Pharmaceutically acceptable salts may also be prepared from other salts including other pharmaceutically acceptable salts of the compounds of Formula (Ia) and (Ib) using conventional methods.

Those skilled in the art of organic chemistry will appreciate that many organic compounds can form complexes with solvents in which they are reacted or from which they are precipitated or crystallized. These complexes are known as "solvates". For example, a complex with water is known as a "hydrate".

The compounds of Formula (I) may readily be isolated in association with solvent molecules by crystallization or evaporation of an appropriate solvent to give the corresponding solvates.

The compounds of Formula (Ia) and (Ib) may be in crystalline form.

Certain groups/substituents included in the present invention may be present as isomers or in one or more tautomeric forms. Accordingly, in certain embodiments, the compounds of Formula (Ia) and (Ib) may exist in the form of other tautomers or geometrical isomers in some cases, depending on the kinds of the substituents. In the present specification, the compounds may be described in only one form of such isomers, but the present invention includes all such isomers, isolated forms of the isomers, or a mixture thereof. Furthermore, the compounds of Formula (Ia) and (Ib) may have asymmetric carbon atoms or axial asymmetries in some cases and, correspondingly, they may exist in the form of optical isomers such as an (R)-form, an (S)-form, and the like. The present invention includes such isomers, including racemates, enantiomers and mixtures thereof.

In the present invention are included all stereoisomeric forms, including enantiomers, diastereoisomers, and mixtures thereof, including racemates and the general reference to the compounds of Formula (Ia) and (Ib) includes all the stereoisomeric forms, unless otherwise indicated.

In general, the compounds or salts of the invention should be interpreted as excluding those compounds (if any) which are so chemically unstable, either *per* se or in water, that they are clearly unsuitable for pharmaceutical use through all administration routes, whether oral, parenteral, or otherwise. Such compounds are known to the skilled chemist.

According to a **first aspect** of the invention, compounds of Formula (Ia): 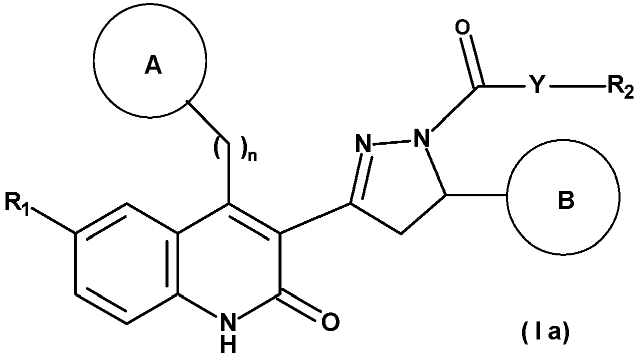 or its pharmaceutically acceptable salts, or solvates thereof are provided.

In the compounds of Formula (Ia):
**R₁** is halogen;
**R₂** is selected from the group consisting of hydrogen, and COOH;
**n** is 1;
**Y** is C₁₋₄alkyl;
**A** is phenyl
**B** is 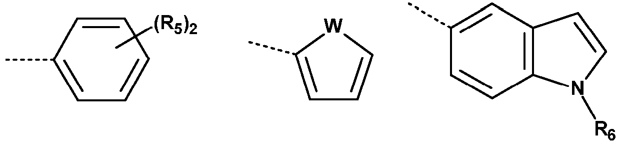 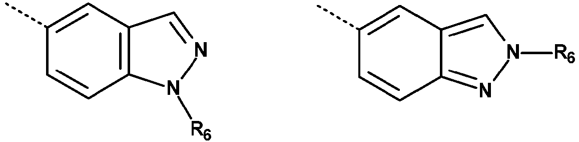 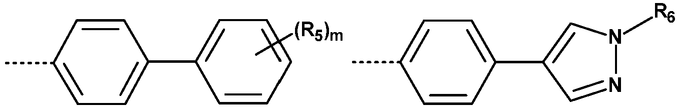 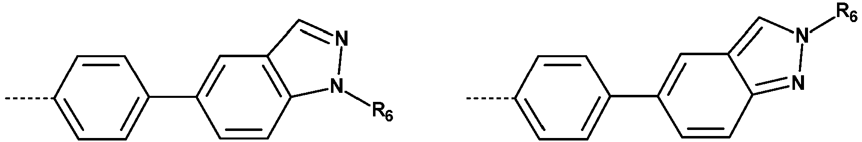 **R₅** is hydrogen, halogen, linear or branched C₁₋₄alkyl, halo-C₁₋₄alkyl, O-C₁₋₄alkyl;
**R₆** is selected from the group consisting of H and C₁₋₄alkyl;
**m** is 1 or 2; and
**W** is selected from the group consisting of O and NH;
or compounds of formula (Ia): 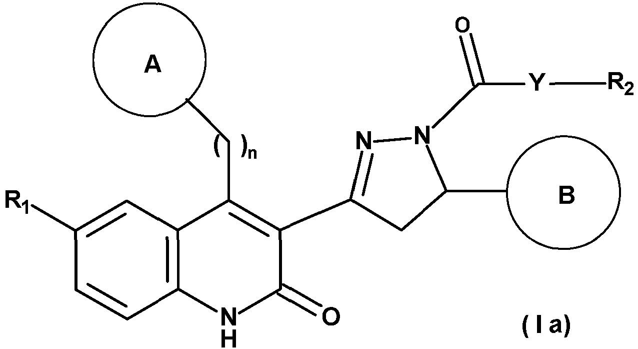
wherein
**R₁** is halogen;
**R₂** is selected from the group consisting of hydrogen and COOH;
**n** is 0;
**Y** is selected from the group consisting of C₁₋₄alkyl;
**A** is morpholino;
**B** is 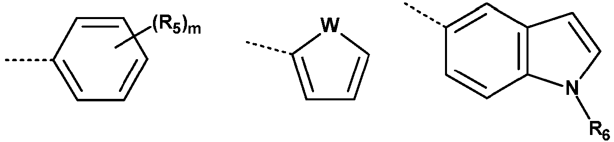 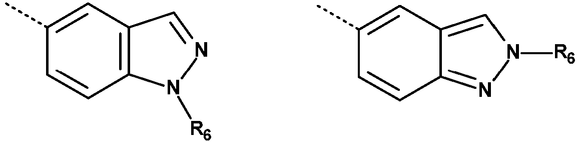 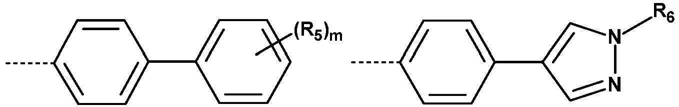 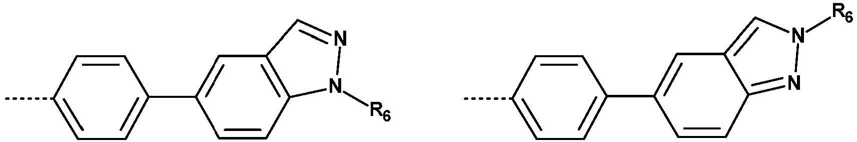
**R₅** is hydrogen, halogen, linear or branched C₁₋₄alkyl, halo-C₁₋₄alkyl, O-C₁₋₄alkyl;
**R₆** is selected from the group consisting of H and C₁₋₄alkyl;
**m** is 1 or 2; and
**W** is selected from the group consisting of O and NH,
or their pharmaceutically acceptable salts or solvates thereof.

According to a **second embodiment,** the compounds of formula (Ia) can be selected from the group consisting of:

| | |
|---|---|
| d.50 | 4-[5-(4-benzyl-6-chloro-2-oxo-1H-quinolin-3-yl)-3-(4-fluorophenyl)-3,4-dihydropyrazol-2-yl]-4-oxo-butanoic acid |
| d.51 | 3-[2-acetyl-3-(4-fluorophenyl)-3,4-dihydropyrazol-5-yl]-4-benzyl-6-chloro-1H-quinolin-2-one |
| d.52 | 3-[2-acetyl-3-(4-methoxyphenyl)-3,4-dihydropyrazol-5-yl]-4-benzyl-6-chloro-1H-quinolin-2-one |
| d.53 | 4-benzyl-6-chloro-3-[3-(4-methoxyphenyl)-2-propanoyl-3,4-dihydropyrazol-5-yl]-1H-quinolin-2-one |
| d.54 | 4-benzyl-6-chloro-3-[3-(4-fluorophenyl)-2-propanoyl-3,4-dihydropyrazol-5-yl]-1H-quinolin-2-one |
| d.55 | 4-[5-(4-benzyl-6-chloro-2-oxo-1H-quinolin-3-yl)-3-(4-chlorophenyl)-3,4-dihydropyrazol-2-yl]-4-oxo-butanoic acid |
| d.56 | 3-[2-acetyl-3-(4-chlorophenyl)-3,4-dihydropyrazol-5-yl]-4-benzyl-6-chloro-1H-quinolin-2-one |
| d.57 | 4-benzyl-6-chloro-3-[3-(4-chlorophenyl)-2-propanoyl-3,4-dihydropyrazol-5-yl]-1H-quinolin-2-one |
| d.58 | 4-[5-(4-benzyl-6-chloro-2-oxo-1H-quinolin-3-yl)-3-(4-bromophenyl)-3,4-dihydropyrazol-2-yl]-4-oxo-butanoic acid |
| d.59 | 3-[2-acetyl-3-(4-bromophenyl)-3,4-dihydropyrazol-5-yl]-4-benzyl-6-chloro-1H-quinolin-2-one |
| d. 60 | 4-benzyl-3-[3-(4-bromophenyl)-2-propanoyl-3,4-dihydropyrazol-5-yl]-6-chloro-1H-quinolin-2-one |
| d.61 | 4-[5-(4-benzyl-6-chloro-2-oxo-1H-quinolin-3-yl)-3-(4-methoxyphenyl)-3,4-dihydropyrazol-2-yl]-4-oxo-butanoic acid |
| d. 62 | 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid |
| d.63 | 3-[2-acetyl-3-[4-(trifluoromethyl)phenyl]-3,4-dihydropyrazol-5-yl]-4-benzyl-6-chloro-1H-quinolin-2-one |
| d.64 | 4-[5-(4-benzyl-6-chloro-2-oxo-1H-quinolin-3-yl)-3-(4-tert-butylphenyl)-3,4-dihydropyrazol-2-yl]-4-oxo-butanoic acid |
| d.65 | 3-[2-acetyl-3-(4-tert-butylphenyl)-3,4-dihydropyrazol-5-yl]-4-benzyl-6-chloro-1H-quinolin-2-one |
| d. 66 | 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid |
| d.67 | 4-[5-(4-benzyl-6-chloro-2-oxo-1H-quinolin-3-yl)-3-(2-furyl)-3,4-dihydropyrazol-2-yl]-4-oxo-butanoic acid |
| d.68 | 3-[2-acetyl-3-(2-furyl)-3,4-dihydropyrazol-5-yl]-4-benzyl-6-chloro-1H-quinolin-2-one |
| d.69 | 4-[5-(4-benzyl-6-chloro-2-oxo-1H-quinolin-3-yl)-3-(1-methylindol-5-yl)-3,4-dihydropyrazol-2-yl]-4-oxo-butanoic acid |
| d.70 | 3-[2-acetyl-3-(1-methylindol-5-yl)-3,4-dihydropyrazol-5-yl]-4-benzyl-6-chloro-1H-quinolin-2-one |
| d.71 | 4-[5-(4-benzyl-6-chloro-2-oxo-1H-quinolin-3-yl)-3-(1-ethylindol-5-yl)-3,4-dihydropyrazol-2-yl]-4-oxo-butanoic acid |
| d.72 | 3-[2-acetyl-3-(1-ethylindol-5-yl)-3,4-dihydropyrazol-5-yl]-4-benzyl-6-chloro-1H-quinolin-2-one |
| d.73 | 4-[5-(4-benzyl-6-chloro-2-oxo-1H-quinolin-3-yl)-3-(1-methylindazol-5-yl)-3,4-dihydropyrazol-2-yl]-4-oxo-butanoic acid |
| d.74 | 3-[2-acetyl-3-(1-methylindazol-5-yl)-3,4-dihydropyrazol-5-yl]-4-benzyl-6-chloro-1H-quinolin-2-one |
| d.75 | 4-[5-(4-benzyl-6-chloro-2-oxo-1H-quinolin-3-yl)-3-(2-methylindazol-5-yl)-3,4-dihydropyrazol-2-yl]-4-oxo-butanoic acid |
| d.76 | 4-[5-(4-benzyl-6-chloro-2-oxo-1H-quinolin-3-yl)-3-(2-ethylindazol-5-yl)-3,4-dihydropyrazol-2-yl]-4-oxo-butanoic acid |
| d.77 | 3-[2-acetyl-3-(2-ethylindazol-5-yl)-3,4-dihydropyrazol-5-yl]-4-benzyl-6-chloro-1H-quinolin-2-one |
| d.78 | 4-[5-(4-benzyl-6-chloro-2-oxo-1H-quinolin-3-yl)-3-(1-ethylindazol-5-yl)-3,4-dihydropyrazol-2-yl]-4-oxo-butanoic acid |
| d.79 | 3-[2-acetyl-3-(1-ethylindazol-5-yl)-3,4-dihydropyrazol-5-yl]-4-benzyl-6-chloro-1H-quinolin-2-one |
| d.80 | 4-[5-(4-benzyl-6-chloro-2-oxo-1H-quinolin-3-yl)-3-(1-propylindazol-5-yl)-3,4-dihydropyrazol-2-yl]-4-oxo-butanoic acid |
| d.81 | 3-[2-acetyl-3-(1-propylindazol-5-yl)-3,4-dihydropyrazol-5-yl]-4-benzyl-6-chloro-1H-quinolin-2-one |
| d.82 | 4-[5-(4-benzyl-6-chloro-2-oxo-1H-quinolin-3-yl)-3-(2-propylindazol-5-yl)-3,4-dihydropyrazol-2-yl]-4-oxo-butanoic acid |
| d.83 | 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid |
| d.84 | 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid |
| d.85 | 3-[2-acetyl-3-[4-(4-chlorophenyl)phenyl]-3,4-dihydropyrazol-5-yl]-4-benzyl-6-chloro-1H-quinolin-2-one |
| d.86 | 4-[5-(4-benzyl-6-chloro-2-oxo-1H-quinolin-3-yl)-3-[4-(4-bromophenyl)phenyl]-3,4-dihydropyrazol-2-yl]-4-oxo-butanoic acid |
| d.87 | 3-[2-acetyl-3-[4-(4-bromophenyl)phenyl]-3,4-dihydropyrazol-5-yl]-4-benzyl-6-chloro-1H-quinolin-2-one |
| d.88 | 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid |
| d.89 | 3-[2-acetyl-3-[4-(4-methoxyphenyl)phenyl]-3,4-dihydropyrazol-5-yl]-4-benzyl-6-chloro-1H-quinolin-2-one |
| d.90 | 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid |
| d.91 | 3-[2-acetyl-3-[4-(1-propylpyrazol-4-yl)phenyl]-3,4-dihydropyrazol-5-yl]-4-benzyl-6-chloro-1H-quinolin-2-one |
| d.92 | 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid |
| d.93 | 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid |
| d.94 | 3-(2-acetyl-3-phenyl-3,4-dihydropyrazol-5-yl)-4-benzyl-6-chloro-1H-quinolin-2-one |
| d.95 | 4-benzyl-6-chloro-3-(3-phenyl-2-propanoyl-3,4-dihydropyrazol-5-yl)-1H-quinolin-2-one |
| d.96 | 3-[2-acetyl-3-(p-tolyl)-3,4-dihydropyrazol-5-yl]-4-benzyl-6-chloro-1H-quinolin-2-one |
| d.101 | 4-[5-(4-benzyl-6-chloro-2-oxo-1H-quinolin-3-yl)-3-(3-pyridyl)-3,4-dihydropyrazol-2-yl]-4-oxo-butanoic acid |
| d.105 | 4-[5-(4-benzyl-6-chloro-2-oxo-1H-quinolin-3-yl)-3-(2-chlorophenyl)-3,4-dihydropyrazol-2-yl]-4-oxo-butanoic acid |
| d.106 | 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid |
| d.109 | 4-[5-(6-chloro-4-morpholino-2-oxo-1H-quinolin-3-yl)-3-(4-methoxyphenyl)-3,4-dihydropyrazol-2-yl]-4-oxo-butanoic acid |

A second aspect of the present invention relates to a pharmaceutical composition comprising a compound of Formula (Ia) or pharmaceutically acceptable salts, hydrates, solvates, clathrates, thereof and a pharmaceutically acceptable carrier, stabilizer, diluent or excipient thereof. A person skilled in the art is aware of a whole variety of such carrier, diluent or excipient compounds suitable to formulate a pharmaceutical composition.

Compounds of Formula (Ia), together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral administration (including subcutaneous and intravenous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Pharmaceutical compositions containing a compound of this invention can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the present invention can be administered by a variety of routes including oral, rectal, subcutaneous, intravenous, intramuscular, intranasal and pulmonary routes. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include pre-filled, pre-measured ampoules or syringes of the liquid compositions or pills, tablets, capsules or the like in the case of solid compositions.

Liquid forms suitable for oral administration may include a suitable aqueous or non-aqueous vehicle with buffers, suspending and dispensing agents, colorants, flavours and the like. Solid forms may include, for example, any of the following ingredients, or compounds of a similar nature: a binder such as microcrystalline cellulose, gum tragacanth, acacia, corn starch or gelatine; an excipient such as starch, dicalcium phosphate or lactose, a disintegrating agent such as alginic acid, Primogel or corn starch; a lubricant such as magnesium stearate; a glidant such as colloidal silicon dioxide; a sweetening agent such as sucrose, lactose or saccharin; or a flavouring agent such as peppermint, methyl salicylate, or orange flavouring.

Injectable compositions are typically based upon injectable sterile saline or phosphate-buffered saline or other injectable carriers known in the art.

The pharmaceutical compositions may be in the form of tablets, pills, capsules, solutions, suspensions, emulsion, powders, suppository and as sustained release formulations.

If desired, tablets may be coated by standard aqueous or non-aqueous techniques. In certain embodiments, such compositions and preparations can contain at least 0.1 percent of active compound. The percentage of active compound in these compositions may, of course, be varied and may conveniently be between about 1 percent to about 60 percent of the weight of the unit. The amount of active compound in such therapeutically useful compositions is such that therapeutically active dosage will be obtained.

When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring agent such as cherry or orange flavor. To prevent breakdown during transit through the upper portion of the gastrointestinal tract, the composition be an enteric coated formulation.

The active compounds can also be administered intranasally as, for example, liquid drops or spray.

Compositions for pulmonary administration include, but are not limited to, dry powder compositions consisting of the powder of a compound of Formula (Ia) or a salt thereof, and the powder of a suitable carrier and/or lubricant. The compositions for pulmonary administration can be inhaled from any suitable dry powder inhaler device known to a person skilled in the art.

Administration of the compositions is performed under a protocol and at a dosage sufficient to reduce the inflammation and pain in the subject. In some embodiments, in the pharmaceutical compositions of the present invention the active principle or active principles are generally formulated in dosage units. The dosage unit may contain from 0.1 to 1000 mg of a compound of Formula (Ia) per dosage unit for daily administration.

In some embodiments, the amounts effective for a specific formulation will depend on the severity of the disease, disorder or condition, previous therapy, the individual's health status and response to the drug. In some embodiments, the dose is in the range from 0.001% by weight to about 60% by weight of the formulation.

When used in combination with one or more other active ingredients, the compound of the present invention and the other active ingredient may be used in lower doses than when each is used singly.

Concerning formulations with respect to any variety of routes of administration, methods and formulations for the administration of drugs are disclosed in Remington's Pharmaceutical Sciences, 17th Edition, Gennaro et al. Eds., Mack Publishing Co., 1985, and Remington's Pharmaceutical Sciences, Gennaro AR ed. 20th Edition, 2000, Williams & Wilkins PA, USA, and Remington: The Science and Practice of Pharmacy, 21st Edition, Lippincott Williams & Wilkins Eds., 2005; and in Loyd V. Allen and Howard C. Ansel, Ansel's Pharmaceutical Dosage Forms and Drug Delivery Systems, 10th Edition, Lippincott Williams & Wilkins Eds., 2014.

The above described components for orally administered or injectable compositions are merely representative.

The compounds of this invention can also be administered in sustained release forms or from sustained release drug delivery systems.

A **third aspect** of the present invention relates to compounds of Formula (Ia) as disclosed above or the pharmaceutical composition thereof, for the use as a medicament.

A **fourth aspect** of the present invention relates to compounds of Formula (Ia) or pharmaceutically acceptable salts, or solvates thereof, for the use in the treatment of diseases or disorders selected from the group consisting of primary and metastatic neoplastic diseases, hyperplasia, metaplasia, dysplasia, cancer, cancer metastasis, benign tumors, hyperproliferative disorders, preferably selected from the group consisting of ovarian, breast and pancreatic cancer.

In particular, the following compounds can be used:

| | |
|---|---|
| d.50 | 4-[5-(4-benzyl-6-chloro-2-oxo-1H-quinolin-3-yl)-3-(4-fluorophenyl)-3,4-dihydropyrazol-2-yl]-4-oxo-butanoic acid |
| d.51 | 3-[2-acetyl-3-(4-fluorophenyl)-3,4-dihydropyrazol-5-yl]-4-benzyl-6-chloro-1H-quinolin-2-one |
| d.52 | 3-[2-acetyl-3-(4-methoxyphenyl)-3,4-dihydropyrazol-5-yl]-4-benzyl-6-chloro-1H-quinolin-2-one |
| d.53 | 4-benzyl-6-chloro-3-[3-(4-methoxyphenyl)-2-propanoyl-3,4-dihydropyrazol-5-yl]-1H-quinolin-2-one |
| d.54 | 4-benzyl-6-chloro-3-[3-(4-fluorophenyl)-2-propanoyl-3,4-dihydropyrazol-5-yl]-1H-quinolin-2-one |
| d.55 | 4-[5-(4-benzyl-6-chloro-2-oxo-1H-quinolin-3-yl)-3-(4-chlorophenyl)-3,4-dihydropyrazol-2-yl]-4-oxo-butanoic acid |
| d.56 | 3-[2-acetyl-3-(4-chlorophenyl)-3,4-dihydropyrazol-5-yl]-4-benzyl-6-chloro-1H-quinolin-2-one |
| d.57 | 4-benzyl-6-chloro-3-[3-(4-chlorophenyl)-2-propanoyl-3,4-dihydropyrazol-5-yl]-1H-quinolin-2-one |
| d.58 | 4-[5-(4-benzyl-6-chloro-2-oxo-1H-quinolin-3-yl)-3-(4-bromophenyl)-3,4-dihydropyrazol-2-yl]-4-oxo-butanoic acid |
| d.59 | 3-[2-acetyl-3-(4-bromophenyl)-3,4-dihydropyrazol-5-yl]-4-benzyl-6-chloro-1H-quinolin-2-one |
| d.60 | 4-benzyl-3-[3-(4-bromophenyl)-2-propanoyl-3,4-dihydropyrazol-5-yl]-6-chloro-1H-quinolin-2-one |
| d.61 | 4-[5-(4-benzyl-6-chloro-2-oxo-1H-quinolin-3-yl)-3-(4-methoxyphenyl)-3,4-dihydropyrazol-2-yl]-4-oxo-butanoic acid |
| d.62 | 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid |
| d.63 | 3-[2-acetyl-3-[4-(trifluoromethyl)phenyl]-3,4-dihydropyrazol-5-yl]-4-benzyl-6-chloro-1H-quinolin-2-one |
| d.64 | 4-[5-(4-benzyl-6-chloro-2-oxo-1H-quinolin-3-yl)-3-(4-tert-butylphenyl)-3,4-dihydropyrazol-2-yl]-4-oxo-butanoic acid |
| d.65 | 3-[2-acetyl-3-(4-tert-butylphenyl)-3,4-dihydropyrazol-5-yl]-4-benzyl-6-chloro-1H-quinolin-2-one |
| d.66 | 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid |
| d.67 | 4-[5-(4-benzyl-6-chloro-2-oxo-1H-quinolin-3-yl)-3-(2-furyl)-3,4-dihydropyrazol-2-yl]-4-oxo-butanoic acid |
| d.68 | 3-[2-acetyl-3-(2-furyl)-3,4-dihydropyrazol-5-yl]-4-benzyl-6-chloro-1H-quinolin-2-one |
| d.69 | 4-[5-(4-benzyl-6-chloro-2-oxo-1H-quinolin-3-yl)-3-(1-methylindol-5-yl)-3,4-dihydropyrazol-2-yl]-4-oxo-butanoic acid |
| d.70 | 3-[2-acetyl-3-(1-methylindol-5-yl)-3,4-dihydropyrazol-5-yl]-4-benzyl-6-chloro-1H-quinolin-2-one |
| d.71 | 4-[5-(4-benzyl-6-chloro-2-oxo-1H-quinolin-3-yl)-3-(1-ethylindol-5-yl)-3,4-dihydropyrazol-2-yl]-4-oxo-butanoic acid |
| d.72 | 3-[2-acetyl-3-(1-ethylindol-5-yl)-3,4-dihydropyrazol-5-yl]-4-benzyl-6-chloro-1H-quinolin-2-one |
| d.73 | 4-[5-(4-benzyl-6-chloro-2-oxo-1H-quinolin-3-yl)-3-(1-methylindazol-5-yl)-3,4-dihydropyrazol-2-yl]-4-oxo-butanoic acid |
| d.74 | 3-[2-acetyl-3-(1-methylindazol-5-yl)-3,4-dihydropyrazol-5-yl]-4-benzyl-6-chloro-1H-quinolin-2-one |
| d.75 | 4-[5-(4-benzyl-6-chloro-2-oxo-1H-quinolin-3-yl)-3-(2-methylindazol-5-yl)-3,4-dihydropyrazol-2-yl]-4-oxo-butanoic acid |
| d.76 | 4-[5-(4-benzyl-6-chloro-2-oxo-1H-quinolin-3-yl)-3-(2-ethylindazol-5-yl)-3,4-dihydropyrazol-2-yl]-4-oxo-butanoic acid |
| d.77 | 3-[2-acetyl-3-(2-ethylindazol-5-yl)-3,4-dihydropyrazol-5-yl]-4-benzyl-6-chloro-1H-quinolin-2-one |
| d.78 | 4-[5-(4-benzyl-6-chloro-2-oxo-1H-quinolin-3-yl)-3-(1-ethylindazol-5-yl)-3,4-dihydropyrazol-2-yl]-4-oxo-butanoic acid |
| d.79 | 3-[2-acetyl-3-(1-ethylindazol-5-yl)-3,4-dihydropyrazol-5-yl]-4-benzyl-6-chloro-1H-quinolin-2-one |
| d.80 | 4-[5-(4-benzyl-6-chloro-2-oxo-1H-quinolin-3-yl)-3-(1-propylindazol-5-yl)-3,4-dihydropyrazol-2-yl]-4-oxo-butanoic acid |
| d.81 | 3-[2-acetyl-3-(1-propylindazol-5-yl)-3,4-dihydropyrazol-5-yl]-4-benzyl-6-chloro-1H-quinolin-2-one |
| d.82 | 4-[5-(4-benzyl-6-chloro-2-oxo-1H-quinolin-3-yl)-3-(2-propylindazol-5-yl)-3,4-dihydropyrazol-2-yl]-4-oxo-butanoic acid |
| d.83 | 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid |
| d.84 | 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid |
| d.85 | 3-[2-acetyl-3-[4-(4-chlorophenyl)phenyl]-3,4-dihydropyrazol-5-yl]-4-benzyl-6-chloro-1H-quinolin-2-one |
| d.86 | 4-[5-(4-benzyl-6-chloro-2-oxo-1H-quinolin-3-yl)-3-[4-(4-bromophenyl)phenyl]-3,4-dihydropyrazol-2-yl]-4-oxo-butanoic acid |
| d.87 | 3-[2-acetyl-3-[4-(4-bromophenyl)phenyl]-3,4-dihydropyrazol-5-yl]-4-benzyl-6-chloro-1H-quinolin-2-one |
| d.88 | 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid |
| d.89 | 3-[2-acetyl-3-[4-(4-methoxyphenyl)phenyl]-3,4-dihydropyrazol-5-yl]-4-benzyl-6-chloro-1H-quinolin-2-one |
| d.90 | 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid |
| d.91 | 3-[2-acetyl-3-[4-(1-propylpyrazol-4-yl)phenyl]-3,4-dihydropyrazol-5-yl]-4-benzyl-6-chloro-1H-quinolin-2-one |
| d.92 | 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid |
| d.93 | 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid |
| d.95 | 4-benzyl-6-chloro-3-(3-phenyl-2-propanoyl-3,4-dihydropyrazol-5-yl)-1H-quinolin-2-one |
| d.96 | 3-[2-acetyl-3-(p-tolyl)-3,4-dihydropyrazol-5-yl]-4-benzyl-6-chloro-1H-quinolin-2-one |
| d.98 | 4-[5-(6-chloro-2-oxo-4-phenyl-1H-quinolin-3-yl)-3-(3-pyridyl)-3,4-dihydropyrazol-2-yl]-4-oxo-butanoic acid |
| d.99 | 4-[5-(6-chloro-2-oxo-4-phenyl-1H-quinolin-3-yl)-3-(2-pyridyl)-3,4-dihydropyrazol-2-yl]-4-oxo-butanoic acid |
| d.101 | 4-[5-(4-benzyl-6-chloro-2-oxo-1H-quinolin-3-yl)-3-(3-pyridyl)-3,4-dihydropyrazol-2-yl]-4-oxo-butanoic acid |
| d.105 | 4-[5-(4-benzyl-6-chloro-2-oxo-1H-quinolin-3-yl)-3-(2-chlorophenyl)-3,4-dihydropyrazol-2-yl]-4-oxo-butanoic acid |
| d.106 | 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid |
| d.109 | 4-[5-(6-chloro-4-morpholino-2-oxo-1H-quinolin-3-yl)-3-(4-methoxyphenyl)-3,4-dihydropyrazol-2-yl]-4-oxo-butanoic acid |

Advantageously, the compounds of the invention block homologous recombination DNA repairing mechanism by disrupting Rad51-BRCA2 interaction.

Pharmacological inhibition of homologous recombination can be used to "chemically induce" synthetic lethality effect, especially useful in cancer treatment because makes cancer cells more sensitive to drugs acting as DNA repairing mechanism inhibitors (i.e.PARPi), DNA-damaging agents, and radiotherapy. The compounds of the invention are potent disruptors of Rad51-BRCA2 interaction and, at the same time, they show promising results in blocking the proliferation of cancer cells either alone or in combination with other chemotherapeutic compounds, for example PARP inhibitors such as Olaparib, or radiotherapy. Moreover, the compounds of the invention are potent inhibitors towards Rad51 enzyme, showing EC₅₀ values in the micromolar range in a cell-based assay measuring cancer cells proliferation. These results suggest that these compounds are able to penetrate the cell membrane in a complex biological environment.

In the following, the present invention shall be illustrated by means of some examples,

The following abbreviations are hereinafter used in the accompanying examples: acetic acid (AcOH), aryl (Ar), apparent triplet *(app-t),* apparent doublet of triplet (*app-*dt), apparent doublet (*app-d*), apparent singlet (app-s), aqueous (aq.), atmospheres (atm), benzyl (Bn), broad signal (bs), tert-butyl (tBu), tert-butylmethyl ether (TBME); normal-butyl lithium (nBuLi), calculated (calcd), carbon nuclear magnetic resonance spectroscopy (¹³C NMR), m-chloro perbenzoic acid (m-CPBA), cyclohexane (CyH or Cy), Deuterium (D), doublet (d), dichloromethane (DCM), doublet of doublet (dd), doublet of doublet of triplets (ddt), diisopropyl azodicarboxylate (DIAD), ethyldiisopropylamine (DIPEA), doublet of quartet (dq), N,N-dimethylformamide (DMF), dimethyl sulfoxide (DMSO), hexadeuterodimethyl sulfoxide (DMSO-*d*₆), doublet of triplet (dt), N-(3-dimethylamino propyl)-N'-ethylcarbodiimide (EDC), half maximal effective concentration (EC50), equivalents (equiv. or eq.), enantiomeric excess (e.e.), electrospray ionization (ESI), ethyl (Et), diethyl ether (Et₂O), ethyl acetate (EtOAc), hour (h), proton nuclear magnetic resonance spectroscopy (¹H NMR), 1-[bis (dimethylamino)methylene]-1H-1,2,3-triazolo[4,5-b] pyridinium 3-oxid hexafluorophosphate (HATU), 1-hydroxybenzotriazole hydrate (HOBt), high performance liquid chromatography (HPLC), hertz (Hz), infrared spectroscopy (IR), half maximal inhibitory concentration (IC50), coupling constant (J), potassium carbonate (K₂CO₃), liter (L), lithium hydroxide (LiOH), molarity (M), multiplet (m), methyl (Me), acetonitrile (MeCN), methanol (MeOH), milligram (mg), megahertz (MHz), minutes (min), milliliter (mL), millimole (mmol), Mass Spectrometry (MS), molecular weight (MW), sodium hydride (NaH), sodium bicarbonate (NaHCO₃), sodium carbonate (Na₂CO₃), sodium sulphate (Na₂SO₄), ammonium chloride (NH₄Cl), not determined (nd), nanomolar (nM), Nuclear Magnetic Resonace (NMR), protecting group (Pg), parts per million (ppm), triphenylphospine (PPh₃), isopropyl (i-Pr), quartet (q), substituent (R), racemic (rac-), room temperature (rt), singlet (s), temperature (T), triplet or time (t), retention time (tr), triethylamine (TEA or Et₃N), trifluoroacetic acid (TFA), tetrahydrofuran (THF), thin-layer chromatography (TLC), ultraviolet (UV), Ultra-Performance Liquid Chromotography - Mass Spectroscopy (UPLC-MS), anionic ligand, halide, substituent, or number (X), chemical shift (δ), microliter (µL), micromolar (µM), Watt (W), deuterated chloroform (CDCl₃), ammonium acetate (NH₄OAc).

### 1.Chemical Synthesis

Automated column chromatography purifications were conducted using a Teledyne ISCO apparatus (CombiFlashTM Rf) with prepacked silica gel columns of different sizes (from 4 g to 120 g). Mixtures of increasing polarity of cyclohexane and ethyl acetate or dichloromethane and methanol or dichloromethane and ethanol were used as eluents. Preparative TLCs were performed using Macherey-Nagel precoated 0.05 mm TLC plates (SIL G-50 UV254). Microwave heating was performed using Explorer-48 positions instrument (CEM). NMR experiments were run on a Bruker Avance III 400 MHz spectrometer (400.13 MHz for ¹H, and 100.62 MHz for ¹³C), equipped with a BBI probe and Z-gradients, or on a Bruker FT NMR Avance III 600-MHz spectrometer (600.130 MHz for ¹H, and 150.903 MHz for ¹³C) equipped with a 5-mm CryoProbe QCI quadruple resonance, a shielded Z-gradient coil and the automatic sample changer SampleJet NMR system. Spectra were acquired at 300 K, using deuterated dimethyl sulfoxide (DMSO-*d*₆) or deuterated chloroform (CDCl₃) as solvents. Chemical shifts for ¹H and ¹³C spectra were recorded in parts per million using the residual non-deuterated solvent as the internal standard (for CDCl₃: ¹H 7.26 ppm, ¹³C 77.16 ppm; for DMSO-*d₆*: ¹H 2.50 ppm, ¹³C 39.52 ppm). UPLC-MS analyses were run on a Waters ACQUITY UPLC/MS system consisting of a SQD (Single Quadropole Detector) Mass Spectrometer equipped with an Electrospray Ionization interface and a Photodiode Array Detector. The PDA range was 210-400 nm. The analyses were performed on either an ACQUITY UPLC HSS T3 C18 column (50x2.1 mmID, particle size 1.8µm) with a VanGuard HSS T3 C18 pre-column (5x2.1mmID, particle size 1.8µm) (LogD<1) or an ACQUITY UPLC BEH C18 column (50x2.1mmID, particle size 1.7µm) with a VanGuard BEH C18 pre-column (5x2.1mmID, particle size 1.7µm) (LogD>1). The mobile phase was 10 mM NH₄OAc in H₂O at pH 5 adjusted with AcOH (A) and 10 mM NH₄OAc in MeCN-H₂O (95:5) at pH 5 (B). Electrospray ionization in positive and negative mode was applied in the mass scan range 100-500 Da. Methods and gradients used:
Polar method:
   Column: Waters ACQUITY UPLC HSS T3 C18, 1.8 µm, 50x2.1 mmID
   Pre-column: VanGuard HSS T3 C18, 1.8 µm, 5x2.1 mmID
   Linear gradient: 0-0.2 min: 0% B, 0.2-2.7 min: 0-50%B, 2.7-2.8 min: 50-100% B, 2.8-3.0 min: 100% B
   Flow rate: 0.5 mL/min
Generic method:
   Column: Waters ACQUITY UPLC BEH C18, 1.7 µm, 50x2.1 mmID
   Pre-column: VanGuard BEH C18, 1.7 µm, 5x2.1 mmID
   Linear gradient: 0-0.2 min: 5% B, 0.2-2.7 min: 5-95% B, 2.7-2.8 min: 95-100% B, 2.8-3.0 min: 100% B
   Flow rate: 0.5 mL/min
Apolar method:
   Column: Waters ACQUITY UPLC BEH C18, 1.7 µm, 50x2.1 mmID
   Pre-column: VanGuard BEH C18, 1.7 µm, 5x2.1 mmID
   Gradient: 0-0.2 min: 50% B, 0.2-2.7 min: 50-100% B, 2.7-3.0 min: 100% B
   Flow rate: 0.5 mL/min 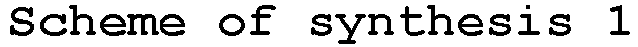 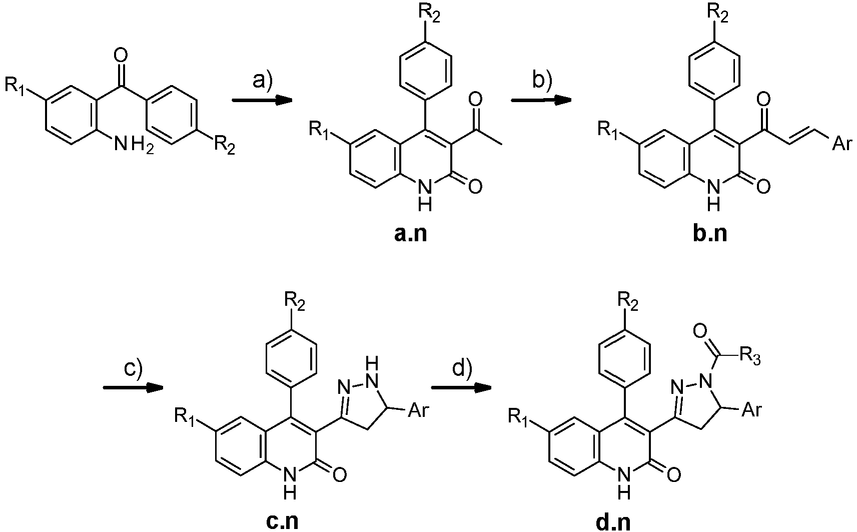 (a) Ethyl acetoacetate, DMF, 120 °C; (b) ArCHO, KOH, 4:3 EtOH/H₂O 0 °C to rt; (c) hydrazine monohydrate, EtOH, 110 °C; (d) anhydride, THF (dry), heating or propionic acid, HOBt, EDCI, DCM, rt 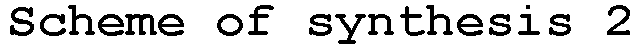 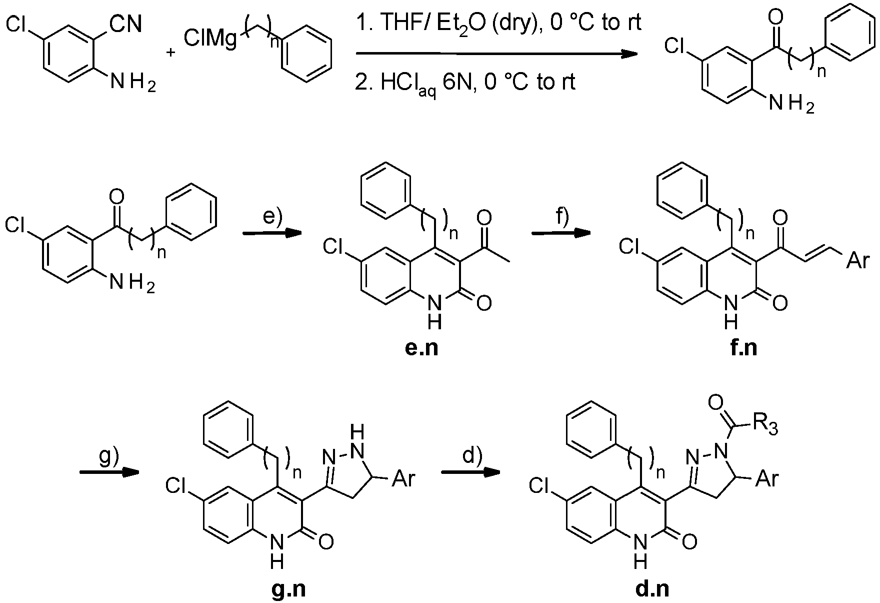 (e) Ethyl acetoacetate, THF (dry), 4A ms, 140 °C, 75 min (f) ArCHO, KOH, 4:3 EtOH/H₂O 0 °C to rt; (g) hydrazine monohydrate, EtOH, 110 °C; (d) anhydride, THF (dry), heating or propionic acid, HOBt, EDCI, DCM, 0 °C to rt

### Synthetic procedures

### General procedures A: synthesis of quinolin-2(1H)-one

**A1)** An appropriate 2-aminobenzophenone (1.0 eq) and the ethyl acetoacetate (1.5 eq) were dissolved in DMF (1.00 M) in an appropriately sized microwaveable vessel and microwaved at 120 °C (200 W) for 1.5 hours. The solvent was concentrated under reduced pressure, the residue was diluted with DCM and washed three times with water. The organic layer was dried over Na₂SO₄, filtered and the solvent removed. The title compound was obtained after purification over silica gel, unless otherwise noted.

A2) An appropriate 2-aminobenzophenone (1.0 eq) and the ethyl acetoacetate (3 eq) were dissolved in DMF (1.00 M) in an appropriately sized screw-capped pressure tube. The mixture was heated to reflux and stirred for 19 hours. Finally the solvent was removed under reduced pressure.

### General procedure B: synthesis of quinolin-2(1H)-one acrolyl intermediates

In a round bottom flask, the quinolin-2(1H)-one (1.00 eq) and potassium hydroxide (25 eq) were stirred in EtOH/H₂O (4:3 v/v, 0.05 M) at 0°C for 45 minutes prior to the addition of an appropriately substituted arylaldehyde (1 eq). The reaction was stirred overnight while gradually reached room temperature. The reaction was quenched by slow addition of acetic acid (equimolar to KOH), the crude was extracted with DCM/H₂O, the organic layer was then anhydrified over a phase separator and the organic solvent removed. The title compound was obtained after purification over silica gel, unless otherwise noted.

### General procedure C: synthesis of pyrazol-3-yl-quinolin-2(1H)-one intermediates

In an appropriately sized microwaveable vessel, the quinolin-2(1H)-one acrolyl intermediate (1 eq) was dissolved in EtOH (0.2 M, 190 proof or 200 proof) and hydrazine monohydrate (2 eq) was added. The mixture was microwaved for 45 minutes at 110 °C (200 W). Ethanol was removed under vacuum, followed by purification over silica gel, unless otherwise noted.

### General procedures D: synthesis of acylated quinolinone pyrazoline products

**D1)** In an appropriately sized microwaveable vessel, the pyrazol-3-ylquinolin-2 (1H) -one intermediate (1.00 eq) was dissolved in anhydrous THF (0.5 M). The appropriate anhydride (2.00 equiv) was added. The solution was microwaved (200 W) with stirring for 45 min at the appropriate temperature. The THF was removed under vacuum, and the organics were dissolved in dichloromethane (DCM), washed three times with aqueous HCl (pH 2), anhydrified over a phase separator, the solvent removed and purified over silica gel.

**D2)** In a round bottomed flask, propionic acid (1.8 eq), HOBt (1.8 eq) and EDCI (1.8 eq) were stirred in DCM (0.50 M) at room temperature for 1 hour. Then a solution of the pyrazol-3-ylquinolin-2(1H)-one intermediate (1.0 eq) and Et₃N (2.50 eq) in DCM (0.50 M) were added. The reaction was stirred at rt for 3 hours. The organic layer was washed with aqueous NaHCO₃ (1M), citric acid 10% and water, dried over Na₂SO₄ and the solvent removed under reduced pressure. Title compound was obtained after purification over silica gel, unless otherwise noted.

All examples compounds are summarized in Table 1 and the synthetic procedures are described in detail below.

### Example d.1. 4-[5-(6-chloro-2-oxo-4-phenyl-1H-quinolin-3-yl)-3-(4-fluorophenyl)-3,4-dihydropyrazol-2-yl]-4-oxo-butanoic acid (example not falling within the scope of the present invention).

### Step 1. Synthesis of 3-acetyl-6-chloro-4-phenyl-1H-quinolin-2-one (a.1)

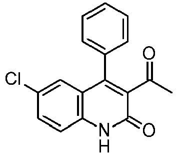

Compound a.1 was synthesized following the general procedure A2 with (2-amino-5-chlorophenyl)-phenyl-methanone (1.460 g, 6.3 mmol) yielding title compound (1.875 g, quantitative yield). ¹H NMR(400 MHz, DMSO-*d₆*) δ 12.38 (s, 1H), 7.64 (dd, J = 8.8, 2.4 Hz, 1H), 7.59 - 7.48 (m, 3H), 7.44 (d, J = 8.8 Hz, 1H), 7.39 - 7.28 (m, 2H), 6.95 (d, J = 2.3 Hz, 1H), 2.22 (s, 3H). Rt 2.06 min (generic method). ESI-MS for C₁₇H₁₂ClNO₂: calculated 297.1, found m/z 298.0 [M+H]⁺, 296.0 [M-H]⁻.

### Step 2. Synthesis of 6-chloro-3-[(E)-3-(4-fluorophenyl)prop-2-enoyl]-4-phenyl-1H-quinolin-2-one (b.1)

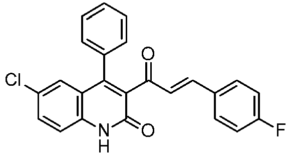

Compound b.1 was synthesized following the general procedure B using a.1 (2.517 g, 8.45 mmol) and 4-fluorobenzaldehyde. Purification by normal phase flash column chromatography (solvent A: DCM, solvent B: EtOH, gradient: 0-20% solvent B) yielded title compound (2.548 g, yield 75%). ¹H NMR(400 MHz, DMSO-*d₆*) δ 12.35 (s, 1H), 7.81 - 7.70 (m, 2H), 7.66 (dd, J = 8.8, 2.4 Hz, 1H), 7.54 - 7.41 (m, 5H), 7.36 - 7.30 (m, 2H), 7.28 - 7.19 (m, 2H), 6.98 (d, J = 2.4 Hz, 1H), 6.73 (d, J = 16.4 Hz, 1H). Rt 2.42 min (generic method). ESI-MS for C₂₄H₁₅ClFNO₂: calculated 403.1, found m/z 404.1 [M+H]⁺, 402.1 [M-H]⁻.

### Step 3. Synthesis of 6-chloro-3-[5-(4-fluorophenyl)-4,5-dihydro-1H-pyrazol-3-yl]-4-phenyl-1H-quinolin-2-one (c.1).

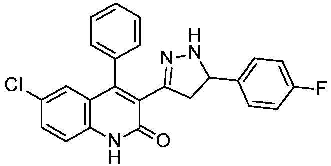

Compound c.1 was synthesized following the general procedure C using b.1 (317 mg, 0.78 mmol). Purification by normal phase flash column chromatography (solvent A: DCM, solvent B: DCM/EtOH 8:2 v/v - gradient: 0-20% solvent B) yielded title compound (303 mg, yield 92%). ¹H NMR(400 MHz, DMSO-*d₆*) δ 12.19 (s, 1H), 7.57 (dd, J = 8.8, 2.4 Hz, 1H), 7.50 (m, 2H), 7.41 (d, J = 8.8 Hz, 1H), 7.33 (dt, J = 6.9, 1.5 Hz, 1H), 7.23 (dt, J = 6.8, 2.0 Hz, 1H), 7.14 (d, J = 3.2 Hz, 1H), 7.13 - 7.02 (m, 4H), 6.89 (d, J = 2.3 Hz, 1H), 4.59 (td, J = 10.9, 10.2, 3.1 Hz, 1H), 3.23 (dd, J = 16.5, 11.0 Hz, 1H), 2.59 - 2.52 (m, 1H). Rt 2.31 min (generic method). ESI-MS for C₂₄H₁₇ClFN₃O: calculated 417.1, found m/z 418 [M+H]⁺, 416 [M-H]⁻.

### Step 4. Synthesis of 4-[5-(6-chloro-2-oxo-4-phenyl-1H-quinolin-3-yl)-3-(4-fluorophenyl)-3,4-dihydropyrazol-2-yl]-4-oxo-butanoic acid (d.1).

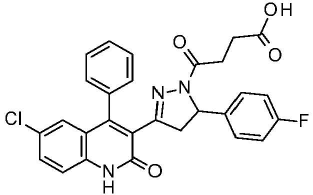

Compound d.1 was synthesized following the general procedure D1 using c.1 (270 mg, 0.65 mmol) with succinic anhydride (120 °C, 200 W). Purification by normal phase flash column chromatography (solvent A: DCM, solvent B: DCM/EtOH 8:2 v/v - gradient: 0-50% solvent B) yielded title compound (208 mg, yield 62%). ¹H NMR(400 MHz, DMSO-*d₆*) δ 12.38 (s, 1H), 12.02 (s, 1H), 7.64 (dd, J = 8.8, 2.4 Hz, 1H), 7.60 - 7.37 (m, 5H), 7.28 (dt, J = 6.8, 2.0 Hz, 1H), 7.04 (t, J = 8.9 Hz, 2H), 6.94 (d, J = 2.3 Hz, 1H), 6.88 - 6.76 (m, 2H), 5.32 (dd, J = 12.0, 4.5 Hz, 1H), 3.73 (dd, J = 18.5, 12.0 Hz, 1H), 2.79 (dd, J = 18.4, 4.5 Hz, 1H), 2.48 - 2.40 (m, 2H), 2.32 - 2.26 (m, 2H). Rt 2.04 min (generic method). ESI-MS for C₂₈H₂₁ClFN₃O₄ : calculated 517.1, found m/z 518 [M+H]⁺, 516 [M-H]⁻.**Example d.2. 3-[2-acetyl-3-(4-fluorophenyl)-3,4-dihydropyrazol-5-yl]-6-chloro-4-phenyl-1H-quinolin-2-one. (example not falling within the scope of the present invention).**

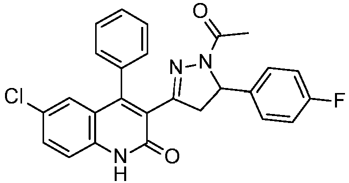

Compound d.2 was synthesized following the general procedure D1 using c.1 (200 mg, 0.48 mmol) with acetic anhydride. Purification by normal phase flash column chromatography (solvent A: DCM, solvent B: MeOH - gradient: 0-20% solvent B) yielded title compound (207 mg, yield 47%). ¹H NMR(400 MHz, DMSO-*d₆*) δ 12.38 (s, 1H), 7.64 (dd, J = 8.8, 2.4 Hz, 1H), 7.61 - 7.50 (m, 3H), 7.46 (d, J = 8.8 Hz, 1H), 7.41 (dt, J = 6.6, 1.9 Hz, 1H), 7.29 (dt, J = 6.4, 1.9 Hz, 1H), 7.06 (t, J = 8.9 Hz, 2H), 6.94 (d, J = 2.3 Hz, 1H), 6.84 (dd, J = 8.6, 5.6 Hz, 2H), 5.32 (dd, J = 12.0, 4.5 Hz, 1H), 3.73 (dd, J = 18.4, 12.1 Hz, 1H), 2.84 (dd, J = 18.5, 4.5 Hz, 1H), 1.88 (s, 3H). Rt 2.38 min (generic method). ESI-MS for C₂₆H₁₉ClFN₃O₂: calculated 459.1, found m/z 460.1 [M+H]⁺, 458.1 [M-H]⁻.

### Example d.3. 6-chloro-3-[3-(4-fluorophenyl)-2-propanoyl-3,4-dihydropyrazol-5-yl]-4-phenyl-1H-quinolin-2-one. (example not falling within the scope of the present invention).

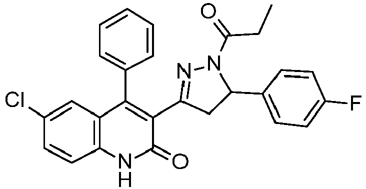

Compound d.3 was synthesized following the general procedure D2 using c.1 (250 mg, 0.60 mmol). Purification by normal phase flash column chromatography (solvent A: DCM, solvent B: MeOH - gradient: 0-1 % solvent B) yielded title compound (60 mg, yield 21%). ¹H NMR(400 MHz, DMSO-*d*₆) δ 12.38 (s, 1H), 7.64 (dd, J = 2.0, 8.0 Hz, 1H), 7.58 - 7.50 (m, 3H), 7.45 (d, *J* = 8.8 Hz, 1H), 7.41 - 7.39 (m, 1H), 7.28 (d, *J* = 5.6 Hz, 1H), 7.06 (t, *J* = 8.8 Hz, 2H), 6.93 (d, *J* = 2.0 Hz, 1H), 6.84 (dd, J = 4.0, 8.0, Hz, 2H), 5.32 (dd, J = 8.0, 12.0 Hz, 1H), 3.73 (dd, J = 12.0, 16.0 Hz, 1H), 2.81 (dd, J = 4.0, 16.0 Hz, 1H), 2.30 - 2.16 (m, 2H), 0.83 (t, J = 7.6 Hz, 3H). ESI-MS for C₂₇H₂₁ClFN₃O₂: calculated 473.1, found m/z 474.1 [M+H]⁺, 472.1 [M-H]⁻.

### Example d.4. 3-[2-(3-aminopropanoyl)-3-(4-fluorophenyl)-3,4-dihydropyrazol-5-yl]-6-chloro-4-phenyl-1H-quinolin-2-one (example not falling within the scope of the present invention).

### Step 1. Synthesis of 3-((tert-Butoxycarbonyl) amino) propanoic acid.

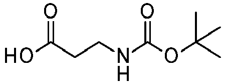

In a round bottom flask, β-alanine (1.0 g, 11.2 mmol) was stirred in a 1:1 *v*/*v* MeOH/1M NaOH mixture (20.0 ml), then Boc₂O (2.94 g, 13.5 mmol) was added at 0 °C. The reaction mixture was left to gradually reach room temperature and stirred overnight. The solvent was removed under reduced pressure and the residue re-dissolved in EtOAc, then washed three times with 1M HCl_{aq} (pH 2). The organic layer was anhydrified over Na₂SO₄ and the solvent removed yielding title compound (2.14 g, yield 99%). ¹H NMR(400 MHz, DMSO-*d*₆) δ 12.14 (s, 1H), 6.78 (s, 1H), 3.12 (dd, *J* = 8.0, 12.0 Hz, 2H), 2.34 (t, *J* = 8.0 Hz, 2H), 1.37 (s, 9H).

### Step 2. Synthesis of tert-butyl N-[3-[5-(6-chloro-2-oxo-4-phenyl-1H-quinolin-3-yl)-3-(4-fluorophenyl)-3,4-dihydropyrazol-2-yl]-3-oxo-propyl]carbamate.

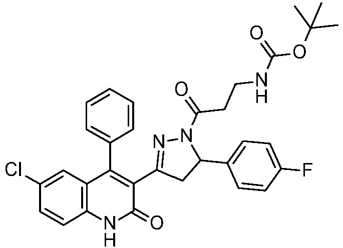

In round bottom flask, 3-((*tert-*butoxycarbonyl)amino)propanoic acid (182 mg, 0.96 mmol), HOBt (156 mg, 1.16 mmol) and EDCI (222 mg, 1.16 mmol) were stirred in DCM (15 ml) at room temperature for 1 hour. Then a solution of the c.1 (400 mg, 0.96 mmol) and Et₃N (296 µl, 2.12 mmol) in DCM (5 ml) was added. The mixture was stirred at room temperature overnight. The solvent was removed under reduced pressure, the residue re-dissolved with EtOAc and then washed with water, 1M NaHCO₃ solution and finally 10% citric acid solution. The organic layer was anhydrified over Na₂SO₄ and evaporated to dryness. Purification by normal phase flash column chromatography (solvent A: DCM, solvent B: EtOAc - gradient: 0- 30% solvent B) yielded title compound (72 mg, yield 25%). ¹H NMR(400 MHz, DMSO-*d*₆) δ 12.40 (s, 1H), 7.65 (dd, J= 4.0, 8.0 Hz, 1H), 7.59-7.50 (m, 4H), 7.46 (d, J = 8.0 Hz, 1H), 7.43-7.42 (m, 1H), 7.28 (d, J = 8.0 Hz, 1H), 7.04 (t, J = 8.0 Hz, 2H), 6.93 (d, J = 2.0 Hz, 1H), 6.80 (dd, J = 4.0, 8.0 Hz, 2H), 6.66 (t, J = 8.0 Hz, 1H), 5.32 (dd, J = 4.0, 12.0 Hz, 1H), 3.73 (dd, J = 12.0, 16.0 Hz, 1H), 2.99 (dd, *J* = 8.0, 12.0 Hz, 2H), 2.76 (dd, *J* = 4.0, 20.0 Hz, 1H) 2.41 (q, J = 8.0 Hz, 2H), 1.38 (s, 9H).

### Step 3. Synthesis of [3-[5-(6-chloro-2-oxo-4-phenyl-1H-quinolin-3-yl)-3-(4-fluorophenyl)-3,4-dihydropyrazol-2-yl]-3-oxo-propyl]ammonium;chloride.

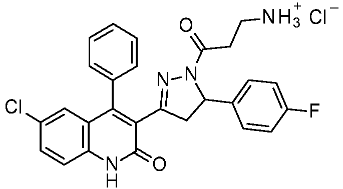

In round bottom flask tert-Butyl (3-(3-(6-chloro-2-oxo-4-phenyl-1,2-dihydroquinolin-3-yl)-5-(4-fluorophenyl)-4,5-dihydro-1H-pyrazol-1-yl)-3-oxopropyl)carbamate (200 mg, 0.34 mmol) was treated with 4M HCl in dioxane (4 ml) and stirred at rt for 15 minutes. The solvent was removed under reduced pressure and the solid washed three times with diethyl ether. The title compound was obtained by filtration (170 mg, yield 95%). ¹H NMR(400 MHz, DMSO-*d*₆) δ 12.46 (s, 1H), 7.81 (br, 3H), 7.66 (dd, J = 2.4, 8.8 Hz, 1H), 7.61-7.53 (m, 3H), 7.49 (d, J = 8.8 Hz), 7.44-7.42 (m, 1H), 7.30 (d, *J* = 6.8 Hz, 1H), 7.05 (t, *J* = 8.8 Hz, 2H), 6.94 (d, *J* = 2.0 Hz, 1H), 6.79 (dd, *J* = 8.8, 5.2 Hz, 2H), 5.35 (dd, *J* = 4.0, 12.0 Hz, 1H), 3.77 (dd, *J* = 12.0, 16.0 Hz, 1H), 2.88 (dd, *J* = 8.0, 12.0 Hz, 2H), 2.76 (dd, *J* = 4.0, 20.0 Hz, 1H), 2.71- 2.62 (m, 2H).

Step 4. Synthesis of 3-[2-(3-aminopropanoyl)-3-(4-fluorophenyl)-3,4-dihydropyrazol-5-yl]-6-chloro-4-phenyl-1H-quinolin-2-one (d.4).

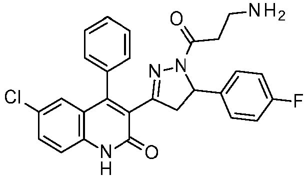

In a round bottom flask 3-(1-(3-Aminopropanoyl)-5-(4-fluorophenyl)-4,5-dihydro-1H-pyrazol-3-yl)-6-chloro-4-phenylquinolin-2 (1*H*) -one hydrochloride (110 mg, 0.21 mmol) was treated with NaOH 0.5 M (420 µl) in EtOAc (5 ml) stirring at rt for 30 minutes. The mixture was then diluted with further EtOAc and washed twice with water. The organic layer was anhydrified over Na₂SO₄, filtered and evaporated to dryness. Purification by normal phase flash column chromatography (solvent A: DCM, solvent B: MeOH, solvent C: NH₄OH - gradient: 0-10% solvent B - gradient: 0-0.1% solvent C) yielded title compound (50 mg, yield 50%) . ¹H NMR(400 MHz, DMSO-*d*₆) δ 7.63 (dd, J = 12.0, 4.0 Hz, 1H), 7.57 - 7.50 (m, 3H), 7.54 (d, J = 4.8 Hz, 1H), 7.42-7.40 (m, 1H), 7.26 (d, J = 7.2 Hz, 1H), 7.03 (t, J = 8.8 Hz, 2H), 6.92 (d, J = 2.0 Hz, 1H), 6.81 (dd, J = 2.0, 8.0 Hz, 2H), 5.32 (dd, J =2.0, 12.0 Hz, 1H), 3.72 (dd, J = 12.0, 20.0 Hz, 1H), 2.76 (dd, J = 4.0, 16.0 Hz, 1H), 2.57 (t, J = 6.6 Hz, 2H), 2.42 - 2.28 (m, 2H). ESI-MS for C₂₇H₂₂ClFN₄O₂: calculated 488.1, found *m*/*z* 489.1 [M+H]⁺, 487.1 [M-H]⁻.

### Example d.5. 4-[5-(6-chloro-2-oxo-4-phenyl-1H-quinolin-3-yl)-3-(4-chlorophenyl)-3,4-dihydropyrazol-2-yl]-4-oxo-butanoic acid (example not falling within the scope of the present invention).

### Step 1. Synthesis of 6-chloro-3-[(E)-3-(4-chlorophenyl)prop-2-enoyl]-4-phenyl-1H-quinolin-2-one (b.2).

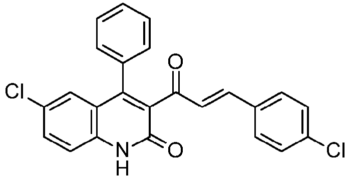

Compound b.2 was synthesized following the general procedure B using a.1 (304 mg, 1.0 mmol) and 4-chlorobenzaldehyde. Title compound was obtained by precipitation and filtration from the reaction crude (429 mg, quantitative yield). ¹H NMR(400 MHz, DMSO-*d₆*) δ 7.70 (d, J = 8.6 Hz, 2H), 7.63 (dd, J = 8.8, 2.4 Hz, 1H), 7.55 - 7.37 (m, 7H), 7.32 (dd, J = 7.6, 1.9 Hz, 2H), 6.96 (d, J = 2.4 Hz, 1H), 6.78 (d, J = 16.4 Hz, 1H). Rt 2.55 min (generic method) . ESI-MS for C₂₄H₁₅C₁₂NO₂: calculated 419.0, found m/z 420.0 [M+H]⁺, 418.0 [M-H]⁻.

### Step 2. Synthesis of 6-chloro-3-[5-(4-chlorophenyl)-4,5-dihydro-1H-pyrazol-3-yl]-4-phenyl-1H-quinolin-2-one (c.2).

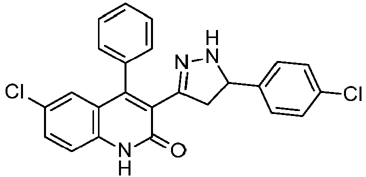

Compound c.2 was synthesized following the general procedure C using b.2 (429 mg, 1.0 mmol). Purification by normal phase flash column chromatography (solvent A: DCM, solvent B: DCM/EtOH 8:2 v/v - gradient: 0-50% solvent B) yielded title compound (301 mg, yield 68%). ¹H NMR(400 MHz, DMSO-*d₆*) δ 12.20 (s, 1H), 7.58 (dd, J = 8.8, 2.4 Hz, 1H), 7.50 (tt, J = 7.5, 2.9 Hz, 3H), 7.42 (d, J = 8.8 Hz, 1H), 7.34 (dt, J = 7.0, 1.7 Hz, 1H), 7.30 (d, J = 8.4 Hz, 2H), 7.23 (dt, J = 7.3, 2.0 Hz, 1H), 7.18 (d, J = 3.2 Hz, 1H), 7.09 (d, J = 8.4 Hz, 2H), 6.90 (d, J = 2.3 Hz, 1H), 4.59 (td, J = 11.6, 9.3, 3.1 Hz, 1H), 3.25 (dd, J = 16.5, 11.1 Hz, 1H), 2.58 - 2.53 (m, 1H). Rt 2.45 min (generic method) . ESI-MS for C₂₄H₁₇C₁₂N₃O: calculated 433.1, found m/z 434.1 [M+H]⁺, 432.1 [M-H]⁻.

### Step 3. Synthesis of 4-[5-(6-chloro-2-oxo-4-phenyl-1H-quinolin-3-yl)-3-(4-chlorophenyl)-3,4-dihydropyrazol-2-yl]-4-oxo-butanoic acid (d.5).

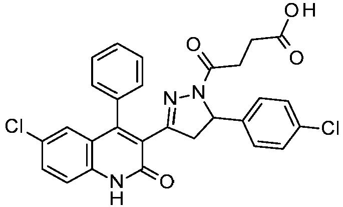

Compound d.5 was synthesized following the general procedure D1 using c.2 (253 mg, 0.58 mmol) with succinic anhydride (120 °C, 200 W). Purification by normal phase flash column chromatography (solvent A: DCM, solvent B: DCM/EtOH 8:2 v/v - gradient: 0-60% solvent B) yielded title compound (231 mg, yield 74%). ¹H NMR(400 MHz, DMSO-*d₆*) δ 12.38 (s, 1H), 12.03 (s, 1H), 7.64 (dd, J = 8.8, 2.4 Hz, 1H), 7.59 - 7.48 (m, 3H), 7.45 (d, J = 8.8 Hz, 1H), 7.41 (dt, J = 6.3, 2.0 Hz, 1H), 7.31 - 7.23 (m, 3H), 6.93 (d, J = 2.4 Hz, 1H), 6.83 - 6.76 (m, 2H), 5.32 (dd, J = 12.0, 4.6 Hz, 1H), 3.74 (dd, J = 18.5, 12.1 Hz, 1H), 2.78 (dd, J = 18.5, 4.6 Hz, 1H), 2.48 - 2.41 (m, 2H), 2.29 (t, J = 7.1 Hz, 2H). Rt 2.16 min (generic method) . ESI-MS for C₂₈H₂₁C₁₂N₃O₄: calculated 533.1, found m/z 534.1 [M+H]⁺, 532.1 [M-H]⁻.

### Example d.6. 3-[2-acetyl-3-(4-chlorophenyl)-3,4-dihydropyrazol-5-yl]-6-chloro-4-phenyl-1H-quinolin-2-one (example not falling within the scope of the present invention).

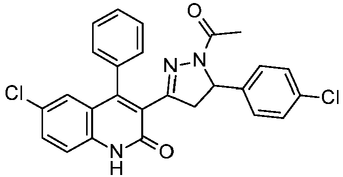

Compound d.6 was synthesized following the general procedure D1 using c.2 (210 mg, 0.48 mmol) with acetic anhydride. Purification by normal phase flash column chromatography (solvent A: DCM, solvent B: MeOH - gradient: 0-1.5% solvent B) yielded title compound (200 mg, yield 94%). ¹H NMR (400 MHz, DMSO-*d*₆) δ 12.41 (s, 1H), 7.64 (dt, *J* = 8.0, 1.2 Hz, 1H), 7.58 - 7.53 (m, 3H), 7.44 (d, *J* = 8.0 Hz, 1H), 7.41-7.39 (m, 1H), 7.30 - 7.28 (m, 3H), 6.93 (d, *J* = 0.8 Hz, 1H), 6.80 (d, *J* = 7.6 Hz, 2H), 5.32 (dd, *J* = 12.0, 8.0 Hz, 1H), 3.74 (dd, *J* = 20.0, 12.0 Hz, 1H), 2.82 (dd, *J* = 16.0, 4.0 Hz, 1H), 1.87 (s, 3H). ESI-MS for C₂₆H₁₉Cl₂N₃O₂: calculated 475.1, found *m*/*z* 476.1 [M+H]⁺, 474.1 [M-H]⁻.

### Example d.7. 6-chloro-3-[3-(4-chlorophenyl)-2-propanoyl-3,4-dihydropyrazol-5-yl]-4-phenyl-1H-quinolin-2-one (example not falling within the scope of the present invention).

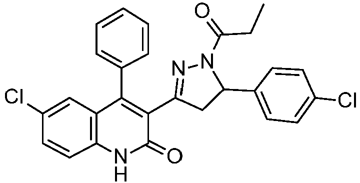

Compound d.7 was synthesized following the general procedure D2 using c.2 (200 mg, 0.46 mmol). Purification by normal phase flash column chromatography (solvent A: DCM, solvent B: MeOH - gradient: 0-2 % solvent B) yielded title compound (76 mg, yield 34%). ¹H NMR(400 MHz, DMSO-*d*₆) δ 12.38 (s, 1H), 7.63 (dd, *J* = 9.0, 2.4 Hz, 1H), 7.54 - 7.49 (m, 3H), 7.44 (d, *J* = 8.8 Hz, 1H), 7.41 - 7.39 (m, 1H), 7.30 - 7.27 (m, 3H), 6.92 (d, *J* = 2.4 Hz, 1H), 6.80 (d, *J* = 8.8 Hz, 2H), 5.31 (dd, *J* = 12.2, 4.6 Hz, 1H), 3.74 (dd, J = 18.4, 12.0 Hz, 1H), 2.80 (dd, *J* = 18.4, 4.0 Hz, 1H), 2.31 - 2.14 (m, 2H), 0.82 (t, *J* = 7.2 Hz, 3H). ESI-MS for C₂₇H₂₁Cl₂N₃O₂: calculated 489.1, found m/z 490.1 [M+H]⁺, 488.1 [M-H]⁻.

### Example d.8. 4-[3-(4-bromophenyl)-5-(6-chloro-2-oxo-4-phenyl-1H-quinolin-3-yl)-3,4-dihydropyrazol-2-yl]-4-oxo-butanoic acid (example not falling within the scope of the present invention).

### Step 1. Synthesis of 3-[(E)-3-(4-bromophenyl)prop-2-enoyl]-6-chloro-4-phenyl-1H-quinolin-2-one (b.3).

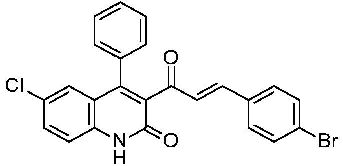

Compound b.3 was synthesized following the general procedure B using a.1 (328 mg, 1.1 mmol) and 4-bromobenzaldehyde. Title compound was obtained by precipitation and filtration from the reaction crude (511 mg, yield quantitative). ¹H NMR(400 MHz, DMSO-*d*₆) δ 7. 67 - 7.47 (m, 6H), 7.46 - 7.35 (m, 4H), 7.30 (dd, *J* = 7.7, 1.8 Hz, 2H), 6.93 (d, *J* = 2.3 Hz, 1H), 6.80 (d, *J* = 16.4 Hz, 1H). Rt 2.59 min (generic method). ESI-MS for C₂₄H₁₅BrClNO₂: calculated 462.0/464.0, found *m*/*z* 463.0/465.0 [M+H]⁺, 461.0/463.0 [M-H]⁻.

### Step 2. Synthesis of 3-[5-(4-bromophenyl)-4,5-dihydro-1H-pyrazol-3-yl]-6-chloro-4-phenyl-1H-quinolin-2-one (c.3).

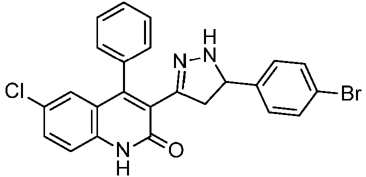

Compound c.3 was synthesized following the general procedure C using b.3 (510 mg, 1.1 mmol). Purification by normal phase flash column chromatography: solvent A: DCM, solvent B: DCM/EtOH 8:2 *v*/*v* - gradient: 0-40% solvent B. Yielding title compound 349 mg, 64%. ¹H NMR(400 MHz, DMSO-*d*₆) δ 12.20 (s, 1H), 7.58 (dd, *J* = 8.8, 2.4 Hz, 1H), 7.56 - 7.45 (m, 3H), 7.42 (dd, *J* = 8.6, 5.5 Hz, 3H), 7.34 (dt, *J* = 7.2, 1.6 Hz, 1H), 7.23 (dt, *J* = 7.2, 2.0 Hz, 1H), 7.18 (d, *J* = 3.2 Hz, 1H), 7.07 - 6.99 (m, 2H), 6.89 (d, *J* = 2.3 Hz, 1H), 4.58 (td, *J* = 11.6, 9.3, 3.2 Hz, 1H), 3.25 (dd, *J* = 16.5, 11.1 Hz, 1H), 2.57 - 2.53 (m, 1H). Rt 2.50 min (generic method). ESI-MS for C₂₄H₁₇BrClN₃O: calculated 476.0/478.0, found *m*/*z* 477.0/479.0 [M+H]⁺, 475.0/477.0 [M-H]⁻.

### Step 3. Synthesis of 4-[3-(4-bromophenyl)-5-(6-chloro-2-oxo-4-phenyl-1H-quinolin-3-yl)-3,4-dihydropyrazol-2-yl]-4-oxo-butanoic acid (d.8).

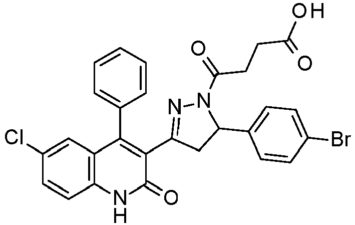

Compound d.8 was synthesized following the general procedure D1 using c.3 (330 mg, 0.69 mmol) with succinic anhydride (120 °C, 200 W). Purification by normal phase flash column chromatography (solvent A: DCM, solvent B: DCM/EtOH *8:2 v*/*v -* gradient: 0-50% solvent B) yielded title compound (275 mg, yield 68%). ¹H NMR(400 MHz, DMSO-*d*₆) δ 12.38 (s, 1H), 12.03 (s, 1H), 7.63 (dd, *J* = 8.8, 2.3 Hz, 1H), 7.59 - 7.47 (m, 3H), 7.45 (d, *J* = 8.8 Hz, 1H), 7.40 (d, *J* = 8.4 Hz, 2H), 7.27 (dd, *J* = 6.5, 1.8 Hz, 1H), 6.93 (d, *J* = 2.3 Hz, 1H), 6.73 (d, *J* = 8.5 Hz, 2H), 5.30 (dd, *J* = 12.0, 4.6 Hz, 1H), 3.74 (dd, *J* = 18.5, 12.1 Hz, 1H), 2.77 (dd, *J* = 18.5, 4.6 Hz, 1H), 2.48 - 2.36 (m, 2H), 2.28 (t, *J* = 7.1 Hz, 2H). Rt 2.18 min (generic method). ESI-MS for C₂₈H₂₁BrClN₃O₄: calculated 576.0/579.0, found *m*/*z* 577.0/580.0 [M+H]⁺, 575.0/578.0 [M-H]⁻.

### Example d.9. 3-[2-acetyl-3-(4-bromophenyl)-3,4-dihydropyrazol-5-yl]-6-chloro-4-phenyl-1H-quinolin-2-one (example not falling within the scope of the present invention).

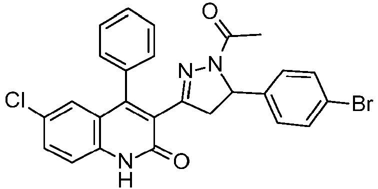

Compound d.9 was synthesized following the general procedure D1 using c.3 (100 mg, 0.21 mmol) with acetic anhydride. Purification by normal phase flash column chromatography (solvent A: DCM, solvent B: MeOH - gradient: 0-2% solvent B) yielded title compound (80 mg, yield 73%). ¹H NMR (400 MHz, DMSO-*d*₆) δ 12.39 (s, 1H), 7.64 (dd, *J* = 8.0, 4.0 Hz, 1H), 7.57 - 7.53 (m, 3H), 7.45-7.39 (m, 4H), 7.28 (dd, *J* = 6.0, 1.6 Hz, 1H), 6.93 (d, *J* = 2.0 Hz, 1H), 6.75 (d, *J* = 8.8 Hz, 2H), 5.30 (dd, *J* = 12.0, 4.4 Hz, 1H), 3.74 (dd, *J* = 18.5, 12.0 Hz, 1H), 2.82 (dd, *J* = 4.0, 20.0 Hz, 1H), 1.86 (s, 3H). ESI-MS for C₂₆H₁₉BrClN₃O₂: calculated 518.0/520.0, found m/z 519.0/521.0 [M+H]⁺, 517.0/519.0 [M-H]⁻.

### Example d.10. 3-[3-(4-bromophenyl)-2-propanoyl-3,4-dihydropyrazol-5-yl]-6-chloro-4-phenyl-1H-quinolin-2-one (example not falling within the scope of the present invention).

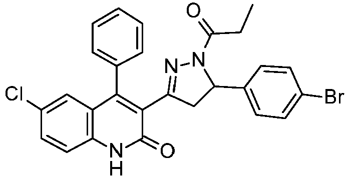

Compound d.10 was synthesized following the general procedure D2 using c.3 (150 mg, 0.31 mmol). Purification by normal phase flash column chromatography (solvent A: DCM, solvent B: MeOH - gradient: 0-0.5 % solvent B) yielded title compound (60 mg, yield 36%). ¹H NMR(400 MHz, DMSO-*d*₆) δ 12.41 (s, 1H), 7.64 (dd, *J* = 12.8, 1.6 Hz, 1H), 7.55 - 7.50 (m, 3H), 7.46-7.41 (m, 4H), 7.28 (d, *J* = 6.8 Hz, 1H), 6.93 (d, *J* = 2.0 Hz, 1H), 6.75 (d, *J* = 8.0 Hz, 2H), 5.30 (dd, *J* = 12.4, 4.0 Hz, 1H), 3.75 (dd, *J* = 18.4, 12.0 Hz, 1H), 2.80 (dd, *J* = 18.2, 4.6 Hz, 1H), 2.31 - 2.14 (m, 2H), 0.83 (t, *J* = 7.6 Hz, 3H). ESI-MS for C₂₇H₂₁BrClN₃O₂: calculated 532.1/534.1, found *m*/*z* 533.1/535.1 [M+H]⁺, 531.1/533.1 [M-H]⁻.

### Example d.11. 4-[5-(6-chloro-2-oxo-4-phenyl-1H-quinolin-3-yl)-3-(4-methoxyphenyl)-3,4-dihydropyrazol-2-yl]-4-oxo-butanoic acid (example not falling within the scope of the present invention).

### Step 1. Synthesis of 6-chloro-3-[(E)-3-(4-methoxyphenyl)prop-2-enoyl]-4-phenyl-1H-quinolin-2-one (b.4).

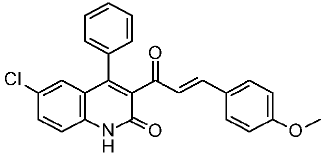

Compound b.4 was synthesized following the general procedure B using a.1 (611 mg, 2.0 mmol) and *p*-anisaldehyde. Purification by normal phase flash column chromatography (solvent A: DCM, solvent B: EtOH - gradient: 0-5% solvent B) yielded title compound (867 mg, quantitative yield). ¹H NMR(400 MHz, DMSO-*d*₆) δ 12.31 (s, 1H), 7.68 - 7.58 (m, 3H), 7.49 - 7.38 (m, 5H), 7.34 - 7.29 (m, 2H), 6.99 - 6.89 (m, 3H), 6.60 (d, *J* = 16.3 Hz, 1H), 3.78 (s, 3H). Rt 1.19 min (apolar method). ESI-MS for C₂₅H₁₈ClNO₃: calculated 415.1, found *m*/*z* 416.1 [M+H]⁺, 414.1 [M-H]⁻.

### Step 2. Synthesis of 6-chloro-3-[5-(4-methoxyphenyl)-4,5-dihydro-1H-pyrazol-3-yl]-4-phenyl-1H-quinolin-2-one (c. 4) .

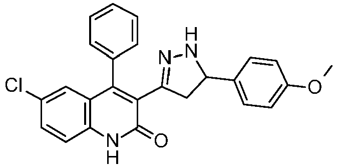

Compound c.4 was synthesized following the general procedure C using b.4 (780 mg, 1.9 mmol). Purification by normal phase flash column chromatography (solvent A: DCM, solvent B: EtOH - gradient: 0-10% solvent B) yielded title compound (611 mg, yield 75%). ¹H NMR(400 MHz, DMSO-*d*₆) δ 12.19 (s, 1H), 7.57 (dd, *J* = 8.8, 2.3 Hz, 1H), 7.53 - 7.47 (m, 3H), 7.42 (d, *J* = 8.8 Hz, 1H), 7.34 (dd, *J* = 10.3, 3.8 Hz, 1H), 7.27 - 7.19 (m, 1H), 7.02 (dd, *J* = 8.9, 5.9 Hz, 3H), 6.90 (d, *J* = 2.3 Hz, 1H), 6.80 (d, *J* = 8.7 Hz, 2H), 4.52 (td, *J* = 10.4, 2.9 Hz, 1H), 3.73 (s, 3H), 3.18 (dd, *J* = 16.4, 10.9 Hz, 1H), 2.58 - 2.53 (m, 1H). Rt 2.24 min (generic method). ESI-MS for C₂₅H₂₀ClN₃O₂: calculated 429.1, found *m*/*z* 430.1 [M+H]⁺, 428.1 [M-H]⁻.

### Step 3. Synthesis of 4-[5-(6-chloro-2-oxo-4-phenyl-1H-quinolin-3-yl)-3-(4-methoxyphenyl)-3,4-dihydropyrazol-2-yl]-4-oxo-butanoic acid (d.11).

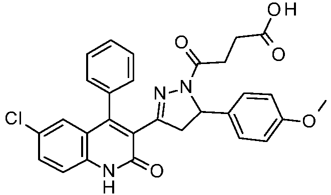

Compound d.11 was synthesized following the general procedure D1 using c.4 (320 mg, 0.74 mmol) with succinic anhydride (120 °C, 200 W). Purification by normal phase flash column chromatography (solvent A: DCM, solvent B: EtOH - gradient: 0-15% solvent B) yielded title compound (339 mg, yield 86%). ¹H NMR(400 MHz, DMSO-*d*₆) δ 12.19 (s, 1H), 7.57 (dd, *J* = 8.8, 2.3 Hz, 1H), 7.51 (m, 3H), 7.42 (d, *J* = 8.8 Hz, 1H), 7.36 - 7.31 (m, 1H), 7.27 - 7.21 (m, 1H), 7.02 (dd, *J* = 8.9, 5.9 Hz, 3H), 6.90 (d, *J* = 2.3 Hz, 1H), 6.80 (d, *J* = 8.7 Hz, 2H), 4.52 (td, *J* = 10.4, 2.9 Hz, 1H), 3.73 (s, 3H), 3.18 (dd, *J* = 16.4, 10.9 Hz, 1H), 2.58 - 2.52 (m, 5H). Rt 1.99 min (generic method). ESI-MS for C₂₉H₂₄ClN₃O₅: calculated 529.1, found *m*/*z* 530.1 [M+H]⁺, 528.1 [M-H]⁻.

### Example d.12. 6-chloro-3-[3-(4-methoxyphenyl)-2-propanoyl-3,4-dihydropyrazol-5-yl]-4-phenyl-1H-quinolin-2-one (example not falling within the scope of the present invention).

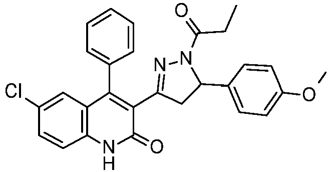

Compound d.12 was synthesized following the general procedure D2 using c.4 (150 mg, 0.358 mmol). Purification by normal phase flash column chromatography (solvent A: DCM, solvent B: MeOH - gradient: 0-2 % solvent B) yielded title compound (100 mg, yield 59%). ¹H NMR(400 MHz, DMSO-*d*₆) δ 12.36 (s, 1H), 7.64 (dd, *J* = 8.6, 2.4 Hz, 1H), 7.56 - 7.52 (m, 3H), 7.45 (d, *J* = 8.4 Hz, 1H), 7.40 - 7.38 (m, 1H), 7.29 - 7.27 (m, 1H), 6.92 (d, *J* = 2.0 Hz, 1H), 6.75 (dd, *J* = 15.2, 9.2 Hz, 4H), 5.24 (dd, *J* = 12.0, 4.8 Hz, 1H), 3.72 (s, 3H), 3.70 (dd, *J* = 16.0, 12.0 Hz, 1H), 2.81 (dd, *J* = 20.0, 4.4 Hz, 1H), 2.29 - 2.12 (m, 2H), 0.82 (t, *J* = 8.0 Hz, 3H). ESI-MS for C₂₈H₂₄ClN₃O₃: calculated 485.2, found m/z 486.2 [M+H]⁺, 484.3 [M-H]⁻.

### Example d.14. 4-[5-(6-chloro-2-oxo-4-phenyl-1H-quinolin-3-yl)-3-[4-(trifluoromethyl)phenyl]-3,4-dihydropyrazol-2-yl]-4-oxo-butanoic acid (example not falling within the scope of the present invention).

### Step 1. Synthesis of 6-chloro-4-phenyl-3-[(E)-3-[4-(trifluoromethyl)phenyl]prop-2-enoyl]-1H-quinolin-2-one (b.6).

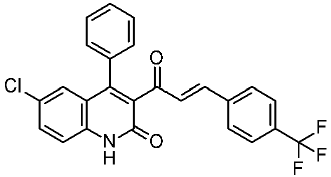

Compound b.6 was synthesized following the general procedure B using a.1 (760 mg, 2.5 mmol) and 4-(trifluoromethyl)benzaldehyde. Purification by normal phase flash column chromatography (solvent A: DCM, solvent B: EtOH - gradient: 0-5% solvent B) yielded title compound (820 mg, yield 71%). ¹H NMR (400 MHz, DMSO-*d*₆) δ 12.38 (s, 1H), 7.89 (d, *J* = 8.2 Hz, 2H), 7.74 (d, *J* = 8.4 Hz, 2H), 7.67 (dd, J = 8.8, 2.4 Hz, 1H), 7.62 - 7.57 (m, 1H), 7.53 - 7.40 (m, 4H), 7.36 - 7.31 (m, 2H), 6.99 (d, *J* = 2.3 Hz, 1H), 6.91 (d, *J* = 16.5 Hz, 1H). Rt 1.65 min (apolar method). ESI-MS for C₂₅H₁₅ClF₃NO₂: calculated 453.1, found *m*/*z* 454.1 [M+H]⁺, 452.1 [M-H]⁻.

### Step 2. Synthesis of 6-chloro-4-phenyl-3-[5-[4-(trifluoromethyl)phenyl]-4,5-dihydro-1H-pyrazol-3-yl]-1H-quinolin-2-one (c.6).

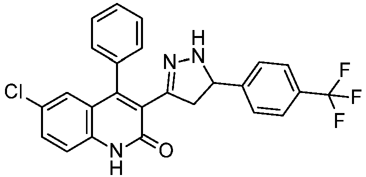

Compound c.6 was synthesized following the general procedure C using b.6 (752 mg, 1.7 mmol). Purification by normal phase flash column chromatography (solvent A: DCM, solvent B: EtOH - gradient: 0-5% solvent B) yielded title compound (696 mg, yield 90%). ¹H NMR(400 MHz, DMSO-*d*₆) δ 12.21 (s, 1H), 7.63 - 7.56 (m, 3H), 7.56 - 7.44 (m, 3H), 7.42 (d, *J* = 8.8 Hz, 1H), 7.37 - 7.32 (m, 1H), 7.29 (d, *J* = 8.5 Hz, 3H), 7.25 - 7.20 (m, 1H), 6.90 (d, *J* = 2.3 Hz, 1H), 4.75 - 4.64 (m, 1H), 3.39 - 3.25 (m, 1H), 2.63 - 2.54 (m, 1H). Rt 2.52 min (generic method). ESI-MS for C₂₅H₁₇ClF₃N₃O: calculated 467.1, found m/z 468.1 [M+H]⁺, 466.1 [M-H]⁻.

### Step 3. Synthesis of 4-[5-(6-chloro-2-oxo-4-phenyl-1H-quinolin-3-yl)-3-[4-(trifluoromethyl)phenyl]-3,4-dihydropyrazol-2-yl]-4-oxo-butanoic acid (d.14).

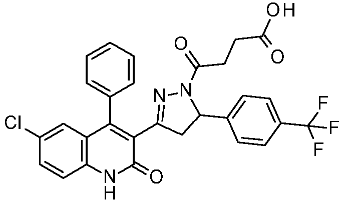

Compound d.14 was synthesized following the general procedure D1 using c.6 (309 mg, 0.66 mmol) with succinic anhydride (120 °C, 200 W) . Purification by normal phase flash column chromatography (solvent A: DCM, solvent B: EtOH - gradient: 0-10% solvent B) yielded title compound (266 mg, yield 71%). ¹H NMR(400 MHz, DMSO-*d*₆) δ 12.38 (s, 1H), 12.05 (s, 1H), 7.63 (dd, *J* = 8.8, 2.4 Hz, 1H), 7.60 - 7.39 (m, 7H), 7.31 - 7.19 (m, 1H), 7.00 (d, *J* = 8.1 Hz, 2H), 6.93 (d, *J* = 2.3 Hz, 1H), 5.42 (dd, *J* = 12.1, 4.7 Hz, 1H), 3.78 (dd, *J* = 18.5, 12.2 Hz, 1H), 2.80 (dd, *J* = 18.5, 4.7 Hz, 1H), 2.59 - 2.38 (m, 2H), 2.36 - 2.26 (m, 2H). Rt 2.22 min (generic method) . ESI-MS for C₂₉H₂₁ClF₃N₃O₄: calculated 567.1, found *m*/*z* 568.1 [M+H]⁺, 566.1 [M-H]⁻.

### Example d.15. 3-[2-acetyl-3-[4-(trifluoromethyl)phenyl]-3,4-dihydropyrazol-5-yl]-6-chloro-4-phenyl-1H-quinolin-2-one (example not falling within the scope of the present invention).

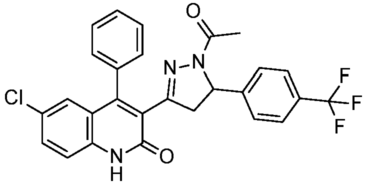

Compound d.15 was synthesized following the general procedure D1 using c.6 (326 mg, 0.70 mmol) with acetic anhydride (165 °C, 200 W). Purification by normal phase flash column chromatography (solvent A: DCM, solvent B: EtOH - gradient: 0-5% solvent B) yielded title compound (268 mg, yield 74%). ¹H NMR(400 MHz, DMSO-*d*₆) δ 12.39 (s, 1H), 7.67 - 7.49 (m, 6H), 7.46 (d, *J* = 8.8 Hz, 1H), 7.44 - 7.39 (m, 1H), 7.32 - 7.25 (m, 1H), 7.03 (d, *J* = 8.0 Hz, 2H), 6.95 (d, *J* = 2.4 Hz, 1H), 5.42 (dd, *J* = 12.1, 4.7 Hz, 1H), 3.79 (dd, *J* = 18.5, 12.1 Hz, 1H), 2.87 (dd, *J* = 18.5, 4.8 Hz, 1H), 1.89 (s, 3H). Rt 2.55 min (generic method). ESI-MS for C₂₇H₁₉ClF₃N₃O₂: calculated 509.1, found m/z 510.1 [M+H]⁺, 508.1 [M-H]⁻.

### Example d.16. 4-[3-(4-tert-butylphenyl)-5-(6-chloro-2-oxo-4-phenyl-1H-quinolin-3-yl)-3,4-dihydropyrazol-2-yl]-4-oxo-butanoic acid (example not falling within the scope of the present invention).

### Step 1. Synthesis of 3-[(E)-3-(4-tert-butylphenyl)prop-2-enoyl]-6-chloro-4-phenyl-1H-quinolin-2-one (b.7).

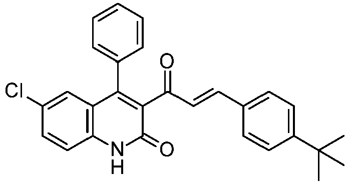

Compound b.7 was synthesized following the general procedure B using a.1 (543 mg, 1.8 mmol) and 4-tert-buthylbenzaldehyde. Purification by normal phase flash column chromatography (solvent A: DCM, solvent B: EtOH - gradient: 0-10% solvent B) yielded title compound (441 mg, yield 55%). ¹H NMR(400 MHz, DMSO-*d*₆) δ 12.33 (s, 1H), 7.64 (dd, *J* = 8.8, 2.4 Hz, 1H), 7.58 (d, *J* = 8.5 Hz, 2H), 7.50 - 7.37 (m, 7H), 7.32 (dd, *J* = 7.7, 1.9 Hz, 2H), 6.97 (d, *J* = 2.4 Hz, 1H), 6.69 (d, *J* = 16.4 Hz, 1H), 1.26 (s, 9H). Rt 2.18 min (generic method). ESI-MS for C₂₈H₂₄ClNO₂: calculated 441.1, found m/z 442.1 [M+H]⁺, 440.1 [M-H]⁻.

### Step 2. Synthesis of 3-[5-(4-tert-butylphenyl)-4,5-dihydro-1H-pyrazol-3-yl]-6-chloro-4-phenyl-1H-quinolin-2-one (c.7).

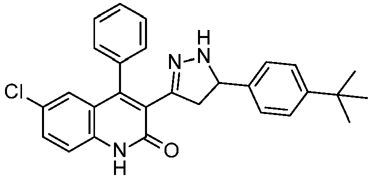

Compound c.7 was synthesized following the general procedure C using b.7 (369 mg, 0.83 mmol). Purification by normal phase flash column chromatography (solvent A: CHX, solvent B: AcOEt - gradient: 0-90% solvent B) yielded title compound (351 mg, yield 93%). ¹H NMR(400 MHz, DMSO-*d*₆) δ 12.20 (s, 1H), 7.56 (dd, *J* = 8.8, 2.4 Hz, 1H), 7.49 (dtd, J = 9.6, 7.2, 5.3 Hz, 3H), 7.42 (d, *J* = 8.8 Hz, 1H), 7.33 (dt, *J* = 7.2, 1.7 Hz, 1H), 7.25 (d, *J* = 8.3 Hz, 3H), 7.06 (d, *J* = 3.0 Hz, 1H), 7.02 (d, *J* = 8.4 Hz, 2H), 6.90 (d, *J* = 2.3 Hz, 1H), 4.54 (t, *J* = 10.4 Hz, 1H), 3.19 (dd, *J* = 16.4, 10.9 Hz, 1H), 2.58 (dd, *J* = 16.4, 9.6 Hz, 1H), 1.26 (s, 9H). Rt 1.82 min (generic method). ESI-MS for C₂₈H₂₆ClN₃O: calculated 455.2, found m/z 456.2 [M+H]⁺, 454.2 [M-H]⁻.

### Step 3. Synthesis of 4-[3-(4-tert-butylphenyl)-5-(6-chloro-2-oxo-4-phenyl-1H-quinolin-3-yl)-3,4-dihydropyrazol-2-yl]-4-oxo-butanoic acid (d.16).

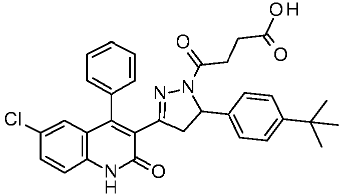

Compound d.16 was synthesized following the general procedure D1 using c.7 (124 mg, 0.27 mmol) with succinic anhydride (120 °C, 200 W). Purification by normal phase flash column chromatography (solvent A: DCM, solvent B: EtOH - gradient: 0-20% solvent B) yielded title compound (121 mg, yield 81%). ¹H NMR(400 MHz, DMSO-*d*₆) δ 12.27 (d, *J* = 78.0 Hz, 1H), 12.08 (s, 1H), 7.64 (dd, *J* = 8.8, 2.3 Hz, 1H), 7.59 - 7.47 (m, 3H), 7.47 - 7.43 (m, 1H), 7.40 (dd, *J* = 4.6, 2.1 Hz, 1H), 7.28 (d, *J* = 7.5 Hz, 1H), 7.23 (d, *J* = 8.3 Hz, 2H), 6.93 (d, *J* = 2.3 Hz, 1H), 6.73 (d, *J* = 8.3 Hz, 2H), 5.26 (dd, *J* = 11.9, 4.5 Hz, 1H), 3.69 (dt, *J* = 24.9, 12.5 Hz, 1H), 2.85 (ddd, *J* = 23.0, 18.2, 4.3 Hz, 1H), 2.47 (dd, *J* = 6.7, 4.0 Hz, 2H), 2.36 - 2.22 (m, 2H), 1.27 (s, 9H). Rt 2.39 min (generic method). ESI-MS for C₃₂H₃₀ClN₃O₄: calculated 555.2, found m/z 556.2 [M+H]⁺, 554.2 [M-H]⁻.

### Example d.17. 3-[2-acetyl-3-(4-tert-butylphenyl)-3,4-dihydropyrazol-5-yl]-6-chloro-4-phenyl-1H-quinolin-2-one (example not falling within the scope of the present invention)

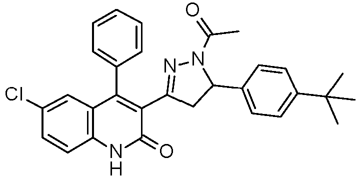

Compound d.17 was synthesized following the general procedure D1 using c.7 (141 mg, 0.31 mmol) with acetic anhydride (165 °C, 200 W). Purification by normal phase flash column chromatography (solvent A: DCM, solvent B: EtOH - gradient: 0-5% solvent B) yielded title compound (105 mg, yield 68%). ¹H NMR(400 MHz, DMSO-*d₆*) δ 12.36 (s, 1H), 7.63 (dt, J = 9.8, 4.9 Hz, 1H), 7.60 - 7.48 (m, 3H), 7.44 (t, J = 9.5 Hz, 1H), 7.41 - 7.36 (m, 1H), 7.30 (d, J = 7.1 Hz, 1H), 7.24 (d, J = 8.3 Hz, 2H), 6.94 (d, J = 2.3 Hz, 1H), 6.75 (d, J = 8.3 Hz, 2H), 5.32 - 5.17 (m, 1H), 3.69 (dt, J = 28.5, 14.3 Hz, 1H), 2.86 (dd, J = 18.4, 4.6 Hz, 1H), 1.86 (s, 3H), 1.27 (s, 9H). Rt 1.83 min (apolar method). ESI-MS for C₃₀H₂₈ClN₃O₂: calculated 497.2, found m/z 498.2 [M+H]⁺, 496.2 [M-H]⁻.

### Example d.18. 4-[5-(6-chloro-2-oxo-4-phenyl-1H-quinolin-3-yl)-3-(3-fluoro-4-methoxy-phenyl)-3,4-dihydropyrazol-2-yl]-4-oxo-butanoic acid (example not falling within the scope of the present invention).

### Step 1. Synthesis of 3-fluoro-4-methoxy-benzaldehyde.

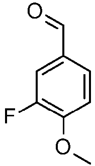

In a screw capped pressure tube 3-fluoro-4-hydroxybenzaldehyde (461 mg, 3.29 mmol) was dissolved with 3.0 ml of anhydrous DMF then potassium carbonate (1.49 g, 10.8 mmol) was added and the mixture kept under stirring for 5 minutes at rt MeI (245 µl, 3.95 mmol) was then added. The reaction was thus heated to 70 °C and stirred for 3.5 hours. The crude mixture was diluted with CHCl₃ and washed three times with water. The organic layer was anhydrified through a phase separator and the solvent removed under reduced pressure. Purification by normal phase flash column chromatography (solvent A: CHX - Solvent B: AtOAc - gradient: 0-20% solvent B) yielded title compound (440 mg, yield 87%). ¹H NMR(400 MHz, DMSO-*d*₆) δ 9.87 (d, *J* = 2.1 Hz, 1H), 7.79 (ddd, *J* = 8.4, 2.0, 1.0 Hz, 1H), 7.69 (dd, *J* = 11.4, 2.0 Hz, 1H), 7.39 (t, *J* = 8.4 Hz, 1H), 3.95 (s, 3H). Rt 1.59 min (generic method) . ESI-MS for C₈H₇FO₂: calculated 154.0, found *m*/*z* 156.0 [M+H]⁺.

### Step 2. Synthesis of 6-chloro-3-[(E)-3-(3-fluoro-4-methoxy-phenyl)prop-2-enoyl]-4-phenyl-1H-quinolin-2-one (b.8).

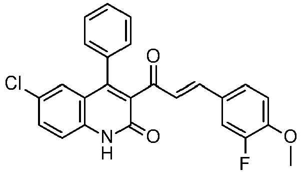

Compound b.8 was synthesized following the general procedure B using a.1 (473 mg, 1.6 mmol) and 3-Fluoro-4-methoxybenzaldehyde. Title compound was obtained after precipitation and filtration from the reaction crude (547 mg, yield 79%). ¹H NMR(600 MHz, DMSO-*d*₆) δ 12.38 (s, 1H), 7.70 - 7.62 (m, 2H), 7.52 - 7.40 (m, 6H), 7.35 - 7.30 (m, 2H), 7.18 (t, *J* = 8.7 Hz, 1H), 6.97 (d, *J* = 2.4 Hz, 1H), 6.69 (d, *J* = 16.3 Hz, 1H), 3.88 (s, 3H). ESI-MS for C₂₅H₁₇ClFNO₃: calculated 433.1, found m/z 434.1 [M+H]⁺, 432.1 [M-H]⁻.

### Step 3. Synthesis of 6-chloro-3-[5-(3-fluoro-4-methoxyphenyl)-4,5-dihydro-1H-pyrazol-3-yl]-4-phenyl-1H-quinolin-2-one (c.8).

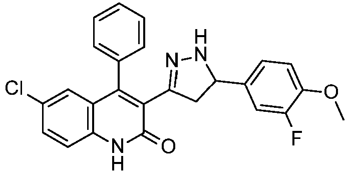

Compound c.8 was synthesized following the general procedure C using b.8 (203 mg, 0.50 mmol). Purification by normal phase flash column chromatography (solvent A: DCM, solvent B: EtOH - gradient: 0-5% solvent B) yielded title compound (213 mg, quantitative yield). ¹H NMR(400 MHz, DMSO-*d*₆) δ 12.20 (s, 1H), 7.63 - 7.39 (m, 5H), 7.35 - 7.29 (m, 1H), 7.23 (dt, *J* = 7.0, 2.0 Hz, 1H), 7.12 (d, *J* = 3.1 Hz, 1H), 7.03 (t, *J* = 8.6 Hz, 1H), 6.97 - 6.85 (m, 3H), 4.55 (ddd, *J* = 11.5, 9.0, 2.9 Hz, 1H), 3.81 (s, 3H), 3.19 (dd, *J* = 16.5, 11.0 Hz, 1H), 2.60 - 2.53 (m, 1H). Rt 2.27 min (generic method). ESI-MS for C₂₅H₁₉ClFN₃O₂: calculated 447.1, found *m*/*z* 448.1 [M+H]⁺, 446.1 [M-H]⁻.

### Step 4. Synthesis of 4-[5-(6-chloro-2-oxo-4-phenyl-1H-quinolin-3-yl)-3-(3-fluoro-4-methoxy-phenyl)-3,4-dihydropyrazol-2-yl]-4-oxo-butanoic acid (d.18).

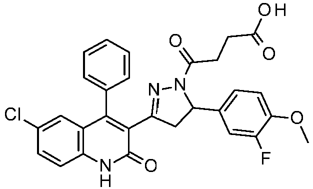

Compound d.18 was synthesized following the general procedure D1 using c.8 (184 mg, 0.41 mmol) with succinic anhydride (120 °C, 200 W). Purification by normal phase flash column chromatography (solvent A: DCM, solvent B: EtOH - gradient: 0-10% solvent B) yielded title compound (115 mg, yield 51%). ¹H NMR (400 MHz, DMSO-*d*₆) δ 12.38 (s, 1H), 12.06 (s, 1H), 7.64 (dd, *J* = 8.8, 2.4 Hz, 1H), 7.59 - 7.43 (m, 4H), 7.43 - 7.38 (m, 1H), 7.29 (dd, *J* = 7.1, 1.9 Hz, 1H), 7.01 (t, *J* = 8.8 Hz, 1H), 6.94 (d, *J* = 2.4 Hz, 1H), 6. 69 - 6.61 (m, 2H), 5.27 (dd, *J* = 12.0, 4.5 Hz, 1H), 3.82 (s, 3H), 3.70 (dd, *J* = 18.5, 12.0 Hz, 1H), 2.82 (dd, *J* = 18.5, 4.5 Hz, 1H), 2.46 (dd, *J* = 14.3, 7.0 Hz, 2H), 2.29 (t, *J* = 7.2 Hz, 2H). Rt 2.00 min (generic method). ESI-MS for C₂₉H₂₃ClFN₃O₅: calculated 547.1, found m/z 548.1 [M+H]⁺, 546.1 [M-H]⁻.

### Example d.19. 4-[5-[6-chloro-4-(4-methoxyphenyl)-2-oxo-1H-quinolin-3-yl]-3-(4-fluorophenyl)-3,4-dihydropyrazol-2-yl]-4-oxo-butanoic acid (example not falling within the scope of the present invention).

### Step 1. Synthesis of 3-acetyl-6-chloro-4-(4-methoxyphenyl)-1H-quinolin-2-one (a.2).

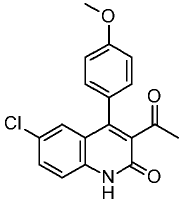

Compound a.2 was synthesized following the general procedure A1 with (2-Amino-5-chlorophenyl) (4-methoxyphenyl)methanone (495 mg, 1.9 mmol). Purification by normal phase flash column chromatography (solvent A: DCM, solvent B: DCM/EtOH 8:2 v/v - gradient: 0-20% solvent B) yielded title compound (343 mg, yield 56%). ¹H NMR(400 MHz, DMSO-*d*₆) δ 12.33 (s, 1H), 7.63 (dd, J = 8.8, 2.3 Hz, 1H), 7.43 (d, J = 8.8 Hz, 1H), 7.26 (d, J = 8.6 Hz, 2H), 7.08 (d, J = 8.7 Hz, 2H), 7.03 (d, J = 2.3 Hz, 1H), 3.84 (s, 3H), 2.19 (s, 3H). Rt 2.04 min (generic method). ESI-MS for C₁₈H₁₄ClNO₃: calculated 327.1, found m/z 328.1 [M+H]⁺, 326.1 [M-H]⁻.

### Step 2. Synthesis of 6-chloro-3-[(E)-3-(4-fluorophenyl)prop-2-enoyl]-4-(4-methoxyphenyl)-1H-quinolin-2-one (b.9).

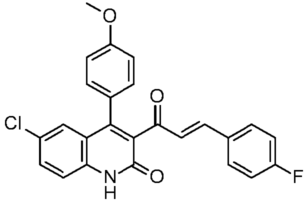

Compound b.9 was synthesized following the general procedure B using a.2 (231 mg, 0.7 mmol) and 4-fluorobenzaldehyde. Title compound was obtained after precipitation and filtration from the reaction crude (253 mg, yield 83%). ¹H NMR(400 MHz, DMSO-*d₆*) δ 7.74 (dd, J = 8.6, 5.5 Hz, 2H), 7.58 (dd, J = 8.8, 2.4 Hz, 1H), 7.51 (d, J = 8.9 Hz, 1H), 7.43 (d, J = 16.4 Hz, 1H), 7.23 (t, J = 9.1 Hz, 4H), 7.06 - 6.95 (m, 3H), 6.73 (d, J = 16.4 Hz, 1H), 3.76 (s, 3H) . Rt 2.35 min (generic method) . ESI-MS for C₂₅H₁₇ClFNO₃ : calculated 433.1, found m/z 434.1 [M+H]⁺, 432.1 [M-H]⁻.

### Step 3. Synthesis of 6-chloro-3-[5-(4-fluorophenyl)-4,5-dihydro-1H-pyrazol-3-yl]-4-(4-methoxyphenyl)-1H-quinolin-2-one (c.9).

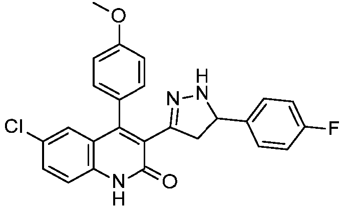

Compound c.9 was synthesized following the general procedure C using b.9 (250 mg, 0.60 mmol). Purification by normal phase flash column chromatography (solvent A: DCM, solvent B: DCM/EtOH 8:2 v/v - gradient: 0-25% solvent B) yielded title compound (179 mg, yield 69%). ¹H NMR(400 MHz, DMSO-*d₆*) δ 12.15 (s, 1H), 7.57 (dd, J = 8.8, 2.4 Hz, 1H), 7.40 (d, J = 8.8 Hz, 1H), 7.26 (dd, J = 8.4, 2.3 Hz, 1H), 7.20 - 6.93 (m, 9H), 4.60 (dd, J = 11.0, 9.2 Hz, 1H), 3.85 (s, 3H), 3.20 (dd, J = 16.4, 11.0 Hz, 1H), 2.55-2.53 (s, 1H). Rt 2.30 min (generic method). ESI-MS for C₂₅H₁₉ClFN₃0₂: calculated 447.1, found m/z 448.1 [M+H]⁺, 446.1 [M-H]⁻.

### Step 4. Synthesis of 4-[5-[6-chloro-4-(4-methoxyphenyl)-2-oxo-1H-quinolin-3-yl]-3-(4-fluorophenyl)-3,4-dihydropyrazol-2-yl]-4-oxo-butanoic acid acid (d.19).

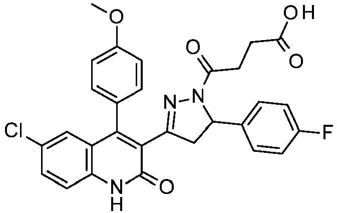

Compound d.19 was synthesized following the general procedure D1 using c.9 (164 mg, 0.36 mmol) with succinic anhydride (120 °C, 200 W). Purification by normal phase flash column chromatography (solvent A: DCM, solvent B: DCM/EtOH 8:2 v/v - gradient: 0-80% solvent B) yielded title compound (136 mg, yield 69%). ¹H NMR(400 MHz, DMSO-*d₆*) δ 12.34 (s, 1H), 12. 08 (s, 1H), 7.63 (dd, J = 8.8, 2.3 Hz, 1H), 7.44 (d, J = 8.8 Hz, 1H), 7.32 (dd, J = 8.5, 2.3 Hz, 1H), 7.20 (dd, J = 8.4, 2.2 Hz, 1H), 7.10 (dd, J = 8.5, 2.7 Hz, 1H), 7.02 (td, J = 8.9, 6.9 Hz, 4H), 6.91 - 6.83 (m, 2H), 5.33 (dd, J = 11.9, 4.4 Hz, 1H), 3.86 (s, 3H), 3.66 (dd, J = 18.4, 12.0 Hz, 1H), 2.73 (dd, J = 18.4, 4.4 Hz, 1H), 2.59 (tt, J = 15.5, 6.8 Hz, 2H), 2.33 (t, J = 6.8 Hz, 2H). Rt 2.05 min (generic method). ESI-MS for C₂₉H₂₃ClFN₃0₅: calculated 547.1, found m/z 548.1 [M+H]⁺, 546.1 [M-H]⁻.

### Example d.20. 4-[5-(6-chloro-2-oxo-4-phenyl-1H-quinolin-3-yl)-3-(2-furyl)-3,4-dihydropyrazol-2-yl]-4-oxo-butanoic acid (example not falling within the scope of the present invention).

### Step 1. Synthesis of 6-chloro-3- [ (E) -3- (2-furyl) prop-2-enoyl]-4-phenyl-1H-quinolin-2-one (b.10).

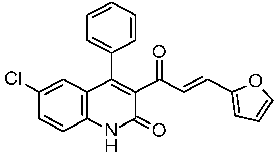

Compound b.10 was synthesized following the general procedure B using a.1 (503 mg, 1.7 mmol) and furan-2-carbaldehyde. Purification by normal phase flash column chromatography (solvent A: DCM, solvent B: EtOH - gradient: 0-5% solvent B) yielded title compound (471 mg, yield 74%). ¹H NMR(400 MHz, DMSO-*d*₆) δ 12.34 (s, 1H), 7.85 (d, *J* = 1.7 Hz, 1H), 7.64 (dd, *J* = 8.8, 2.4 Hz, 1H), 7.51 - 7.40 (m, 4H), 7.35 (s, 1H), 7.33 - 7.26 (m, 2H), 6.96 (dd, *J* = 6.9, 2.9 Hz, 2H), 6.63 (dd, *J* = 3.5, 1.8 Hz, 1H), 6.37 (d, *J* = 16.1 Hz, 1H). Rt 2.24 min (generic method). ESI-MS for C₂₂H₁₄ClNO₃: calculated 375.1, found m/z 376.0 [M+H]⁺, 374.0 [M-H]⁻.

### Step 2. Synthesis of 6-chloro-3-[5-(2-furyl)-4,5-dihydro-1H-pyrazol-3-yl]-4-phenyl-1H-quinolin-2-one (c.10).

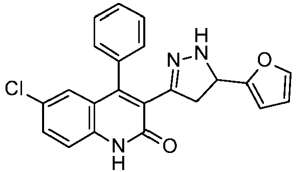

Compound c.10 was synthesized following the general procedure C using b.10 (442 mg, 1.2 mmol). Purification by normal phase flash column chromatography (solvent A: DCM, solvent B: EtOH - gradient: 0-7.5% solvent B) yielded title compound (422 mg, yield 91%). ¹H NMR(400 MHz, DMSO-*d*₆) δ 12.21 (s, 1H), 7.57 (dd, *J* = 8.8, 2.3 Hz, 1H), 7.54 - 7.51 (m, 1H), 7.50 - 7.38 (m, 4H), 7.31 - 7.23 (m, 2H), 7.07 (d, *J* = 2.8 Hz, 1H), 6. 90 (d, *J* = 2.3 Hz, 1H), 6.34 (dd, *J* = 3.1, 1.9 Hz, 1H), 6.17 (d, *J* = 3.2 Hz, 1H), 4.61 - 4.53 (m, 1H), 3. 04 (dd, *J* = 16. 2, 10.6 Hz, 1H), 2.87 (dd, *J* = 16.2, 9.2 Hz, 1H). Rt 2.14 min (generic method). ESI-MS for C₂₂H₁₆ClN₃0₂: calculated 389.1, found *m*/*z* 390.1 [M+H]⁺, 388.1 [M-H]⁻.

### Step 3. Synthesis of 4-[5-(6-chloro-2-oxo-4-phenyl-1H-quinolin-3-yl)-3-(2-furyl)-3,4-dihydropyrazol-2-yl]-4-oxo-butanoic acid (d.20).

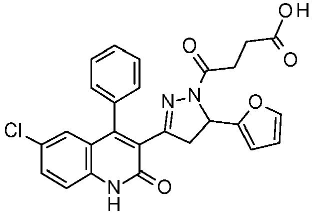

Compound d.20 was synthesized following the general procedure D1 using c.10 (201 mg, 0.51 mmol) with succinic anhydride (120 °C, 200 W). Purification by normal phase flash column chromatography (solvent A: DCM, solvent B: EtOH - gradient: 0-10% solvent B) yielded title compound (226 mg, yield 90%). ¹H NMR(400 MHz, DMSO-*d*₆) δ 12.38 (s, 1H), 12.06 (s, 1H), 7.64 (dd, *J* = 8.8, 2.3 Hz, 1H), 7.54 - 7.43 (m, 5H), 7.32 (t, *J* = 6.4 Hz, 2H), 6.96 (d, *J* = 2.3 Hz, 1H), 6.33 (dd, *J* = 3.1, 1.9 Hz, 1H), 5.99 (d, *J* = 3.2 Hz, 1H), 5.39 (dd, *J* = 11.8, 4.5 Hz, 1H), 3.58 (dd, *J* = 18.2, 11.9 Hz, 1H), 3.17 (dd, *J* = 18.2, 4.6 Hz, 1H), 2.41 - 2.18 (m, 4H). Rt 1.90 min (generic method). ESI-MS for C₂₆H₂ₒClN₃0₅: calculated 489.1, found m/z 490.1 [M+H]⁺, 488.1 [M-H]⁻.

### Example d.21. 3-[2-acetyl-3-(2-furyl)-3,4-dihydropyrazol-5-yl]-6-chloro-4-phenyl-1H-quinolin-2-one (example not falling within the scope of the present invention).

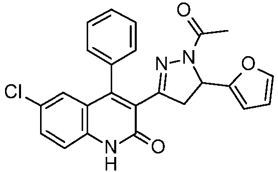

Compound d.21 was synthesized following the general procedure D1 using c.10 (202 mg, 0.52 mmol) with acetic anhydride (165 °C, 200 W). Purification by normal phase flash column chromatography (solvent A: DCM, solvent B: EtOH - gradient: 0-5% solvent B) yielded title compound (162 mg, yield 73%). ¹H NMR(400 MHz, DMSO-*d*₆) δ 12.36 (s, 1H), 7.63 (dd, *J* = 8.8, 2.3 Hz, 1H), 7.52 - 7.42 (m, 5H), 7.35 - 7.29 (m, 2H), 6.96 (d, *J* = 2.3 Hz, 1H), 6.33 (dd, *J* = 3.3, 1.9 Hz, 1H), 6.03 (d, *J* = 3.2 Hz, 1H), 5.38 (dd, *J* = 11.9, 4.6 Hz, 1H), 3.57 (dd, *J* = 18.2, 11.9 Hz, 1H), 3.20 (dd, *J* = 18.2, 4.7 Hz, 1H), 1.75 (s, 3H). ESI-MS for C₂₄H₁ₑC1N₃0₃: calculated 431.1, found *m*/*z* 432.3 [M+H]⁺, 430.1 [M-H]⁻.

### Example d.22. 4-[5-(6-chloro-2-oxo-4-phenyl-1H-quinolin-3-yl)-3-(1-methylindol-5-yl)-3,4-dihydropyrazol-2-yl]-4-oxo-butanoic acid (example not falling within the scope of the present invention).

### Step 1. Synthesis of 1-methylindole-5-carbaldehyde.

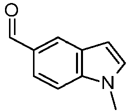

In a screw capped pressure tube 1*H*-indole-5-carboxaldehyde (333 mg, 2.3 mmol) was dissolved with 2.0 ml of anhydrous DMF then potassium carbonate (630 mg, 4.6 mmol) was added and the mixture kept under stirring for 10 minutes at rt. MeI (647 mg, d = 2.28 g/ml, 284 µl, 4.6 mmol) was then added. The reaction was thus heated to 35 °C and stirred for 18 hours. The crude mixture was diluted with DCM and washed three times with water. The organic layer was anhydrified through a phase separator and the solvent removed under reduced pressure. Purification by normal phase flash column chromatography (solvent A: CHX, solvent B: EtOAc - gradient: 0-7.5% solvent B) yielded title compound (286 mg, yield 81%). ¹H NMR(400 MHz, DMSO-*d*₆) δ 9.98 (s, 1H), 8.18 (d, *J* = 1.5 Hz, 1H), 7.74 - 7.56 (m, 2H), 7.49 (d, *J* = 3.2 Hz, 1H), 6.67 (dd, *J* = 3.1, 0.8 Hz, 1H), 3.85 (d, *J* = 1.3 Hz, 3H). Rt 1.75 min (generic method) . ESI-MS for C₁₀H₉NO: calculated 159.1, found *m*/*z* 160.1 [M+H]⁺.

### Step 2. Synthesis of 6-chloro-3-[(E)-3-(1-methylindol-5-yl)prop-2-enoyl]-4-phenyl-1H-quinolin-2-one (b.11).

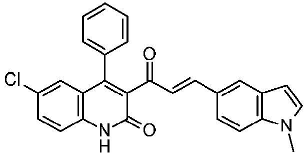

Compound b.11 was synthesized following the general procedure B using a.1 (545 mg, 1.8 mmol) and 1-Methyl-2*H-*indole-5-carbaldehyde. Purification by normal phase flash column chromatography (solvent A: DCM, solvent B: EtOH - gradient: 0-10% solvent B) yielded title compound (427 mg, 1-Methyl-2*H*-indole-5-carbaldehyde 53%). ¹H NMR(400 MHz, DMSO-*d*₆) δ 12.31 (s, 1H), 7.86 (d, *J* = 1.5 Hz, 1H), 7.65 (dd, *J* = 8.8, 2.3 Hz, 1H), 7.56 (d, *J* = 16.3 Hz, 1H), 7.51 - 7.30 (m, 9H), 6.97 (d, *J* = 2.3 Hz, 1H), 6.65 (d, *J* = 16.3 Hz, 1H), 6.44 (dd, *J* = 3.2, 0.7 Hz, 1H), 3.79 (s, 3H). Rt 2.45 min (generic method) . ESI-MS for C₂₇H₁₉ClN₂O₂: calculated 438.1, found *m*/*z* 439.1 [M+H]⁺, 437.1 [M-H]⁻.

### Step 3. Synthesis of 6-chloro-3-[5-(1-methylindol-5-yl)-4,5-dihydro-1H-pyrazol-3-yl]-4-phenyl-1H-quinolin-2-one (c.11).

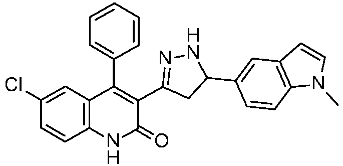

Compound c .11 was synthesized following the general procedure C using b.11 (370 mg, 0.84 mmol). Purification by normal phase flash column chromatography (solvent A: DCM, solvent B: EtOH - gradient: 0-5% solvent B) yielded title compound (325 mg, yield 85%). ¹H NMR(400 MHz, DMSO-*d*₆) δ 12.20 (s, 1H), 7.57 - 7.46 (m, 4H), 7.41 (d, *J* = 8.8 Hz, 1H), 7.35 (dt, *J* = 7.2, 1.7 Hz, 1H), 7.31 - 7.21 (m, 4H), 7.02 (s, 1H), 6.91 (td, *J* = 3.9, 1.7 Hz, 2H), 6.34 (dd, *J* = 3.0, 0.7 Hz, 1H), 4.66 (t, *J* = 10.5 Hz, 1H), 3.75 (s, 3H), 3.22 (dd, *J* = 16.4, 11.0 Hz, 1H), 2.63 (dd, *J* = 16.4, 9.9 Hz, 1H). Rt 2.38 min (generic method). ESI-MS for C₂₇H₂₁ClN₄O: calculated 452.1, found *m*/*z* 453.1 [M+H]⁺, 451.1 [M-H]⁻.

### Step 4. Synthesis of 4-[5-(6-chloro-2-oxo-4-phenyl-1H-quinolin-3-yl)-3-(1-methylindol-5-yl)-3,4-dihydropyrazol-2-yl]-4-oxo-butanoic acid (d.22).

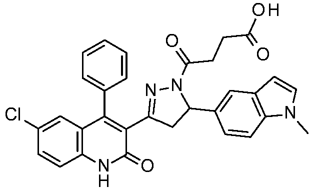

Compound d.22 was synthesized following the general procedure D1 using c.11 (153 mg, 0.34 mmol) with succinic anhydride (120 °C, 200 W). Purification by normal phase flash column chromatography (solvent A: DCM, solvent B: EtOH - gradient: 0-20% solvent B) yielded title compound (150 mg, yield 79%). ¹H NMR(400 MHz, DMSO-*d*₆) δ 12.37 (s, 1H), 12.18 (s, 1H), 7.63 (dd, *J* = 8.8, 2.3 Hz, 1H), 7.60 - 7.55 (m, 2H), 7.55 - 7.49 (m, 1H), 7.44 (dd, *J* = 7.7, 4.3 Hz, 2H), 7.31 - 7.20 (m, 3H), 6.98 (d, *J* = 1.7 Hz, 1H), 6.93 (d, *J* = 2.3 Hz, 1H), 6.61 (dd, *J* = 8.5, 1.7 Hz, 1H), 6.34 (dd, *J* = 3.0, 0.8 Hz, 1H), 5.35 (dd, *J* = 12.0, 4.6 Hz, 1H), 3.84 - 3.69 (m, 4H), 2.85 (dd, *J* = 18.4, 4.6 Hz, 1H), 2.53 - 2.45 (m, 2H), 2.26 (t, *J* = 6.9 Hz, 2H). Rt 2.09 min (generic method). ESI-MS for C₃₁H₂₅ClN₄O₄: calculated 552.2, found *m*/*z* 553.2 [M+H]⁺, 551.2 [M-H]⁻.

### Example d.23. 3-[2-acetyl-3-(1-methylindol-5-yl)-3,4-dihydropyrazol-5-yl]-6-chloro-4-phenyl-1H-quinolin-2-one (example not falling within the scope of the present invention).

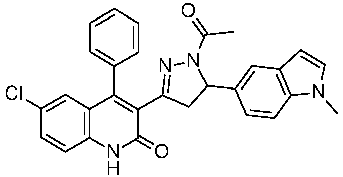

Compound d.23 was synthesized following the general procedure D1 using c.11 (127 mg, 0.28 mmol) with acetic anhydride (165 °C, 200 W). Purification by normal phase flash column chromatography (solvent A: DCM, solvent B: EtOH - gradient: 0-10% solvent B) yielded title compound (107 mg, yield 75%). ¹H NMR (400 MHz, DMSO-*d*₆) δ 12.36 (s, 1H), 7.64 - 7.56 (m, 3H), 7.53 (ddd, *J* = 8.9, 5.6, 3.3 Hz, 1H), 7.47 - 7.40 (m, 2H), 7.31 - 7.23 (m, 3H), 6.99 (d, *J* = 1.7 Hz, 1H), 6.94 (d, *J* = 2.3 Hz, 1H), 6.65 (dd, *J* = 8.5, 1.7 Hz, 1H), 6.35 (dd, *J* = 3.1, 0.8 Hz, 1H), 5.36 (dd, *J* = 12.0, 4.5 Hz, 1H), 3.75 (s, 4H), 2.90 (dd, *J* = 18.4, 4.6 Hz, 1H), 1.86 (s, 3H). Rt 2.41 min (generic method) . ESI-MS for C₂₉H₂₃ClN₄O₂: calculated 494.2, found m/z 495.2 [M+H]⁺, 493.2 [M-H]⁻.

### Example d.24. 4-[5-(6-chloro-2-oxo-4-phenyl-1H-quinolin-3-yl)-3-(1-ethylindol-5-yl)-3,4-dihydropyrazol-2-yl]-4-oxo-butanoic acid (example not falling within the scope of the present invention).

### Step 1. Synthesis of 1-ethylindole-5-carbaldehyde.

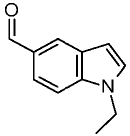

In a screw capped pressure tube 1*H*-indole-5-carboxaldehyde (338 mg, 2.3 mmol) was dissolved with 2.0 ml of anhydrous DMF then potassium carbonate (638 mg, 4.6 mmol) was added and the mixture kept under stirring for 10 minutes at rt. Iodoethane (503 mg, d = 1.94 g/ml, 344 µl, 4.6 mmol) was then added. The reaction was thus heated to 35 °C and stirred for 18 hours. The crude mixture was diluted with DCM and washed three times with water. The organic layer was anhydrified through a phase separator and the solvent removed under reduced pressure. Purification by normal phase flash column chromatography (solvent A: CHX, solvent B: EtOAc - gradient: 0-5% solvent B) yielded title compound (363 mg, yield 91%). ¹H NMR(400 MHz, DMSO-*d*₆) δ 9.98 (s, 1H), 8.18 (t, *J* = 1.1 Hz, 1H), 7.71 - 7.62 (m, 2H), 7.57 (d, *J* = 3.2 Hz, 1H), 6.67 (dd, *J* = 3.2, 0.7 Hz, 1H), 4.27 (q, *J* = 7.2 Hz, 2H), 1.37 (t, *J* = 7.2 Hz, 3H). Rt 1.94 min (generic method). ESI-MS for C₁₁H₁₁NO: calculated 173.1, found m/z 174.1 [M+H]⁺.

### Step 2. Synthesis of 6-chloro-3-[(E)-3-(1-ethylindol-5-yl)prop-2-enoyl]-4-phenyl-1H-quinolin-2-one (b.12).

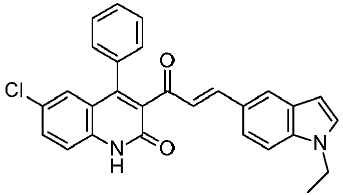

Compound b.12 was synthesized following the general procedure B using a.1 (402 mg, 1.3 mmol) and 1-Ethyl-indole-5-carbaldehyde. Purification by normal phase flash column chromatography (solvent A: DCM, solvent B: EtOH - gradient: 0-5% solvent B) yielded title compound (395 mg, yield 65%). ¹H NMR (400 MHz, DMSO-*d*₆) δ 12.31 (s, 1H), 7.85 (d, *J* = 1.4 Hz, 1H), 7.65 (dd, *J* = 8.8, 2.4 Hz, 1H), 7.56 (d, *J* = 16.3 Hz, 1H), 7.50 - 7.45 (m, 3H), 7.45 - 7.38 (m, 4H), 7.33 (dd, *J* = 7.7, 1.8 Hz, 2H), 6.97 (d, *J* = 2.3 Hz, 1H), 6.65 (d, *J* = 16.2 Hz, 1H), 6.45 (d, *J* = 3.1 Hz, 1H), 4.20 (q, *J* = 7.2 Hz, 2H), 1.34 (t, *J* = 7.2 Hz, 3H). Rt 2.56 min (generic method). ESI-MS for C₂₈H₂₁ClN₂O₂: calculated 452.1291, found *m*/*z* 453 [M+H]⁺, 451 [M-H]⁻.

### Step 3. Synthesis of 6-chloro-3-[5-(1-ethylindol-5-yl)-4,5-dihydro-1H-pyrazol-3-yl]-4-phenyl-1H-quinolin-2-one (c.12).

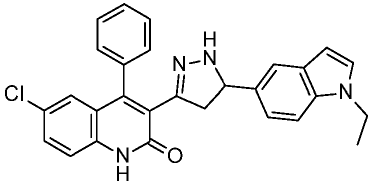

Compound c.12 was synthesized following the general procedure C using b.12 (336 mg, 0.79 mmol). Purification by normal phase flash column chromatography (solvent A: DCM, solvent B: EtOH - gradient: 0-10% solvent B) yielded title compound (322 mg, yield 87%). ¹H NMR(400 MHz, DMSO-*d*₆) δ 12.18 (s, 1H), 7.60 - 7.48 (m, 4H), 7.41 (d, *J* = 8.8 Hz, 1H), 7.38 - 7.30 (m, 3H), 7.25 (dt, *J* = 6.6, 2.1 Hz, 2H), 7.00 (d, *J* = 3.2 Hz, 1H), 6.89 (dd, *J* = 9.7, 2.0 Hz, 2H), 6.34 (dd, *J* = 3.2, 0.8 Hz, 1H), 4.64 (td, *J* = 10.4, 3.2 Hz, 1H), 4.17 (q, *J* = 7.2 Hz, 2H), 3. 21 (dd, *J* = 16.4, 11.0 Hz, 1H), 2.63 (dd, *J* = 16. 4, 9.9 Hz, 1H), 1.33 (t, *J* = 7.2 Hz, 3H). Rt 2.51 min (generic method). ESI-MS for C₂₈H₂₃ClN₄O: calculated 466.2, found *m*/*z* 467 [M+H]⁺, 465 [M-H]⁻.

### Step 4. Synthesis of 4-[5-(6-chloro-2-oxo-4-phenyl-1H-quinolin-3-yl)-3-(1-ethylindol-5-yl)-3,4-dihydropyrazol-2-yl]-4-oxo-butanoic acid (d.24).

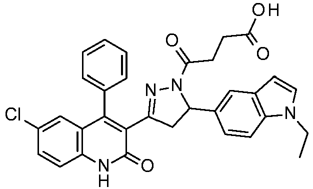

Compound d.26 was synthesized following the general procedure D1 using c.12 (153 mg, 0.33 mmol) with succinic anhydride (120 °C, 200 W). Purification by normal phase flash column chromatography (solvent A: DCM, solvent B: EtOH - gradient: 0-10% solvent B) yielded title compound (92 mg, yield 49%). ¹H NMR(400 MHz, DMSO-*d*₆) δ 12.37 (s, 1H), 12.02 (s, 1H), 7.63 (dd, *J* = 8.8, 2.4 Hz, 1H), 7.58 (dd, *J* = 5.5, 3.3 Hz, 2H), 7.52 (ddd, *J* = 8.9, 5.2, 3.1 Hz, 1H), 7.44 (dd, *J* = 8.7, 4.4 Hz, 2H), 7.35 (d, *J* = 3.1 Hz, 1H), 7.29 (dd, J = 8.5, 2.2 Hz, 2H), 6.95 (dd, *J* = 15.5, 2.0 Hz, 2H), 6. 60 (dd, *J* = 8.5, 1.7 Hz, 1H), 6.34 (d, *J* = 3.1 Hz, 1H), 5.34 (dd, *J* = 12.0, 4.7 Hz, 1H), 4.17 (q, *J* = 7.2 Hz, 2H), 3.77 (dd, *J* = 18.4, 12.1 Hz, 1H), 2.86 (dd, *J* = 18.4, 4.7 Hz, 1H), 2.48 - 2.42 (m, 2H), 2.26 (t, *J* = 6.9 Hz, 2H), 1.34 (t, *J* = 7.2 Hz, 3H). Rt 2.20 min (generic method). ESI-MS for C₃₂H₂₇ClN₄O₄: calculated 566.2, found m/z 567.2 [M+H]⁺, 565.2 [M-H]⁻.

### Example d.25. 3-[2-acetyl-3-(1-ethylindol-5-yl)-3,4-dihydropyrazol-5-yl]-6-chloro-4-phenyl-1H-quinolin-2-one (example not falling within the scope of the present invention).

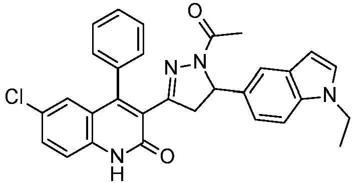

Compound d.25 was synthesized following the general procedure D₁ using c.12 (168 mg, 0.36 mmol) with acetic anhydride (165 °C, 200 W). Purification by normal phase flash column chromatography (solvent A: DCM, solvent B: EtOH - gradient: 0-10% solvent B) yielded title compound (128 mg, yield 70%). ¹H NMR(400 MHz, DMSO-*d*₆) δ 12.36 (s, 1H), 7.64 - 7.50 (m, 4H), 7.47 - 7.40 (m, 2H), 7.35 (d, *J* = 3.1 Hz, 1H), 7.32 - 7.27 (m, 2H), 6.97 (dd, *J* = 22.0, 2.0 Hz, 2H), 6.64 (dd, *J* = 8.5, 1.7 Hz, 1H), 6.35 (dd, *J* = 3.1, 0.8 Hz, 1H), 5.35 (dd, *J* = 12.0, 4.6 Hz, 1H), 4.16 (q, *J* = 7.2 Hz, 2H), 3.78 (dd, *J* = 18.4, 12.0 Hz, 1H), 2.91 (dd, *J* = 18.4, 4.6 Hz, 1H), 1.86 (s, 3H), 1.33 (t, *J* = 7.2 Hz, 3H). Rt 2.51 min (generic method). ESI-MS for C₃₀H₂₅ClN₄O₂: calculated 508.2, found *m*/*z* 509.2 [M+H]⁺, 507.2 [M-H]⁻.

### Example d.26. 4-[5-(6-chloro-2-oxo-4-phenyl-1H-quinolin-3-yl)-3-(1-methylindazol-5-yl)-3,4-dihydropyrazol-2-yl]-4-oxo-butanoic acid (example not falling within the scope of the present invention).

### Step 1. Synthesis of 1-methylindazole-5-carbaldehyde and 2-methylindazole-5-carbaldehyde.

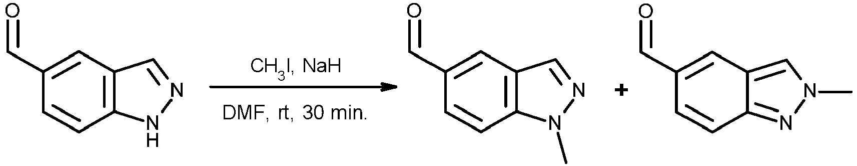

In a screw capped pressure tube 1H-indazole-5-carboxaldehyde (73 mg, 0.5 mmol) was dissolved with 500 µl of anhydrous DMF then sodium hydride (36 mg, 1.5 mmol) was added and the mixture kept under stirring for 30 minutes at rt. MeI (68 µl, 1.1 mmol) was then added. The reaction was thus stirred at rt for 30 minutes. The crude mixture was diluted with DCM and washed three times with water. The organic layer was anhydrified through a phase separator and the solvent removed under reduced pressure. Purification by normal phase flash column chromatography (solvent A: DCM, solvent B: DCM/EtOH 9:1 *v*/*v* - gradient: 0-35% solvent B) yielded both title compounds separately. Mono- and bi-dimensional ¹H- and ¹³C-NMR (HMBC) analyses confirmed the structure of the title compounds 1-methyl-1*H*-indazole-5-carbaldehyde, which showed a positive ¹H-¹³C correlation between CH₃ at 4.11 ppm and a quaternary C at 141.7 ppm, and 2-methyl-1*H*-indazole-5-carbaldehyde, which showed a positive ¹H-¹³C correlation between CH₃ at 4.22 ppm and the C₃ at 128.3 ppm.
1-methyl-indazole-5-carbaldehyde: 31 mg, yield 38%. ¹H NMR (400 MHz, DMSO-*d*₆) δ 10.03 (s, 1H), 8.43 - 8.42 (m, 1H), 8.32 (d, *J* = 1.0 Hz, 1H), 7.95 - 7.72 (m, 2H), 4.10 (s, 3H). ¹³C NMR (101 MHz, DMSO-*d*₆) δ 192.24, 141.77, 135.03, 129.97, 127.62, 124.37, 123.22, 110.58, 35.64. Rt 1.41 min (generic method) . ESI-MS for C₉H₈N₂O: calculated 160.1, found m/z 161.1 [M+H]⁺;
2-methyl-indazole-5-carbaldehyde: 18 mg, yield 22%. ¹H NMR (400 MHz, DMSO-*d*₆) δ 9.96 (s, 1H), 8.69 (s, 1H), 8.44 (t, *J* = 1.2 Hz, 1H), 7.72 - 7.63 (m, 2H), 4.22 (s, 3H) . ¹³C NMR (101 MHz, DMSO-*d*₆) δ 192.19, 149.84, 130.51, 130.18, 128.36, 122.02, 120.96, 117.56, 40.31. Rt 1.22 min (generic method) . ESI-MS for C₉H₈N₂O: calculated 160.1, found m/z 161.1 [M+H]⁺.

### Step 2. Synthesis of 6-chloro-3-[(E)-3-(1-methylindazol-5-yl)prop-2-enoyl]-4-phenyl-1H-quinolin-2-one (b.13).

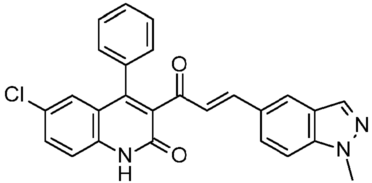

Compound b.13 was synthesized following the general procedure B using a.1 (229 mg, 0.77 mmol) and 1-methylindazole-5-carbaldehyde. Purification by normal phase flash column chromatography (solvent A: DCM, solvent B: EtOH - gradient: 0-5% solvent B) yielded title compound (275 mg, yield 81%). ¹H NMR(400 MHz, DMSO-*d*₆) δ 12.33 (s, 1H), 8.07 (dd, *J* = 5.0, 1.2 Hz, 2H), 7.73 (dd, *J* = 8.9, 1.6 Hz, 1H), 7.68 - 7.57 (m, 3H), 7.53 - 7.30 (m, 6H), 6.98 (d, *J* = 2.3 Hz, 1H), 6.74 (d, *J* = 16.3 Hz, 1H), 4.04 (s, 3H). Rt 2.26 min (generic method). ESI-MS for C₂₆H₁₈ClN₃O₂: calculated 439.1, found *m*/*z* 440.1 [M+H]⁺, 438.1 [M-H]⁻.

### Step 3. Synthesis of 6-chloro-3-[5-(1-methylindazol-5-yl)-4,5-dihydro-1H-pyrazol-3-yl]-4-phenyl-1H-quinolin-2-one (c.13).

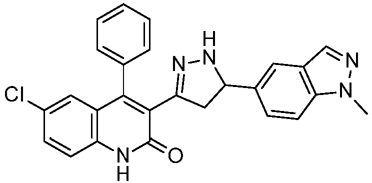

Compound c.13 was synthesized following the general procedure C using b.13 (268 mg, 0.61 mmol). Purification by normal phase flash column chromatography (solvent A: DCM, solvent B: EtOH - gradient: 0-10% solvent B) yielded title compound (245 mg, yield 88%). ¹H NMR(400 MHz, DMSO-*d*₆) δ 12.19 (s, 1H), 7.95 (d, *J* = 0.9 Hz, 1H), 7.60 - 7.45 (m, 5H), 7.43 - 7.39 (m, 2H), 7.36 (dt, *J* = 6.7, 1.7 Hz, 1H), 7.27 - 7.20 (m, 1H), 7.16 - 7.09 (m, 2H), 6.90 (d, *J* = 2.3 Hz, 1H), 4.70 (ddd, *J* = 11.0, 9.3, 3.1 Hz, 1H), 4.01 (s, 3H), 3.27 (dd, *J* = 16.8, 11.4 Hz, 2H), 2.60 (dd, *J* = 16.5, 9.4 Hz, 1H). Rt 0.83 min (apolar method). ESI-MS for C₂₆H₂₀ClN₅O: calculated 453.1, found *m*/*z* 454.1 [M+H]⁺.

### Step 4. Synthesis of 4-[5-(6-chloro-2-oxo-4-phenyl-1H-quinolin-3-yl)-3-(1-methylindazol-5-yl)-3,4-dihydropyrazol-2-yl]-4-oxo-butanoic acid (d.26).

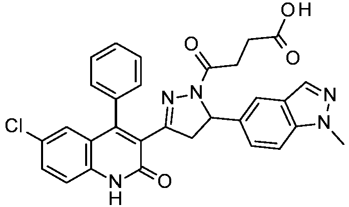

Compound d.26 was synthesized following the general procedure D1 using c.13 (102 mg, 0.22 mmol) with succinic anhydride (120 °C, 200 W). Purification by normal phase flash column chromatography (solvent A: DCM, solvent B: EtOH - gradient: 0-20% solvent B) yielded title compound (103 mg, yield 86%). ¹H NMR(400 MHz, DMSO-*d*₆) δ 12.38-12.11 (m, 2H), 7.96 (d, *J* = 1.0 Hz, 1H), 7.65 - 7.56 (m, 3H), 7.54 - 7.41 (m, 4H), 7.29 - 7.24 (m, 1H), 7.15 - 7.11 (m, 1H), 6.93 (d, *J* = 2.3 Hz, 1H), 6.83 (dd, *J* = 8.7, 1.6 Hz, 1H), 5.42 (dd, *J* = 12.0, 4.6 Hz, 1H), 4.01 (s, 3H), 3.80 (dd, *J* = 18.5, 12.1 Hz, 1H), 2.84 (dd, *J* = 18.5, 4.6 Hz, 1H), 2.56 - 2.44 (m, 2H), 2.28 (t, *J* = 6.9 Hz, 2H). Rt 1.93 min (generic method) . ESI-MS for C₃₀H₂₄ClN₅O₄: calculated 553.2, found m/z 554.2 [M+H]⁺, 552.2 [M-H]⁻.

### Example d.27. 3-[2-acetyl-3-(1-methylindazol-5-yl)-3,4-dihydropyrazol-5-yl]-6-chloro-4-phenyl-1H-quinolin-2-one (example not falling within the scope of the present invention).

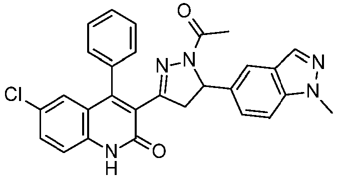

Compound d.27 was synthesized following the general procedure D1 using c.13 (131 mg, 0.30 mmol) with acetic anhydride (165 °C, 200 W). Purification by normal phase flash column chromatography (solvent A: DCM, solvent B: EtOH - gradient: 0-10% solvent B) yielded title compound (126 mg, yield 86%). ¹H NMR(400 MHz, DMSO-*d*₆) δ 12.37 (s, 1H), 7.97 (d, *J* = 0.9 Hz, 1H), 7.66 - 7.40 (m, 7H), 7.32 - 7.23 (m, 1H), 7.17 - 7.12 (m, 1H), 6.94 (d, *J* = 2.4 Hz, 1H), 6.86 (dd, *J* = 8.7, 1.6 Hz, 1H), 5.42 (dd, *J* = 12.0, 4.5 Hz, 1H), 4.01 (s, 3H), 3.80 (dd, *J* = 18.5, 12.1 Hz, 1H), 2.88 (dd, *J* = 18.4, 4.6 Hz, 1H), 1.88 (s, 3H). Rt 2.19 min (generic method). ESI-MS for C₂₈H₂₂ClN₅O₂: calculated 495.1, found *m*/*z* 496.1 [M+H]⁺, 494.4 [M-H]⁻.

### Example d.28. 4-[5-(6-chloro-2-oxo-4-phenyl-1H-quinolin-3-yl)-3-(2-methylindazol-5-yl)-3,4-dihydropyrazol-2-yl]-4-oxo-butanoic acid (example not falling within the scope of the present invention).

### Step 1. Synthesis of 6-chloro-3-[(E)-3-(2-methylindazol-5-yl)prop-2-enoyl]-4-phenyl-1H-quinolin-2-one (b.14).

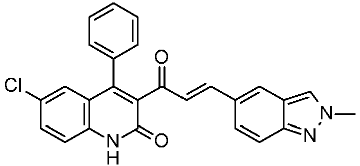

Compound b.14 was synthesized following the general procedure B using a.1 (229 mg, 0.77 mmol) and 2-methyl-1H-indazole-5-carbaldehyde. Purification by normal phase flash column chromatography (solvent A: DCM, solvent B: EtOH - gradient: 0-10% solvent B) yielded title compound (160 mg, yield 72%). ¹H NMR(400 MHz, DMSO-*d*₆) δ 12.33 (s, 1H), 8.43 (s, 1H), 8.02 (d, *J* = 1.5 Hz, 1H), 7.65 (dd, *J* = 8.8, 2.4 Hz, 1H), 7.59 - 7.53 (m, 3H), 7.48 (d, *J* = 8.8 Hz, 1H), 7.44 - 7.40 (m, 3H), 7.34 (dd, *J* = 7.8, 1.8 Hz, 2H), 6.98 (d, *J* = 2.4 Hz, 1H), 6.69 (d, *J* = 16.3 Hz, 1H), 4.15 (s, 3H). Rt 2.11 min (generic method) . ESI-MS for C₂₆H₁₈ClN₃O₂: calculated 439.1, found m/z 440.1 [M+H]⁺, 438.1 [M-H]⁻

### Step 2. Synthesis of 6-chloro-3-[5-(2-methylindazol-5-yl)-4,5-dihydro-1H-pyrazol-3-yl]-4-phenyl-1H-quinolin-2-one (c.14).

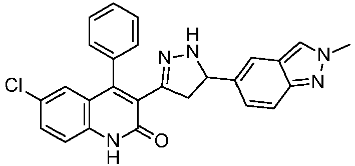

Compound c.14 was synthesized following the general procedure C using b.14 (147 mg, 0.33 mmol). Purification by normal phase flash column chromatography (solvent A: DCM, solvent B: EtOH - gradient: 0-10% solvent B) yielded title compound (140 mg, yield 94%). ¹H NMR(400 MHz, DMSO-*d*₆) δ 12.19 (s, 1H), 8.23 (s, 1H), 7.60 - 7.38 (m, 6H), 7.35 (dd, *J* = 6.1, 1.7 Hz, 2H), 7.24 (dt, *J* = 6.3, 2.0 Hz, 1H), 7.09 (d, *J* = 3.2 Hz, 1H), 6.97 - 6.88 (m, 2H), 4.64 (td, *J* = 10.4, 3.2 Hz, 1H), 4.14 (s, 3H), 3.23 (dd, *J* = 16.5, 11.1 Hz, 1H), 2.59 (dd, *J* = 16.5, 9.7 Hz, 1H). Rt 1.98 min (generic method). ESI-MS for C₂₆H₂₀ClN₅O: calculated 453.1, found *m*/*z* 454.1 [M+H]⁺ , 452.1 [M-H]⁻.

### Step 3. Synthesis of 4-[5-(6-chloro-2-oxo-4-phenyl-1H-quinolin-3-yl)-3-(2-methylindazol-5-yl)-3,4-dihydropyrazol-2-yl]-4-oxo-butanoic acid (d.28).

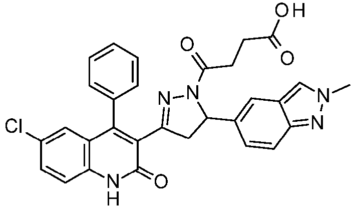

Compound d.28 was synthesized following the general procedure D1 using c.14 (129 mg, 0.28 mmol) with succinic anhydride (120 °C, 200 W). Purification by normal phase flash column chromatography (solvent A: DCM, solvent B: EtOH - gradient: 0-20% solvent B) yielded title compound (119 mg, yield 77%). ¹H NMR(400 MHz, DMSO-*d*₆) δ 12.37 (s, 1H), 12.05 (s, 1H), 8.25 (s, 1H), 7.65 - 7.55 (m, 3H), 7.55 - 7.47 (m, 1H), 7.47 - 7.39 (m, 3H), 7.27 (dd, *J* = 7.5, 1.8 Hz, 1H), 7.09 - 7.04 (m, 1H), 6.93 (d, *J* = 2.3 Hz, 1H), 6.63 (dd, *J* = 9.0, 1.7 Hz, 1H), 5.35 (dd, *J* = 12.0, 4.6 Hz, 1H), 4.15 (s, 3H), 3.77 (dd, *J* = 18.5, 12.1 Hz, 1H), 2.82 (dd, *J* = 18.4, 4.6 Hz, 1H), 2.54 - 2.46 (m, 2H), 2.28 (t, *J* = 6.9 Hz, 2H). Rt 1.77 min (generic method) . ESI-MS for C₃₀H₂₄ClN₅O₄: calculated 553.2, found *m*/*z* 554.2 [M+H]⁺, 552.2 [M-H]⁻.

### Example d.29. 4-[5-(6-chloro-2-oxo-4-phenyl-1H-quinolin-3-yl)-3-(2-ethylindazol-5-yl)-3,4-dihydropyrazol-2-yl]-4-oxo-butanoic acid (example not falling within the scope of the present invention).

### Step 1. Synthesis of 1-ethylindazole-5-carbaldehyde and 2-ethylindazole-5-carbaldehyde.

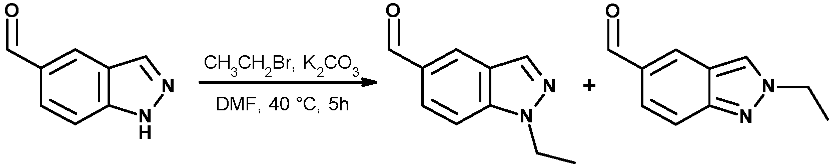

In a screw capped pressure tube 1*H*-indazole-5-carboxaldehyde (300 mg, 2.84 mmol) was dissolved with 3.0 ml of anhydrous DMF then potassium carbonate (1.13 g, 8.2 mmol) was added and the mixture kept under stirring for 10 minutes at rt. Ethyl bromide (230 µl, 3.1 mmol) was then added. The reaction was thus heated to 40 °C and stirred for 5 hours. The crude mixture was diluted with DCM and washed three times with water. The organic layer was anhydrified through a phase separator and the solvent removed under reduced pressure. Purification by normal phase flash column chromatography (solvent A: DCM, solvent B: DCM/EtOH 8:2 *v*/*v* - gradient: 0-5% solvent B) yielded both title compounds separately. Mono- and bi-dimensional ¹H- and ¹³C-NMR (HMBC) analyses confirmed the structure of the title compounds named 1-ethyl-1*H-*indazole-5-carbaldehyde, which showed a positive ¹H-¹³C correlation between CH₂ at 4.49 ppm and a quaternary C at 141.7 ppm, and 2-ethyl-1*H*-indazole-5-carbaldehyde, which showed a positive ¹H-¹³C correlation between CH₂ at 4.51 ppm and the C₃ at 127.5 ppm.

1-ethyl-indazole-5-carbaldehyde: 179 mg, yield 50%. ¹H NMR (400 MHz, DMSO-*d*₆) δ 10.04 (s, 1H), 8.43 (t, *J* = 1.1 Hz, 1H), 8.34 (d, *J* = 0.8 Hz, 1H), 7.92 - 7.81 (m, 2H), 4.50 (q, *J* = 7.2 Hz, 2H), 1.42 (t, *J* = 7.2 Hz, 3H). ¹³C NMR (101 MHz, DMSO-*d*₆) δ 192.67, 141.43, 135.64, 130.49, 128.16, 124.80, 123.78, 110.94, 43.85, 15.32. Rt 1.56 min (generic method). ESI-MS for C₁₀H₁₀N₂O: calculated 174.1, found *m*/*z* 175.1 [M+H]⁺.

2-ethyl-indazole-5-carbaldehyde: 121 mg, yield 34%. ¹H NMR(400 MHz, DMSO-*d*₆) δ 9.98 (s, 1H), 8.75 (s, 1H), 8.45 (d, *J* = 1.2 Hz, 1H), 7.69 (qd, *J* = 9.0, 1.3 Hz, 2H), 4.52 (q, *J* = 7.3 Hz, 2H), 1.54 (t, *J* = 7.3 Hz, 3H). ¹³C NMR (101 MHz, DMSO-*d*₆) δ 192.69, 150.17, 130.99, 130.81, 127.49, 122.46, 121.23, 118.24, 48.63, 16.02. Rt 1.36 min (generic method). ESI-MS for C₁₀H₁₀N₂O: calculated 174.1, found *m*/*z* 175.1 [M+H]⁺.

### Step 2. Synthesis of 6-chloro-3-[(E)-3-(2-ethylindazol-5-yl)prop-2-enoyl]-4-phenyl-1H-quinolin-2-one (b.15).

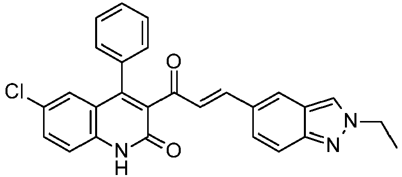

Compound b.15 was synthesized following the general procedure B using a.1 (307 mg, 1.0 mmol) and 2-ethyl-indazole-5-carbaldehyde. Purification by normal phase flash column chromatography (solvent A: DCM, solvent B: EtOH - gradient: 0-5% solvent B) yielded title compound (386 mg, yield 82%). ¹H NMR(400 MHz, DMSO-*d*₆) δ 12.33 (s, 1H), 8.48 (s, 1H), 8.02 (s, 1H), 7.65 (dd, *J* = 8.8, 2.4 Hz, 1H), 7.61 - 7.51 (m, 3H), 7.51 - 7.38 (m, 4H), 7.34 (dd, *J* = 7.7, 1.9 Hz, 2H), 6.98 (d, *J* = 2.3 Hz, 1H), 6.68 (d, *J* = 16.3 Hz, 1H), 4.44 (q, *J* = 7.3 Hz, 2H), 1.49 (t, *J* = 7.2 Hz, 3H). Rt 2.22 min (generic method) . ESI-MS for C₂₇H₂₀ClN₃O₂: calculated 453.1, found *m*/*z* 454.1 [M+H]⁺, 452.1 [M-H]⁻

### Step 3. Synthesis of 6-chloro-3-[5-(2-ethylindazol-5-yl)-4,5-dihydro-1H-pyrazol-3-yl]-4-phenyl-1H-quinolin-2-one (c.15).

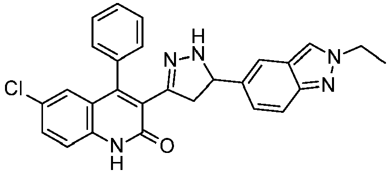

Compound c.15 was synthesized following the general procedure C using b.15 (302 mg, 0.6 mmol). Purification by normal phase flash column chromatography (solvent A: DCM, solvent B: EtOH - gradient: 0-10% solvent B) yielded title compound (236 mg, yield 76%). ¹H NMR(400 MHz, DMSO-*d*₆) δ 12.19 (s, 1H), 8.27 (d, *J* = 0.9 Hz, 1H), 7.59 - 7.38 (m, 6H), 7.35 (td, *J* = 3.1, 1.5 Hz, 2H), 7.28 - 7.20 (m, 1H), 7.08 (d, *J* = 3.2 Hz, 1H), 6.98 - 6.86 (m, 2H), 4.65 (td, *J* = 10.3, 3.1 Hz, 1H), 4.42 (q, *J* = 7.3 Hz, 2H), 3.24 (dd, *J* = 16.5, 11.1 Hz, 1H), 2.60 (dd, *J* = 16.5, 9.6 Hz, 1H), 1.48 (t, *J* = 7.3 Hz, 3H). Rt 2.06 min (generic method). ESI-MS for C₂₇H₂₂ClN₅O: calculated 467.1, found *m*/*z* 468.1 [M+H]⁺, 466.1 [M-H]⁻.

### Step 4. Synthesis of 4-[5-(6-chloro-2-oxo-4-phenyl-1H-quinolin-3-yl)-3-(2-ethylindazol-5-yl)-3,4-dihydropyrazol-2-yl]-4-oxo-butanoic acid (d.29).

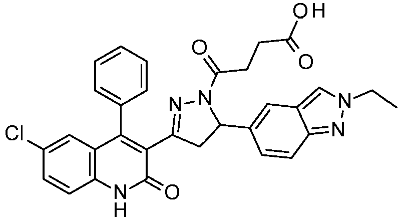

Compound d.29 was synthesized following the general procedure D1 using c.15 (198 mg, 0.42 mmol) with succinic anhydride (120 °C, 200 W). Purification by normal phase flash column chromatography (solvent A: DCM, solvent B: EtOH - gradient: 0-20% solvent B) yielded title compound (120 mg, yield 50%). ¹H NMR(400 MHz, DMSO-*d*₆) δ 12.37 (s, 1H), 12.04 (s, 1H), 8.29 (s, 1H), 7.66 - 7.55 (m, 3H), 7.51 (td, *J* = 6.6, 5.5, 3.3 Hz, 1H), 7.44 (dd, *J* = 10.0, 6.8 Hz, 3H), 7.28 (d, *J* = 7.5 Hz, 1H), 7.07 (d, *J* = 1.6 Hz, 1H), 6.93 (d, *J* = 2.4 Hz, 1H), 6.61 (dd, *J* = 8.9, 1.7 Hz, 1H), 5.35 (dd, *J* = 12.0, 4.6 Hz, 1H), 4.43 (q, *J* = 7.3 Hz, 2H), 3.77 (dd, *J* = 18.4, 12.1 Hz, 1H), 2.82 (dd, *J* = 18.4, 4.6 Hz, 1H), 2.54 - 2.44 (m, 2H), 2.28 (t, *J* = 6.9 Hz, 2H), 1.49 (t, *J* = 7.2 Hz, 3H). Rt 1.85 min (generic method). ESI-MS for C₃₁H₂₆ClN₅O₄: calculated 567.2, found *m*/*z* 568.2 [M+H]⁺, 566.2 [M-H]⁻.

### Example d.30. 3-[2-acetyl-3-(2-ethylindazol-5-yl)-3,4-dihydropyrazol-5-yl]-6-chloro-4-phenyl-1H-quinolin-2-one (example not falling within the scope of the present invention).

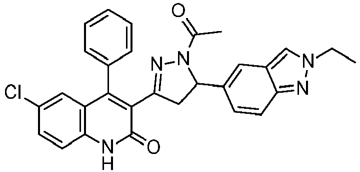

Compound d.30 was synthesized following the general procedure D1 using c.15 (80 mg, 0.17 mmol) with acetic anhydride (165 °C, 200 W). Purification by normal phase flash column chromatography (solvent A: DCM, solvent B: EtOH - gradient: 0-10% solvent B) yielded title compound (67 mg, yield 77%). ¹H NMR(400 MHz, DMSO-*d*₆) δ 12.37 (s, 1H), 8.31 (d, *J* = 0.9 Hz, 1H), 7.67 - 7.57 (m, 3H), 7.57 - 7.51 (m, 1H), 7.49 - 7.42 (m, 3H), 7.32 - 7.26 (m, 1H), 7.10 - 7.06 (m, 1H), 6.94 (d, *J* = 2.4 Hz, 1H), 6.65 (dd, *J* = 9.0, 1.7 Hz, 1H), 5.36 (dd, *J* = 12.0, 4.6 Hz, 1H), 4.44 (q, *J* = 7.3 Hz, 2H), 3.78 (dd, *J* = 18.4, 12.0 Hz, 1H), 2.87 (dd, *J* = 18.4, 4.6 Hz, 1H), 1.89 (s, 3H), 1.50 (t, *J* = 7.2 Hz, 3H). Rt 2.14 min (generic method). ESI-MS for C₂₉H₂₄ClN₅O₂: calculated 509.2, found *m*/*z* 510.2 [M+H]⁺, 508.2 [M-H]⁻.

### Example d.31. 4-[5-(6-chloro-2-oxo-4-phenyl-1H-quinolin-3-yl)-3-(1-ethylindazol-5-yl)-3,4-dihydropyrazol-2-yl]-4-oxo-butanoic acid (example not falling within the scope of the present invention).

### Step 1. Synthesis of 6-chloro-3-[(E)-3-(1-ethylindazol-5-yl)prop-2-enoyl]-4-phenyl-1H-quinolin-2-one (b.16)

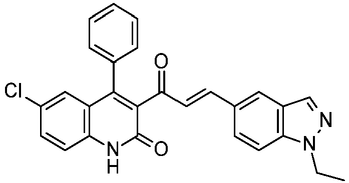

Compound b.16 was synthesized following the general procedure B using a.1 (292 mg, 0.98 mmol) and 1-ethyl-indazole-5-carbaldehyde. Purification by normal phase flash column chromatography (solvent A: DCM, solvent B: EtOH - gradient: 0-5% solvent B) yielded title compound (275 mg, yield 61%). ¹H NMR(400 MHz, DMSO-*d*₆) δ 12.33 (s, 1H), 8.09 (s, 1H), 8.07 (d, *J* = 1.4 Hz, 1H), 7.72 (dd, *J* = 8.9, 1.6 Hz, 1H), 7.69 - 7.57 (m, 3H), 7.48 (d, *J* = 8.8 Hz, 1H), 7.46 - 7.36 (m, 3H), 7.34 (dd, *J* = 7.8, 1.8 Hz, 2H), 6.98 (d, *J* = 2.3 Hz, 1H), 6.74 (d, *J* = 16.3 Hz, 1H), 4.43 (q, *J* = 7.2 Hz, 2H), 1.38 (t, *J* = 7.2 Hz, 3H). Rt 2.35 min (generic method) . ESI-MS for C₂₇H₂₀ClN₃O₂: calculated 453.1, found *m*/*z* 454.1 [M+H]⁺, 452.1 [M-H]⁻.

### Step 2. Synthesis of 6-chloro-3-[5-(1-ethylindazol-5-yl)-4,5-dihydro-1H-pyrazol-3-yl]-4-phenyl-1H-quinolin-2-one (c.16).

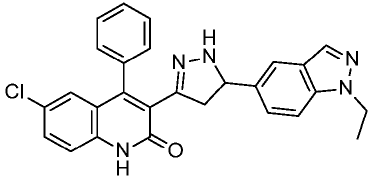

Compound c.16 was synthesized following the general procedure C using b.16 (260 mg, 0.57 mmol). Purification by normal phase flash column chromatography (solvent A: DCM, solvent B: EtOH - gradient: 0-10% solvent B) yielded title compound (228 mg, yield 86%). ¹H NMR(400 MHz, DMSO-*d*₆) δ 12.21 (s, 1H), 7.97 (d, *J* = 0.9 Hz, 1H), 7.59 - 7.46 (m, 5H), 7.42 (d, *J* = 8.8 Hz, 2H), 7.38 - 7.34 (m, 1H), 7.27 - 7.21 (m, 1H), 7.17 - 7.08 (m, 2H), 6.91 (d, *J* = 2.4 Hz, 1H), 4.71 (ddd, *J =* 11.3, 9.3, 2.5 Hz, 1H), 4.41 (q, *J* = 7.2 Hz, 2H), 3.27 (dd, *J* = 16.5, 11.1 Hz, 1H), 2.62 (dd, *J* = 16.5, 9.4 Hz, 1H), 1.38 (t, *J* = 7.2 Hz, 3H). Rt 2.19 min (generic method). ESI-MS for C₂₇H₂₂ClN₅O: calculated 467.2, found m/z 468.2 [M+H]⁺, 466.2 [M-H]⁻.

### Step 3. Synthesis of 4-[5-(6-chloro-2-oxo-4-phenyl-1H-quinolin-3-yl)-3-(1-ethylindazol-5-yl)-3,4-dihydropyrazol-2-yl]-4-oxo-butanoic acid (d.31).

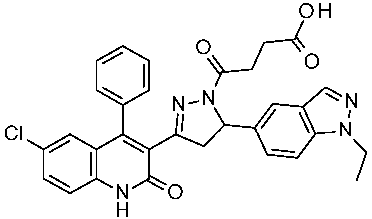

Compound d.31 was synthesized following the general procedure D1 using c.16 (196 mg, 0.42 mmol) with succinic anhydride (120 °C, 200 W) . Purification by normal phase flash column chromatography (solvent A: DCM, solvent B: EtOH - gradient: 0-10% solvent B) yielded title compound (177 mg, yield 74%). ¹H NMR (400 MHz, DMSO-*d*₆) δ 12.39 (s, 1H), 12.03 (s, 1H), 7.98 (s, 1H), 7.64 (dd, *J* = 8.8, 2.4 Hz, 1H), 7.59 (dd, *J* = 5.4, 3.5 Hz, 2H), 7.52 (dd, *J* = 10.0, 6.6 Hz, 2H), 7.48 - 7.43 (m, 2H), 7.29 (d, *J* = 7.5 Hz, 1H), 7.14 (d, *J* = 1.5 Hz, 1H), 6.94 (d, *J* = 2.3 Hz, 1H), 6.83 (dd, *J* = 8.7, 1.6 Hz, 1H), 5.42 (dd, *J* = 12.0, 4.6 Hz, 1H), 4.42 (q, *J* = 7.2 Hz, 2H), 3.80 (dd, *J* = 18.5, 12.1 Hz, 1H), 2.85 (dd, *J* = 18.5, 4.6 Hz, 1H), 2.54 - 2.48 (m, 2H), 2.28 (t, *J* = 6.9 Hz, 2H), 1.38 (t, *J* = 7.2 Hz, 3H). Rt 1.95 min (generic method). ESI-MS for C₃₁H₂₆ClN₅O₄: calculated 567.2, found m/z 568.2 [M+H]⁺, 566.2 [M-H]⁻.

### Example d.32. 3-[2-acetyl-3-(1-ethylindazol-5-yl)-3,4-dihydropyrazol-5-yl]-6-chloro-4-phenyl-1H-quinolin-2-one (example not falling within the scope of the present invention).

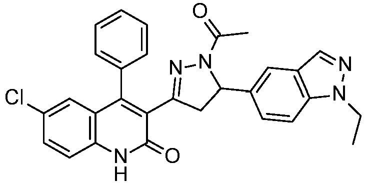

Compound d.32 was synthesized following the general procedure D1 using c.16 (131 mg, 0.28 mmol) with acetic anhydride (165 °C, 200 W) . Purification by normal phase flash column chromatography (solvent A: DCM, solvent B: EtOH - gradient: 0-5% solvent B) yielded title compound (118 mg, 82%). ¹H NMR(400 MHz, DMSO-*d*₆) δ 12.37 (s, 1H), 7.98 (d, J = 0.9 Hz, 1H), 7.66 - 7.57 (m, 3H), 7.57 - 7.49 (m, 2H), 7.48 - 7.40 (m, 2H), 7.29 (ddt, *J* = 7.6, 2.0, 1.0 Hz, 1H), 7.15 (t, *J* = 1.1 Hz, 1H), 6.94 (d, *J* = 2.3 Hz, 1H), 6.84 (dd, J = 8.7, 1.6 Hz, 1H), 5.41 (dd, *J* = 12.0, 4.5 Hz, 1H), 4.41 (q, *J* = 7.2 Hz, 2H), 3.79 (dd, *J* = 18.5, 12.1 Hz, 1H), 2.88 (dd, *J* = 18.4, 4.6 Hz, 1H), 1.88 (s, 3H), 1.37 (t, *J* = 7.2 Hz, 3H). Rt 2.29 min (generic method) . ESI-MS for C₂₉H₂₄ClN₅O₂: calculated 509.2, found *m*/*z* 510.2 [M+H]⁺, 508.2 [M-H]⁻.

### Example d.33. 4-[5-(6-chloro-2-oxo-4-phenyl-1H-quinolin-3-yl)-3-(1-propylindazol-5-yl)-3,4-dihydropyrazol-2-yl]-4-oxo-butanoic acid (example not falling within the scope of the present invention).

### Step 1. Synthesis of 1-propylindazole-5-carbaldehyde and 2-propylindazole-5-carbaldehyde.

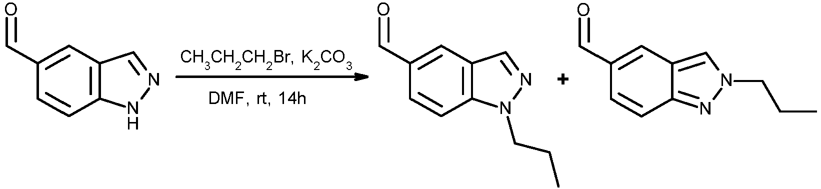

In a screw capped pressure tube 1*H*-indazole-5-carboxyaldehyde (152 mg, 1.0 mmol) was dissolved with 1.1 ml of anhydrous DMF then potassium carbonate (359 mg, 2.6 mmol) was added and the mixture kept under stirring for 30 minutes at rt. 1-Bromopropane (189 µl, 2.0 mmol) was then added. The reaction was thus stirred for 14 hours at rt. The crude mixture was diluted with DCM and washed three times with water. The organic layer was anhydrified through a phase separator and the solvent removed under reduced pressure. Purification by normal phase flash column chromatography (solvent A: DCM, solvent B: DCM/EtOH 9:1 *v*/*v* - gradient: 0-70% solvent B) yielded both title compounds separately. Mono- and bi-dimensional ¹H- and ¹³C-NMR (HMBC) analyses confirmed the structure of the title compounds named 1-Propylindazole-5-carbaldehyde, which showed a positive ¹H-¹³C correlation between CH₂ at 4.42 ppm and a quaternary C at 142.0 ppm, and 2-Propyl-indazole-5-carbaldehyd, which showed a positive ¹H-¹³C correlation between CH₂ at 4.44 ppm and the C₃ at 128.2 ppm.

1-Propyl-indazole-5-carbaldehyde: 99 mg, yield 50%. ¹H NMR(400 MHz, DMSO-*d*₆) δ 10.02 (s, 1H), 8.42 (d, *J* = 1.2 Hz, 1H), 8.33 (s, 1H), 7.88 - 7.80 (m, 2H), 4.42 (t, *J* = 6.9 Hz, 2H), 1.85 (h, *J* = 7.2 Hz, 2H), 0.85 - 0.77 (m, 3H). Rt 1.82 min (generic method). ESI-MS for C₁₁H₁₂N₂O: calculated 188.1, found m/z 189.1 [M+H]⁺.

2-Propyl-indazole-5-carbaldehyd: 66 mg, yield 34%. ¹H NMR(400 MHz, DMSO-*d*₆) δ 9.97 (s, 1H), 8.73 (s, 1H), 8.44 (d, *J* = 1.4 Hz, 1H), 7.78 - 7.62 (m, 2H), 4.43 (t, *J* = 7.0 Hz, 2H), 1.95 (h, *J* = 7.2 Hz, 2H), 0.85 (t, *J =* 7.4 Hz, 3H). Rt 1.61 min (generic method). ESI-MS for C₁₁H₁₂N₂O: calculated 188.1, found m/z 189.1 [M+H]⁺.

### Step 2. Synthesis of 6-chloro-4-phenyl-3-[(E)-3-(1-propylindazol-5-yl)prop-2-enoyl]-1H-quinolin-2-one (b.17).

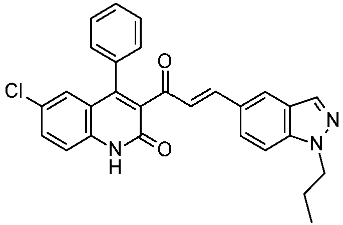

Compound b.17 was synthesized following the general procedure B using a.1 (402 mg, 1.35 mmol) and 1-Propylindazole-5-carbaldehyde. Purification by normal phase flash column chromatography (solvent A: DCM, solvent B: EtOH - gradient: 0-7% solvent B) yielded title compound (401 mg, yield 64%). ¹H NMR (400 MHz, DMSO-*d*₆) δ 12.33 (s, 1H), 8.10 (d, *J* = 0.8 Hz, 1H), 8.07 (d, *J* = 1.4 Hz, 1H), 7.72 (dd, *J* = 9.0, 1.6 Hz, 1H), 7.70 - 7.57 (m, 3H), 7.48 (d, *J* = 8.8 Hz, 1H), 7.44 - 7.38 (m, 3H), 7.34 (dd, *J* = 7.7, 1.8 Hz, 2H), 6.98 (d, *J* = 2.4 Hz, 1H), 6.74 (d, *J* = 16.3 Hz, 1H), 4.36 (t, *J* = 6.9 Hz, 2H), 1.82 (h, *J* = 7.2 Hz, 2H), 0.80 (t, *J* = 7.4 Hz, 3H). Rt 2.51 min (generic method). ESI-MS for C₂₈H₂₂ClN₃O₂: calculated 467.1, found m/z 468.1 [M+H]⁺, 466.1 [M-H]⁻.

### Step 3. Synthesis of 6-chloro-4-phenyl-3-[5-(1-propylindazol-5-yl)-4,5-dihydro-1H-pyrazol-3-yl]-1H-quinolin-2-one (c.17).

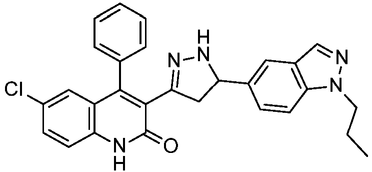

Compound c.17 was synthesized following the general procedure C using b.17 (395 mg, 0.84 mmol). Purification by normal phase flash column chromatography (solvent A: DCM, solvent B: EtOH - gradient: 0-10% solvent B) yielded title compound (381 mg, yield 94%). ¹H NMR(400 MHz, DMSO-*d*₆) δ 12.19 (s, 1H), 8.00 - 7.91 (m, 1H), 7.59 - 7.45 (m, 6H), 7.42 (d, *J* = 8.7 Hz, 2H), 7.38 - 7.31 (m, 1H), 7.24 (dd, *J* = 6.8, 1.9 Hz, 1H), 7.12 (dd, *J* = 8.7, 1.6 Hz, 1H), 6.90 (d, *J* = 2.3 Hz, 1H), 4.70 (t, *J =* 10.3 Hz, 1H), 4.33 (t, *J* = 6.9 Hz, 2H), 3.25 (dd, *J* = 16.5, 11.1 Hz, 1H), 2.62 (dd, *J* = 16.5, 9.5 Hz, 1H), 1.88 - 1.77 (m, 2H), 0.81 (t, *J* = 7.4 Hz, 3H). Rt 2.34 min (generic method) . ESI-MS for C₂₈H₂₄ClN₅O: calculated 481.2, found m/z 482.2 [M+H]⁺, 480.2 [M-H]⁻.

### Step 4. Synthesis of 4-[5-(6-chloro-2-oxo-4-phenyl-1H-quinolin-3-yl)-3-(1-propylindazol-5-yl)-3,4-dihydropyrazol-2-yl]-4-oxo-butanoic acid (d.33).

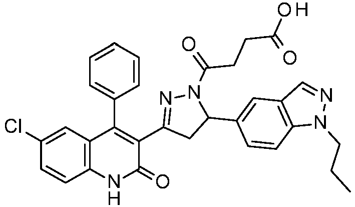

Compound d.33 was synthesized following the general procedure D1 using c.17 (176 mg, 0.36 mmol) with succinic anhydride (120 °C, 200 W) . Purification by normal phase flash column chromatography (solvent A: DCM, solvent B: EtOH - gradient: 0-25% solvent B) yielded title compound (178 mg, yield 86%). ¹H NMR(400 MHz, DMSO-*d*₆) δ 12.36 (s, 1H), 12.05 (s, 1H), 7.97 (d, *J* = 0.9 Hz, 1H), 7.62 (dd, *J* = 8.8, 2.4 Hz, 1H), 7.57 (qd, *J* = 4.0, 1.0 Hz, 2H), 7.53 - 7.47 (m, 2H), 7.47 - 7.41 (m, 2H), 7.30 - 7.25 (m, 1H), 7.16 - 7.11 (m, 1H), 6.94 (d, *J* = 2.3 Hz, 1H), 6.84 (dd, *J* = 8.8, 1.6 Hz, 1H), 5.42 (dd, *J* = 12.0, 4.6 Hz, 1H), 4.33 (t, *J* = 6.9 Hz, 2H), 3.78 (dd, *J* = 18.5, 12.0 Hz, 1H), 2.85 (dd, *J* = 18.5, 4.6 Hz, 1H), 2.54 - 2.48 (m, 2H), 2.28 (t, *J* = 6.9 Hz, 2H), 1.83 (h, *J* = 7.2 Hz, 2H), 0.83 (t, *J* = 7.4 Hz, 3H). Rt 2.06 min (generic method). ESI-MS for C₃₂H₂₈ClN₅O₄: calculated 581.2, found m/z 582.2 [M+H]⁺, 580.2 [M-H]⁻.

### Example d.34. 3-[2-acetyl-3-(1-propylindazol-5-yl)-3,4-dihydropyrazol-5-yl]-6-chloro-4-phenyl-1H-quinolin-2-one (example not falling within the scope of the present invention).

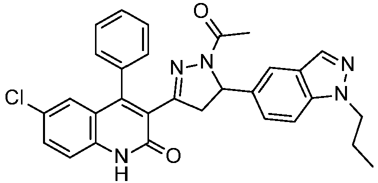

Compound d.36 was synthesized following the general procedure D1 using c.17 (188 mg, 0.39 mmol) with acetic anhydride (165 °C, 200 W) . Purification by normal phase flash column chromatography (solvent A: DCM, solvent B: EtOH - gradient: 0-10% solvent B) yielded title compound (184 mg, yield 90%). ¹H NMR(400 MHz, DMSO-*d*₆) δ 12.37 (s, 1H), 7.99 (d, *J* = 0.9 Hz, 1H), 7.65 - 7.57 (m, 3H), 7.56 - 7.50 (m, 2H), 7.47 - 7.41 (m, 2H), 7.36 - 7.25 (m, 1H), 7.17 - 7.09 (m, 1H), 6.94 (d, *J* = 2.3 Hz, 1H), 6.85 (dd, *J* = 8.8, 1.7 Hz, 1H), 5.41 (dd, *J* = 12.0, 4.6 Hz, 1H), 4.33 (t, *J* = 6.9 Hz, 2H), 3.78 (dd, *J* = 18.5, 12.0 Hz, 1H), 2.89 (dd, *J* = 18.5, 4.6 Hz, 1H), 1.90 - 1.77 (m, 5H), 0.82 (t, *J* = 7.4 Hz, 3H). Rt 1.21 min (generic method). ESI-MS for C₃₀H₂₆ClN₅O₂: calculated 523.2, found m/z 524.2 [M+H]⁺, 522.2 [M-H]⁻.

### Example d.35. 4-[5-(6-chloro-2-oxo-4-phenyl-1H-quinolin-3-yl)-3-(2-propylindazol-5-yl)-3,4-dihydropyrazol-2-yl]-4-oxo-butanoic acid (example not falling within the scope of the present invention).

### Step 1. Synthesis of 6-chloro-4-phenyl-3-[(E)-3-(2-propylindazol-5-yl)prop-2-enoyl]-1H-quinolin-2-one (b.18).

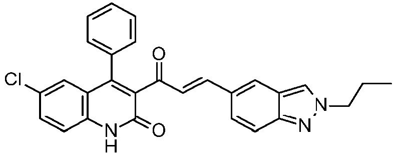

Compound b.18 was synthesized following the general procedure B using a.1 (292 mg, 0.98 mmol) and 2-Propylindazole-5-carbaldehyd. Purification by normal phase flash column chromatography (solvent A: DCM, solvent B: EtOH - gradient: 0-7% solvent B) yielded title compound (372 mg, yield 81%). ¹H NMR(400 MHz, DMSO-*d*₆) δ 12.33 (s, 1H), 8.47 (s, 1H), 8.04 - 8.00 (m, 1H), 7.65 (dd, *J* = 8.8, 2.3 Hz, 1H), 7.60 - 7.51 (m, 3H), 7.47 (d, *J* = 8.8 Hz, 1H), 7.44 - 7.38 (m, 3H), 7.36 - 7.32 (m, 2H), 6.97 (d, *J* = 2.3 Hz, 1H), 6.68 (d, *J* = 16.2 Hz, 1H), 4.36 (t, *J* = 6.9 Hz, 2H), 1.92 (h, *J =* 7.2 Hz, 2H), 0.83 (t, *J* = 7.4 Hz, 3H). Rt 2.37 min (generic method) . ESI-MS for C₂₈H₂₂ClN₃O₂: calculated 467.1, found m/z 468.1 [M+H]⁺, 466.1 [M-H]⁻.

### Step 2. Synthesis of 6-chloro-4-phenyl-3-[5-(2-propylindazol-5-yl)-4,5-dihydro-1H-pyrazol-3-yl]-1H-quinolin-2-one (c.18).

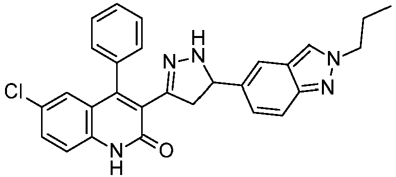

Compound c.18 was synthesized following the general procedure C using b.18 (342 mg, 0.73 mmol). Purification by normal phase flash column chromatography (solvent A: DCM, solvent B: EtOH - gradient: 0-10% solvent B) yielded title compound (316 mg, yield 89%). ¹H NMR(400 MHz, DMSO-*d*₆) δ 12.19 (s, 1H), 8.27 (d, *J* = 0.9 Hz, 1H), 7.57 (dd, *J* = 8.8, 2.3 Hz, 1H), 7.55 - 7.44 (m, 4H), 7.41 (d, *J* = 8.8 Hz, 1H), 7.38 - 7.33 (m, 2H), 7.24 (dt, *J* = 6.6, 1.9 Hz, 1H), 7.08 (d, *J* = 3.2 Hz, 1H), 6.92 (dd, *J* = 8.9, 1.6 Hz, 1H), 6.90 (d, *J* = 2.4 Hz, 1H), 4.70 - 4.59 (m, 1H), 4.35 (t, *J* = 6.9 Hz, 2H), 3.24 (dd, *J* = 16.5, 11.1 Hz, 1H), 2.60 (dd, *J* = 16.4, 9.6 Hz, 1H), 1.91 (h, *J* = 7.2 Hz, 2H), 0.82 (t, *J* = 7.4 Hz, 3H). Rt 2.23 min (generic method). ESI-MS for C₂₈H₂₄ClN₅O: calculated 481.2, found m/z 482.2 [M+H]⁺, 480.2 [M-H]⁻.

### Step 3. Synthesis of 4-[5-(6-chloro-2-oxo-4-phenyl-1H-quinolin-3-yl)-3-(2-propylindazol-5-yl)-3,4-dihydropyrazol-2-yl]-4-oxo-butanoic acid (d.35).

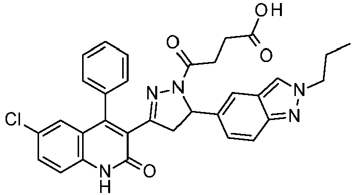

Compound d.35 was synthesized following the general procedure D₁ using c.18 (173 mg, 0.36 mmol) with succinic anhydride (120 °C, 200 W) . Purification by normal phase flash column chromatography (solvent A: DCM, solvent B: EtOH - gradient: 0-20% solvent B) yielded title compound (162 mg, yield 78%). ¹H NMR(400 MHz, DMSO-*d*₆) δ 12.37 (os, 2H), 8.28 (d, *J* = 0.9 Hz, 1H), 7.63 - 7.54 (m, 3H), 7.50 (td, *J* = 6.6, 5.5, 3.3 Hz, 1H), 7.47 - 7.40 (m, 3H), 7.30 - 7.24 (m, 1H), 7.08 (t, *J* = 1.2 Hz, 1H), 6.93 (d, *J* = 2.4 Hz, 1H), 6.62 (dd, *J* = 8.9, 1.7 Hz, 1H), 5.36 (dd, *J* = 12.0, 4.6 Hz, 1H), 4.35 (t, *J* = 6.9 Hz, 2H), 3.77 (dd, *J* = 18.5, 12.0 Hz, 1H), 2.83 (dd, *J* = 18.5, 4.6 Hz, 1H), 2.54 - 2.50 (m, 2H), 2.29 (t, *J* = 6.9 Hz, 2H), 1.91 (h, *J* = 7.2 Hz, 2H), 0.83 (t, *J* = 7.4 Hz, 3H). Rt 2.00 min (generic method). ESI-MS for C₃₂H₂₈ClN₅O₄: calculated 581.2, found m/z 582.2 [M+H]⁺, 580.2 [M-H]⁻.

### Example d.36. 4-[5-(6-chloro-2-oxo-4-phenyl-1H-quinolin-3-yl)-3-[4-(4-fluorophenyl)phenyl]-3,4-dihydropyrazol-2-yl]-4-oxo-butanoic acid (example not falling within the scope of the present invention).

### Step 1. Synthesis of 4-(4-fluorophenyl)benzaldehyde.

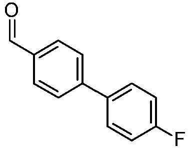

In a screw capped pressure tube 4-iodobenzaldehyde (255 mg, 1.1 mmol), (4-fluorophenyl)boronic acid (231 mg, 1.5 mmol) and anhydrous sodium carbonate (350 mg, 3.3 mmol) were added. The tube was deoxygenated with three cycles vacuum/Ar, then DMF (2 ml) and H₂O (400 µl) were added. The solution was stirred at rt, under Ar flux, for 15 minutes. Tetrakis(triphenylphosphine)palladium(0) (185 mg, 0.16 mmol) was thus added, the mixture was heated to 110 °C and stirred for 20 hours. The crude was then diluted with DCM and washed three times with water. The solvent was then removed under reduced pressure. Purification by normal phase flash column chromatography (solvent A: CHX, solvent B: EtOAc - gradient: 0-7% solvent B) yielded title compound (226 mg, yield 66%). ¹H NMR (400 MHz, DMSO-*d*₆) δ 10.06 (s, 1H), 8.03 - 7.97 (m, 2H), 7.94 - 7.88 (m, 2H), 7.87 - 7.81 (m, 2H), 7.40 - 7.32 (m, 2H). Rt 2.30 min (generic method).

### Step 2. Synthesis of 6-chloro-3-[(E)-3-[4-(4-fluorophenyl)phenyl]prop-2-enoyl]-4-phenyl-1H-quinolin-2-one (b.19).

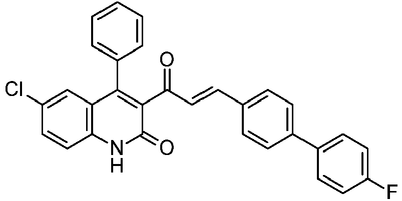

Compound b.19 was synthesized following the general procedure B using a.1 (214 mg, 0.72 mmol) and 4'-Fluoro-[1,1'-biphenyl]-4-carbaldehyde. Purification by normal phase flash column chromatography (solvent A: CHCl₃, solvent B: EtOH - gradient: 0-7% solvent B) yielded title compound (305 mg, yield 89%). ¹H NMR(400 MHz, DMSO-*d*₆) δ 12.35 (s, 1H), 7.82 - 7.62 (m, 7H), 7.57 - 7.40 (m, 5H), 7.36 - 7.26 (m, 4H), 6.98 (d, *J =* 2.3 Hz, 1H), 6.80 (d, *J* = 16.4 Hz, 1H). Rt 1.90 min (apolar method) . ESI-MS for C₃₀H₁₉ClFNO₂: calculated 479.1, found *m*/*z* 480.1 [M+H]⁺.

### Step 3. Synthesis of 6-chloro-3-[5-[4-(4-fluorophenyl)phenyl]-4,5-dihydro-1H-pyrazol-3-yl]-4-phenyl-1H-quinolin-2-one (c.19).

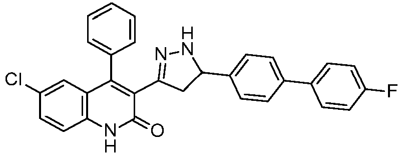

Compound c.19 was synthesized following the general procedure C using b.19 (273 mg, 0.57 mmol). Purification by normal phase flash column chromatography (solvent A: DCM, solvent B: EtOH - gradient: 0-10% solvent B. Yielding title compound 188 mg, 67%. ¹H NMR(400 MHz, DMSO-*d*₆) δ 12.18 (s, 1H), 7.71 - 7.64 (m, 2H), 7.60 - 7.46 (m, 6H), 7.42 (d, *J* = 8.8 Hz, 1H), 7.37 - 7.32 (m, 1H), 7.31 - 7.21 (m, 3H), 7.19 - 7.13 (m, 3H), 6.90 (d, *J* = 2.3 Hz, 1H), 4.67 - 4.57 (m, 1H), 3.26 (dd, *J* = 16.6, 11.1 Hz, 1H), 2.60 (dd, *J* = 16.4, 9.5 Hz, 1H). Rt 1.72 min (apolar method). ESI-MS for C₃₀H₂₁ClFN₃O: calculated 493.1, found *m*/*z* 494 [M+H]⁺, 492.1 [M-H]⁻.

### Step 4. Synthesis of 4-[5-(6-chloro-2-oxo-4-phenyl-1H-quinolin-3-yl)-3-[4-(4-fluorophenyl)phenyl]-3,4-dihydropyrazol-2-yl]-4-oxo-butanoic acid (d.36).

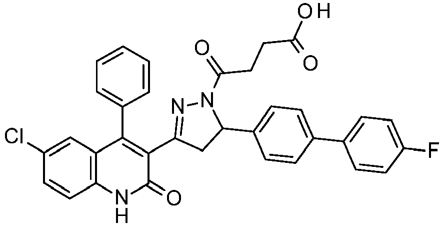

Compound d.36 was synthesized following the general procedure D1 using c.19 (171 mg, 0.35 mmol) with succinic anhydride (120 °C, 200 W). Purification by normal phase flash column chromatography (solvent A: DCM, solvent B: EtOH - gradient: 0-10% solvent B) yielded title compound (167 mg, yield 80%). ¹H NMR(400 MHz, DMSO-*d*₆) δ 12.38 (s, 1H), 12.08 (s, 1H), 7.71 - 7.61 (m, 3H), 7.55 (ddt, *J* = 10.6, 9.5, 4.0 Hz, 3H), 7.50 - 7.40 (m, 4H), 7.33 - 7.25 (m, 3H), 6.94 (d, *J* = 2.3 Hz, 1H), 6.87 (d, *J* = 8.3 Hz, 2H), 5.35 (dd, *J* = 12.0, 4.6 Hz, 1H), 3.77 (dd, *J* = 18.5, 12.1 Hz, 1H), 2. 84 (dd, *J* = 18.4, 4.7 Hz, 1H), 2.57 - 2.44 (m, 2H), 2.30 (t, *J* = 6.8 Hz, 2H). ESI-MS for C₃₄H₂₅ClFN₃O₄: calculated 593.2, found m/z 594.2 [M+H]⁺, 592.2 [M-H]⁻.

### Example d.37. 4-[5-(6-chloro-2-oxo-4-phenyl-1H-quinolin-3-yl)-3-[4-(4-chlorophenyl)phenyl]-3,4-dihydropyrazol-2-yl]-4-oxo-butanoic acid (example not falling within the scope of the present invention).

### Step 1. Synthesis of 6-chloro-3-[(E)-3-[4-(4-chlorophenyl)phenyl]prop-2-enoyl]-4-phenyl-1H-quinolin-2-one (b.20).

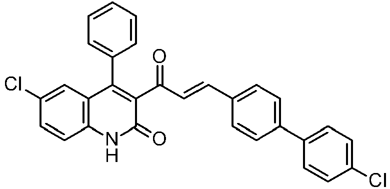

Compound b.20 was synthesized following the general procedure B using a.1 (339 mg, 1.1 mmol) and 4'-chloro-[1,1'-biphenyl]-4-carbaldehyde. Purification by normal phase flash column chromatography (solvent A: DCM, solvent B: EtOH - gradient: 0-5% solvent B) yielded title compound (567 mg, quantitative yield). ¹H NMR(400 MHz, DMSO-*d*₆) δ 12.35 (s, 1H), 7.78 - 7.68 (m, 6H), 7.65 (dd, *J* = 8.8, 2.4 Hz, 1H), 7.57 - 7.48 (m, 3H), 7.48 - 7.40 (m, 4H), 7.34 (dd, *J* = 7.8, 1.8 Hz, 2H), 6.99 (d, *J* = 2.3 Hz, 1H), 6.81 (d, J = 16.4 Hz, 1H). Rt 2.16 min (apolar method) . ESI-MS for C₃₀H₁₉Cl₂NO₂: calculated 495.1, found *m*/*z* 496.1 [M+H]⁺, 494.1 [M-H]⁻.

### Step 2. Synthesis of 6-chloro-3-[5-[4-(4-chlorophenyl)phenyl]-4,5-dihydro-1H-pyrazol-3-yl]-4-phenyl-1H-quinolin-2-one (c.20).

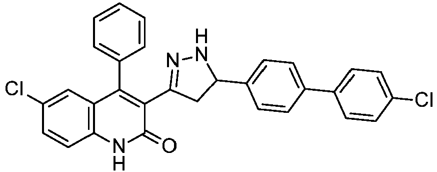

Compound c.20 was synthesized following the general procedure C using b.20 (558 mg, 1.1 mmol). Purification by normal phase flash column chromatography (solvent A: DCM, solvent B: EtOH - gradient: 0-15% solvent B) yielded title compound (501 mg, yield 87%). ¹H NMR(400 MHz, DMSO-*d*₆) δ 12.20 (s, 1H), 7.69 - 7.63 (m, 2H), 7.59 - 7.46 (m, 8H), 7.41 (d, *J* = 8.7 Hz, 1H), 7.37 - 7.31 (m, 1H), 7.26 - 7.20 (m, 1H), 7.17 (d, *J =* 8.3 Hz, 2H), 6.90 (d, *J* = 2.3 Hz, 1H), 4.63 (ddd, *J* = 11.3, 9.5, 3.0 Hz, 1H), 3.27 (dd, *J* = 16.4, 11.1 Hz, 1H), 2.60 (dd, *J* = 16.4, 9.4 Hz, 1H). Rt 1.99 min (apolar method) . ESI-MS for C₃₀H₂₁Cl₂N₃O: calculated 509.1, found m/z 510.1 [M+H]⁺, 508.1 [M-H]⁻.

### Step 3. Synthesis of 4-[5-(6-chloro-2-oxo-4-phenyl-1H-quinolin-3-yl)-3-[4-(4-chlorophenyl)phenyl]-3,4-dihydropyrazol-2-yl]-4-oxo-butanoic acid (d.37).

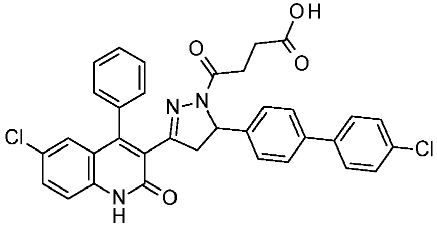

Compound d.37 was synthesized following the general procedure D1 using c.20 (227 mg, 0.44 mmol) with succinic anhydride (120 °C, 200 W). Purification by normal phase flash column chromatography (solvent A: DCM, solvent B: EtOH - gradient: 0-10% solvent B) yielded title compound (205 mg, yield 77%). ¹H NMR(400 MHz, DMSO-*d*₆) δ 12.38 (s, 1H), 12.04 (s, 1H), 7.67 (d, *J* = 8.6 Hz, 2H), 7.63 (dd, *J* = 8.8, 2.4 Hz, 1H), 7.59 - 7.48 (m, 7H), 7.47 - 7.39 (m, 2H), 7.29 (dd, *J* = 6.5, 2.0 Hz, 1H), 6.94 (d, *J* = 2.4 Hz, 1H), 6.88 (d, *J* = 8.3 Hz, 2H), 5.35 (dd, *J =* 12.0, 4.6 Hz, 1H), 3.77 (dd, *J* = 18.5, 12.1 Hz, 1H), 2.84 (dd, *J* = 18.5, 4.6 Hz, 1H), 2.50 (m, 2H), 2.29 (t, *J* = 6.8 Hz, 2H). Rt 2.51 min (generic method). ESI-MS for C₃₄H₂₅Cl₂N₃O₄: calculated 609.1, found *m*/*z* 610.1 [M+H]⁺, 608.1 [M-H]⁻.

### Example d.38. 3-[2-acetyl-3-[4-(4-chlorophenyl)phenyl]-3,4-dihydropyrazol-5-yl]-6-chloro-4-phenyl-1H-quinolin-2-one (example not falling within the scope of the present invention).

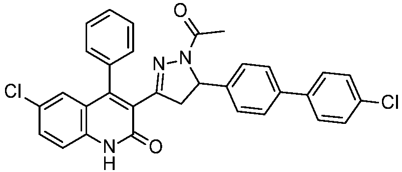

Compound d.38 was synthesized following the general procedure D1 using c.20 (224 mg, 0.44 mmol) with acetic anhydride (165 °C, 200 W). Purification by normal phase flash column chromatography (solvent A: DCM, solvent B: EtOH - gradient: 0-10% solvent B) yielded title compound (245 mg, quantitative yield). ¹H NMR(400 MHz, DMSO-*d*₆) δ 12.37 (s, 1H), 7.71 - 7.48 (m, 10H), 7.48 - 7.39 (m, 2H), 7.29 (dt, *J* = 5.2, 2.0 Hz, 1H), 6.94 (d, *J* = 2.4 Hz, 1H), 6.90 (d, *J* = 8.3 Hz, 2H), 5.35 (dd, *J* = 12.1, 4.6 Hz, 1H), 3.78 (dd, *J* = 18.4, 12.1 Hz, 1H), 2.88 (dd, *J* = 18.5, 4.6 Hz, 1H), 1.88 (s, 3H). Rt 2.00 min (apolar method) . ESI-MS for C₃₂H₂₃Cl₂N₃O₂: calculated 551.1, found m/z 552.1 [M+H]⁺, 550.1 [M-H]⁻.

### Example d.39. 4-[3-[4-(4-bromophenyl)phenyl]-5-(6-chloro-2-oxo-4-phenyl-1H-quinolin-3-yl)-3,4-dihydropyrazol-2-yl]-4-oxo-butanoic acid (example not falling within the scope of the present invention).

### Step 1. Synthesis of 3-[(E)-3-[4-(4-bromophenyl)phenyl]prop-2-enoyl]-6-chloro-4-phenyl-1H-quinolin-2-one (b.21).

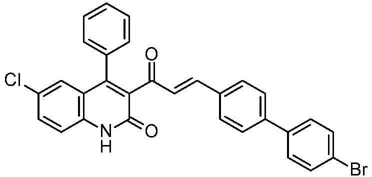

Compound b.21 was synthesized following the general procedure B using a.1 (337 mg, 1.1 mmol) and 4'-bromo-[1,1'-biphenyl]-4-carbaldehyde. Purification by normal phase flash column chromatography (solvent A: DCM, solvent B: EtOH - gradient: 0-5% solvent B) yielded title compound (576 mg, yield 89%). ¹H NMR(400 MHz, DMSO-*d*₆) δ 12.35 (s, 1H), 7.80 - 7.68 (m, 4H), 7.68 - 7.62 (m, 5H), 7.57 - 7.39 (m, 5H), 7.34 (dd, *J* = 7.7, 1.8 Hz, 2H), 6.99 (d, *J* = 2.3 Hz, 1H), 6.81 (d, *J* = 16.4 Hz, 1H). Rt 2.20 min (apolar method). ESI-MS for C₃₀H₁₉BrClNO₂: calculated 538.0/540.0, found *m*/z 539.0/541.0 [M+H]⁺, 537.0/539.0 [M-H]⁻.

### Step 2. Synthesis of 3-[5-[4-(4-bromophenyl)phenyl]-4,5-dihydro-1H-pyrazol-3-yl]-6-chloro-4-phenyl-1H-quinolin-2-one (c.21).

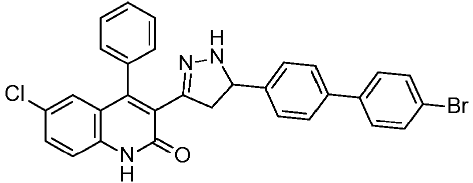

Compound c.21 was synthesized following the general procedure C using b.21 (570 mg, 1.0 mmol). Purification by normal phase flash column chromatography (solvent A: DCM, solvent B: EtOH - gradient: 0-10% solvent B) yielded title compound (472 mg, yield 81%). ¹H NMR(400 MHz, DMSO-*d*₆) δ 12.20 (s, 1H), 7.68 - 7.46 (m, 10H), 7.41 (d, *J* = 8.8 Hz, 1H), 7.37 - 7.33 (m, 1H), 7.26 - 7.21 (m, 1H), 7.19 - 7.15 (m, 3H), 6.90 (d, *J* = 2.4 Hz, 1H), 4.63 (ddd, *J =* 11.3, 9.5, 3.1 Hz, 1H), 3.27 (dd, *J* = 16.4, 11.0 Hz, 1H), 2.60 (dd, J = 16.4, 9.4 Hz, 1H). Rt 2.05 min (apolar method). ESI-MS for C₃ₒH₂₁BrClN₃O: calculated 552.1/554.1, found m/z 553.1/555.1 [M+H]⁺.

### Step 3. Synthesis of 4-[3-[4-(4-bromophenyl)phenyl]-5-(6-chloro-2-oxo-4-phenyl-1H-quinolin-3-yl)-3,4-dihydropyrazol-2-yl]-4-oxo-butanoic acid (d.39)

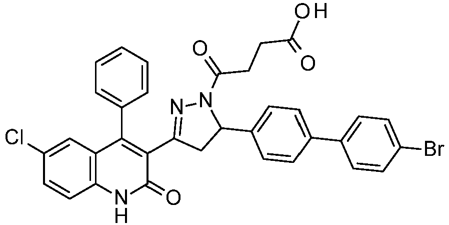

Compound d.39 was synthesized following the general procedure D1 using c.21 (214 mg, 0.38 mmol) with succinic anhydride (120 °C, 200 W) . Purification by normal phase flash column chromatography (solvent A: DCM, solvent B: EtOH - gradient: 0-20% solvent B) yielded title compound (197 mg, yield 79%). ¹H NMR(400 MHz, DMSO-*d*₆) δ 12.37 (s, 1H), 12.08 (s, 1H), 7.68 - 7.38 (m, 12H), 7.30 - 7.26 (m, 1H), 6.94 (d, J = 2.4 Hz, 1H), 6.89 (d, *J* = 8.4 Hz, 2H), 5.35 (dd, *J* = 12.0, 4.6 Hz, 1H), 3.78 (dd, *J* = 18.5, 12.1 Hz, 1H), 2.84 (dd, *J* = 18.5, 4.7 Hz, 1H), 2.58 - 2.43 (m, *J* = 3.7 Hz, 2H), 2.30 (t, *J =* 6.9 Hz, 2H). Rt 2.52 min (generic method). ESI-MS for C₃₄H₂₅BrClN₃O₄: calculated 652.1/654.1, found *m*/z 653.1/655.1 [M+H]⁺, 651.1/653.1 [M-H]⁻.

### Example d.40. 3-[2-acetyl-3-[4-(4-bromophenyl)phenyl]-3,4-dihydropyrazol-5-yl]-6-chloro-4-phenyl-1H-quinolin-2-one (example not falling within the scope of the present invention).

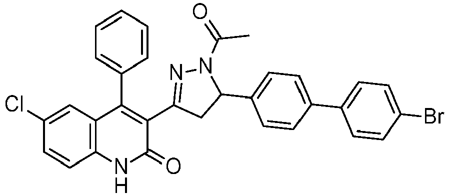

Compound d.40 was synthesized following the general procedure D1 using c.21 (203 mg, 0.36 mmol) with acetic anhydride (165 °C, 200 W). Purification by normal phase flash column chromatography: solvent A: DCM, solvent B: EtOH - gradient: 0-10% solvent B. Yielding title compound 175 mg, 80%. ¹H NMR(400 MHz, DMSO-*d*₆) δ 12.37 (s, 1H), 7.66 - 7.36 (m, 12H), 7.32 - 7.24 (m, 1H), 6.98 - 6.85 (m, 3H), 5.35 (dd, *J* = 12.0, 4.6 Hz, 1H), 3.78 (dd, *J* = 18.4, 12.1 Hz, 1H), 2.90 (dd, *J* = 18.4, 4.6 Hz, 1H), 1.88 (s, 3H). Rt 2.52 min (apolar method). ESI-MS for C₃₂H₂₃BrClN₃O₂: calculated 594.1/596.1, found *m*/*z* 595.1/597.1 [M+H]⁺, 593.1/595.1 [M-H]⁻.

### Example d.41. 4-[5-(6-chloro-2-oxo-4-phenyl-1H-quinolin-3-yl)-3-[4-(4-methoxyphenyl)phenyl]-3,4-dihydropyrazol-2-yl]-4-oxo-butanoic acid (example not falling within the scope of the present invention).

### Step 1. Synthesis of 6-chloro-3-[(E)-3-[4-(4-methoxyphenyl)phenyl]prop-2-enoyl]-4-phenyl-1H-quinolin-2-one (b.22).

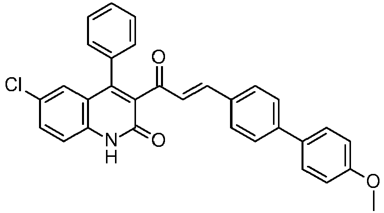

Compound b.22 was synthesized following the general procedure B using a.1 (430 mg, 1.4 mmol) and 4'-methoxy-[1,1'-biphenyl]-4-carbaldehyde. Purification by normal phase flash column chromatography (solvent A: DCM, solvent B: EtOH - gradient: 0-10% solvent B) yielded title compound (528 mg, yield 74%). ¹H NMR(400 MHz, DMSO-*d*₆) δ 12.35 (s, 1H), 7.75 - 7.62 (m, 6H), 7.55 - 7.40 (m, 6H), 7.33 (ddt, *J* = 6.7, 3.2, 1.8 Hz, 2H), 7.05 - 7.00 (m, 2H), 6.98 (d, *J* = 2.4 Hz, 1H), 6.77 (d, *J* = 16.4 Hz, 1H), 3.79 (s, 3H). Rt 1.81 min (apolar method). ESI-MS for C₃₁H₂₂ClNO₃: calculated 491.1, found *m*/*z* 492.1 [M+H]⁺, 490.1 [M-H]⁻.

### Step 2. Synthesis of 6-chloro-3-[5-[4-(4-methoxyphenyl)phenyl]-4,5-dihydro-1H-pyrazol-3-yl]-4-phenyl-1H-quinolin-2-one (c.22).

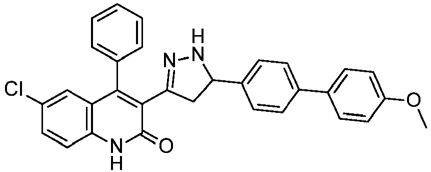

Compound c.22 was synthesized following the general procedure C using b.22 (522 mg, 1.1 mmol). Purification by normal phase flash column chromatography (solvent A: DCM, solvent B: EtOH - gradient: 0-10% solvent B) yielded title compound (412 mg, yield 77%). ¹H NMR(400 MHz, DMSO-*d*₆) δ 12.20 (s, 1H), 7.57 (dd, *J* = 8.9, 2.4 Hz, 3H), 7.54 - 7.44 (m, 5H), 7.41 (d, *J* = 8.8 Hz, 1H), 7.38 - 7.31 (m, 1H), 7.24 (td, *J* = 4.2, 3.7, 1.8 Hz, 1H), 7.16 - 7.11 (m, 3H), 7.01 (d, *J* = 8.8 Hz, 2H), 6.90 (d, J = 2.3 Hz, 1H), 4.60 (ddd, *J* = 11.0, 9.5, 3.1 Hz, 1H), 3.79 (s, 3H), 3.25 (dd, *J* = 16.4, 11.0 Hz, 1H), 2.60 (dd, *J* = 16.4, 9.5 Hz, 1H). Rt 2.60 min (generic method). ESI-MS for C₃₁H₂₄ClN₃O₂: calculated 505.2, found *m*/*z* 506.2 [M+H]⁺, 504.2 [M-H]⁻.

### Step 3. Synthesis of 4-[5-(6-chloro-2-oxo-4-phenyl-1H-quinolin-3-yl)-3-[4-(4-methoxyphenyl)phenyl]-3,4-dihydropyrazol-2-yl]-4-oxo-butanoic acid (d.41).

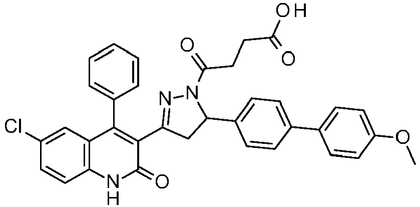

Compound d.41 was synthesized following the general procedure D1 using c.22 (545 mg, 1.1 mmol) with succinic anhydride (120 °C, 200 W). Purification by normal phase flash column chromatography (solvent A: DCM, solvent B: EtOH - gradient: 0-10% solvent B) yielded title compound (359 mg, yield 55%). ¹H NMR (400 MHz, DMSO-*d*₆) δ 12.38 (s, 1H), 12.03 (s, 1H), 7.67 - 7.50 (m, 6H), 7.44 (dt, *J* = 7.7, 5.9 Hz, 4H), 7.29 (dd, *J* = 6.7, 2.1 Hz, 1H), 7.02 (d, *J* = 8.8 Hz, 2H), 6.94 (d, *J* = 2.3 Hz, 1H), 6.84 (d, *J* = 8.3 Hz, 2H), 5.33 (dd, *J* = 12.0, 4.6 Hz, 1H), 3.79 (s, 4H), 2.84 (dd, J = 18.4, 4.6 Hz, 1H), 2.50 (p, *J* = 1.9 Hz, 2H), 2.30 (t, *J* = 6.9 Hz, 2H). Rt 2.30 min (generic method). ESI-MS for C₃₅H₂₈ClN₃O₅: calculated 605.2, found m/z 606.2 [M+H]⁺, 604.2 [M-H]⁻.

### Example d.42. 3-[2-acetyl-3-[4-(4-methoxyphenyl)phenyl]-3,4-dihydropyrazol-5-yl]-6-chloro-4-phenyl-1H-quinolin-2-one (example not falling within the scope of the present invention).

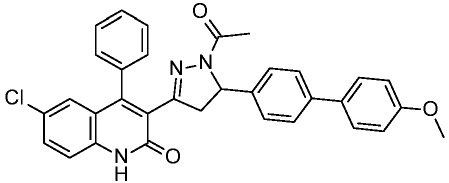

Compound d.42 was synthesized following the general procedure D1 using c.22 (179 mg, 0.35 mmol) with acetic anhydride (165 °C, 200 W). Purification by normal phase flash column chromatography (solvent A: DCM, solvent B: EtOH - gradient: 0-8% solvent B) yielded title compound (158 mg, yield 83%) . ¹H NMR(600 MHz, DMSO-*d*₆) δ 12.39 (s, 1H), 7.63 (dd, *J* = 8.8, 2.4 Hz, 1H), 7.60 - 7.53 (m, 5H), 7.45 (d, *J* = 8.5 Hz, 3H), 7.43 - 7.39 (m, 1H), 7.31 - 7.27 (m, 1H), 7.03 - 6.99 (m, 2H), 6.94 (d, *J* = 2.4 Hz, 1H), 6.86 (d, *J* = 8.3 Hz, 2H), 5.33 (dd, *J* = 12.1, 4.6 Hz, 1H), 3.79 (s, 4H), 2.88 (dd, *J* = 18.5, 4.6 Hz, 1H), 1.88 (s, 3H). Rt 1.65 min (apolar method) . ESI-MS for C₃₃H₂₆ClN₃O₃: calculated 547.2, found m/z 548 [M+H]⁺, 546 [M-H]⁻.

### Example d.43. 4-[5-(6-chloro-2-oxo-4-phenyl-1H-quinolin-3-yl)-3-[4-(1-propylpyrazol-4-yl)phenyl]-3,4-dihydropyrazol-2-yl]-4-oxo-butanoic acid (d.46) (example not falling within the scope of the present invention).

### Step 1. Synthesis of 4-(1-propylpyrazol-4-yl)benzaldehyde.

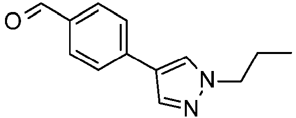

In a Schlenk tube 4-iodobenzaldehyde (326 mg, 1.4 mmol), (1-propyl-pyrazol-4-yl)boronic acid (323 mg, 2.1 mmol) and anhydrous sodium carbonate (445 mg, 4.2 mmol) were added. The tube was deoxygenated with three cycles vacuum/Ar, then anhydrous DMF (1.1 ml) and H₂O (300 µl) were added. The solution was stirred at rt, under Ar flux, for 15 minutes. Tetrakis(triphenylphosphine)palladium(0) (243 mg, 0.2 mmol) was thus added, the mixture was heated to 110 °C and stirred for 18 hours. The reaction mixture was diluted with DCM and washed three times with water; the solvent was thus removed under reduced pressure. Purification by normal phase flash column chromatography (solvent A: CHX, solvent B: EtOAc - gradient: 0-30% solvent B) yielded title compound (238 mg, yield 97%). ¹H NMR(400 MHz, DMSO-*d*₆) δ 9.95 (s, 1H), 8.37 (s, 1H), 8.03 (s, 1H), 7.94 - 7.75 (m, 4H), 4.10 (t, *J* = 6.9 Hz, 2H), 1.83 (h, *J* = 7.2 Hz, 2H), 0.85 (t, *J =* 7.4 Hz, 3H). Rt 1.86 min (generic method). ESI-MS for C₁₃H₁₄N₂O: calculated 214.1, found *m*/*z* 215 [M+H]⁺.

### Step 2. Synthesis of 6-chloro-4-phenyl-3-[(E)-3-[4-(1-propylpyrazol-4-yl)phenyl]prop-2-enoyl]-1H-quinolin-2-one (b.23).

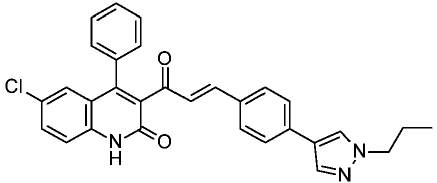

Compound b.23 was synthesized following the general procedure B using a.1 (324 mg, 1.1 mmol) and 4-(1-Propyl-1*H-*pyrazol-4-yl)benzaldehyde. Purification by normal phase flash column chromatography (solvent A: DCM, solvent B: EtOH - gradient: 0-8% solvent B) yielded title compound (387 mg, yield 72%). ¹H NMR(400 MHz, DMSO-*d*₆) δ 12.33 (s, 1H), 8.26 (d, *J* = 0.8 Hz, 1H), 7.94 (d, *J* = 0.8 Hz, 1H), 7.68 - 7.55 (m, 5H), 7.50 - 7.38 (m, 5H), 7.33 (dd, *J* = 7.7, 1.8 Hz, 2H), 6.98 (d, *J* = 2.3 Hz, 1H), 6.73 (d, *J* = 16.4 Hz, 1H), 4.06 (t, *J* = 7.0 Hz, 2H), 1.81 (h, *J* = 7.3 Hz, 2H), 0.84 (t, J = 7.4 Hz, 3H). Rt 2.46 min (generic method). ESI-MS for C₃₀H₂₄ClN₃O₂: calculated 493.2, found *m*/*z* 494.2 [M+H]⁺, 492.2 [M-H]⁻.

### Step 3. Synthesis of 6-chloro-4-phenyl-3-[5-[4-(1-propylpyrazol-4-yl)phenyl]-4,5-dihydro-1H-pyrazol-3-yl]-1H-quinolin-2-one (c.23).

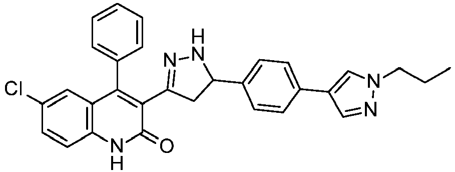

Compound c.23 was synthesized following the general procedure C using b.23 (358 mg, 0.72 mmol). Purification by normal phase flash column chromatography (solvent A: DCM, solvent B: EtOH - gradient: 0-10% solvent B) yielded title compound (326 mg, yield 89%). ¹H NMR(400 MHz, DMSO-*d*₆) δ 12.18 (s, 1H), 8.13 (d, *J* = 0.8 Hz, 1H), 7.83 (d, *J* = 0.8 Hz, 1H), 7.57 (dd, *J* = 8.8, 2.4 Hz, 1H), 7.51 (tdd, *J =* 6.6, 3.8, 1.8 Hz, 3H), 7.42 (dd, *J* = 8.5, 6.2 Hz, 3H), 7.34 (dt, *J* = 6.8, 1.7 Hz, 1H), 7.26 - 7.22 (m, 1H), 7.09 (d, *J* = 3.2 Hz, 1H), 7.08 - 7.03 (m, 2H), 6.90 (d, *J* = 2.3 Hz, 1H), 4.61 - 4.49 (m, 1H), 4.06 (t, *J* = 7.0 Hz, 2H), 3.22 (dd, *J* = 16.4, 11.0 Hz, 1H), 2.56 (dd, *J* = 16.4, 9.6 Hz, 1H), 1.81 (h, *J* = 7.3 Hz, 2H), 0.85 (t, *J* = 7.4 Hz, 3H). Rt 2.34 min (generic method). ESI-MS for C₃₀H₂₆ClN₅O: calculated 507.2, found m/z 508.2 [M+H]⁺, 506.2 [M-H]⁻.

### Step 4. Synthesis of 4-[5-(6-chloro-2-oxo-4-phenyl-1H-quinolin-3-yl)-3-[4-(1-propylpyrazol-4-yl)phenyl]-3,4-dihydropyrazol-2-yl]-4-oxo-butanoic acid (d.43).

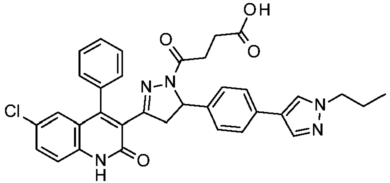

Compound d.43 was synthesized following the general procedure D1 using c.23 (155 mg, 0.30 mmol) with succinic anhydride (120 °C, 200 W) . Purification by normal phase flash column chromatography (solvent A: DCM, solvent B: EtOH - gradient: 0-30% solvent B) yielded title compound (189 mg, quantitative yield). ¹H NMR(400 MHz, DMSO-*d*₆) δ 12.37 (s, 1H), 12.10 (s, 1H), 8.14 (s, 1H), 7.84 (s, 1H), 7.62 (dd, *J* = 8.8, 2.4 Hz, 1H), 7.59 - 7.50 (m, 3H), 7.48 - 7.36 (m, 4H), 7.31 - 7.25 (m, 1H), 6.94 (d, *J* = 2.3 Hz, 1H), 6.77 (d, *J* = 8.0 Hz, 2H), 5.28 (dd, *J* = 12.0, 4.6 Hz, 1H), 4.07 (t, *J* = 7.0 Hz, 2H), 3.75 (dd, *J =* 18.4, 12.1 Hz, 1H), 2.82 (dd, *J* = 18.5, 4.6 Hz, 1H), 2.48 - 2.41 (m, 2H), 2.28 (t, *J* = 6.9 Hz, 2H), 1.81 (h, *J* = 7.2 Hz, 2H), 0.85 (t, *J* = 7.3 Hz, 3H). Rt 2.09 min (generic method). ESI-MS for C₃₄H₃₀ClN₅O₄ : calculated 607.2, found *m*/*z* 608.2 [M+H]⁺, 606.2 [M-H]⁻.

### Example d.44. 3-[2-acetyl-3-[4-(1-propylpyrazol-4-yl)phenyl]-3,4-dihydropyrazol-5-yl]-6-chloro-4-phenyl-1H-quinolin-2-one (example not falling within the scope of the present invention).

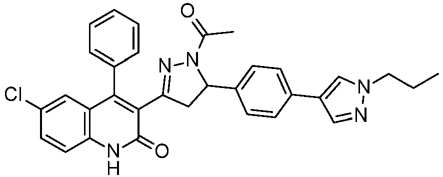

Compound d.44 was synthesized following the general procedure D1 using c.23 (146 mg, 0.31 mmol) with acetic anhydride (165 °C, 200 W) . Purification by normal phase flash column chromatography (solvent A: DCM, solvent B: EtOH - gradient: 0-8% solvent B) yielded title compound (138 mg, yield 81%). ¹H NMR (400 MHz, DMSO-*d*₆) δ 12.36 (s, 1H), 8.13 (s, 1H), 7.83 (d, *J* = 0.7 Hz, 1H), 7.62 (dd, *J* = 8.8, 2.4 Hz, 1H), 7.60 - 7.52 (m, 3H), 7.45 (d, *J* = 8.8 Hz, 1H), 7.43 - 7.38 (m, 3H), 7.30 (dt, *J* = 6.2, 1.7 Hz, 1H), 6.94 (d, *J* = 2.3 Hz, 1H), 6.79 (d, *J =* 8.3 Hz, 2H), 5.28 (dd, *J* = 12.0, 4.5 Hz, 1H), 4.07 (t, *J* = 7.0 Hz, 2H), 3.75 (dd, *J* = 18. 4, 12.0 Hz, 1H), 2.86 (dd, *J* = 18.4, 4.6 Hz, 1H), 1.91 - 1.75 (m, 5H), 0.85 (t, *J* = 7.4 Hz, 3H). Rt 1.22 min (apolar method) . ESI-MS for C₃₂H₂₈ClN₅O₂: calculated 549.2, found m/z 550.2 [M+H]⁺, 548.2 [M-H]⁻.

### Example d.45. 4-[5-(6-chloro-2-oxo-4-phenyl-1H-quinolin-3-yl)-3-[4-(1-ethylindazol-5-yl)phenyl]-3,4-dihydropyrazol-2-yl]-4-oxo-butanoic acid (example not falling within the scope of the present invention).

### Step 1. Synthesis of 4-(1-ethylindazol-5-yl)benzaldehyde and 4-(2-ethylindazol-5-yl)benzaldehyde

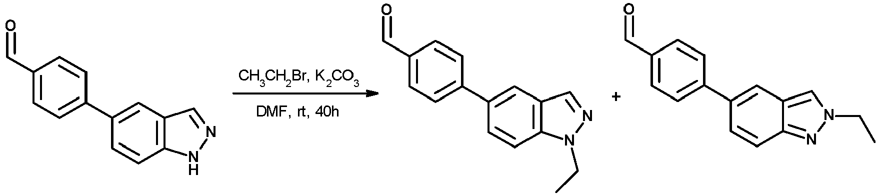

In a screw capped pressure tube 4-(1*H*-indazol-5-yl)benzaldehyde (204 mg, 0.92 mmol) was dissolved with 1.0 ml of anhydrous DMF then potassium carbonate (254 mg, 1.8 mmol) was added and the mixture kept under stirring for 15 minutes at rt. Ethyl bromide (172 µl, 2.3 mmol) was then added. The reaction was thus stirred at rt for 40 hours. The crude mixture was diluted with DCM and washed three times with water. The organic layer was dried through a phase separator and the solvent removed under reduced pressure. Purification by normal phase flash column chromatography (solvent A: DCM, solvent B: DCM/EtOH 9:1 *v*/*v* - gradient: 0-50% solvent B) yielded both title compounds separately. Mono- and bi-dimensional ¹H- and ¹³C-NMR analyses confirmed the structure of the title compounds named 4-(1-ethylindazol-5-yl)benzaldehyde and 4-(2-ethylindazol-5-yl)benzaldehyde.

4-(1-ethylindazol-5-yl)benzaldehyde: 125 mg, yield 54%. ¹H NMR(400 MHz, DMSO-*d*₆) δ 10.05 (s, 1H), 8.17 (t, *J* = 1.3 Hz, 1H), 8.15 (s, 1H), 8.02 - 7.94 (m, 4H), 7.80 (d, *J* = 1.3 Hz, 2H), 4.48 (q, *J* = 7.2 Hz, 2H), 1.42 (t, *J =* 7.2 Hz, 3H). ¹³C NMR (101 MHz, DMSO-*d*₆) δ 192.65, 146.47, 138.64, 134.57, 133.34, 131.34, 130.17, 127.36, 125.54, 124.24, 119.64, 110.32, 43.20, 14.93. Rt 2.19 min (generic method). ESI-MS for C₁₆H₁₄N₂O: calculated 250.1, found *m*/*z* 251.1 [M+H]⁺.

4-(2-ethylindazol-5-yl)benzaldehyde: 82 mg, yield 39%. ¹H NMR (400 MHz, DMSO-*d*₆) δ 10.05 (s, 1H), 8.49 (d, *J* = 0.9 Hz, 1H), 8.13 (dd, *J* = 1.8, 0.9 Hz, 1H), 8.02 - 7.91 (m, 4H), 7.78 - 7.60 (m, 2H), 4.49 (q, *J* = 7.3 Hz, 2H), 1.53 (t, *J* = 7.3 Hz, 3H). ¹³C NMR (101 MHz, DMSO-*d*₆) δ 192.63, 147.68, 146.74, 134.52, 131.49, 130.14, 127.19, 124.97, 124.34, 121.86, 119.45, 117.72, 47.88, 15.72. Rt 1.97 min (generic method). ESI-MS for C₁₆H₁₄N₂O: calculated 250.1, found *m*/*z* 251.1 [M+H]⁺.

### Step 2. Synthesis of 6-chloro-3-[(E)-3-[4-(1-ethylindazol-5-yl)phenyl]prop-2-enoyl]-4-phenyl-1H-quinolin-2-one (b.24).

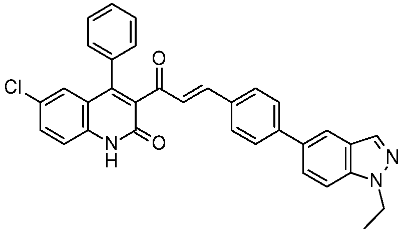

Compound b.24 was synthesized following the general procedure B using a.1 (137 mg, 0.46 mmol) and 4-(1-ethylindazol-5-yl)benzaldehyde. Purification by normal phase flash column chromatography (solvent A: DCM, solvent B: EtOH - gradient: 0-2% solvent B) yielded title compound (244 mg, quantitative yield). ¹H NMR(400 MHz, DMSO-*d*₆) δ 12.35 (s, 1H), 8.10 (d, *J* = 7.3 Hz, 2H), 7.74 (s, 5H), 7.65 (dd, *J* = 8.8, 2.4 Hz, 1H), 7.59 - 7.39 (m, 6H), 7.34 (dd, *J* = 7.8, 1.8 Hz, 2H), 6.99 (d, *J* = 2.4 Hz, 1H), 6.80 (d, *J* = 16.3 Hz, 1H), 4.46 (q, *J* = 7.2 Hz, 2H), 1.40 (t, *J* = 7.2 Hz, 3H). Rt 2.64 min (generic method) . ESI-MS for C₃₃H₂₄ClN₃O₂: calculated 529.2, found m/z 530.2 [M+H]⁺, 528.2 [M-H]⁻.

### Step 3. Synthesis of 6-chloro-3-[5-[4-(1-ethylindazol-5-yl)phenyl]-4,5-dihydro-1H-pyrazol-3-yl]-4-phenyl-1H-quinolin-2-one (c.24).

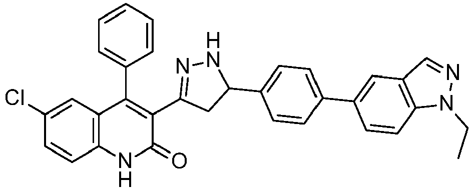

Compound c.24 was synthesized following the general procedure C using b.24 (233 mg, 0.44 mmol). Purification by normal phase flash column chromatography (solvent A: DCM, solvent B: EtOH - gradient: 0-8% solvent B) yielded title compound (181 mg, yield 75%). ¹H NMR(400 MHz, DMSO-*d*₆) δ 12.20 (s, 1H), 8.10 (d, *J* = 0.9 Hz, 1H), 7.98 (t, *J* = 1.2 Hz, 1H), 7.74 (d, *J* = 8.8 Hz, 1H), 7.67 (dd, *J* = 8.8, 1.7 Hz, 1H), 7.60 - 7.47 (m, 6H), 7.42 (d, *J* = 8.8 Hz, 1H), 7.36 (dt, *J =* 6.4, 1.8 Hz, 1H), 7.28 - 7.24 (m, 1H), 7.20 - 7.14 (m, 3H), 6.90 (d, *J* = 2.4 Hz, 1H), 4.63 (td, *J =* 10.9, 10.3, 3.2 Hz, 1H), 4.46 (q, *J* = 7.2 Hz, 2H), 3.29 - 3.20 (m, 1H), 2.62 (dd, *J* = 16.4, 9.4 Hz, 1H), 1.41 (t, *J* = 7.2 Hz, 3H). Rt 1.49 min (apolar method) . ESI-MS for C₃₃H₂₆ClN₅O: calculated 543.2, found *m*/*z* 544.2 [M+H]⁺, 542.2 [M-H]⁻.

### Step 4. Synthesis of 4-[5-(6-chloro-2-oxo-4-phenyl-1H-quinolin-3-yl)-3-[4-(1-ethylindazol-5-yl)phenyl]-3,4-dihydropyrazol-2-yl]-4-oxo-butanoic acid (d.45).

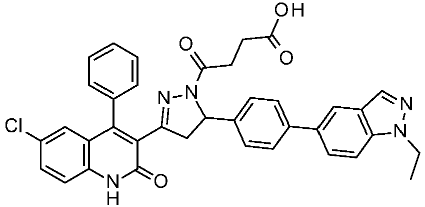

Compound d.45 was synthesized following the general procedure D1 using c.24 (170 mg, 0.31 mmol) with succinic anhydride (120 °C, 200 W). Purification by normal phase flash column chromatography (solvent A: DCM, solvent B: EtOH - gradient: 0-20% solvent B) yielded title compound (163 mg, yield 81%). ¹H NMR(400 MHz, DMSO-*d*₆) δ 12.38 (s, 1H), 12.07 (s, 1H), 8.10 (d, *J =* 0.8 Hz, 1H), 7.99 (d, *J =* 1.6 Hz, 1H), 7.74 (d, *J* = 8.8 Hz, 1H), 7.67 (dd, *J* = 8.8, 1.7 Hz, 1H), 7.63 (dd, *J* = 8.8, 2.4 Hz, 1H), 7.60 - 7.51 (m, 5H), 7.48 - 7.40 (m, 2H), 7.30 (dd, *J* = 5.9, 1.8 Hz, 1H), 6.94 (d, *J* = 2.3 Hz, 1H), 6.89 (d, *J* = 8.3 Hz, 2H), 5.35 (dd, *J* = 12.0, 4.6 Hz, 1H), 4.46 (q, *J* = 7.2 Hz, 2H), 3.78 (dd, *J* = 18.5, 12.1 Hz, 1H), 2.86 (dd, *J* = 18.5, 4.6 Hz, 1H), 2.59 - 2.44 (m, 2H), 2.31 (t, *J* = 6.8 Hz, 2H), 1.41 (t, *J* = 7.2 Hz, 3H). Rt 2.24 min (generic method). ESI-MS for C₃₇H₃₀ClN₅O₄ calculated 643.2, found *m*/*z* 644.2 [M+H]⁺, 642.2 [M-H]⁻.

### Example d.46. 4-[5-(6-chloro-2-oxo-4-phenyl-1H-quinolin-3-yl)-3-[4-(2-ethylindazol-5-yl)phenyl]-3,4-dihydropyrazol-2-yl]-4-oxo-butanoic acid (example not falling within the scope of the present invention).

### Step 1. Synthesis of 6-chloro-3-[(E)-3-[4-(2-ethylindazol-5-yl)phenyl]prop-2-enoyl]-4-phenyl-1H-quinolin-2-one (b.25).

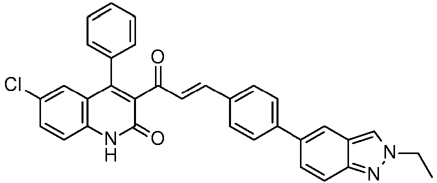

Compound b.25 was synthesized following the general procedure B using a.1 (92 mg, 0.31 mmol) and 4-(2-ethylindazol-5-yl)benzaldehyde. Purification by normal phase flash column chromatography (solvent A: DCM, solvent B: EtOH - gradient: 0-10% solvent B) yielded title compound (150 mg, yield 91%). ¹H NMR(400 MHz, DMSO-*d*₆) δ 12.35 (s, 1H), 8.44 (d, *J* = 0.8 Hz, 1H), 8.04 (t, *J* = 1.3 Hz, 1H), 7.73 (s, 4H), 7.70 - 7.63 (m, 2H), 7.59 (dd, *J* = 9.1, 1.8 Hz, 1H), 7.57 - 7.42 (m, 5H), 7.34 (dd, *J* = 7.8, 1.8 Hz, 2H), 6.99 (d, *J* = 2.3 Hz, 1H), 6.79 (d, *J* = 16.4 Hz, 1H), 4.47 (q, *J =* 7.3 Hz, 2H), 1.52 (t, *J =* 7.3 Hz, 3H) . Rt 2.48 min (generic method) . ESI-MS for C₃₃H₂₄ClN₃O₂: calculated 529.2, found *m*/*z* 530.2 [M+H]⁺, 528.2 [M-H]⁻.

### Step 2. Synthesis of 6-chloro-3-[5-[4-(2-ethylindazol-5-yl)phenyl]-4,5-dihydro-1H-pyrazol-3-yl]-4-phenyl-1H-quinolin-2-one (c.25).

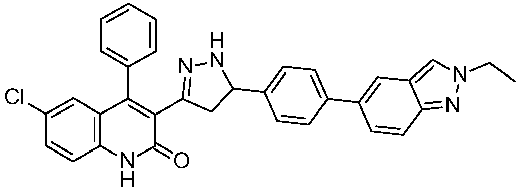

Compound c.25 was synthesized following the general procedure C using b.25 (141 mg, 0.27 mmol). Purification by normal phase flash column chromatography (solvent A: DCM, solvent B: EtOH - gradient: 0-15% solvent B) yielded title compound (123 mg, yield 83%). ¹H NMR(400 MHz, DMSO-*d*₆) δ 8.41 (d, *J* = 0.9 Hz, 1H), 7.92 - 7.88 (m, 1H), 7.67 (d, *J* = 9.0 Hz, 1H), 7.59 - 7.48 (m, 7H), 7.42 (d, *J =* 8.8 Hz, 1H), 7.37 - 7.33 (m, 1H), 7.25 (dq, *J* = 4.9, 1.9 Hz, 1H), 7.16 (dd, *J* = 9.3, 2.7 Hz, 3H), 6.90 (d, *J* = 2.4 Hz, 1H), 4.62 (ddd, *J* = 10.9, 9.4, 3.1 Hz, 1H), 4.47 (q, *J* = 7.3 Hz, 2H), 3.27 (dd, *J* = 16.5, 11.1 Hz, 1H), 2. 62 (dd, *J* = 16.4, 9.5 Hz, 1H), 1.52 (t, *J =* 7.3 Hz, 3H). Rt 2.35 min (generic method). ESI-MS for C₃₃H₂₆ClN₅O: calculated 543.2, found *m*/*z* 544.2 [M+H]⁺, 542.2 [M-H]⁻.

### Step 3. Synthesis of 4-[5-(6-chloro-2-oxo-4-phenyl-1H-quinolin-3-yl)-3-[4-(2-ethylindazol-5-yl)phenyl]-3,4-dihydropyrazol-2-yl]-4-oxo-butanoic acid (d.46).

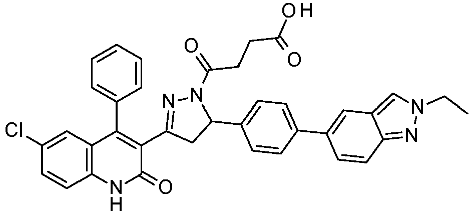

Compound d.46 was synthesized following the general procedure D1 using c.25 (117 mg, 0.21 mmol) with succinic anhydride (120 °C, 200 W) . Purification by normal phase flash column chromatography (solvent A: DCM, solvent B: EtOH - gradient: 0-20% solvent B) yielded title compound (66 mg, yield 49%). ¹H NMR (400 MHz, DMSO-*d*₆) δ 12.38 (s, 1H), 12.15 (s, 1H), 8.42 (d, *J =* 0.9 Hz, 1H), 7.92 (t, *J =* 1.3 Hz, 1H), 7.68 (d, *J* = 8.9 Hz, 1H), 7.63 (dd, *J* = 8.8, 2.4 Hz, 1H), 7.60 - 7.50 (m, 6H), 7.48 - 7.41 (m, 2H), 7.30 (dd, *J =* 6.1, 1.9 Hz, 1H), 6.94 (d, *J* = 2.3 Hz, 1H), 6.87 (d, *J* = 8.4 Hz, 2H), 5.35 (dd, *J* = 12.0, 4.6 Hz, 1H), 4.47 (q, *J* = 7.3 Hz, 2H), 3.78 (dd, *J* = 18.5, 12.1 Hz, 1H), 2.86 (dd, *J* = 18.5, 4.7 Hz, 1H), 2.57 - 2.43 (m, 2H), 2.30 (t, *J* = 6.9 Hz, 2H), 1.52 (t, *J =* 7.3 Hz, 3H Rt 2.10 min (generic method). ESI-MS for C₃₇H₃₀ClN₅O₄: calculated 643.2, found *m*/*z* 644.2 [M+H]⁺, 642.2 [M-H]⁻.

### Example d.47. 3-(2-acetyl-3-phenyl-3,4-dihydropyrazol-5-yl)-6-chloro-4-phenyl-1H-quinolin-2-one (example not falling within the scope of the present invention).

### Step 1. Synthesis of 6-chloro-4-phenyl-3-[(E)-3-phenylprop-2-enoyl]-1H-quinolin-2-one (b.26)

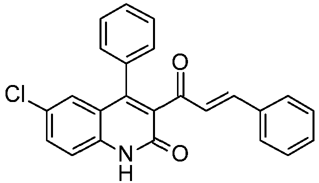

In a round bottom flask, 3-acetyl-6-chloro-4-phenylquinolin-2(1H)-one (1.5 g, 5.04 mmol) was suspended in a mixture of ethanol and water (4:3) (100.0 ml), cooled at 0°C and potassium hydroxide (7.07 g, 126.0 mmol) was added. After stirring at 0°C for 45 minutes, benzaldehyde (1.02 ml, 10.08 mmol) was added and the reaction mixture stirred at room temperature for 16 h. After cooling down to 0°C, acetic acid (7.2 ml, 126.0 mmol) was added and the mixture stirred for 30 minutes. Resulting yellow solid was recovered by filtration under vacuum, dissolved in dichloromethane, the organic phase dried over Na₂SO₄ and evaporated to dryness to give title compound as a yellow solid (1.93 g, yield 99%). ¹H NMR(400 MHz, DMSO-*d*₆) δ 12.38 (s, 1H), 7.68 (dd, J=2.0, 8.0 Hz, 2H), 7.54- 7.39 (m, 7H), 7.35 (dd, J=2.0, 8.0 Hz, 2H), 7.00 (d, J= 2.0 Hz, 1H), 6.78 (d, J= 16.0 Hz, 1H).

### Step 2. Synthesis of 6-chloro-4-phenyl-3-(5-phenyl-4,5-dihydro-1H-pyrazol-3-yl)-1H-quinolin-2-one (c.26)

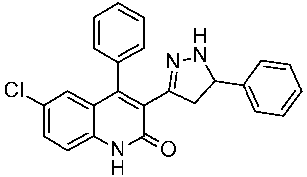

In appropriately sized microwaveable vessel, b.26 (1.90 g, 4.92 mmol) was suspended in ethanol (9 ml), and then hydrazine hydrate (431 µL, 8.86 mmol) was added. The reaction mixture was microwaved at 110°C for 25 minutes, then title compound was recovered by filtration under vacuum at room temperature as a yellow solid (1.53 g, yield 78%). ¹H NMR(400 MHz, CDCl₃) δ 12.56 (s, 1H), 7.54-7.48 (m, 3H), 7.41 (dd, J=4.0, 12.0 Hz, 1H), 7.33 (s, 1H), 7.32-7.31 (m, 1H), 7.30-7.28 (m, 4H), 7.20-7.17 (m, 3H), 5.81 (s, 1H), 4.75 (dd, J= 8.0, 12.0 Hz, 1H), 3.23 (dd, J=12.0, 20.0 Hz, 1H), 2.86 (dd, J= 8.0, 16.0 Hz, 1H).

### Step 3. Synthesis of 3-(2-acetyl-3-phenyl-3,4-dihydropyrazol-5-yl)-6-chloro-4-phenyl-1H-quinolin-2-one (d.47)

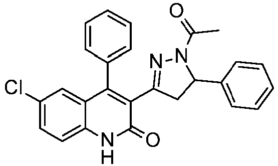

In appropriately-sized microwaveable vessel, c.26 (400 mg, 1.00 mmol) was suspended in anhydrous tetrahydrofuran (2 ml) in presence of 4Å molecular sieves. Acetic anhydride (113 µL, 1.20 mmol) was added and the reaction mixture was microwaved at 165°C for 25 minutes. After cooling down to rt, the mixture was diluted with dichloromethane (7 ml) and the organic phase was washed with water (2 x 5 ml), dried over Na₂SO₄ and evaporated to dryness. The crude product was purified by flash silica gel chromatography (methanol: dichloromethane gradient up to 2:8) to obtain title compound as a yellow solid (365 mg, yield 83%). ¹H NMR(400 MHz, DMSO-*d₆*) δ 12.39 (s, 1H), 7.62 (d, J=8.0 Hz, 1H), 7.57-7.55 (m, 2H), 7.52-7.49 (m, 1H), 7.44 (d, J=8.0 Hz, 1H), 7.40 (d, J=4.0 Hz, 1H), 7.27 (d, J=8.0 Hz, 1H), 7.22-7.19 (m, 3H), 6.93 (d, J= 4.0 Hz, 1H), 6.79 (d, J= 8.0 Hz, 2H), 5.29 (dd, J= 4.0, 12.0 Hz, 1H), 3.75 (dd, J= 12.0, 20.0 Hz, 1H), 2.83 (dd, J=4.0, 20.0 Hz, 1H), 1.87 (s, 3H). Rt 2.40 min (generic method) . ESI-MS for C₂₆H₂₀ClN₃O₂: calculated 441.1, found 442.0 [M+H]⁺.

### Example d.48. 6-chloro-4-phenyl-3-(3-phenyl-2-propanoyl-3,4-dihydropyrazol-5-yl)-1H-quinolin-2-one (example not falling within the scope of the present invention).

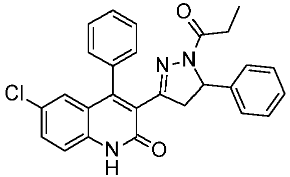

Compound d.48 was synthesized following the general procedure D2 using c.26 (400 mg, 1.00 mmol). Purification by normal phase flash column chromatography (solvent A: DCM, solvent B: MeOH - gradient: 0-2 % solvent B) yielded title compound (70 mg, yield 15%). ¹H NMR(400 MHz, DMSO-*d*₆) δ 12.34 (s, 1H), 7.64-7.61 (m, 1H), 7.58 - 7.52 (m, 2H), 7.51 - 7.48 (m, 1H), 7.44 (d, J = 8.0 Hz, 1H), 7.41-7.39 (m, 1H), 7.26 (d, J = 8.0 Hz, 1H), 7.24-7.19 (m, 3H), 6.91 (d, J = 2.0 Hz, 1H), 6.80-6.78 (m, 2H), 5.28 (dd, J = 12.0, 4.8 Hz, 1H), 3.74 (dd, J = 20.0, 12.0 Hz, 1H), 2.80 (dd, J = 20.0, 4.8 Hz, 1H), 2.28-2.18 (m, 2H), 0.83 (t, J = 7.4 Hz, 3H). Rt 2.52 min (generic method) .ESI-MS for C₂₇H₂₂ClN₃O₂: calculated 455.1, found *m*/*z* 456.1 [M+H]⁺, 454.1 [M-H]⁻.

### Example d.49. 3-[2-acetyl-3-(p-tolyl)-3,4-dihydropyrazol-5-yl]-6-chloro-4-phenyl-1H-quinolin-2-one (example not falling within the scope of the present invention).

### Step 1. Synthesis of 6-chloro-4-phenyl-3-[(E)-3-(p-tolyl)prop-2-enoyl]-1H-quinolin-2-one (b.27)

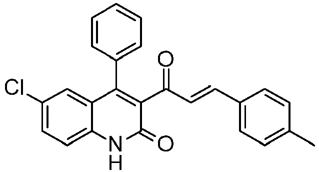

In round bottom flask, 3-acetyl-6-chloro-4-phenylquinolin-2(1H)-one (1.0 g, 3.36 mmol) was suspended in a mixture of ethanol and water (4:3) (67.0 ml), cooled at 0°C and potassium hydroxide (4.70 g, 84.0 mmol) was added. After stirring at 0°C for 45 minutes, p-tolualdehyde (792 µL, 6.72 mmol) was added and the reaction was stirred at room temperature for 16 h. After cooling down to 0°C, acetic acid (4.8 mL, 84.0 mmol) was added and the mixture stirred for 30 minutes. Resulting yellow solid was recovered by filtration under vacuum at room temperature, solved in dichloromethane, the organic phase dried over Na₂SO₄ and evaporated to dryness. Final normal flash silica gel chromatography purification (methanol: dichloromethane gradient up 1:9) yielded title compound as a yellow solid (1.18 g, yield 88%). ¹H NMR (400 MHz, CDCl₃) δ 12.72 (s, 1H), 7.42-7.40 (m, 5H), 7.34 (d, J=16.0 Hz, 1H), 7.33-7.28 (m, 4H), 7.26-7.25 (m, 1H), 7.15 (d, J=8.0 Hz, 2H), 6.70 (d, J= 16.0 Hz, 1H), 2.36 (s, 3H).

### Step 2. Synthesis of 6-chloro-4-phenyl-3-[5-(p-tolyl)-4,5-dihydro-1H-pyrazol-3-yl]-1H-quinolin-2-one (c.27)

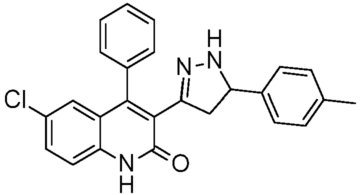

In appropriately sized microwaveable vessel, b.27 (1.16 g, 2.90 mmol) was suspended in ethanol (8 ml), and then hydrazine hydrate (254 µl, 5.22 mmol) was added. The reaction mixture was microwaved at 110°C for 30 minutes, then title compound was recovered by filtration under vacuum at room temperature as a yellow solid (750 mg, yield 63%). ¹H NMR(400 MHz, CDCl₃) δ 12.36 (s, 1H), 7.52-7.49 (m, 3H), 7.41 (dd, J= 2.0, 8.0 Hz, 1H), 7.33-7.31 (m, 2H), 7.30-7.27 (m, 1H), 7.17 (d, J=2.0 Hz, 1H), 7.08 (dd, J= 8.4, 10.8 Hz, 4H), 4.70 (dd, J= 8.0, 12.0 Hz, 1H), 3.21 (dd, J= 8.0, 16.0 Hz, 1H), 2.82 (dd, J= 8.0, 20.0 Hz, 1H), 2.34 (s, 3H).

### Step 3. Synthesis of 3-[2-acetyl-3-(p-tolyl)-3,4-dihydropyrazol-5-yl]-6-chloro-4-phenyl-1H-quinolin-2-one (d.49).

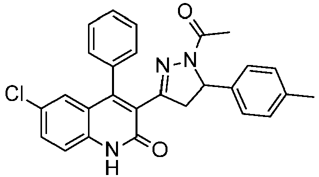

In appropriately-sized microwaveable vessel, c.27 (350 mg, 0.85 mmol) was suspended in anhydrous tetrahydrofuran (2 ml) in presence of 4Å molecular sieves. Acetic anhydride (96 µL, 1.01 mmol) was added and the reaction mixture was microwaved at 165°C for 25 minutes. After cooling down to rt, the mixture was diluted with dichloromethane (5 ml) and the organic phase was washed with water (2 x 5 ml), dried over Na₂SO₄ and evaporated to dryness. The crude product was purified by flash silica gel chromatography (methanol: dichloromethane gradient up to 1.5:8.5) yielding title compound as yellow solid (258 mg, yield 67%). ¹H NMR(400 MHz, DMSO-*d*₆) δ 12.38 (s, 1H), 7.63 (dd, J=8.0, 16.0 Hz, 1H), 7.59-7.50 (m, 3H), 7.44 (d, J=8.0 Hz, 1H), 7.41-7.40 (m, 1H), 7.28 (d, J= 8.0 Hz, 1H), 7.01 (d, J=8.0 Hz, 2H), 6.93 (d, J=2.0 Hz, 1H), 6.67 (d, J=8.0 Hz, 2H), 5.24 (dd, J=4.0, 12.0 Hz, 1H), 3.72 (dd, J=12.0, 20.0 Hz, 1H), 2.80 (dd, J=4.0, 16.0 Hz, 1H), 2.24 (s, 3H), 1.86 (s, 3H). Rt 2.51 min (generic method) .ESI-MS for C₂₇H₂₂ClN₃O₂: calculated 455.1, found 456.1 [M+H]⁺.

### Example d.50. 4-[5-(4-benzyl-6-chloro-2-oxo-1H-quinolin-3-yl)-3-(4-fluorophenyl)-3,4-dihydropyrazol-2-yl]-4-oxo-butanoic acid

### Step 1. Synthesis of 1-(2-amino-5-chloro-phenyl)-2-phenyl-ethanone

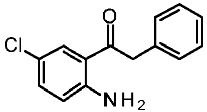

Title compound was synthesized following the procedure described by Patterson S. et al (J. Med. Chem. 2011, 54, 6514-6530) :
In a dried three-neck round-bottom flask, 2-amino-5-chlorobenzonitrile (3.0 g, 19.66 mmol) was suspended in anhydrous diethyl ether (9.8 ml) under nitrogen atmosphere. The mixture was cooled to 0 °C, and then benzyl magnesium chloride was added dropwise as 2 M solution in anhydrous tetrahydrofuran (28 ml, 56.00 mmol). After stirring for 6 hours at room temperature, the reaction was cooled to -60 °C and 6 N HCl (15 ml) was added while stirring vigorously. After 1 hour at room temperature, the mixture was diluted with ethyl acetate (50 ml), the aqueous layer extracted with ethyl acetate (3 x 50 ml), the combined organic phases were dried over Na₂SO₄ and evaporated to dryness. Purification by normal phase chromatography (solvent A: petroleum ether, solvent B: ethyl acetate - gradient: 0-4% solvent B) yielded title compound (4.22 g, yield 87 %). ¹H NMR(400 MHz, CDCl₃) δ 7.80 (d, J= 2.4 Hz, 1H), 7.35 (t, J=8.0 Hz, 2H), 7.29-7.23 (m, 3H), 7.20 (dd, J= 2.4, 9.2 Hz, 1H), 6.61 (d, J= 9.2 Hz, 1H) 6.29 (br, 2H), 4.23 (s, 2H).

### Step 1. Synthesis of 3-acetyl-4-benzyl-6-chloro-1H-quinolin-2-one (e.1)

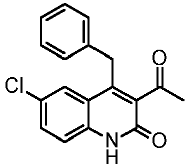

In dried microwaveable vessel, 1-(2-amino-5-chlorophenyl)-2-phenyl-ethanone (1.75 g, 7.12 mmol) was dissolved in anhydrous tetrahydrofuran (11 ml) in presence of 4Å molecular sieves. Then ethyl acetoacetate (2.3 mL, 17.8 mmol) was added. The reaction mixture was microwaved at 140 °C (200 W) for 75 minutes, cooled at room temperature, resulting white-off solid filtered and rinsed with cold tetrahydrofuran to finally yield title compound (1.06 g, yield 48 %). ¹H NMR (400 MHz, DMSO-*d*₆) δ 12.52 (s, 1H), 7.69 (d, J= 2.0 Hz, 1H), 7.55 (dd, J= 2.0, 8.8 Hz, 1H), 7.35 (d, J= 8.8 Hz, 1H), 7.30- 7.24 (m, 4H), 7.19 (t, J= 6.8 Hz, 1H), 4.14 (s, 2H), 2.42 (s, 3H).

### Step 2. Synthesis of 4-benzyl-6-chloro-3-[(E)-3-(4-fluorophenyl)prop-2-enoyl]-1H-quinolin-2-one (f.1)

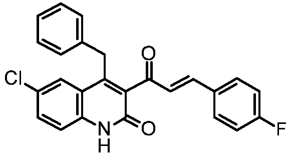

In a round-bottom flask, e.1 (1.06 g, 3.40 mmol) was suspended in a 4:3 mixture of ethanol (39.0 ml) and water (29.0 ml). The mixture was cooled to 0 °C and potassium hydroxide (4.80 g, 85.0 mmol) was added. After stirring for 45 minutes at 0 °C, 4-fluorobenzaldehyde (657 µL, 6.12 mmol) was added. The reaction was stirred at room temperature for 16 hours, afterwards acetic acid (4.9 ml, 85.0 mmol) was added at 0 °C, resulting white-off solid filtered and purified by gravity silica gel chromatography (solvent A: dichloromethane, solvent B: methanol - isocratic 5 % solvent B) to finally yield title compound as a white solid (1.08 g, yield 76%). ¹H NMR(400 MHz, DMSO-*d*₆) δ 12.14 (s, 1H), 7.81 (dd, J= 5.6, 8.4 Hz, 2H), 7.64 (d, J= 1.6 Hz, 1H), 7.59-7.55 (m, 2H), 7.39 (d, J= 8.4 Hz, 1H), 7.27-7.24 (m, 6H), 7.14 (t, J= 6.8 Hz, 1H), 7.05 (d, J= 16.4 Hz, 1H), 4.13 (s, 2H).

### Step 3. Synthesis of 4-benzyl-6-chloro-3-[5-(4-fluorophenyl)-4,5-dihydro-1H-pyrazol-3-yl]-1H-quinolin-2-one (g.1).

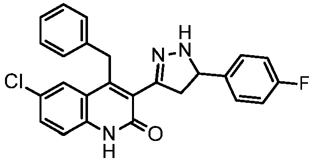

In appropriately sized microwaveable vessel, f.1 (1.0 g, 2.39 mmol) was suspended in ethanol (5.0 ml), then hydrazine hydrate (198 µL, 4.07 mmol) added, the reaction mixture microwaved at 110 °C (200 W) for 20 minutes, cooled at room temperature and resulting yellow solid filtered to yield title compound (855 mg, yield 83%). ¹H NMR(400 MHz, DMSO-*d*₆) δ 11.98 (s, 1H), 7.66 (d, J= 2.0 Hz, 1H), 7.52-7.47 (m, 2H), 7.42 (dd, J= 6.0, 8.0 Hz, 2H), 7.33 (d, J= 8.4 Hz, 1H), 7.25 (d, J= 4.4 Hz, 4H), 7.17-7.09 (m, 3H), 4.80 (dd, J= 4.0, 8.0 Hz, 1H), 4.43 (d, J=12.0 Hz, 1H), 4.40 (d, J=12.0 Hz, 1H), 3.44 (dd, J= 8.0, 12.0 Hz, 1H), 2.84 (dd, J= 12.0, 16.0 Hz, 1H).

### Step 4. Synthesis of 4-[5-(4-benzyl-6-chloro-2-oxo-1H-quinolin-3-yl)-3-(4-fluorophenyl)-3,4-dihydropyrazol-2-yl]-4-oxo-butanoic acid (d.50).

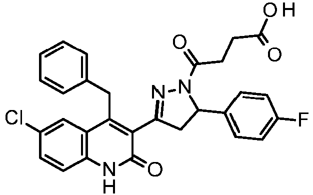

Compound d.50 was synthesized following the general procedure D1 using g.1 (300 mg, 0.69 mmol) with succinic anhydride (90.4 mg, 0.90 mmol) at 165 °C for 20 minutes. Purification by normal phase flash column chromatography (solvent A: DCM, solvent B: MeOH - gradient: 0-2 % solvent B) yielded title compound as a white solid (155 mg, yield 42 %). ¹H NMR (400 MHz, DMSO-*d*₆) δ 12.21 (s, 1H), 12.03 (br, 1H), 7.77 (d, J= 2.4 Hz, 1H), 7.56 (dd, J= 4.0, 8.0 Hz, 1H), 7.38 (d, J= 8.0 Hz, 1H), 7.27-7.24 (m, 6H), 7.21-7.15 (m, 1H), 7.09 (t, J= 8.0 Hz, 2H), 5.45 (dd, J= 4.8, 11.6 Hz, 1H), 4.46 (d, J=16.0 Hz, 1H), 4.39 (d, J=16.0 Hz, 1H), 3.70 (dd, J= 12.0, 18.4 Hz, 1H), 3.08 (dd, J= 4.8, 18.4 Hz, 1H), 3.65-2.58 (m, 2H), 2.32 (t, J= 8.0 H, 2H). Rt 2.05 min (generic method). ESI-MS for C₂₉H₂₃ClFN₃O₄: calculated 531.1, found m/z 532.1 [M+H]⁺, 530.1 [M-H]⁻.

### Example d.51. 3-[2-acetyl-3-(4-fluorophenyl)-3,4-dihydropyrazol-5-yl]-4-benzyl-6-chloro-1H-quinolin-2-one

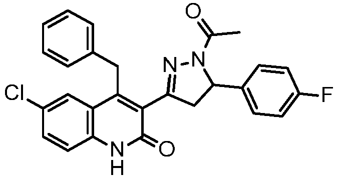

Compound d.51 was synthesized following the general procedure D1 using g.1 (300 mg, 0.69 mmol) with acetic anhydride (85 µL, 0.90 mmol) at 165 °C for 20 minutes. Purification by normal phase flash column chromatography (solvent A: DCM, solvent B: MeOH - gradient: 0-20% solvent B) yielded title compound as a white solid (245 mg, yield 75%). ¹H NMR(400 MHz, DMSO-*d*₆) δ 12.19 (s, 1H), 7.35 (d, J= 1.6 Hz, 1H), 7.55 (dd, J= 2.2, 9.0 Hz, 1H), 7.37 (d, J= 8.4 Hz, 1H), 7.28- 7.24 (m, 6H), 7.17 (t, J= 4.4 Hz, 1H), 7.12-7.07 (m, 2H), 5.46 (dd, J= 4.7, 11.9 Hz, 1H), 4.42 (d, J= 15.6 Hz, 1H), 4.39 (d, J= 15.6 Hz, 1H), 3.72 (dd, J= 18.3, 12.0 Hz, 1H), 3.12 (dd, J= 18.3, 4.8 Hz, 1H), 1.96 (s, 3H). Rt 2.37 min (generic method). ESI-MS for C₂₇H₂₁ClFN₃O₂: calculated 473.1, found m/z 474.1 [M+H]⁺, 472.1 [M-H]⁻.

### Example d.52. 3-[2-acetyl-3-(4-methoxyphenyl)-3,4-dihydropyrazol-5-yl]-4-benzyl-6-chloro-1H-quinolin-2-one

### Step 1. Synthesis of 4-benzyl-6-chloro-3-[(E)-3-(4-methoxyphenyl)prop-2-enoyl]-1H-quinolin-2-one (f.2)

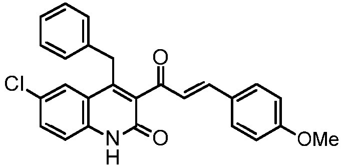

In a round-bottom flask, e.1 (1.06 g, 3.40 mmol) was suspended in a 4:3 mixture of ethanol (65.7 ml) and water (49.3 ml). The mixture was cooled to 0 °C and potassium hydroxide (8.01 g, 142.75 mmol) was added. After stirring for 45 minutes at 0 °C, 4-methoxybenzaldehyde (1.39 mL, 11.42 mmol) was added. The reaction was stirred at room temperature for 16 hours, afterwards acetic acid (8.17 mL, 142.75 mmol) was added at 0 °C, resulting yellow solid filtered and purified by normal phase chromatography (solvent A: dichloromethane, solvent B: methanol - gradient: 0-3 % solvent B) to finally yield title compound as a white-yellow solid (1.62 g, yield 66%). ¹H NMR(400 MHz, DMSO-*d*₆) δ 12.19 (s, 1H), 7.68 (d, J= 8.4 Hz, 2H), 7.62 (d, J= 2.0 Hz, 1H), 7.55 (dd, J= 2.0, 9.0 Hz, 1H), 7.51 (d, J= 16.4 Hz, 1H), 7.38 (d, J= 9.0 Hz, 1H), 7.29- 7.22 (m, 4H), 7.14 (t, J= 6.8 Hz, 1H), 6.98- 6.92 (m, 3H), 4.12 (s, 2H), 3.80 (s, 3H).

### Step 2. Synthesis of 4-benzyl-6-chloro-3-[5-(4-methoxyphenyl)-4,5-dihydro-1H-pyrazol-3-yl]-1H-quinolin-2-one (g.2).

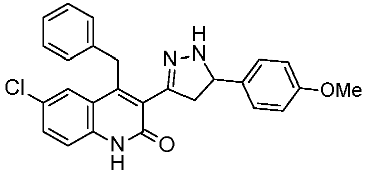

In appropriately sized microwaveable vessel, f.2 (1.6 g, 3.72 mmol) was suspended in ethanol (5.0 ml), then hydrazine hydrate (308 µL, 6.32 mmol) added, the reaction mixture microwaved at 110 °C (200 W) for 25 minutes, cooled at room temperature and resulting yellow solid filtered to yield title compound (1.385 g, yield 84%). ¹H NMR(400 MHz, DMSO-*d*₆) δ 12.01 (s, 1H), 7.65 (d, J= 2.0 Hz, 1H), 7.49 (dd, J= 2.0, 8.8 Hz, 1H), 7.41 (d, J= 3.2 Hz, 1H), 7.34- 7.25 (m, 7H), 7.20-7.15 (m, 1H), 6.85 (d, J= 8.4 Hz, 2H), 4.74 (dd, J= 4.0, 12.0 Hz, 1H), 4.44 (d, J=16.0 Hz, 1H), 4.40 (d, J=16.0 Hz, 1H), 3.72 (s, 3H), 3.34-3.27 (m, 1H), 2.84 (dd, J=8.0, 16.0 Hz, 1H).

### Step 4. Synthesis of 3-[2-acetyl-3-(4-methoxyphenyl)-3,4-dihydropyrazol-5-yl]-4-benzyl-6-chloro-1H-quinolin-2-one(d.52).

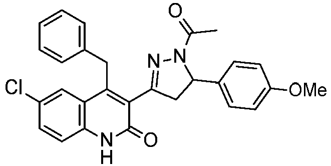

Compound d.52 was synthesized following the general procedure D1 using g.1 (300 mg, 0.69 mmol) with acetic anhydride (85 µL, 0.90 mmol) at 165 °C for 20 minutes. Purification by normal phase flash column chromatography (solvent A: DCM, solvent B: MeOH - gradient: 0-20% solvent B) yielded title compound as a white solid (245 mg, yield 75%). ¹H NMR(400 MHz, DMSO-*d₆*) δ 12.20 (s, 1H), 7.73 (d, J = 2.2 Hz, 1H), 7.56 (dd, J = 8.8, 2.2 Hz, 1H), 7.38 (d, J = 8.8 Hz, 1H), 7.28 (d, J = 4.4 Hz, 4H), 7.19 (dd, J = 9.0, 4.8 Hz, 1H), 7.15 (d, J = 8.7 Hz, 2H), 6.83 (d, J = 8.7 Hz, 2H), 5.40 (dd, J = 11.9, 4.6 Hz, 1H), 4.44 (d, J = 15.5 Hz, 1H), 4.39 (d, J = 15.5 Hz, 1H), 3.78 - 3.65 (m, 4H), 3.12 (dd, J = 18.3, 4.6 Hz, 1H), 1.95 (s, 3H). Rt 2.36 min (generic method) . ESI-MS for C₂₈H₂₄ClN₃O₃: calculated 485.1, found m/z 486.2 [M+H]⁺, 484.3 [M-H]⁻.

### Example d.53. 4-benzyl-6-chloro-3-[3-(4-methoxyphenyl)-2-propanoyl-3,4-dihydropyrazol-5-yl]-1H-quinolin-2-one

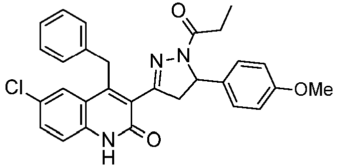

Compound d.53 was synthesized following the general procedure D2 using g.1 (450 mg, 1.01 mmol). Purification by normal phase flash column chromatography (solvent A: DCM, solvent B: MeOH - gradient: 0-1.5 % solvent B) yielded title compound as a yellow solid(260 mg, yield 51%). ¹H NMR(400 MHz, DMSO-*d₆*) δ 12.20 (s, 1H), 7.72 (d, J = 1.9 Hz, 1H), 7.56 (dd, J = 8.8, 1.9 Hz, 1H), 7.38 (d, J = 8.8 Hz, 1H), 7.28 (d, J = 4.1 Hz, 3H), 7.24 - 7.17 (m, 2H), 7.15 (d, J = 8.6 Hz, 2H), 6.84 (d, J = 8.5 Hz, 2H), 5.40 (dd, J = 11.8, 4.5 Hz, 1H), 4.46 (d, J = 15.6 Hz, 1H), 4.39 (d, J = 15.6 Hz, 1H), 3.81 - 3.63 (m, 4H), 3.12 (dd, J = 18.3, 4.5 Hz, 1H), 2.39 - 2.21 (m, 2H), 0.83 (t, J = 7.5 Hz, 3H). Rt 2.42 min (generic method). ESI-MS for C₂₉H₂₆ClN₃O₃: calculated 499.2, found *m*/*z* 500.3 [M+H]⁺, 498.4 [M-H]⁻.

### Example d.54. 4-benzyl-6-chloro-3-[3-(4-fluorophenyl)-2-propanoyl-3,4-dihydropyrazol-5-yl]-1H-quinolin-2-one

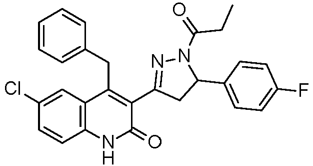

Compound d.54 was synthesized following the general procedure D2 using g.1 (100 mg, 0.23 mmol). Purification by normal phase flash column chromatography (solvent A: DCM, solvent B: MeOH - gradient: 2-3 % solvent B) yielded title compound as a solid (80 mg., yield 70%). ¹H NMR(400 MHz, DMSO-*d₆*) δ 12.20 (s, 1H), 7.73 (d, J = 2.3 Hz, 1H), 7.56 (dd, J = 8.8, 2.2 Hz, 1H), 7.38 (d, J = 8.8 Hz, 1H), 7.33 - 7.21 (m, 6H), 7.25 - 7.16 (m, 1H), 7.19 - 7.06 (m, 2H), 5.46 (dd, J = 11.9, 4.8 Hz, 1H), 4.49 - 4.35 (m, 2H), 3.73 (dd, J = 18.3, 12.0 Hz, 1H), 3.13 (dd, J = 18.3, 4.8 Hz, 1H), 2.40 - 2.27 (m, 2H), 0.84 (t, J = 7.5 Hz, 3H). Rt 2.46 min (generic method) . ESI-MS for C₂₈H₂₃ClFN₃O₂: calculated 487.1, found *m*/*z* 488.2 /490.2 [M+H]⁺.

### Example d.55. 4-[5-(4-benzyl-6-chloro-2-oxo-1H-quinolin-3-yl)-3-(4-chlorophenyl)-3,4-dihydropyrazol-2-yl]-4-oxo-butanoic acid

### Step 1. Synthesis of 4-benzyl-6-chloro-3-[(E)-3-(4-chlorophenyl)prop-2-enoyl]-1H-quinolin-2-one (f.3)

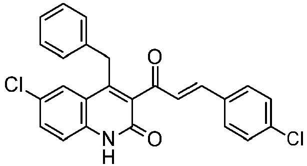

In a round-bottom flask, e.1 (380 mg, 1.22 mmol) was suspended in a 4:3 mixture of ethanol (13.9 ml) and water (10.5 ml). The mixture was cooled to 0 °C and potassium hydroxide (1.71 g, 30.5 mmol) was added. After stirring for 45 minutes at 0 °C, 4-chlorobenzaldehyde (309 mg, 2.20 mmol) was added. The reaction was stirred at room temperature for 16 hours, afterwards acetic acid (1.76 mL, 30.5 mmol) was added at 0 °C, resulting solid filtered, washed with ethanol (20 mL), the solid was dissolved in CH₂Cl₂ (50 mL), washed with water (3 x 50 mL) and brine, concentrated to dryness in vacuum to finally yield the title compound as a yellow solid (458 mg, yield 86 %). ¹H NMR(400 MHz, DMSO-*d₆*) is 12.28 (s, 1H), 7.75 (d, J = 8.6 Hz, 2H), 7.64 (d, J = 2.3 Hz, 1H), 7.60 - 7.51 (m, 2H), 7.47 (d, J = 8.6 Hz, 2H), 7.39 (d, J = 8.8 Hz, 1H), 7.30 - 7.20 (m, 4H), 7.18 - 7.05 (m, 2H), 4.12 (s, 2H).Rt 2.53 min (generic method) .ESI-MS for C₂₅H₁₇Cl₂NO₂: calculated 433.1, found m/z 434.1/436.0/438.1.

### Step 2. Synthesis of 4-benzyl-6-chloro-3-[5-(4-chlorophenyl)-4,5-dihydro-1H-pyrazol-3-yl]-1H-quinolin-2-one (g.3).

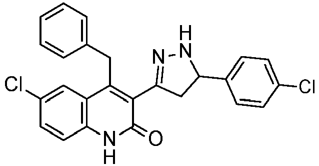

In appropriately sized microwaveable vessel, f.3 (442 mg, 1.02 mmol) was suspended in ethanol (4 ml), then hydrazine hydrate (84 µL, 1.73 mmol) added, the reaction mixture microwaved at 110 °C (200 W) for 25 minutes, cooled at room temperature and resulting solid filtered to yield title compound as a yellow solid (361 mg, yield 79%). ¹H NMR(400 MHz, DMSO-*d₆*) δ 12.04 (s, 1H), 7.66 (d, J = 2.3 Hz, 1H), 7.54 (d, J = 3.5 Hz, 1H), 7.49 (dd, J = 8.8, 2.2 Hz, 1H), 7.41 (d, J = 8.6 Hz, 2H), 7.37 - 7.30 (m, 3H), 7.30 - 7.22 (m, 4H), 7.21 - 7.12 (m, 1H), 4.80 (td, J = 10.7, 3.6 Hz, 1H), 4.51 - 4.35 (m, 2H), 3.36 (dd, J = 16.4, 10.6 Hz, 1H), 2.84 (dd, J = 16.3, 10.7 Hz, 1H). ESI-MS for C₂₅H₁₉Cl₂N₃O: calculated 447.1, found m/z 448.1/450.0/452.1 [M+H]⁺.

### Step 3. Synthesis of 4-[5-(4-benzyl-6-chloro-2-oxo-1H-quinolin-3-yl)-3-(4-chlorophenyl)-3,4-dihydropyrazol-2-yl]-4-oxo-butanoic acid (d.55).

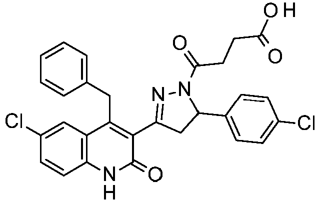

Compound d.55 was synthesized following the general procedure D1 using g.3 (100 mg, 0.22 mmol) with succinic anhydride (165 °C, 200 W). Purification by normal phase flash column chromatography (solvent A: DCM, solvent B: MeOH - gradient: 0-5 % solvent B) yielded title compound (71 mg, yield 63 %). ¹H NMR (400 MHz, DMSO-*d*₆) δ 12.21 (s, 1H), 12.09 (s, 1H), 7.77 (d, J = 2.3 Hz, 1H), 7.56 (dd, J = 8.8, 2.2 Hz, 1H), 7.38 (d, J = 8.8 Hz, 1H), 7.32 (d, J = 8.5 Hz, 2H) , 7.28 - 7.21 (m, 6H), 7.20 - 7.15 (m, 1H), 5.45 (dd, J = 12.0, 4.9 Hz, 1H), 4.47 (d, J = 15.6 Hz, 1H), 4.39 (d, J = 15.6 Hz, 1H), 3.71 (dd, J = 18.4, 12.0 Hz, 1H), 3.08 (dd, J = 18.4, 4.9 Hz, 1H), 2.73 - 2.54 (m, 2H), 2.38 - 2.27 (m, 2H). Rt 2.09 min (generic method). ESI-MS for C₂₉H₂₃Cl₂N₃O₄: calculated 547.1, found m/z 548.1/550.1/552.1 [M+H]⁺.

### Example d.56. 3-[2-acetyl-3-(4-chlorophenyl)-3,4-dihydropyrazol-5-yl]-4-benzyl-6-chloro-1H-quinolin-2-one

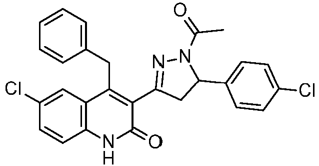

Compound d.56 was synthesized following the general procedure D1 using g.3 (100 mg, 0.22 mmol) with anhydrous THF (0.6 mL) and acetic anhydride (42 µL, 0.44 mmol) at 165 °C for 20 minutes. The THF was removed under vacuum, and the crude dissolved in CH₂Cl₂, washed three times with aqueous HCl (1 M, pH 2) and brine. The organic phase was dried over Na2SO4 and evaporated in vacuo. Purification by normal phase flash column chromatography (solvent A: DCM, solvent B: MeOH - gradient: 0-5 % solvent B) yielded title compound as a white solid (95 mg, yield 88%). ¹H NMR(400 MHz, DMSO-*d₆*) δ 12.21 (s, 1H), 7.74 (d, J = 2.3 Hz, 1H), 7.55 (dd, J = 8.8, 2.2 Hz, 1H), 7.40 - 7.32 (m, 3H), 7.31 - 7.23 (m, 6H), 7.23 - 7.16 (m, 1H), 5.46 (dd, J = 12.0, 4.8 Hz, 1H), 4.45 (d, J = 15.6 Hz, 1H), 4.39 (d, J = 15.6 Hz, 1H), 3.74 (dd, J = 18.4, 12.0 Hz, 1H), 3.12 (dd, J = 18.3, 4.9 Hz, 1H), 1.97 (s, 3H). Rt 1.29 min (apolar method) . ESI-MS for C₂₇H₂₁Cl₂N₃O₂: calculated 489.1, found m/z 490.1/492.2/494.1 [M+H]⁺.

### Example d.57. 4-benzyl-6-chloro-3-[3-(4-chlorophenyl)-2-propanoyl-3,4-dihydropyrazol-5-yl]-1H-quinolin-2-one

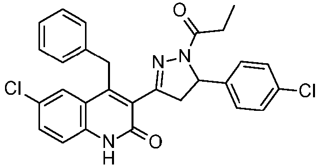

Compound d.57 was synthesized following the general procedure D2 using g.3 (100 mg, 0.22 mmol). Purification by normal phase flash column chromatography (solvent A: DCM, solvent B: MeOH - gradient: 0-5 % solvent B) yielded title compound as a yellow solid (55 mg, yield 50%). ¹H NMR(400 MHz, DMSO-*d₆*) δ 12.21 (s, 1H), 7.74 (d, J = 2.3 Hz, 1H), 7.57 (dd, J = 8.8, 2.2 Hz, 1H), 7.42 - 7.33 (m, 3H), 7.32 - 7.17 (m, 7H), 5.46 (dd, J = 12.0, 4.9 Hz, 1H), 4.50 - 4.33 (m, 2H), 3.74 (dd, J = 18.3, 12.0 Hz, 1H), 3.13 (dd, J = 18.3, 4.9 Hz, 1H), 2.43 - 2.24 (m, J = 7.8 Hz, 2H), 0.85 (t, J = 7.5 Hz, 3H). Rt 2.53 min (generic method). ESI-MS for C₂₈H₂₃Cl₂N₃O₂: calculated 503.1, found m/z 504.1/506.0/508.0 [M+H]⁺.

### Example d.58. 4-[5-(4-benzyl-6-chloro-2-oxo-1H-quinolin-3-yl)-3-(4-bromophenyl)-3,4-dihydropyrazol-2-yl]-4-oxo-butanoic acid

### Step 1. Synthesis of 4-benzyl-3-[(E)-3-(4-bromophenyl)prop-2-enoyl]-6-chloro-1H-quinolin-2-one (f.4)

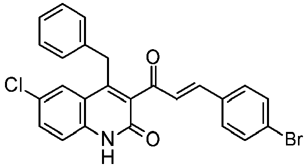

In a round-bottom flask, e.1 (330 mg, 1.10 mmol) was suspended in a 4:3 mixture of ethanol (12.6 ml) and water (9.4 ml). The mixture was cooled to 0 °C and potassium hydroxide (1.54 g, 27.5 mmol) was added. After stirring for 45 minutes at 0 °C, 4-bromobenzaldehyde (366 mg, 1.98 mmol) was added. The reaction was stirred at room temperature for 16 hours, afterwards acetic acid (1.59 mL, 27.5 mmol) was added at 0 °C, resulting solid was dissolved in CH₂Cl₂ (50 mL), washed with water (3 x 50 mL) and brine. The solvent was evaporated in vacuo to yield the title compound as an orange solid (438 mg, yield 83%). ¹H NMR(400 MHz, DMSO-*d₆*) δ 12.22 (s, 1H), 7.70 - 7.59 (m, 5H), 7.59 - 7.52 (m, 2H), 7.39 (d, J = 8.8 Hz, 1H), 7.30 - 7.22 (m, 4H), 7.18 - 7.07 (m, 2H), 4.12 (s, 2H). Rt 2.57 min (generic method) .ESI-MS for C₂₅H₁₇BrClNO₂: calculated 477.0, found m/z 478.0/480.0/482.0 [M+H]⁺, 476.3/478.1/480.1 [M-H]⁻.

### Step 2. Synthesis of 4-benzyl-3-[5-(4-bromophenyl)-4,5-dihydro-1H-pyrazol-3-yl]-6-chloro-1H-quinolin-2-one (g.4).

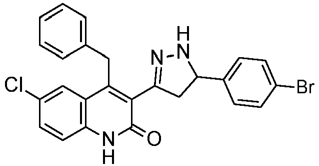

In appropriately sized microwaveable vessel, f.4 (424 mg, 0.89 mmol) was suspended in ethanol (4 ml), then hydrazine hydrate (73 µL, 1.51 mmol) added, the reaction mixture microwaved at 110 °C (200 W) for 25 minutes, cooled at room temperature and resulting yellow solid filtered to yield title compound (310 mg, yield 71%). ¹H NMR(400 MHz, DMSO-*d₆*) δ 11.99 (s, 1H), 7.66 (d, J = 2.3 Hz, 1H), 7.54 (d, J = 3.5 Hz, 1H), 7.54 - 7.46 (m, 3H), 7.37 - 7.31 (m, 3H), 7.26 (d, J = 5.3 Hz, 4H), 7.20 - 7.12 (m, 1H), 4.79 (td, J = 10.7, 3.5 Hz, 1H), 4.48 - 4.35 (m, 2H), 3.42 - 3.26 (m, 1H), 2.83 (dd, J = 16.4, 10.6 Hz, 1H). Rt 2.47 min (generic method) . ESI-MS for C₂₅H₁₉BrClN₃O: calculated 491.0, found m/z 492.0/494.0/496.1 [M+H]⁺.

### Step 3. Synthesis of 4-[5-(4-benzyl-6-chloro-2-oxo-1H-quinolin-3-yl)-3-(4-bromophenyl)-3,4-dihydropyrazol-2-yl]-4-oxo-butanoic acid (d.58).

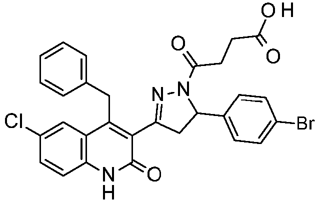

Compound d.58 was synthesized following the general procedure D1 using g.4 (100 mg, 0.2 mmol) with succinic anhydride (165 °C, 200 W). Purification by normal phase flash column chromatography (solvent A: DCM, solvent B: MeOH - gradient: 0-5% solvent B) yielded title compound (53 mg, yield 45%). ¹H NMR(400 MHz, DMSO-*d*₆) δ 12.22 (s, 2H), 7.77 (d, J = 2.3 Hz, 1H), 7.56 (dd, J = 8.8, 2.2 Hz, 1H), 7.46 (d, J = 8.4 Hz, 2H), 7.38 (d, J = 8.8 Hz, 1H), 7.30 - 7.23 (m, 4H), 7.21 - 7.13 (m, 3H), 5.43 (dd, J = 12.0, 4.9 Hz, 1H), 4.47 (d, J = 15.6 Hz, 1H), 4.39 (d, J = 15.5 Hz, 1H), 3.71 (dd, J = 18.3, 12.0 Hz, 1H), 3.08 (dd, J = 18.3, 4.9 Hz, 1H), 2.74 - 2.53 (m, 2H), 2.33 (t, J = 6.7 Hz, 2H). Rt 2.11 min (generic method). ESI-MS for C₂₉H₂₃BrClN₃O₄: calculated 591.1, found m/z 592.1/594.0.

### Example d.59. 3-[2-acetyl-3-(4-bromophenyl)-3,4-dihydropyrazol-5-yl]-4-benzyl-6-chloro-1H-quinolin-2-one

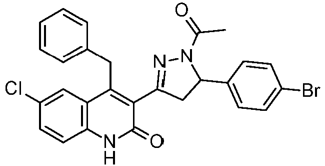

Compound d.59 was synthesized following the general procedure D1 using g.4 (86 mg, 0.17 mmol) with acetic anhydride (33 µL, 0.35 mmol) at 165 °C for 20 minutes. The THF was removed under vacuum, and the crude dissolved in CH₂Cl₂ (50 mL), washed (3 x 50 mL) with aqueous HCl (1 M, pH 2) and brine. The organic phase was dried over Na₂SO₄, evaporated in vacuo and the resulting solid triturated with Et₂O (10 mL) to yield the title compound as a white solid. (65 mg, yield 71%). ¹H NMR(400 MHz, DMSO-*d₆*) δ 12.21 (s, 1H), 7.74 (d, J = 2.3 Hz, 1H), 7.55 (dd, J = 8.8, 2.3 Hz, 1H), 7.48 (d, J = 8.4 Hz, 2H), 7.38 (d, J = 8.8 Hz, 1H), 7.30 - 7.23 (m, 4H), 7.23 - 7.14 (m, 3H), 5.44 (dd, J = 12.0, 4.9 Hz, 1H), 4.49 - 4.34 (m, 2H), 3.73 (dd, J = 18.3, 12.0 Hz, 1H), 3.12 (dd, J = 18.3, 4.9 Hz, 1H), 1.97 (s, 3H). Rt 2.52 min (generic method) . ESI-MS for C₂₇H₂₁BrClN₃O: calculated 533.1, found m/z 534.1/536.2/538.1 [M+H]⁺.

### Example d.60. 4-benzyl-3-[3-(4-bromophenyl)-2-propanoyl-3,4-dihydropyrazol-5-yl]-6-chloro-1H-quinolin-2-one

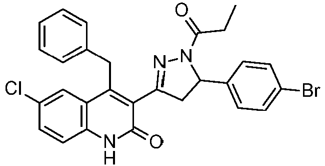

Compound d.60 was synthesized following the general procedure D2 using g.4 (97 mg, 0.20 mmol). Purification by normal phase flash column chromatography (solvent A: DCM, solvent B: MeOH - gradient: 0-5 % solvent B) yielded title compound as a yellow solid (53 mg, yield 48%). ¹H NMR(400 MHz, DMSO-*d₆*) δ 12.20 (s, 1H), 7.73 (d, J = 2.3 Hz, 1H), 7.56 (dd, J = 8.8, 2.2 Hz, 1H), 7.48 (d, J = 8.4 Hz, 2H), 7.38 (d, J = 8.8 Hz, 1H), 7.32 - 7.24 (m, 4H), 7.24 - 7.13 (m, 3H), 5.44 (dd, J = 12.0, 4.9 Hz, 1H), 4.49 - 4.35 (m, 2H), 3.73 (dd, J = 18.3, 12.0 Hz, 1H), 3.12 (dd, J = 18.3, 4.9 Hz, 1H), 2.43 - 2.23 (m, 2H), 0.84 (t, J = 7.5 Hz, 3H). Rt 1.53 min (apolar method) . ESI-MS for C₂₈H₂₃BrClN₃O₂: calculated 547.1, found m/z 548.0 /550.0/552.0 [M+H]⁺.

### Example d.61. 4-[5-(4-benzyl-6-chloro-2-oxo-1H-quinolin-3-yl)-3-(4-methoxyphenyl)-3,4-dihydropyrazol-2-yl]-4-oxo-butanoic acid

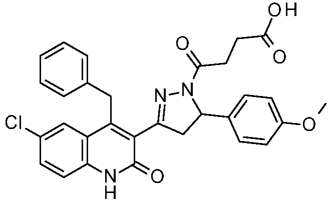

Compound d.61 was synthesized following the general procedure D1 using g.2 (104 mg, 0.23 mmol) with succinic anhydride (165 °C, 200 W). THF was evaporated and the resulted solid was triturated in MeOH (10 mL), CH₂Cl₂ (10 mL), cold CHCl₃ (10 mL), Et₂O (10 mL), cyclohexane (10 mL), and petroleum ether (10 mL) to obtain the title product as an off-white solid (82 mg, yield 66 %). ¹H NMR(400 MHz, DMSO-*d*₆) δ 12.20 (s, 1H), 12.00 (s, 1H), 7.76 (d, J = 2.3 Hz, 1H), 7.56 (dd, J = 8.8, 2.2 Hz, 1H), 7.38 (d, J = 8.8 Hz, 1H), 7.31 - 7.23 (m, 4H), 7.21 - 7.16 (m, 1H), 7.14 (d, J = 8.7 Hz, 2H) , 6.81 (d, J = 8.7 Hz, 2H), 5.39 (dd, J = 11.8, 4.7 Hz, 1H), 4.47 (d, J = 15.5 Hz, 1H), 4.39 (d, J = 15.5 Hz, 1H), 3.72 (s, 3H), 3.69 - 3.61 (m, 1H), 3.08 (dd, J = 18.3, 4.7 Hz, 1H), 2.70 - 2.56 (m, 2H), 2.31 (t, J = 6.7 Hz, 2H). Rt 1.97 min (Generic method). ESI-MS for C₃₀H₂₆ClN₃O₅: calculated 543.2, found m/z 544.2/546.2 [M+H]⁺.

### Example d.62. 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid

### Step 1. Synthesis of 4-benzyl-6-chloro-3-[(E)-3-[4-(trifluoromethyl)phenyl]prop-2-enoyl]-1H-quinolin-2-one (f.5)

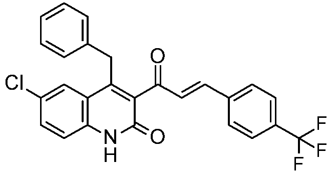

In a round-bottom flask, e.1 (350 mg, 1.12 mmol) was suspended in a 4:3 mixture of ethanol (12.8 ml) and water (9.6 ml). The mixture was cooled to 0 °C and potassium hydroxide (1.573 g, 28.1 mmol) was added. After stirring for 45 minutes at 0 °C, 4-(trifluoromethyl)benzaldehyde (275 µL, 2.02 mmol) was added. The reaction was stirred at room temperature for 16 hours, afterwards acetic acid (1.62 mL, 28.1 mmol) was added at 0 °C, resulting yellow solid filtered The solid was dissolved in CH₂Cl₂ (50 mL), washed with water (50 mL) and brine (50 mL). The solvent was evaporated in vacuo to yield the title compound as a yellow solid. (453 mg, yield 86%). ¹H NMR (400 MHz, DMSO-*d₆*) δ 12.25 (s, 1H), 7.94 (d, J = 8.1 Hz, 2H), 7.76 (d, J = 8.2 Hz, 2H), 7.70 - 7.61 (m, 2H), 7.57 (dd, J = 8.8, 2.3 Hz, 1H), 7.40 (d, J = 8.8 Hz, 1H), 7.31 - 7.19 (m, 5H), 7.17 - 7.11 (m, 1H), , 4.14 (s, 2H). Rt 2.55 min (generic method).ESI-MS for C₂₆H₁₇ClF₃NO₂: calculated 467.1, found m/z 468.1/470.1 [M+H]⁺.

### Step 2. Synthesis of 4-benzyl-6-chloro-3-[5-[4-(trifluoromethyl)phenyl]-4,5-dihydro-1H-pyrazol-3-yl]-1H-quinolin-2-one (g.5).

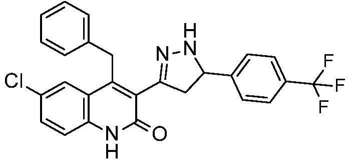

In appropriately sized microwaveable vessel, f.5 (430 mg, 0.92 mmol) was suspended in ethanol (5 ml), then hydrazine hydrate (76 µL, 1.56 mmol) added, the reaction mixture microwaved at 110 °C (200 W) for 20 minutes, cooled at room temperature and the solvent evaporated in vacuo. The crude was diluted with CHCl₃ (50 mL), washed with H₂O (50 mL) and brine (50 mL), dried over Na₂SO₄ and the solvent removed under pressure to yield the title compound as a yellow solid (321 mg, yield 72%). ¹H NMR (400 MHz, DMSO-*d₆*) δ 12.07 (s, 1H), 7.72 - 7.58 (m, 6H), 7.50 (dd, J = 8.7, 2.3 Hz, 1H), 7.33 (d, J = 8.8 Hz, 1H), 7.30 - 7.23 (m, 4H), 7.20 - 7.13

(m, 1H), 4.90 (td, J = 10.7, 3.6 Hz, 1H), 4.51 - 4.37 (m, 2H), 3.43 (dd, J = 16.4, 10.7 Hz, 1H), 2.87 (dd, J = 16.4, 10.6 Hz, 1H). Rt 2.46 min (generic method). ESI-MS for C₂₆H₁₉ClF₃N₃O: calculated 481.1, found m/z 482.2/484.1 [M+H]⁺.

### Step 3. Synthesis of 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid (d.62).

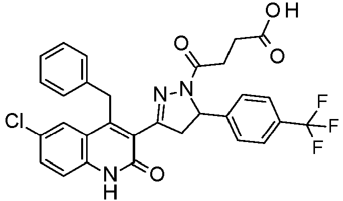

Compound d.62 was synthesized following the general procedure D1 using g.5 (168 mg, 0.35 mmol) with succinic anhydride (120 °C, 200 W). Purification by normal phase flash column chromatography (solvent A: CH₂Cl₂, solvent B: MeOH - gradient: 0-5% solvent B) yielded title compound as a yellow-orange solid (65 mg, yield 32%). ¹H NMR(400 MHz, DMSO-*d*₆) δ 12.22 (s, 1H), 12.06 (s, 1H), 7.78 (d, J = 2.3 Hz, 1H), 7.64 (d, J = 8.1 Hz, 2H), 7.57 (dd, J = 8.8, 2.3 Hz, 1H), 7.42 (d, J = 8.1 Hz, 2H), 7.38 (d, J = 8.8 Hz, 1H), 7.29 - 7.23 (m, 4H), 7.21 - 7.14 (m, 1H), 5.55 (dd, J = 12.1, 5.0 Hz, 1H), 4.49 (d, J = 15.6 Hz, 1H), 4.41 (d, J = 15.6 Hz, 1H), 3.76 (dd, J = 18.4, 12.1 Hz, 1H), 3.09 (dd, J = 18.4, 5.1 Hz, 1H), 2.81 - 2.53 (m, 2H), 2.40 - 2.29 (m, 2H). Rt 1.08 min (apolar method). ESI-MS for C₃₀H₂₃ClF₃N₃O: calculated 581.1, found m/z 582.2/584.2 [M+H]⁺.

### Example d.63. 3-[2-acetyl-3-[4-(trifluoromethyl)phenyl]-3,4-dihydropyrazol-5-yl]-4-benzyl-6-chloro-1H-quinolin-2-one

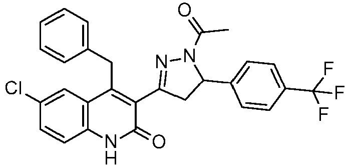

Compound d.63 was synthesized following the general procedure D1 using g.5 (136 mg, 0.28 mmol) with acetic anhydride (53 µL, 0.56 mmol) at 165 °C for 20 minutes. Purification by normal phase flash column chromatography (solvent A: DCM, solvent B: MeOH - gradient: 0-2 % solvent B) yielded title compound as a yellow solid (74 mg, yield 50%). ¹H NMR(400 MHz, DMSO-*d₆*) δ 12.21 (s, 1H), 7.75 (d, J = 2.3 Hz, 1H), 7.66 (d, J = 8.1 Hz, 2H), 7.56 (dd, J = 8.8, 2.2 Hz, 1H), 7.44 (d, J = 8.1 Hz, 2H), 7.38 (d, J = 8.8 Hz, 1H), 7.30 - 7.25 (m, 4H), 7.21 - 7.15 (m, 1H), 5.55 (dd, J = 12.1, 5.0 Hz, 1H), 4.47 (d, J = 15.6 Hz, 1H), 4.40 (d, J = 15.6 Hz, 1H), 3.78 (dd, J = 18.4, 12.1 Hz, 1H), 3.14 (dd, J = 18.3, 5.0 Hz, 1H), 1.99 (s, 3H). Rt 1.50 min (apolar method) . ESI-MS for C₂₈H₂₁ClF₃N₃O₂: calculated 523.1, found m/z 524.2/526.2 [M+H]⁺.

### Example d.65. 3-[2-acetyl-3-(4-tert-butylphenyl)-3,4-dihydropyrazol-5-yl]-4-benzyl-6-chloro-1H-quinolin-2-one

### Step 1. Synthesis of 4-benzyl-3-[(E)-3-(4-tert-butylphenyl)prop-2-enoyl]-6-chloro-1H-quinolin-2-one (f.6)

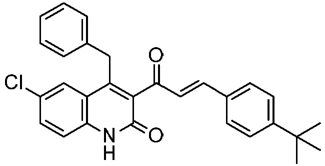

In a round-bottom flask, e.1 (150 mg, 0.48 mmol) was suspended in a 4:3 mixture of ethanol (5.5 ml) and water (4.1 ml). The mixture was cooled to 0 °C and potassium hydroxide (673 mg, 12 mmol) was added. After stirring for 45 minutes at 0 °C, 4-tert-butylbenzaldehyde (144 µL, 0.86 mmol) was added. The reaction was stirred at room temperature for 16 hours, afterwards acetic acid (692 µL, 12 mmol) was added at 0 °C, resulting in a precipitate that was filtered and washed with ethanol (20 mL) and water (20 mL). The solid was dissolved in CH₂Cl₂ (50 mL), washed with water (50 mL) and brine (50 mL). The solvent was evaporated in vacuo to yield the title compound as a yellow solid (121 mg, yield 55%). ¹H NMR(400 MHz, DMSO-*d₆*) δ 12.21 (s, 1H), 7.67 - 7.61 (m, 3H), 7.59 - 7.51 (m, 2H), 7.45 - 7.36 (m, 3H), 7.31 - 7.23 (m, 4H), 7.19 - 7.13 (m, 1H), 7.03 (d, J = 16.3 Hz, 1H), 4.13 (s, 2H), 1.28 (s, 9H). Rₜ 2.04 min (apolar method) .ESI-MS for C₂₉H₂₆ClNO₂: calculated 455.17, found m/z 456.3/458.2 [M+H]⁺.

### Step 2. Synthesis of 4-benzyl-3-[5-(4-tert-butylphenyl)-4,5-dihydro-1H-pyrazol-3-yl]-6-chloro-1H-quinolin-2-one (g.6).

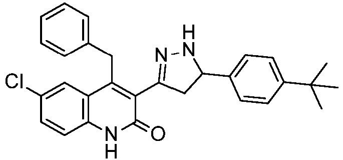

In appropriately sized microwaveable vessel, f.6 (111 mg, 0.24 mmol) was suspended in ethanol (0.5 ml), then hydrazine hydrate (20 µL, 0.41 mmol) added, the reaction mixture microwaved at 110 °C (200 W) for 20 minutes, cooled at room temperature and resulting yellow solid filtered to yield title compound (83 mg, yield 74%). ¹H NMR(400 MHz, DMSO-*d₆*) δ 12.03 (s, 1H), 7.65 (d, J = 2.3 Hz, 1H), 7.49 (dd, J = 8.8, 2.3 Hz, 1H), 7.44 (d, J = 3.3 Hz, 1H), 7.33 (d, J = 8.8 Hz, 1H), 7.31 - 7.25 (m, 8H), 7.21 - 7.14 (m, 1H), 4.75 (td, J = 10.6, 3.3 Hz, 1H), 4.47 (d, J = 15.3 Hz, 1H), 4.40 (d, J = 15.2 Hz, 1H), 3.38 - 3.33 (m, 1H), 2.87 (dd, J = 16.3, 10.6 Hz, 1H), 1.26 (s, 9H). Rt 1.97 min (apolar method). ESI-MS for C₂₉H₂₈ClN₃O: calculated 469.2, found m/z 470.2/472.3 [M+H]⁺.

### Step 3. Synthesis of 3-[2-acetyl-3-(4-tert-butylphenyl)-3,4-dihydropyrazol-5-yl]-4-benzyl-6-chloro-1H-quinolin-2-one (d.65).

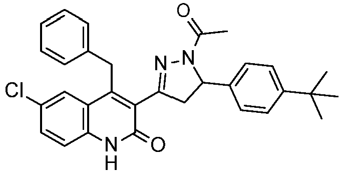

Compound d.65 was synthesized following the general procedure D1 using g.6 (111 mg, 0.24 mmol) with acetic anhydride (29 µL, 0.30 mmol) at 165 °C for 20 minutes. Purification by normal phase flash column chromatography (solvent A: DCM, solvent B: MeOH - gradient: 0-5 % solvent B) yielded title compound as a yellow solid (68 mg, yield 78%). ¹H NMR(400 MHz, DMSO-*d₆*) δ 12.19 (s, 1H), 7.73 (d, J = 2.3 Hz, 1H), 7.56 (dd, J = 8.8, 2.2 Hz, 1H), 7.38 (d, J = 8.8 Hz, 1H), 7.32 - 7.25 (m, 6H), 7.23 - 7.16 (m, 1H), 7.13 (d, J = 8.3 Hz, 2H), 5.41 (dd, J = 11.9, 4.7 Hz, 1H), 4.46 (d, J = 15.6 Hz, 1H), 4.39 (d, J = 15.5 Hz, 1H), 3.73 (dd, J = 18.3, 12.0 Hz, 1H), 3.11 (dd, J = 18.3, 4.7 Hz, 1H), 1.97 (s, 3H), 1.25 (s, 9H). Rt 1.87 min (apolar method). ESI-MS for C₃₁H₃₀ClN₃O₂: calculated 511.2, found m/z 512.1/514.2 [M+H]⁺.

### Example d.66. 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid

### Step 1. Synthesis of 4-benzyl-6-chloro-3-[(E)-3-(3-fluoro-4-methoxy-phenyl)prop-2-enoyl]-1H-quinolin-2-one (f.7)

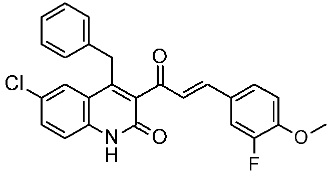

In a round-bottom flask, e.1 (157 mg, 0.5 mmol) was suspended in a 4:3 mixture of ethanol (5.7 ml) and water (4.3 ml). The mixture was cooled to 0 °C and potassium hydroxide (706 mg, 12.6 mmol) was added. After stirring for 45 minutes at 0 °C, 3-fluoro-4-methoxybenzaldehyde (139 mg, 0.9 mmol) was added. The reaction was stirred at room temperature for 16 hours, afterwards acetic acid (0.726 mL, 12.6 mmol) was added at 0 °C, and washed with ethanol (20 mL). The solid was diluted in CH₂Cl₂ (50 mL), washed with water and brine. The solvent was evaporated in vacuo to yield the title compound as a yellow solid (168 mg, yield 75 %). ¹H NMR(400 MHz, DMSO-*d₆*) δ 12.20 (s, 1H), 7.69 (dd, J = 12.7, 2.0 Hz, 1H), 7.63 (d, J = 2.3 Hz, 1H), 7.61 - 7.45 (m, 3H), 7.39 (d, J = 8.8 Hz, 1H), 7.31 - 7.15 (m, 5H), 7.19 - 7.10 (m, 1H), 7.00 (d, J = 16.3 Hz, 1H), 4.11 (s, 2H), 3.88 (s, 3H) Rt 2.45 min (Generic method) .ESI-MS for C₂₆H₁₉ClFNO₃: calculated 447.1, found m/z 448.1/450.2 [M+H]⁺.

### Step 2. Synthesis of 4-benzyl-6-chloro-3-[5-(3-fluoro-4-methoxy-phenyl)-4,5-dihydro-1H-pyrazol-3-yl]-1H-quinolin-2-one (g.7).

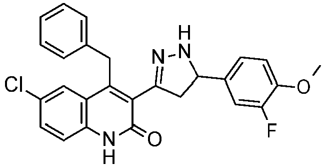

In appropriately sized microwaveable vessel, f.7 (152 mg, 0.34 mmol) was suspended in ethanol (0.7 ml), then hydrazine hydrate (28 µL, 0.58 mmol) added, the reaction mixture microwaved at 110 °C (200 W) for 20 minutes, cooled at room temperature and resulting yellow solid filtered to yield title compound (133 g, yield 85 %). ¹H NMR(400 MHz, DMSO-*d₆*) δ 12.05 (s, 1H), 7.66 (d, J = 2.3 Hz, 1H), 7.53 - 7.45 (m, 2H), 7.33 (d, J = 8.8 Hz, 1H), 7.31 - 7.21 (m, 5H), 7.21 - 7.04 (m, 3H), 4.75 (td, J = 10.4, 3.4 Hz, 1H), 4.47 - 4.36 (m, 2H), 3.81 (s, 3H), 3.36 - 3.27 (m, 1H), 2.85 (dd, J = 16.3, 10.2 Hz, 1H) Rt 2.35 min (Generic method). ESI-MS for C₂₆H₂₁ClFN₃O₂: calculated 461.1, found m/z 462.2/464.2 [M+H]⁺.

### Step 3. Synthesis of 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid (d.66).

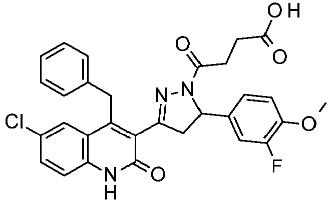

Compound d.66 was synthesized following the general procedure D1 using g.7 (119 mg, 0.26 mmol) with succinic anhydride (165 °C, 200 W). The THF was removed under vacuum, and the crude suspended in CHCl3 (10 mL), filtered and washed with MeOH (3 x 10 mL) to yield the title compound as an off-white solid (47 mg, yield 32 %). ¹H NMR(400 MHz, DMSO-*d*₆) δ 12.21 (s, 1H), 12.00 (s, 1H), 7.77 (d, J = 2.4 Hz, 1H), 7.56 (dd, J = 8.8, 2.3 Hz, 1H), 7.38 (d, J = 8.8 Hz, 1H), 7.31 - 7.22 (m, 4H), 7.22 - 7.16 (m, 1H), 7.14 - 6.98 (m, 3H), 5.41 (dd, J = 11.9, 4.6 Hz, 1H), 4.45 (d, J = 15.5 Hz, 1H), 4.38 (d, J = 15.6 Hz, 1H), 3.80 (s, 3H), 3.65 (dd, J = 18.3, 11.8 Hz, 1H), 3.11 (dd, J = 18.3, 4.7 Hz, 1H), 2.75 - 2.52 (m, 2H), 2.43 - 2.28 (m, 2H). Rt 1.96 min (Generic method). ESI-MS for C₃₀H₂₅ClFN₃O₅: calculated 561.1, found *m*/*z* 562.1/564.2 [M+H]⁺.

### Example d.69. 4-[5-(4-benzyl-6-chloro-2-oxo-1H-quinolin-3-yl)-3-(1-methylindol-5-yl)-3,4-dihydropyrazol-2-yl]-4-oxo-butanoic acid

### Step 1. Synthesis of 4-benzyl-6-chloro-3-[(E)-3-(1-methylindol-5-yl)prop-2-enoyl]-1H-quinolin-2-one (f.8)

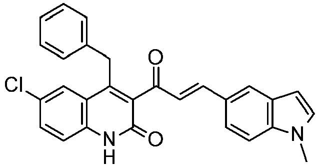

In a round-bottom flask, e.1 (350 g, 1.12 mmol) was suspended in a 4:3 mixture of ethanol (13 ml) and water (10 ml). The mixture was cooled to 0 °C and potassium hydroxide (1.571 g, 28 mmol) was added. After stirring for 45 minutes at 0 °C, 1-methyl-1H-indole-5-carbaldehyde (322 mg, 2.02 mmol) was added. The reaction was stirred at room temperature for 16 hours, afterwards acetic acid (1.614 mL, 28 mmol) was added at 0 °C, resulting in a precipitate that was filtered and washed with ethanol (20 mL) and water (20 mL). The solid was dissolved in CH₂Cl₂ (50 mL) and washed with brine (50 mL). The solvent was evaporated in vacuo to yield the title compound as an orange solid (407 g, yield 80 %). ¹H NMR(400 MHz, DMSO-*d₆*) δ 12.19 (s, 1H), 7.90 (d, J = 1.6 Hz, 1H), 7.69 - 7.62 (m, 2H), 7.59 - 7.53 (m, 2H), 7.47 (d, J = 8.7 Hz, 1H), 7.41 - 7.36 (m, 2H), 7.32 - 7.21 (m, 4H), 7.18 - 7.11 (m, 1H), 7.00 (d, J = 16.2 Hz, 1H), 6.46 (dd, J = 3.2, 0.8 Hz, 1H), 4.14 (s, 2H), 3.80 (s, 3H). Rt 2.43 min (Generic method) ESI-MS for C₂₈H₂₁ClN₂O₂: calculated 452.1, found m/z 453.1/455.1 [M+H]⁺.

### Step 2 Synthesis of 4-benzyl-6-chloro-3-[5-(1-methylindol-5-yl)-4,5-dihydro-1H-pyrazol-3-yl]-1H-quinolin-2-one (g.8).

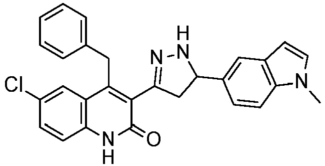

In appropriately sized microwaveable vessel, f.8 (390 g, 0.86 mmol) was suspended in ethanol (2 ml), then hydrazine hydrate (71 µL, 1.46 mmol) added, the reaction mixture microwaved at 110 °C (200 W) for 20 minutes, cooled at room temperature and resulting solid filtered and washed with ethanol (20 mL) to yield title compound as an orange solid (298 mg, yield 72 %). ¹H NMR(400 MHz, DMSO-*d₆*) δ 12.04 (s, 1H), 7.66 (d, J = 2.3 Hz, 1H), 7.56 - 7.45 (m, 2H), 7.41 (d, J = 3.4 Hz, 1H), 7.38 - 7.32 (m, 2H), 7.31 - 7.24 (m, 5H) , 7.22 - 7.13 (m, 2H), 6.33 (d, J = 3.0 Hz, 1H), 4.88 (td, J = 10.7, 3.4 Hz, 1H), 4.54 - 4.36 (m, 2H), 3.76 (s, 3H), 3.44 - 3.33 (m, 1H), 2.93 (dd, J = 16.3, 10.9 Hz, 1H). Rt 2.36 min (Generic method). ESI-MS for C₂₈H₂₃ClN₄O: calculated 466.2, found m/z 467.2 /469.3 [M+H]⁺.

### Step 3. Synthesis of 4-[5-(4-benzyl-6-chloro-2-oxo-1H-quinolin-3-yl)-3-(1-methylindol-5-yl)-3,4-dihydropyrazol-2-yl]-4-oxo-butanoic acid (d.69).

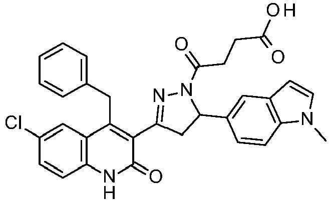

Compound d.69 was synthesized following the general procedure D1 using g.8 (150 mg, 0.32 mmol) with succinic anhydride (165 °C, 200 W). The THF was removed under vacuum, and the crude triturated in CH₂Cl₂ (10 mL) and MeOH (10 mL), filtered and washed with petroleum ether (10 mL) to obtain the title compound as a red solid (115 mg, yield 63 %). ¹H NMR(400 MHz, DMSO-*d*₆) δ 12.20 (s, 1H), 11.97 (s, 1H), 7.76 (d, J = 2.3 Hz, 1H), 7.56 (dd, J = 8.8, 2.2 Hz, 1H), 7.41 - 7.36 (m, 2H), 7.34 - 7.24 (m, 6H), 7.22 - 7.15 (m, 1H), 7.03 (dd, J = 8.5, 1.7 Hz, 1H), 6.29 (d, J = 3.0 Hz, 1H), 5.51 (dd, J = 11.9, 4.7 Hz, 1H), 4.49 (d, J = 15.5 Hz, 1H), 4.41 (d, J = 15.6 Hz, 1H), 3.79 - 3.70 (m, 4H), 3.16 (dd, J = 18.3, 4.7 Hz, 1H), 2.62 (t, J = 6.7 Hz, 2H), 2.30 (t, J = 6.7 Hz, 2H). Rt 2.08 min (Generic method). ESI-MS for C₃₂H₂₇ClN₄O₄: calculated 566.2, found *m*/*z* 567.2 /569.3 [M+H]⁺.

### Example d.70. 3-[2-acetyl-3-(1-methylindol-5-yl)-3,4-dihydropyrazol-5-yl]-4-benzyl-6-chloro-1H-quinolin-2-one

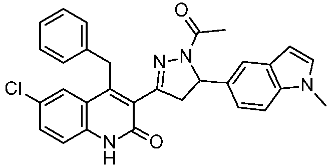

Compound d.70 was synthesized following the general procedure D1 using g.8 (135 mg, 0.29 mmol) with acetic anhydride (55 µL, 0.58 mmol) at 165 °C for 20 minutes. Purification by normal phase flash column chromatography (solvent A: DCM, solvent B: MeOH - gradient: 0-5 % solvent B) yielded title compound as a white solid (91 mg, yield 62%). ¹H NMR (400 MHz, DMSO-*d₆*) δ 12.20 (s, 1H), 7.73 (d, J = 2.3 Hz, 1H), 7.55 (dd, J = 8.8, 2.3 Hz, 1H), 7.41 - 7.35 (m, 2H), 7.35 - 7.25 (m, 6H), 7.24 - 7.15 (m, 1H), 7.04 (dd, J = 8.5, 1.7 Hz, 1H), 6.31 (dd, J = 3.1, 0.8 Hz, 1H), 5.53 (dd, J = 12.0, 4.6 Hz, 1H), 4.47 (d, J = 15.6 Hz, 1H), 4.40 (d, J = 15.5 Hz, 1H), 3.77 (dd, J = 18.3, 11.9 Hz, 1H), 3.76 (s, 3H), 3.19 (dd, J = 18.3, 4.7 Hz, 1H), 1.96 (s, 3H). Rt 2.34 min (generic method). ESI-MS for C₃₀H₂₅ClN₄O₂: calculated 508.2, found m/z 509.2/511.2 [M+H]⁺.

### Example d.73. 4-[5-(4-benzyl-6-chloro-2-oxo-1H-quinolin-3-yl)-3-(1-methylindazol-5-yl)-3,4-dihydropyrazol-2-yl]-4-oxo-butanoic acid

### Step 1. Synthesis of 4-benzyl-6-chloro-3-[(E)-3-(1-methylindazol-5-yl)prop-2-enoyl]-1H-quinolin-2-one (f.9)

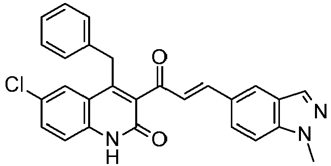

In a round-bottom flask, e.1 (300 mg, 0.96 mmol) was suspended in a 4:3 mixture of ethanol (11 ml) and water (8 ml). The mixture was cooled to 0 °C and potassium hydroxide (1.35 g, 24.06 mmol) was added. After stirring for 45 minutes at 0 °C, 1-methyl-1H-indazole-5-carbaldehyde (277 mg, 1.73 mmol) was added. The reaction was stirred at room temperature for 16 hours, afterwards acetic acid (1.387 mL, 24.06 mmol) was added at 0 °C, resulting in a precipitate that was filtered and washed with ethanol (20 mL) and water (20 mL). The solid was dissolved in CH₂Cl₂ (50 mL), washed with water (50 mL) and brine (50 mL). The solvent was evaporated in vacuo to yield the title compound as a yellow solid. (274 mg, yield 57 %). ¹H NMR(400 MHz, DMSO-*d₆*) δ 12.21 (s, 1H), 8.16 - 8.08 (m, 2H), 7.82 (dd, J = 9.0, 1.6 Hz, 1H), 7.73 - 7.62 (m, 3H), 7.56 (dd, J = 8.8, 2.3 Hz, 1H), 7.40 (d, J = 8.8 Hz, 1H), 7.33 - 7.21 (m, 4H), 7.16 - 7.12 (m, 1H), 7.08 (d, J = 16.3 Hz, 1H), 4.14 (s, 2H), 4.05 (s, 3H). Rt 2.28 min (Generic method) .ESI-MS for C₂₇H₂₀ClN₃O₂: calculated 453.1, found m/z 454.1/456.1 [M+H]⁺.

### Step 2. Synthesis of 4-benzyl-6-chloro-3-[5-(1-methylindazol-5-yl)-4,5-dihydro-1H-pyrazol-3-yl]-1H-quinolin-2-one (g.9).

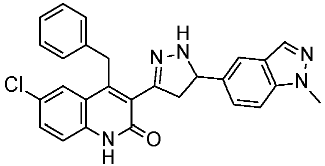

In appropriately sized microwaveable vessel, f.9 (230 mg, 0.51 mmol) was suspended in ethanol (1.1 ml), then hydrazine hydrate (42 µL, 0.86 mmol) added, the reaction mixture microwaved at 110 °C (200 W) for 20 minutes, cooled at room temperature and resulting yellow solid filtered and washed with ethanol (20 mL) to yield title compound as an off-white solid (166 mg, yield 70 %). ¹H NMR(400 MHz, DMSO-*d₆*) δ 12.00 (s, 1H), 7.94 (s, 1H), 7.73 - 7.65 (m, 2H), 7.57 (d, J = 8.7 Hz, 1H), 7.53 - 7.43 (m, 3H), 7.33 (d, J = 8.8 Hz, 1H), 7.30 - 7.23 (m, 4H), 7.20 - 7.14 (m, 1H), 4.92 (td, J = 10.6, 3.5 Hz, 1H), 4.51 - 4.39 (m, 2H), 4.02 (s, 3H), 3.38 (dd, J = 16.4, 10.6 Hz, 1H), 2.92 (dd, J = 16.3, 10.7 Hz, 1H). Rt 2.17 min (Generic method). ESI-MS for C₂₇H₂₂ClN₅O: calculated 467.2, found m/z 468.2/470.2 [M+H]⁺.

### Step 3. Synthesis of 4-[5-(4-benzyl-6-chloro-2-oxo-1H-quinolin-3-yl)-3-(1-methylindazol-5-yl)-3,4-dihydropyrazol-2-yl]-4-oxo-butanoic acid (d.73).

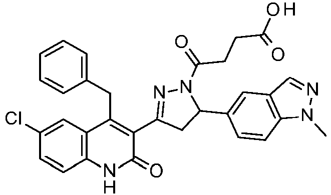

Compound d.73 was synthesized following the general procedure D1 using g.9 (75 mg, 0.16 mmol) with succinic anhydride (165 °C, 200 W). The THF was removed under vacuum, and the crude dissolved in CH₂Cl₂ (50 mL), washed three times (3 x 50 mL) with aqueous HCl (1 M, pH 2) and brine. The organic phase was dried over Na₂SO₄ and evaporated in vacuo. The crude was then precipitated in CH₂Cl₂, filtered and washed with CH₂Cl₂ (10 mL) and Et₂O (10 mL) to obtain the title product as a white solid (46 mg, yield 84 %). ¹H NMR (400 MHz, DMSO-*d*₆) δ 12.21 (s, 1H), 12.00 (s, 1H), 7.92 (d, J = 0.9 Hz, 1H), 7.77 (d, J = 2.3 Hz, 1H), 7.59 - 7.51 (m, 3H), 7.38 (d, J = 8.8 Hz, 1H), 7.33 - 7.23 (m, 5H), 7.22 - 7.15 (m, 1H), 5.56 (dd, J = 11.9, 4.8 Hz, 1H), 4.49 (d, J = 15.6 Hz, 1H), 4.41 (d, J = 15.6 Hz, 1H), 4.01 (s, 3H), 3.74 (dd, J = 18.3, 11.9 Hz, 1H), 3.16 (dd, J = 18.3, 4.8 Hz, 1H), 2.70 - 2.54 (m, 2H), 2.31 (t, J = 6.7 Hz, 2H). Rt 1.86 min (Generic method) . ESI-MS for C₃₁H₂₆ClN₅O₄ : calculated 567.2, found m/z 568.3/570.2 [M+H]⁺.

### Example d.74. 3-[2-acetyl-3-(1-methylindazol-5-yl)-3,4-dihydropyrazol-5-yl]-4-benzyl-6-chloro-1H-quinolin-2-one

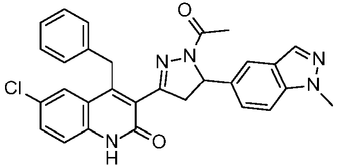

Compound d.74 was synthesized following the general procedure D1 using g.9 (79 mg, 0.17 mmol) with acetic anhydride (32 µL, 0.34 mmol) at 165 °C for 20 minutes. The white precipitate was filtered and washed with MeOH (10 mL) and Et₂O (10 mL) to yield the title compound as a white solid (63 mg, yield 73%). ¹H NMR(400 MHz, DMSO-*d₆*) δ 12.21 (s, 1H), 7.94 (d, J = 0.9 Hz, 1H), 7.74 (d, J = 2.3 Hz, 1H), 7.60 - 7.53 (m, 3H), 7.38 (d, J = 8.8 Hz, 1H), 7.33 - 7.26 (m, 5H), 7.23 - 7.16 (m, 1H), 5.57 (dd, J = 11.9, 4.7 Hz, 1H), 4.50 - 4.38 (m, 2H), 4.01 (s, 3H), 3.77 (dd, J = 18.3, 11.9 Hz, 1H), 3.20 (dd, J = 18.3, 4.7 Hz, 1H), 1.97 (s, 3H). Rt 2.14 min (generic method). ESI-MS for C₂₉H₂₄ClN₅O₂: calculated 509.2, found m/z 510.2/512.1 [M+H]⁺.

### Example d.75. 4-[5-(4-benzyl-6-chloro-2-oxo-1H-quinolin-3-yl)-3-(2-methylindazol-5-yl)-3,4-dihydropyrazol-2-yl]-4-oxo-butanoic acid

### Step 1. Synthesis of 4-benzyl-6-chloro-3-[(E)-3-(2-methylindazol-5-yl)prop-2-enoyl]-1H-quinolin-2- (f.10)

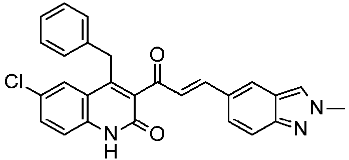

In a round-bottom flask, e.1 (130 mg, 0.42 mmol) was suspended in a 4:3 mixture of ethanol (5 ml) and water (4 ml). The mixture was cooled to 0 °C and potassium hydroxide (590 mg, 10.5 mmol) was added. After stirring for 45 minutes at 0 °C, 2-methylindazole-5-carbaldehyde (100 mg, 0.63 mmol) was added. The reaction was stirred at room temperature for 16 hours, afterwards acetic acid (606 µL, 10.5 mmol) was added at 0 °C, resulting in a precipitate that was filtered and washed with ethanol (20 mL). The solid was dissolved in CH₂Cl₂ (50 mL) and washed with water (50 mL) and brine (50 mL). The solvent was evaporated in vacuo to yield the title compound as a yellow/orange solid (137 mg, yield 72%). ¹H NMR(400 MHz, DMSO-*d₆*) δ 12.20 (s, 1H), 8.44 (s, 1H), 8.04 (s, 1H), 7.68 - 7.61 (m, 3H), 7.61 - 7.52 (m, 2H), 7.40 (d, J = 8.8 Hz, 1H), 7.33 - 7.20 (m, 4H), 7.18 - 7.10 (m, 1H), 7.01 (d, J = 16.2 Hz, 1H), 4.18 - 4.12 (m, 5H). Rt 2.04 min (generic method) .ESI-MS for C₂₇H₂₀ClN₃O₂: calculated 453.1, found m/z 452.3/454.2 [M+H]⁺.

### Step 2. Synthesis of 4-benzyl-6-chloro-3-[5-(2-methylindazol-5-yl)-4,5-dihydro-1H-pyrazol-3-yl]-1H-quinolin-2-one (g.10).

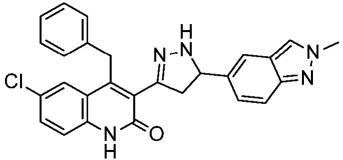

In appropriately sized microwaveable vessel, f.10 (136 g, 0.3 mmol) was suspended in ethanol (3 ml), then hydrazine hydrate (25 µL, 0.51 mmol) added, the reaction mixture microwaved at 110 °C (200 W) for 20 minutes, cooled at room temperature and resulting yellow solid filtered to yield title compound (98 mg, yield 70%). ¹H NMR(400 MHz, DMSO-*d₆*) δ 12.05 (s, 1H), 8.23 (s, 1H), 7.66 (d, J = 2.3 Hz, 1H), 7.62 (d, J = 1.4 Hz, 1H), 7.55 - 7.45 (m, 3H), 7.33 (d, J = 8.8 Hz, 1H), 7.30 - 7.22 (m, 5H), 7.21 - 7.13 (m, 1H), 4.86 (td, J = 10.8, 3.5 Hz, 1H), 4.52 - 4.38 (m, 2H), 4.14 (s, 3H), 3.36 (dd, J = 16.4, 10.6 Hz, 1H), 2.92 (dd, J = 16.3, 11.0 Hz, 1H). Rt 1.91 min (generic method). ESI-MS for C₂₇H₂₂ClN₅O: calculated 467.2, found m/z 468.2/470.2 [M+H]⁺.

### Step 3. Synthesis of 4-[5-(4-benzyl-6-chloro-2-oxo-1H-quinolin-3-yl)-3-(2-methylindazol-5-yl)-3,4-dihydropyrazol-2-yl]-4-oxo-butanoic acid (d.75).

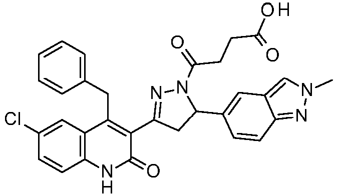

Compound d.75 was synthesized following the general procedure D1 using g.10 (83 mg, 0.18 mmol) with succinic anhydride (120 °C, 200 W). THF was evaporated and the resulted crude solid was triturated in CH₂Cl₂ (10 mL) and cold MeOH (10 mL), filtered and washed with diethyl ether (10 mL) to obtain the title compound as an off-white solid. (38 mg, yield 37%). ¹H NMR (400 MHz, DMSO-*d*₆) δ 12.20 (s, 1H), 12.02 (s, 1H), 8.22 (s, 1H), 7.76 (d, J = 2.3 Hz, 1H), 7.56 (dd, J = 8.8, 2.3 Hz, 1H), 7.51 - 7.43 (m, 2H), 7.38 (d, J = 8.8 Hz, 1H), 7.32 - 7.23 (m, 4H), 7.21 - 7.13 (m, 1H), 7.08 (dd, J = 9.1, 1.6 Hz, 1H), 5.50 (dd, J = 11.9, 4.8 Hz, 1H), 4.49 (d, J = 15.6 Hz, 1H), 4.41 (d, J = 15.6 Hz, 1H), 4.14 (s, 3H), 3.74 (dd, J = 18.4, 11.9 Hz, 1H), 3.14 (dd, J = 18.4, 4.8 Hz, 1H), 2.73 - 2.57 (m, 2H), 2.32 (t, J = 6.7 Hz, 2H). Rt 1.81 min (generic method) . ESI-MS for C₃₁H₂₆ClN₅O₄: calculated 567.2, found m/z 568.3/570.3 [M+H]⁺.

### Example d.83. 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid

### Step 1. Synthesis of 4-benzyl-6-chloro-3-[(E)-3-[4-(4-fluorophenyl)phenyl]prop-2-enoyl]-1H-quinolin-2-one (f.11)

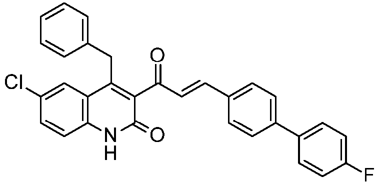

In a round-bottom flask, e.1 (119 g, 0.38 mmol) was suspended in a 4:3 mixture of ethanol (4.3 ml) and water (3.3 ml). The mixture was cooled to 0 °C and potassium hydroxide (533 mg, 9.5 mmol) was added. After stirring for 45 minutes at 0 °C, 4-(4-fluorophenyl)benzaldehyde (137 mg, 0.68 mmol) was added. The reaction was stirred at room temperature for 16 hours, afterwards acetic acid (0.548 mL, 9.5 mmol) was added at 0 °C, resulting off-white solid filtered and washed with ethanol (20 mL) and water (20 mL). The solid was dissolved in CH₂Cl₂ (50 mL), washed with water (3 x 50 mL) and brine. The solvent was evaporated in vacuo to yield the title compound as a yellow solid. (170 mg, yield 89 %). ¹H NMR(400 MHz, DMSO-*d₆*) δ 12.23 (s, 1H), 7.84 - 7.75 (m, 4H), 7.72 (d, J = 8.4 Hz, 2H), 7.66 - 7.55 (m, 3H), 7.40 (d, J = 8.8 Hz, 1H), 7.33 - 7.23 (m, 6H), 7.19 - 7.11 (m, 2H), 4.15 (s, 2H). Rt 1.81 min (Apolar method) .ESI-MS for C₃₁H₂₁ClFNO₂: calculated 493.1, found m/z 494.1/496.1.

### Step 2. Synthesis of 4-benzyl-6-chloro-3-[5-[4-(4-fluorophenyl)phenyl]-4,5-dihydro-1H-pyrazol-3-yl]-1H-quinolin-2-one (g.11).

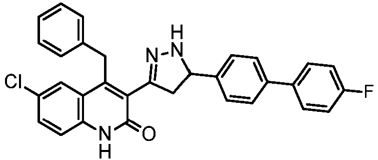

In appropriately sized microwaveable vessel, f.11 (156 mg, 0.32 mmol) was suspended in ethanol (0.7 ml), then hydrazine hydrate (26 µL, 0.54 mmol) added, the reaction mixture microwaved at 110 °C (200 W) for 20 minutes, cooled at room temperature and resulting grey solid filtered to yield title compound (107 mg, yield 71 %). ¹H NMR(400 MHz, DMSO-*d₆*) δ 12.06 (s, 1H), 7.71 - 7.65 (m, 3H), 7.59 - 7.53 (m, 3H), 7.51 - 7.45 (m, 3H), 7.36 - 7.25 (m, 7H), 7.21 - 7.14 (m, 1H), 4.85 (td, J = 10.6, 3.5 Hz, 1H), 4.53 - 4.37 (m, 2H), 3.39 (dd, J = 16.4, 10.6 Hz, 1H), 2.91 (dd, J = 16.3, 10.7 Hz, 1H). Rt 1.68 min (Apolar method). ESI-MS for C₃₁H₂₃ClFN₃O: calculated 507.2, found m/z 508.3/510.2 [M+H]⁺.

### Step 3. Synthesis of 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid (d.83).

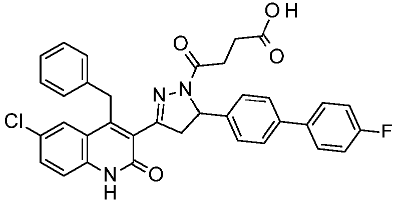

Compound d.83 was synthesized following the general procedure D1 using g.11 (100 mg, 0.20 mmol) with succinic anhydride (165 °C, 200 W). Purification by normal phase flash column chromatography (solvent A: CH₂Cl₂, solvent B: MeOH - gradient: 0-5 % solvent B) yielded title compound (45 mg, yield 45 %). ¹H NMR (400 MHz, DMSO-*d*₆) δ 12.21 (s, 1H), 12.00 (s, 1H), 7.78 (d, J = 2.3 Hz, 1H), 7.72 - 7.62 (m, 2H), 7.60 - 7.51 (m, 3H), 7.38 (d, J = 8.8 Hz, 1H), 7.33 - 7.22 (m, 8H), 7.22 - 7.13 (m, 1H), 5.49 (dd, J = 11.9, 4.9 Hz, 1H), 4.50 (d, J = 15.6 Hz, 1H), 4.41 (d, J = 15.5 Hz, 1H), 3.75 (dd, J = 18.3, 12.0 Hz, 1H), 3.14 (dd, J = 18.3, 4.9 Hz, 1H), 2.74 - 2.56 (m, 2H), 2.34 (t, J = 6.7 Hz, 2H). Rt 2.24 min (Generic method). ESI-MS for C₃₅H₂₇ClFN₃O₄: calculated 607.2, found m/z 608.1/610.1 [M+H]⁺.

### Example d.84. 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid

### Step 1. Synthesis of 4-benzyl-6-chloro-3-[(E)-3-[4-(4-chlorophenyl)phenyl]prop-2-enoyl]-1H-quinolin-2-one (f.12)

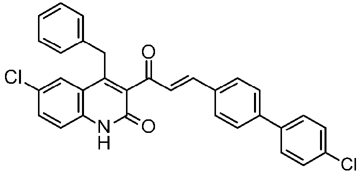

In a round-bottom flask, e.1 (300 mg, 0.96 mmol) was suspended in a 4:3 mixture of ethanol (11 ml) and water (8 ml). The mixture was cooled to 0 °C and potassium hydroxide (1.350 g, 24.06 mmol) was added. After stirring for 45 minutes at 0 °C, 4'-chloro-biphenyl-4-carbaldehyde (374 mg, 1.73 mmol) was added. The reaction was stirred at room temperature for 3 hours, afterwards acetic acid (1.387 mL, 24.06 mmol) was added at 0 °C, resulting solid filtered and washed with ethanol (20 mL) and water (20 mL). The solid was dissolved in CH₂Cl₂ (50 mL), washed with water (50 mL) and brine (50 mL). The solvent was evaporated in vacuo to yield the title compound as a yellow solid (322 mg, yield 66 %). ¹H NMR(400 MHz, DMSO-*d₆*) δ 12.23 (s, 1H), 7.82 (d, J = 8.5 Hz, 2H), 7.80 - 7.72 (m, 4H), 7.68 - 7.50 (m, 5H), 7.39 (d, J = 8.8 Hz, 1H), 7.31 - 7.22 (m, 4H), 7.18 - 7.10 (m, 2H), 4.15 (s, 2H). Rt 2.06 min (Apolar method).ESI-MS for C₃₁H₂₁Cl₂NO₂: calculated 509.10, found m/z 510.2/512.0 [M+H]⁺.

### Step 2. Synthesis of 4-benzyl-6-chloro-3-[5-[4-(4-chlorophenyl)phenyl]-4,5-dihydro-1H-pyrazol-3-yl]-1H-quinolin-2-one (g.12).

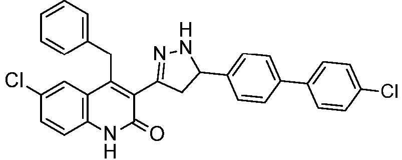

In appropriately sized microwaveable vessel, f.12 (310 mg, 0.61 mmol) was suspended in ethanol (1.3 ml), then hydrazine hydrate (50 µL, 1.03 mmol) added, the reaction mixture microwaved at 110 °C (200 W) for 20 minutes, cooled at room temperature, resulting solid filtered and washed with ethanol (20 mL) to yield title compound as a grey solid (280 g, yield 88 %). ¹H NMR(400 MHz, DMSO-*d₆*) δ 12.04 (s, 1H), 7.71 - 7.64 (m, 3H), 7.61 (d, J = 8.3 Hz, 2H), 7.55 (d, J = 3.4 Hz, 1H), 7.54 - 7.47 (m, 5H), 7.34 (d, J = 8.8 Hz, 1H), 7.30 - 7.25 (m, 4H), 7.21 - 7.16 (m, 1H), 4.85 (td, J = 10.7, 3.5 Hz, 1H), 4.51 - 4.37 (m, 2H), 3.39 (dd, J = 16.3, 10.6 Hz, 1H), 2.91 (dd, J = 16.3, 10.7 Hz, 1H). Rt 2.09 min (Apolar method). ESI-MS for C₃₁H₂₃Cl₂N₃O: calculated 523.1, found m/z 524.2/526.2/528.0 [M+H]⁺.

### Step 3. Synthesis of 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid (d.84).

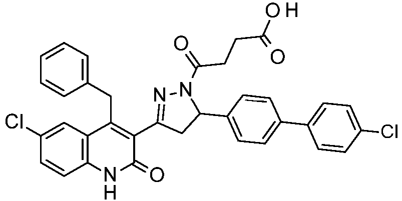

Compound d.84 was synthesized following the general procedure D1 using g.12 (134 mg, 0.26 mmol) with succinic anhydride (165 °C, 200 W). The precipitate formed was triturated with MeOH (10 mL) and Et₂O (10 mL) to obtain the title compound as a white solid (134 mg, yield 83 %). ¹H NMR(400 MHz, DMSO-*d*₆) δ 12.21 (s, 1H), 12.00 (s, 1H), 7.78 (d, J = 2.3 Hz, 1H), 7.66 (d, J = 8.6 Hz, 2H), 7.57 (d, J = 8.2 Hz, 3H), 7.51 (d, J = 8.6 Hz, 2H), 7.38 (d, J = 8.8 Hz, 1H), 7.33 - 7.24 (m, 6H), 7.21 - 7.15 (m, 1H), 5.49 (dd, J = 11.9, 4.9 Hz, 1H), 4.49 (d, J = 15.5 Hz, 1H), 4.41 (d, J = 15.6 Hz, 1H), 3.75 (dd, J = 18.4, 12.0 Hz, 1H), 3.14 (dd, J = 18.4, 4.9 Hz, 1H), 2.74 - 2.55 (m, 2H), 2.34 (t, J = 6.7 Hz, 2H). Rt 2.42 min (Generic method). ESI-MS for C₃₅H₂₇Cl₂N₃O₄: calculated 623.1, found *m*/*z* 624.1/626.1/628.1 [M+H]⁺.

### Example d.85. 3-[2-acetyl-3-[4-(4-chlorophenyl)phenyl]-3,4-dihydropyrazol-5-yl]-4-benzyl-6-chloro-1H-quinolin-2-one

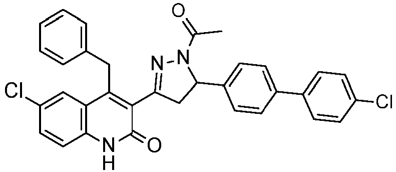

Compound d.85 was synthesized following the general procedure D1 using g.12 (135 mg, 0.26 mmol) with acetic anhydride (49 µL, 0.52 mmol) at 165 °C for 20 minutes. The THF was removed under vacuum, and the crude triturated with MeOH (10 mL) and Et₂O (10 mL) to yield the title compound as a white solid (72 mg, yield 49%). ¹H NMR(400 MHz, DMSO-*d₆*) δ 12.21 (s, 1H), 7.75 (d, J = 2.3 Hz, 1H), 7.67 (d, J = 8.6 Hz, 2H), 7.62 - 7.53 (m, 3H), 7.51 (d, J = 8.5 Hz, 2H), 7.38 (d, J = 8.8 Hz, 1H), 7.34 - 7.25 (m, 6H), 7.23 - 7.14 (m, 1H), 5.50 (dd, J = 11.9, 4.8 Hz, 1H), 4.47 (d, J = 15.6 Hz, 1H), 4.40 (d, J = 15.6 Hz, 1H), 3.77 (dd, J = 18.3, 12.0 Hz, 1H), 3.18 (dd, J = 18.4, 4.9 Hz, 1H), 1.99 (s, 3H). Rt 1.83 min (apolar method). ESI-MS for C₃₃H₂₅Cl₂N₃O₂: calculated 565.1, found m/z 566.1/568.2/570.4 [M+H]⁺.

### Example d.88. 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid

### Step 1. Synthesis of 4-benzyl-6-chloro-3-[(E)-3-[4-(4-methoxyphenyl)phenyl]prop-2-enoyl]-1H-quinolin-2-one (f.13)

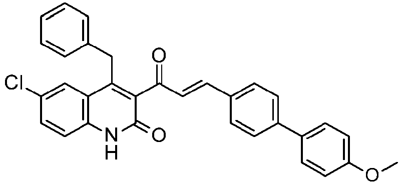

In a round-bottom flask, e.1 (350 mg, 1.12 mmol) was suspended in a 4:3 mixture of ethanol (13 ml) and water (10 ml). The mixture was cooled to 0 °C and potassium hydroxide (1.574 g, 28 mmol) was added. After stirring for 45 minutes at 0 °C, 4'-methoxybiphenyl-4-carbaldehyde (428 mg, 2.02 mmol) was added. The reaction was stirred at room temperature for 16 hours, afterwards acetic acid (1.614 mL, 28 mmol) was added at 0 °C, resulting in a precipitate that was filtered and washed with ethanol (20 mL) and water (20 mL). The solid was dissolved in CH₂Cl₂ (50 mL), washed with water (50 mL) and the solvent evaporated in vacuo to yield the title compound as a yellow/orange solid (529 mg, yield 93 %). ¹H NMR(400 MHz, DMSO-*d₆*) δ 12.22 (s, 1H), 7.77 (d, J = 8.4 Hz, 2H), 7.72 - 7.63 (m, 5H), 7.63 - 7.53 (m, 2H), 7.39 (d, J = 8.8 Hz, 1H), 7.32 - 7.25 (m, 2H), 7.29 - 7.21 (m, 2H), 7.20 - 6.96 (m, 4H), 4.15 (s, 2H), 3.80 (s, 3H). Rt 1.77 min (Apolar method) .ESI-MS for C₃₂H₂₄ClNO₃: calculated 505.1, found m/z 506.2/508.2 [M+H]⁺.

### Step 2. Synthesis of 4-benzyl-6-chloro-3-[5-[4-(4-methoxyphenyl)phenyl]-4,5-dihydro-1H-pyrazol-3-yl]-1H-quinolin-2-one (g.13).

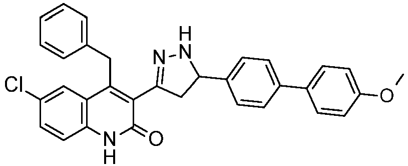

In appropriately sized microwaveable vessel, f.13 (511 mg, 1.01 mmol) was suspended in ethanol (2 ml), then hydrazine hydrate (83 µL, 1.72 mmol) added, the reaction mixture microwaved at 110 °C (200 W) for 20 minutes, cooled at room temperature, resulting solid filtered and washed with ethanol (20 mL) to yield title compound as a yellow solid (496 mg, yield 94%). ¹H NMR (400 MHz, DMSO-*d₆*) δ 12.06 (s, 1H), 7.67 (d, J = 2.3 Hz, 1H), 7.62 - 7.48 (m, 6H), 7.43 (d, J = 8.3 Hz, 2H), 7.34 (d, J = 8.8 Hz, 1H), 7.31 - 7.23 (m, 4H), 7.23 - 7.13 (m, 1H), 7.01 (d, J = 8.8 Hz, 2H), 4.83 (td, J = 10.7, 3.4 Hz, 1H), 4.52 - 4.35 (m, 2H), 3.79 (s, 3H), 3.38 (dd, J = 16.4, 10.6 Hz, 1H), 2.91 (dd, J = 16.4, 10.7 Hz, 1H).

### Step 3. Synthesis of 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid (d.88).

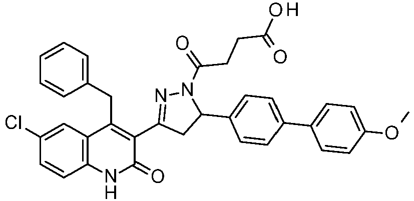

Compound d.88 was synthesized following the general procedure D1 using g.13 (100 mg, 0.19 mmol) with succinic anhydride (120 °C, 200 W). The resulted solid was triturated in MeOH (10 mL), CH₂Cl₂ (10 mL), and Et₂O (10 mL) to obtain the title product as a white solid (80 mg, yield 68%). ¹H NMR(400 MHz, DMSO-*d*₆) δ 12.21 (s, 1H), 12.00 (s, 1H), 7.77 (d, J = 2.3 Hz, 1H), 7.61 - 7.53 (m, 3H), 7.50 (d, J = 8.4 Hz, 2H), 7.38 (d, J = 8.8 Hz, 1H), 7.31 - 7.23 (m, 6H), 7.21 - 7.15 (m, 1H), 7.02 (d, J = 8.8 Hz, 2H), 5.47 (dd, J = 11.9, 4.8 Hz, 1H), 4.49 (d, J = 15.6 Hz, 1H), 4.41 (d, J = 15.5 Hz, 1H), 3.79 (s, 3H), 3.78 - 3.69 (m, 1H), 3.13 (dd, J = 18.3, 4.9 Hz, 1H), 2.74 - 2.56 (m, 2H), 2.34 (t, J = 6.7 Hz, 2H). Rt 2.21 min (Generic method). ESI-MS for C₃₆H₃₀ClN₃O₅: calculated 619.2, found m/z 620.3/622.2 [M+H]⁺.

### Example d.89. 3-[2-acetyl-3-[4-(4-methoxyphenyl)phenyl]-3,4-dihydropyrazol-5-yl]-4-benzyl-6-chloro-1H-quinolin-2-one

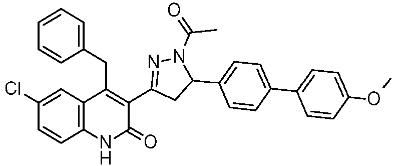

Compound d.89 was synthesized following the general procedure D1 using g.13 (100 mg, 0.19 mmol) with acetic anhydride (39 mg, 0.38 mmol) at 165 °C for 20 minutes. The resulted solid was filtrated and washed with MeOH (10 mL), CH₂Cl₂ (10 mL), and Et₂O (10 mL) to obtain the title product as a white solid (63 mg, yield 59 %). ¹H NMR(400 MHz, DMSO-*d₆*) δ 12.20 (s, 1H), 7.74 (d, J = 2.3 Hz, 1H), 7.62 - 7.49 (m, 5H), 7.38 (d, J = 8.8 Hz, 1H), 7.33 - 7.24 (m, 6H), 7.23 - 7.15 (m, 1H), 7.02 (d, J = 8.8 Hz, 2H), 5.48 (dd, J = 12.0, 4.8 Hz, 1H), 4.48 (d, J = 15.6 Hz, 1H), 4.40 (d, J = 15.6 Hz, 1H), 3.79 (s, 3H), 3.78 - 3.71 (m, 1H), 3.17 (dd, J = 18.3, 4.8 Hz, 1H), 1.99 (s, 3H). Rt 2.48 min (generic method). ESI-MS for C₃₄H₂₈ClN₃O₃: calculated 561.2, found m/z 562.3/564.3 [M+H]⁺.

### Example d.90. 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid

### Step 1. Synthesis of 4-benzyl-6-chloro-3-[(E)-3-[4-(1-propylpyrazol-4-yl)phenyl]prop-2-enoyl]-1H-quinolin-2-one (f.14)

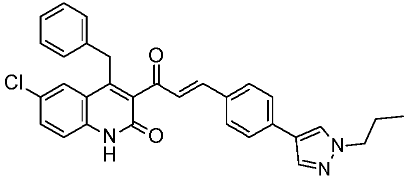

In a round-bottom flask, e.1 (165 mg, 0.53 mmol) was suspended in a 4:3 mixture of ethanol (6 ml) and water (4.5 ml). The mixture was cooled to 0 °C and potassium hydroxide (743 g, 13.25 mmol) was added. After stirring for 45 minutes at 0 °C, 4-(1-propylpyrazol-4-yl)benzaldehyde (206 mg, 0.96 mmol) was added. The reaction was stirred at room temperature for 16 hours, afterwards acetic acid (0.746 mL, 13.25 mmol) was added at 0 °C, resulting in a precipitate that was filtered and washed with ethanol (20 mL) and water (20 mL). The solid was dissolved in CH₂Cl₂ (50 mL) and washed with water and brine. The solvent was evaporated in vacuo to yield the title compound as a yellow solid (225 g, yield 84%). ¹H NMR(400 MHz, DMSO-*d₆*) δ 12.21 (s, 1H), 8.29 (s, 1H), 7.96 (d, J = 0.8 Hz, 1H), 7.70 (d, J = 8.5 Hz, 2H), 7.66 - 7.60 (m, 3H), 7.58 - 7.50 (m, 2H), 7.39 (d, J = 8.8 Hz, 1H), 7.31 - 7.22 (m, 4H), 7.18 - 7.12 (m, 1H), 7.05 (d, J = 16.3 Hz, 1H), 4.14 (s, 2H), 4.07 (t, J = 7.0 Hz, 2H), 1.87 - 1.76 (m, 2H), 0.85 (t, J = 7.4 Hz, 3H). Rt 2.45 min (generic method) .ESI-MS for C₃₁H₂₆ClN₃O: calculated 507.2, found m/z 508.3/510.3 [M+H]⁺.

### Step 2. Synthesis of 4-benzyl-6-chloro-3-[5-[4-(1-propylpyrazol-4-yl)phenyl]-4,5-dihydro-1H-pyrazol-3-yl]-1H-quinolin-2-one (g.14).

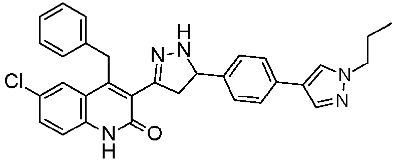

In appropriately sized microwaveable vessel, f.14 (220 g, 0.43 mmol) was suspended in ethanol (4 ml), then hydrazine hydrate (36 µL, 0.74 mmol) added, the reaction mixture microwaved at 110 °C (200 W) for 20 minutes, cooled at room temperature and resulting green/grey solid filtered to yield title compound (192 g, yield 86%). ¹H NMR (400 MHz, DMSO-*d₆*) δ 12.05 (s, 1H), 8.14 (s, 1H), 7.83 (d, J = 0.9 Hz, 1H), 7.66 (d, J = 2.3 Hz, 1H), 7.53 - 7.46 (m, 4H), 7.40 - 7.32 (m, 3H), 7.31 - 7.24 (m, 4H), 7.22 - 7.15 (m, 1H), 4.78 (td, J = 10.7, 3.4 Hz, 1H), 4.51 - 4.37 (m, 2H), 4.06 (t, J = 7.0 Hz, 2H), 3.41 - 3.34 (m, 1H), 2.88 (dd, J = 16.3, 10.8 Hz, 1H), 1.81 (h, J = 7.3 Hz, 2H), 0.85 (t, J = 7.4 Hz, 3H). Rt 2.32 min (generic method). ESI-MS for C₃₁H₂₈ClN₅O: calculated 521.2, found m/z 522.3/524.3 [M+H]⁺.

### Step 3. Synthesis of 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid (d.90).

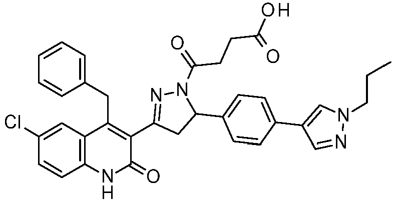

Compound d.90 was synthesized following the general procedure D1 using g.14 (92 mg, 0.18 mmol) with succinic anhydride (120 °C, 200 W). THF was evaporated and the resulted solid was triturated in MeOH (10 mL), CH₂Cl₂ (10 mL), and Et₂O (10 mL) to obtain the title product as a white solid (65 mg, yield 58%). ¹H NMR(400 MHz, DMSO-*d*₆) δ 12.20 (s, 1H), 11.99 (s, 1H), 8.12 (s, 1H), 7.82 (s, 1H), 7.77 (d, J = 2.3 Hz, 1H), 7.56 (dd, J = 8.8, 2.2 Hz, 1H), 7.46 (d, J = 8.0 Hz, 2H), 7.38 (d, J = 8.8 Hz, 1H), 7.31 - 7.24 (m, 4H), 7.22 - 7.14 (m, 3H), 5.43 (dd, J = 12.0, 4.8 Hz, 1H), 4.48 (d, J = 15.6 Hz, 1H), 4.39 (d, J = 15.5 Hz, 1H), 4.06 (t, J = 7.0 Hz, 2H), 3.71 (dd, J = 18.3, 12.0 Hz, 1H), 3.11 (dd, J = 18.3, 4.8 Hz, 1H), 2.72 - 2.55 (m, 2H), 2.33 (t, J = 6.7 Hz, 2H), 1.81 (h, J = 7.3 Hz, 2H), 0.84 (t, J = 7.4 Hz, 3H). Rt 2.05 min (generic method) . ESI-MS for C₃₅H₃₂ClN₅O₄: calculated 621.2, found m/z 622.2/624.2 [M+H]⁺.

### Example d.91. 3-[2-acetyl-3-[4-(1-propylpyrazol-4-yl)phenyl]-3,4-dihydropyrazol-5-yl]-4-benzyl-6-chloro-1H-quinolin-2-one

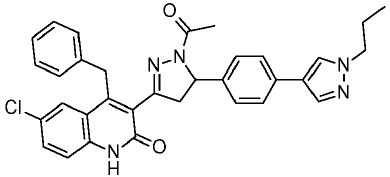

Compound d.91 was synthesized following the general procedure D1 using g.14 (93 mg, 0.18 mmol) with acetic anhydride (37 mg, 0.34 mmol) at 165 °C for 20 minutes. Purification by normal phase flash column chromatography (solvent A: DCM, solvent B: MeOH - gradient: 0-3 % solvent B) yielded title compound as a white solid (84 mg, yield 82%). ¹H NMR(400 MHz, DMSO-*d₆*) δ 12.20 (s, 1H), 8.13 (d, J = 0.8 Hz, 1H), 7.83 (d, J = 0.8 Hz, 1H), 7.74 (d, J = 2.3 Hz, 1H), 7.56 (dd, J = 8.8, 2.3 Hz, 1H), 7.47 (d, J = 8.3 Hz, 2H), 7.38 (d, J = 8.8 Hz, 1H), 7.33 - 7.25 (m, 4H), 7.25 - 7.14 (m, 3H), 5.43 (dd, J = 11.9, 4.7 Hz, 1H), 4.46 (d, J = 15.6 Hz, 1H), 4.39 (d, J = 15.6 Hz, 1H), 4.07 (t, J = 7.0 Hz, 2H), 3.73 (dd, J = 18.3, 12.0 Hz, 1H), 3.15 (dd, J = 18.3, 4.7 Hz, 1H), 1.97 (s, 3H), 1.81 (h, J = 7.3 Hz, 2H), 0.85 (t, J = 7.4 Hz, 3H). Rt 2.32 min (generic method). ESI-MS for C₃₃H₃₀ClN₅O₂: calculated 563.2, found m/z 564.2/566.3 [M+H]⁺.

### Example d.92. 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid

### Step 1. Synthesis of 4-benzyl-6-chloro-3-[(E)-3-[4-(1-ethylindazol-5-yl)phenyl]prop-2-enoyl]-1H-quinolin-2-one (f.15)

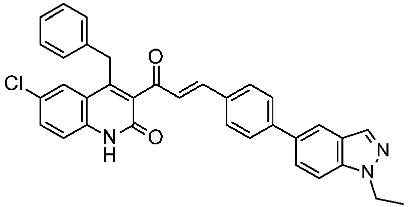

In a round-bottom flask, e.1 (73 mg, 0.24 mmol) was suspended in a 4:3 mixture of ethanol (3 ml) and water (2 ml). The mixture was cooled to 0 °C and potassium hydroxide (337 mg, 6 mmol) was added. After stirring for 45 minutes at 0 °C, 4-(1-ethylindazol-5-yl)benzaldehyde (106 mg, 0.42 mmol) was added. The reaction was stirred at room temperature for 16 hours, afterwards acetic acid (0.346 mL, 6 mmol) was added at 0 °C, resulting in a precipitate that was filtered and washed with ethanol (20 mL) and water (20 mL). The solid was dissolved in CH₂Cl₂ (50 mL) and washed with brine. The solvent was evaporated in vacuo to yield the title compound as a yellow solid (114 mg, yield 87%). ¹H NMR(400 MHz, DMSO-*d₆*) δ 12.23 (s, 1H), 8.14 - 8.10 (m, 2H), 7.84 - 7.75 (m, 6H), 7.68 - 7.53 (m, 3H), 7.40 (d, J = 8.8 Hz, 1H), 7.32 - 7.23 (m, 4H), 7.20 - 7.10 (m, 2H), 4.47 (q, J = 7.1 Hz, 2H), 4.15 (s, 2H), 1.41 (t, J = 7.2 Hz, 3H). Rt 2.64 min (generic method) .ESI-MS for C₃₄H₂₆ClN₃O₂: calculated 543.2, found m/z 544.3/546.2 [M+H]⁺.

### Step 2. Synthesis of 4-benzyl-6-chloro-3-[5-[4-(1-ethylindazol-5-yl)phenyl]-4,5-dihydro-1H-pyrazol-3-yl]-1H-quinolin-2-one (g.15).

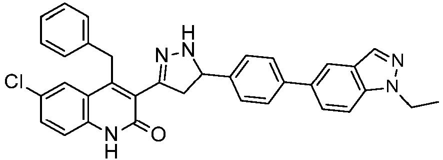

In appropriately sized microwaveable vessel, f.15 (114 mg, 0.21 mmol) was suspended in ethanol (3 ml), then hydrazine hydrate (17 µL, 0.36 mmol) added, the reaction mixture microwaved at 110 °C (200 W) for 20 minutes, cooled at room temperature and resulting solid filtered and washed with ethanol (20 mL) to yield title compound as a green/grey solid (92 mg, yield 79%). ¹H NMR(400 MHz, DMSO-*d₆*) δ 12.06 (s, 1H), 8.10 (d, J = 0.9 Hz, 1H), 7.99 (dd, J = 1.7, 0.8 Hz, 1H), 7.75 (d, J = 8.7 Hz, 1H), 7.71 - 7.60 (m, 4H), 7.56 - 7.44 (m, 4H), 7.34 (d, J = 8.8 Hz, 1H), 7.31 - 7.26 (m, 4H), 7.21 - 7.14 (m, 1H), 4.85 (td, J = 10.6, 3.4 Hz, 1H), 4.52 - 4.41 (m, 4H), 3.40 (dd, J = 16.4, 10.6 Hz, 1H), 2.93 (dd, J = 16.3, 10.7 Hz, 1H), 1.41 (t, J = 7.2 Hz, 3H). Rt 2.52 min (generic method). ESI-MS for C₃₄H₂₈ClN₅O: calculated 557.2, found m/z 558.3/559.9 [M+H]⁺.

### Step 3. Synthesis of 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid (d.92).

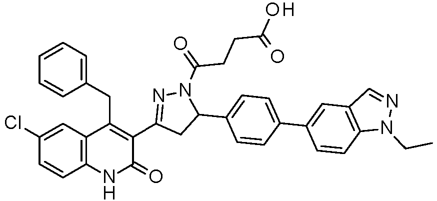

Compound d.92 was synthesized following the general procedure D1 using g.15 (84 mg, 0.15 mmol) with succinic anhydride (120 °C, 200 W The resulted crude solid was triturated in CH₂Cl₂ (10 mL) and MeOH (10 mL), filtered and washed with diethyl ehter (10 mL) to obtain the title compound as a grey-green solid (63 mg, yield 64%). ¹H NMR(400 MHz, DMSO-*d*₆) δ 12.21 (s, 1H), 12.01 (s, 1H), 8.10 (d, J = 0.9 Hz, 1H), 7.98 (d, J = 1.7 Hz, 1H), 7.78 (d, J = 2.3 Hz, 1H), 7.75 (d, J = 8.8 Hz, 1H), 7.67 (dd, J = 8.8, 1.7 Hz, 1H), 7.63 - 7.54 (m, 3H), 7.39 (d, J = 8.8 Hz, 1H), 7.33 - 7.23 (m, 6H), 7.23 - 7.14 (m, 1H), 5.50 (dd, J = 11.9, 4.9 Hz, 1H), 4.55 - 4.38 (m, 4H), 3.76 (dd, J = 18.3, 12.0 Hz, 1H), 3.16 (dd, J = 18.3, 4.9 Hz, 1H), 2.75 - 2.57 (m, 2H), 2.35 (t, J = 6.7 Hz, 2H), 1.41 (t, J = 7.2 Hz, 3H). Rt 2.18 min (Generic method). ESI-MS for C₃₈H₃₂ClN₅O₄: calculated 657.2, found m/z 658.2/660.2 [M+H]⁺.

### Example d.93. 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid

### Step 1. Synthesis of 4-benzyl-6-chloro-3-[(E)-3-[4-(2-ethylindazol-5-yl)phenyl]prop-2-enoyl]-1H-quinolin-2-one (f.16)

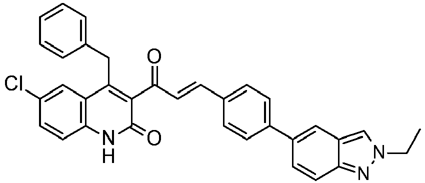

In a round-bottom flask, e.1 (80 mg, 0.25 mmol) was suspended in a 4:3 mixture of ethanol (3 ml) and water (2 ml). The mixture was cooled to 0 °C and potassium hydroxide (351 g, 6.25 mmol) was added. After stirring for 45 minutes at 0 °C, 4-(2-ethylindazol-5-yl)benzaldehyde (71 mg, 0.28 mmol) was added. The reaction was stirred at room temperature for 16 hours, afterwards acetic acid (0.36 mL, 6.25 mmol) was added at 0 °C, resulting precipitate was filtered and washed with ethanol (20 mL) and water (20 mL). The solid was dissolved in CH₂Cl₂ (50 mL) and washed with water (50 mL) and brine (50 mL). The solvent was evaporated in vacuo to yield the title compound as a yellow solid (119 mg, yield 87 %). ¹H NMR(400 MHz, DMSO-*d₆*) δ 12.25 (s, 1H), 8.45 (s, 1H), 8.06 (s, 1H), 7.83 - 7.74 (m, 4H), 7.72 - 7.54 (m, 5H), 7.40 (d, J = 8.8 Hz, 1H), 7.32 - 7.23 (m, 4H), 7.19 - 7.07 (m, 2H), 4.47 (q, J = 7.3 Hz, 2H), 4.15 (s, 2H), 1.52 (t, J = 7.3 Hz, 3H). Rt 2.44 min (Generic method) .ESI-MS for C₃₄H₂₆ClN₃O₂: calculated 543.2, found m/z 544.2/546.3 [M+H]⁺.

### Step 2. Synthesis of 4-benzyl-6-chloro-3-[5-[4-(2-ethylindazol-5-yl)phenyl]-4,5-dihydro-1H-pyrazol-3-yl]-1H-quinolin-2-one (g.16).

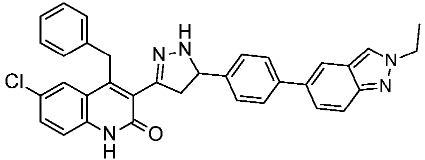

In appropriately sized microwaveable vessel, f.16 (117 mg, 0.22 mmol) was suspended in ethanol (3 ml), then hydrazine hydrate (18 µL, 0.37 mmol) added, the reaction mixture microwaved at 110 °C (200 W) for 20 minutes, cooled at room temperature and resulting solid filtered and washed with ethanol (20 mL) to yield title compound as a green/grey solid (103 mg, yield 84 %). ¹H NMR(400 MHz, DMSO-*d₆*) δ 12.06 (s, 1H), 8.42 (s, 1H), 7.93 (s, 1H), 7.71 - 7.59 (m, 4H), 7.56 - 7.44 (m, 5H), 7.34 (d, J = 8.8 Hz, 1H), 7.32 - 7.25 (m, 4H), 7.22 - 7.14 (m, 1H), 4.85 (td, J = 10.7, 3.3 Hz, 1H), 4.53 - 4.40 (m, 4H), 3.40 (dd, J = 16.4, 10.6 Hz, 1H), 2.93 (dd, J = 16.3, 10.7 Hz, 1H), 1.52 (t, J = 7.3 Hz, 3H). Rt 2.33 min (Generic method). ESI-MS for C₃₄H₂₈ClN₅O: calculated 557.2, found m/z 558.3 /560.3 [M+H]⁺.

### Step 3. Synthesis of 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid (d.93).

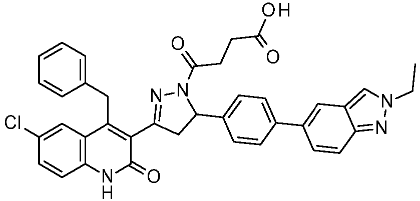

Compound d.93 was synthesized following the general procedure D1 using g.16 (95 mg, 0.17 mmol) with succinic anhydride (165 °C, 200 W). THF was evaporated and the resulted crude solid was triturated in CH₂Cl₂ (10 mL) and MeOH (10 mL), filtered and washed with diethyl ehter (10 mL) to obtain the title compound as a grey solid (98 mg, yield 88 %). ¹H NMR(400 MHz, DMSO-*d*₆) δ 12.22 (s, 1H), 12.01 (s, 1H), 8.42 (s, 1H), 7.91 (s, 1H), 7.78 (d, J = 2.3 Hz, 1H), 7.67 (d, J = 9.0 Hz, 1H), 7.63 - 7.50 (m, 4H), 7.39 (d, J = 8.8 Hz, 1H), 7.33 - 7.24 (m, 6H), 7.23 - 7.13 (m, 1H), 5.49 (dd, J = 11.9, 4.9 Hz, 1H), 4.55 - 4.38 (m, 4H), 3.76 (dd, J = 18.4, 12.0 Hz, 1H), 3.15 (dd, J = 18.3, 4.9 Hz, 1H), 2.73 - 2.59 (m, 2H), 2.35 (t, J = 6.7 Hz, 2H), 1.52 (t, J = 7.3 Hz, 3H). Rt 0.75 min (Apolar method). ESI-MS for C₃₈H₃₂ClN₅O₄: calculated 657.2, found *m*/*z* 658.3/660.2 [M+H]⁺.

### Example d.96. 3-[2-acetyl-3-(p-tolyl)-3,4-dihydropyrazol-5-yl]-4-benzyl-6-chloro-1H-quinolin-2-one

### Step 1. Synthesis of 4-benzyl-6-chloro-3-[(E)-3-(p-tolyl)prop-2-enoyl]-1H-quinolin-2-one (f.17)

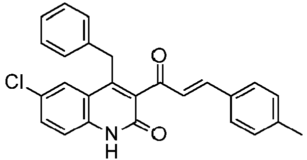

In a round-bottom flask, e.1 (192 mg, 0.62 mmol) was suspended in a 4:3 mixture of ethanol (12 ml) and water (9 ml). The mixture was cooled to 0 °C and potassium hydroxide (870 mg, 15.5 mmol) was added. After stirring for 45 minutes at 0 °C, p-tolualdehyde (131 µL, 1.11 mmol) was added. The reaction was stirred at room temperature for 16 hours, afterwards acetic acid (0.894 mL, 15.5 mmol) was added at 0 °C, resulting in an off-white solid that filtered and washed with ethanol (20 mL). The solid was dissolved in CH₂Cl₂ (50 mL), washed with water and brine. The solvent was evaporated in vacuo to yield the title compound as a yellow solid (225 mg, yield 87%). ¹H NMR(400 MHz, DMSO-*d₆*) δ 12.17 (s, 1H), 7.63 (d, J = 2.3 Hz, 1H), 7.62 - 7.58 (m, 2H), 7.57 - 7.49 (m, 2H), 7.38 (d, J = 8.8 Hz, 1H), 7.29 - 7.20 (m, 6H), 7.18 - 7.11 (m, 1H), 7.01 (d, J = 16.3 Hz, 1H), 4.12 (s, 2H), 2.32 (s, 3H). Rt 2.53 min (generic method).ESI-MS for C₂₆H₂₀ClNO₂: calculated 413.1, found m/z 414.1/416.1 [M+H]⁺.

### Step 2. Synthesis of 4-benzyl-6-chloro-3-[5-(p-tolyl)-4,5-dihydro-1H-pyrazol-3-yl]-1H-quinolin-2-one (g.17).

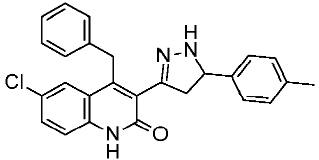

In appropriately sized microwaveable vessel, f.17 (210 mg, 0.51 mmol) was suspended in ethanol (2 ml), then hydrazine hydrate (42 µL, 0.86 mmol) added, the reaction mixture microwaved at 110 °C (200 W) for 20 minutes, cooled at room temperature and resulting yellow solid filtered to yield title compound (155 mg, yield 71%). ¹H NMR(400 MHz, DMSO-*d₆*) δ 12.05 (s, 1H), 7.66 (d, J = 2.3 Hz, 1H), 7.49 (dd, J = 8.8, 2.2 Hz, 1H), 7.44 (d, J = 3.4 Hz, 1H), 7.33 (d, J = 8.8 Hz, 1H), 7.30 - 7.22 (m, 5H), 7.22 - 7.13 (m, 1H), 7.10 (d, J = 7.8 Hz, 2H), 4.75 (td, J = 10.7, 3.5 Hz, 1H), 4.49 - 4.35 (m, 2H), 3.39 - 3.26 (m, 1H), 2.84 (dd, J = 16.3, 10.9 Hz, 1H), 2.27 (s, 3H). Rt 2.44 min (generic method). ESI-MS for C₂₆H₂₂ClN₃O: calculated 427.1, found m/z 428.2/430.1 [M+H]⁺.

### Step 3. Synthesis of 3-[2-acetyl-3-(p-tolyl)-3,4-dihydropyrazol-5-yl]-4-benzyl-6-chloro-1H-quinolin-2-one (d.96) .

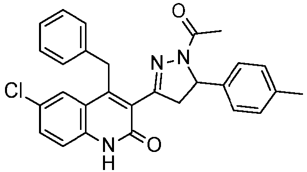

Compound d.96 was synthesized following the general procedure D1 using g.17 (148 mg, 0.35 mmol) with acetic anhydride (65 µL, 0.69 mmol) at 165 °C for 20 minutes. Purification by normal phase flash column chromatography (solvent A: DCM, solvent B: MeOH - gradient: 0-5 % solvent B) yielded title compound as a white solid (80 mg, yield 49%). ¹H NMR(400 MHz, DMSO-*d₆*) δ 12.19 (s, 1H), 7.73 (d, J = 2.3 Hz, 1H), 7.55 (dd, J = 8.8, 2.2 Hz, 1H), 7.38 (d, J = 8.8 Hz, 1H), 7.28 (d, J = 4.3 Hz, 4H), 7.24 - 7.16 (m, 1H), 7.14 - 7.06 (m, 4H), 5.41 (dd, J = 11.9, 4.7 Hz, 1H), 4.45 (d, J = 15.6 Hz, 1H), 4.37 (d, J = 15.5 Hz, 1H), 3.73 (dd, J = 18.3, 11.9 Hz, 1H), 3.10 (dd, J = 18.3, 4.7 Hz, 1H), 2.26 (s, 3H), 1.96 (s, 3H). Rt 2.36 min (generic method). ESI-MS for C₂₈H₂₄ClN₃O₂: calculated 469.2, found m/z 470.1/472.1 [M+H]⁺.

### Example d.97. 3-(1-acetyl-5-(pyridin-4-yl)-4,5-dihydro-1H-pyrazol-3-yl)-6-chloro-4-phenylquinolin-2(1H)-one (example not falling within the scope of the present invention).

### Step 1. Synthesis of (E)-6-chloro-4-phenyl-3-(3-(pyridin-4-yl)acryloyl)quinolin-2(1H)-one (b.28)

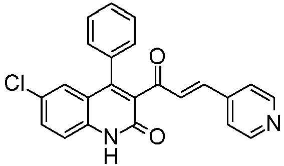

In a round-bottomed flask, the appropriate 3-acetyl-6-chloro-4-phenylquinolin-2(1H)-one (2.1 g, 7.05 mmol) and potassium hydroxide (9.89 g, 176.33 mmol) were stirred in EtOH/H₂O (4:3 v/v, 0.05 M) at 0 °C for 45 min prior to the addition of 4-pyridine carboxaldehyde (1.2 mL, 12.69 mmol). The reaction mixture was stirred for 6 h as it gradually reached room temperature. EtOH was removed under reduced pressure and the aqueous phase was extracted with DCM (3 × 60 mL), the organic layer was then dried over Na₂SO₄, and solvent removed under reduced pressure. Final normal flash silica gel chromatography purification (solvent A: DCM, solvent B: MeOH - gradient: 1-5% solvent B) yielded title compound as a yellow solid (2.02 g, yield 74%). ¹H NMR(400 MHz, CDCl₃) δ 12.40 (s, 1H), 8.60 (dd, J = 4.5, 1.5 Hz, 2H), 7.68 (dd, J = 8.8, 2.4 Hz, 1H), 7.61 (dd, J = 4.6, 1.5 Hz, 2H), 7.54 - 7.38 (m, 5H), 7.33 (dd, J = 7.7, 1.7 Hz, 2H), 6.99 (dd, J = 9.4, 7.1 Hz, 2H).

### Step 2. Synthesis of 6-chloro-4-phenyl-3-(5-(pyridin-4-yl)-4,5-dihydro-1H-pyrazol-3-yl) quinolin-2(1H)-one (c.28)

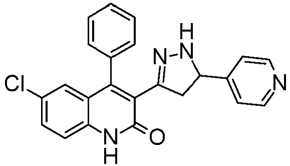

In appropriately sized microwaveable vessel, b.28 (2.01 g, 5.20 mmol) was suspended in ethanol (10 ml), and then hydrazine hydrate (379 µl, 7.79 mmol) was added. The reaction mixture was microwaved at 110°C for 45 minutes, then the EtOH was removed under reduced pressure, diethyl ether was added to the white residue and the filtration of the precipitated afforded the titled compound as yellow solid (1.64 g, yield 79%). ¹H NMR(400 MHz, DMSO-*d₆*) δ 12.21 (s, 1H), 8.41 (dd, J = 4.4, 1.6 Hz, 2H), 7.57 (dd, J = 8.8, 2.4 Hz, 1H), 7.54 - 7.37 (m, 4H), 7.35 - 7.31 (m, 1H), 7.28 (d, J = 2.9 Hz, 1H), 7.22 - 7.16 (m, 1H), 7.03 (dd, J = 4.6, 1.5 Hz, 2H), 6.88 (d, J = 2.3 Hz, 1H), 4.59 (ddd, J = 11.4, 8.8, 2.9 Hz, 1H), 3.28 (d, J = 5.3 Hz, 1H), 2.58 - 2.51 (m, 1H).

### Step 3. Synthesis of 3-(1-acetyl-5-(pyridin-4-yl)-4,5-dihydro-1H-pyrazol-3-yl)-6-chloro-4-phenylquinolin-2(1H)-one (d.97).

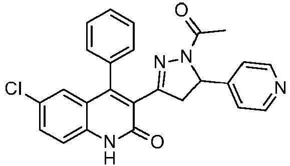

In appropriately-sized microwaveable vessel, c.28 (300 mg, 0.75 mmol) was suspended in anhydrous tetrahydrofuran (1.5 ml). Acetic anhydride (85 µL, 0.90 mmol) was added and the reaction mixture was microwaved at 165°C for 25 minutes. After cooling down to rt, THF was removed under reduced pressure, and the organic residue suspended in MeOH. The filtration of resulting solid afforded title compound as white powder (192 mg, yield 59%). ¹H NMR(400 MHz, DMSO-*d₆*) δ 12.39 (s, 1H), 8.43 (dd, J = 4.5, 1.4 Hz, 2H), 7.64 (dd, J = 8.8, 2.3 Hz, 1H), 7.61 - 7.54 (m, 2H), 7.54 - 7.47 (m, 1H), 7.47 - 7.38 (m, 2H), 7.26 (d, J = 7.5 Hz, 1H), 6.93 (d, J = 2.3 Hz, 1H), 6.77 (d, J = 6.0 Hz, 2H), 5.34 (dd, J = 12.1, 4.5 Hz, 1H), 3.78 (dd, J = 18.5, 12.2 Hz, 1H), 2.82 (dd, J = 18.5, 4.6 Hz, 1H), 1.91 (s, 3H). ESI-MS for C₂₅H₁₉ClN₄O₂: calculated 442.1, found 443.0 [M+H]⁺.

### Example d.98. 4-(3-(6-chloro-2-oxo-4-phenyl-1,2-dihydroquinolin-3-yl)-5-(pyridin-3-yl)-4,5-dihydro-1H-pyrazol-1-yl)-4-oxobutanoic acid

### Step 1. Synthesis of (E)-6-chloro-4-phenyl-3-(3-(pyridin-3-yl)acryloyl)quinolin-2(1H)-one (b.29)

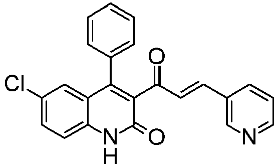

In a round-bottomed flask, the appropriate 3-acetyl-6-chloro-4-phenylquinolin-2(1H)-one (500 mg, 3.36 mmol) and potassium hydroxide (2.4 g, 42.0 mmol) were stirred in EtOH/H₂O (4:3 v/v, 0.05 M) at 0 °C for 45 min prior to the addition of 3-pyridine carboxaldehyde (0.237 mL, 2.52 mmol). The reaction mixture was stirred for 16 h as it gradually reached room temperature. EtOH was removed under reduced pressure and the aqueous phase was extracted with DCM (3 × 15 mL), the organic layer was then dried over Na₂SO₄, and solvent removed under reduced pressure. Final normal flash silica gel chromatography purification (solvent A: DCM, solvent B: MeOH - gradient: 5-10% solvent B) yielded title compound as a yellow solid (670 mg, yield 99%). ¹H NMR(400 MHz, CDCl₃) δ 12.62 (s, 1H), 8.53 - 8.38 (m, 2H), 7.60 (d, J = 8.0 Hz, 1H), 7.35 - 7.27 (m, 5H), 7.27 - 7.19 (m, 1H), 7.19 - 7.11 (m, 4H), 6.66 (d, J = 16.3 Hz, 1H).

### Step 2. Synthesis of 6-chloro-4-phenyl-3-(5-(pyridin-3-yl)-4,5-dihydro-1H-pyrazol-3-yl)quinolin-2(1H)-one (c.29)

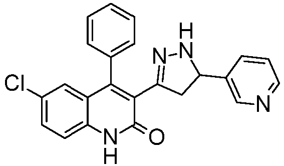

In appropriately sized microwaveable vessel, b.29 (650 mg, 1.68 mmol) was suspended in ethanol (4.0 ml), and then hydrazine hydrate (147 µl, 3.02 mmol) was added. The reaction mixture was microwaved at 110°C for 45 minutes, then the EtOH was removed under reduced pressure, diethyl ether was added to the white residue and the filtration of the precipitated afforded the titled compound as yellow solid (550 mg, yield 82%). ¹H NMR(400 MHz, CDCl₃) δ 11.88 (s, 1H), 8.52 - 8.40 (m, 2H), 7.52 - 7.36 (m, 5H), 7.29 - 7.18 (m, 4H), 7.18 - 7.09 (m, 2H), 4.78 - 4.62 (m, 1H), 3.19 (dd, J = 16.7, 10.9 Hz, 1H), 2.75 (dd, J = 16.7, 7.2 Hz, 1H).

### Step 3. Synthesis of 4-(3-(6-chloro-2-oxo-4-phenyl-1,2-dihydroquinolin-3-yl)-5-(pyridin-3-yl)-4,5-dihydro-1H-pyrazol-1-yl)-4-oxobutanoic acid (d.98).

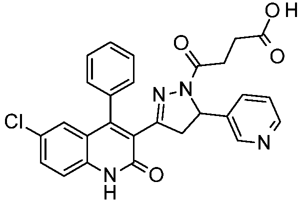

Compound d.98 was synthesized following the general procedure D1 using c.29 (73 mg, 0.18 mmol) with succinic anhydride (120 °C, 200 W). THF was removed under reduced pressure, the organic residue suspended in MeOH and the resulting solid filtered to afford the title compound as yellowish powder (25 mg, yield 27 %). ¹H NMR(400 MHz, DMSO-*d*₆) δ 12.40 (s, 1H), 11.97 (s, 1H), 8.42 (d, J = 4.8 Hz, 1H), 8.23 (s, 1H), 7.64 (d, J = 8.9 Hz, 1H), 7.58 - 7.37 (m, 5H), 7.32 - 7.19 (m, 2H), 7.04 (d, J = 7.7 Hz, 1H), 6.94 (s, 1H), 5.38 (dd, J = 11.7, 4.3 Hz, 1H), 3.74 (dd, J = 18.6, 11.9 Hz, 1H), 2.88 (dd, J = 18.4, 4.3 Hz, 1H), 2.44 (d, J = 6.4 Hz, 2H), 2.28 (t, J = 6.7 Hz, 2H). ESI-MS for C₂₇H₂₁ClN₄O₄: calculated 500.1, found m/z 501.2 [M+H]⁺.

### Example d.99. 4-(3-(6-chloro-2-oxo-4-phenyl-1,2-dihydroquinolin-3-yl)-5-(pyridin-2-yl)-4,5-dihydro-1H-pyrazol-1-yl)-4-oxobutanoic acid

### Step 1. Synthesis of (E)-6-chloro-4-phenyl-3-(3-(pyridin-2-yl)acryloyl)quinolin-2(1H)-one (b.30)

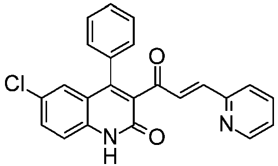

In a round-bottomed flask, the appropriate 3-acetyl-6-chloro-4-phenylquinolin-2(1H)-one (1.0 g, 3.36 mmol) and potassium hydroxide (4.7 g, 84.0 mmol) were stirred in EtOH/H₂O (4:3 v/v, 0.05 M) at 0 °C for 45 min prior to the addition of 2-pyridine carboxaldehyde (0.576 mL, 6.05 mmol). The reaction mixture was stirred for 16 h as it gradually reached room temperature. EtOH was removed under reduced pressure and the aqueous phase was extracted with DCM (3 × 20 mL), the organic layer was then dried over Na₂SO₄, and solvent removed under reduced pressure. Final normal flash silica gel chromatography purification (solvent A: DCM, solvent B: MeOH - gradient: 1-3% solvent B) yielded title compound as a yellow solid (753 mg, yield 58%). ¹H NMR(400 MHz, CDCl₃) δ 12.42 (s, 1H), 8.61 (d, J = 3.9 Hz, 1H), 7.83 (dd, J = 7.7, 1.8 Hz, 1H), 7.73 - 7.62 (m, 2H), 7.54 - 7.36 (m, 6H), 7.32 (dd, J = 7.6, 1.8 Hz, 2H), 7.01 (dd, J = 17.2, 9.2 Hz, 2H) .

### Step 2. Synthesis of 6-chloro-4-phenyl-3-(5-(pyridin-2-yl)-4,5-dihydro-1H-pyrazol-3-yl)quinolin-2(1H)-one (c.30)

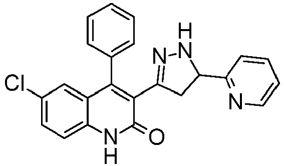

In appropriately sized microwaveable vessel, b.30 (655 mg, 1.69 mmol) was suspended in ethanol (3.5 ml), and then hydrazine hydrate (124 µl, 2.54 mmol) was added. The reaction mixture was microwaved at 110°C for 45 minutes, then the EtOH was removed under reduced pressure, diethyl ether was added to the white residue and the filtration of the precipitated afforded the titled compound as yellow solid (345 g, yield 51%). ¹H NMR(400 MHz, DMSO-*d₆*) δ 12.19 (s, 1H), 8.43 (d, J = 4.0 Hz, 1H), 7.69 (td, J = 7.7, 1.8 Hz, 1H), 7.57 (dd, J = 8.8, 2.4 Hz, 1H), 7.54 - 7.35 (m, 4H), 7.29 (dd, J = 13.7, 5.2 Hz, 2H), 7.26 - 7.20 (m, 1H), 7.18 (d, J = 7.4 Hz, 1H), 7.09 (d, J = 7.9 Hz, 1H), 6.89 (d, J = 2.3 Hz, 1H), 4.77 - 4.50 (m, 1H), 3.27 - 3.08 (m, 1H), 2.77 (dd, J = 16.5, 8.8 Hz, 1H).

### Step 3. Synthesis of 4-(3-(6-chloro-2-oxo-4-phenyl-1,2-dihydroquinolin-3-yl)-5-(pyridin-2-yl)-4,5-dihydro-1H-pyrazol-1-yl)-4-oxobutanoic acid (d.99).

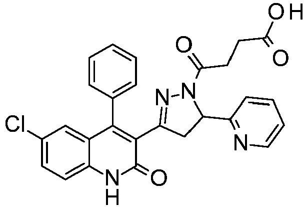

Compound d.99 was synthesized following the general procedure D1 using c.30 (150 mg, 0.37 mmol) with succinic anhydride (120 °C, 200 W). THF was removed under reduced pressure, the organic residue suspended in MeOH and the resulting solid filtered to afford the title compound as white powder (130 mg, yield 70%). ¹H NMR(400 MHz, DMSO-*d₆*) δ 12.36 (s, 1H), 12.04 (s, 1H), 8.45 (d, J = 4.1 Hz, 1H), 7.69 - 7.56 (m, 2H), 7.54 - 7.38 (m, 4H), 7.36 (d, J = 7.2 Hz, 1H), 7.26 - 7.19 (m, 2H), 6.93 (d, J = 2.3 Hz, 1H), 6.66 (d, J = 7.9 Hz, 1H), 5.33 (dd, J = 12.1, 4.9 Hz, 1H), 3.71 (dd, J = 18.3, 12.1 Hz, 1H), 3.01 (dd, J = 18.3, 4.9 Hz, 1H), 2.44 - 2.34 (m, 2H), 2.33 - 2.26 (m, 2H). ESI-MS for C₂₇H₂₁ClN₄O₄: calculated 500.1, found m/z 501.2 [M+H]⁺.

### Example d.100. 4-[5-(4-benzyl-6-chloro-2-oxo-1H-quinolin-3-yl)-3-(3-pyridyl)-3,4-dihydropyrazol-2-yl]-4-oxo-butanoic acid (example not falling within the scope of the present invention).

### Step 1. Synthesis of 4-benzyl-6-chloro-3-[(E)-3-(3-pyridyl)prop-2-enoyl]-1H-quinolin-2-one (f.18)

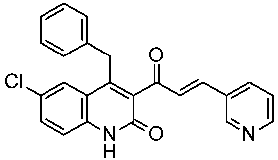

In a round-bottom flask, e.1 (200 mg, 0.64 mmol) was suspended in a 4:3 mixture of ethanol (7 ml) and water (5 ml). The mixture was cooled to 0 °C and potassium hydroxide (898 mg, 16 mmol) was added. After stirring for 45 minutes at 0 °C, 3-pyridinecarbaldehyde (108 µL, 1.15 mmol) was added. The reaction was stirred at room temperature for 16 hours, afterwards acetic acid (0.923 mL, 16 mmol) was added at 0 °C, the solid was dissolved in CH₂Cl₂ (50 mL) and washed with brine (50 mL). The solvent was evaporated in vacuo to yield the title compound as a white solid. (209 mg, yield 81 %). ¹H NMR(400 MHz, DMSO-*d₆*) δ 12.24 (s, 1H), 8.84 (d, J = 2.2 Hz, 1H), 8.58 (dd, J = 4.8, 1.6 Hz, 1H), 8.17 (dt, J = 8.0, 2.0 Hz, 1H), 7.67 (d, J = 2.3 Hz, 1H), 7.64 - 7.51 (m, 2H), 7.44 (dd, J = 8.1, 4.8 Hz, 1H), 7.40 (d, J = 8.8 Hz, 1H), 7.30 - 7.18 (m, 5H), 7.18 - 7.10 (m, 1H), 4.14 (s, 2H). Rt 2.21 min (generic method) .ESI-MS for C₂₄H₁₇ClN₂O₂: calculated 400.1, found m/z 401.0/403.0 [M+H]⁺.

### Step 2. Synthesis of 4-benzyl-6-chloro-3-[5-(3-pyridyl)-4,5-dihydro-1H-pyrazol-3-yl]-1H-quinolin-2-one. (g.18).

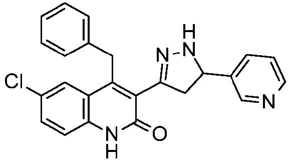

In appropriately sized microwaveable vessel, f.18 (197 mg, 0.49 mmol) was suspended in ethanol (4 ml), then hydrazine hydrate (41 µL, 0.84 mmol) added, the reaction mixture microwaved at 110 °C (200 W) for 20 minutes, cooled at room temperature and resulting yellow solid filtered and washed with EtOH (20 mL) to yield title compound (140 mg, yield 66 %). ¹H NMR(400 MHz, DMSO-*d₆*) δ 12.07 (s, 1H), 8.59 (d, J = 2.3 Hz, 1H), 8.47 (dd, J = 4.8, 1.6 Hz, 1H), 7.80 (dt, J = 8.0, 2.0 Hz, 1H), 7.67 (d, J = 2.3 Hz, 1H), 7.59 (d, J = 3.4 Hz, 1H), 7.50 (dd, J = 8.8, 2.2 Hz, 1H), 7.38 - 7.31 (m, 2H), 7.30 - 7.23 (m, 4H), 7.21 - 7.14 (m, 1H), 4.85 (td, J = 10.5, 3.4 Hz, 1H), 4.47 - 4.38 (m, 2H), 3.44 - 3.33 (m, 1H), 2.92 (dd, J = 16.4, 10.3 Hz, 1H). Rt 1.98 min (generic method) . ESI-MS for C₂₄H₁₉ClN₄O: calculated 414.1, found m/z 415.2/417.2 [M+H]⁺.

### Step 3. Synthesis of 4-[5-(4-benzyl-6-chloro-2-oxo-1H-quinolin-3-yl)-3-(3-pyridyl)-3,4-dihydropyrazol-2-yl]-4-oxo-butanoic acid (d.100).

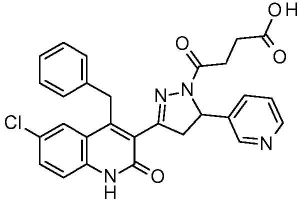

Compound d.100 was synthesized following the general procedure D1 using g.18 (120 mg, 0.29 mmol) with succinic anhydride (120 °C, 200 W). Purification by normal phase flash column chromatography (solvent A: CH₂Cl₂, solvent B: MeOH - gradient: 0-10 % solvent B) yielded title compound (70 mg, yield 47 %). ¹H NMR(400 MHz, DMSO-*d*₆) δ 12.21 (s, 2H), 8.51 (d, J = 2.3 Hz, 1H), 8.45 (dd, J = 4.8, 1.6 Hz, 1H), 7.79 (d, J = 2.3 Hz, 1H), 7.61 (dt, J = 7.9, 2.0 Hz, 1H), 7.57 (dd, J = 8.8, 2.2 Hz, 1H), 7.39 (d, J = 8.8 Hz, 1H), 7.34 - 7.23 (m, 5H), 7.21 - 7.15 (m, 1H), 5.51 (dd, J = 12.0, 4.9 Hz, 1H), 4.53 - 4.33 (m, 2H), 3.72 (dd, J = 18.4, 12.1 Hz, 1H), 3.16 (dd, J = 18.4, 4.9 Hz, 1H), 2.73 - 2.52 (m, 2H), 2.33 (t, J = 6.8 Hz, 2H) . Rt 1.78 min (generic method). ESI-MS for C₂₈H₂₃ClN₄O₄: calculated 514.1, found *m*/*z* 515.2/517.3 [M+H]⁺.

### Example d.101. 4-[5-(4-benzyl-6-chloro-2-oxo-1H-quinolin-3-yl)-3-(2-methylindazol-5-yl)-3,4-dihydropyrazol-2-yl]-4-oxo-butanoic acid

### Step 1. Synthesis of 4-benzyl-6-chloro-3-[(E)-3-(2-methylindazol-5-yl)prop-2-enoyl]-1H-quinolin-2-one (f.19)

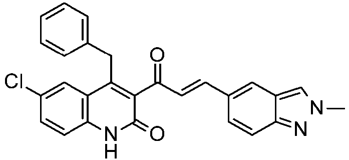

In a round-bottom flask, e.1 (130 mg, 0.42 mmol) was suspended in a 4:3 mixture of ethanol (5 ml) and water (4 ml). The mixture was cooled to 0 °C and potassium hydroxide (590 mg, 10.5 mmol) was added. After stirring for 45 minutes at 0 °C, 2-methylindazole-5-carbaldehyde (100 mg, 0.63 mmol) was added. The reaction was stirred at room temperature for 16 hours, afterwards acetic acid (0.606 mL, 10.5 mmol) was added at 0 °C, resulting precipitate filtered, washed with ethanol (20 mL), dissolved in CH₂Cl₂ (50 mL, washed with water (50 mL) and brine (50 mL). The solvent was evaporated in vacuo to yield the title compound as a yellow/orange solid (137 mg, yield 72 %). ¹H NMR(400 MHz, DMSO-*d₆*) δ 12.20 (s, 1H), 8.44 (s, 1H), 8.04 (s, 1H), 7.68 - 7.61 (m, 3H), 7.61 - 7.52 (m, 2H), 7.40 (d, J = 8.8 Hz, 1H), 7.33 - 7.20 (m, 4H), 7.18 - 7.10 (m, 1H), 7.01 (d, J = 16.2 Hz, 1H), 4.18 - 4.12 (m, 5H). Rt 2.04 min (generic method).ESI-MS for C₂₇H₂₀ClN₃O₂: calculated 453.1, found m/z 452.3/454.2 [M+H]⁺.

### Step 2. Synthesis of 4-benzyl-6-chloro-3-[5-(2-methylindazol-5-yl)-4,5-dihydro-1H-pyrazol-3-yl]-1H-quinolin-2-one (g.19).

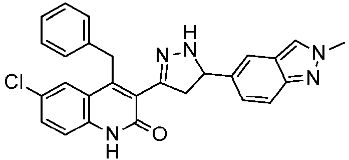

In appropriately sized microwaveable vessel, f.19 (136 mg, 0.30 mmol) was suspended in ethanol (3 ml), then hydrazine hydrate (25 µL, 0.51 mmol) added, the reaction mixture microwaved at 110 °C (200 W) for 20 minutes, cooled at room temperature and resulting yellow solid filtered filtered and washed with ethanol (20 mL) to yield title compound as an orange solid (98 g, yield 70 %). ¹H NMR(400 MHz, DMSO-*d₆*) δ 12.05 (s, 1H), 8.23 (s, 1H), 7.66 (d, J = 2.3 Hz, 1H), 7.62 (d, J = 1.4 Hz, 1H), 7.55 - 7.45 (m, 3H), 7.33 (d, J = 8.8 Hz, 1H), 7.30 - 7.22 (m, 5H), 7.21 - 7.13 (m, 1H), 4.86 (td, J = 10.8, 3.5 Hz, 1H), 4.52 - 4.38 (m, 2H), 4.14 (s, 3H), 3.36 (dd, J = 16.4, 10.6 Hz, 1H), 2.92 (dd, J = 16.3, 11.0 Hz, 1H). Rt 1.91 min (Generic method). ESI-MS for C₂₇H₂₂ClN₅O: calculated 467.2, found m/z 468.2/470.2 [M+H]⁺.

### Step 3. Synthesis of 4-[5-(4-benzyl-6-chloro-2-oxo-1H-quinolin-3-yl)-3-(2-methylindazol-5-yl)-3,4-dihydropyrazol-2-yl]-4-oxo-butanoic acid. (d.101).

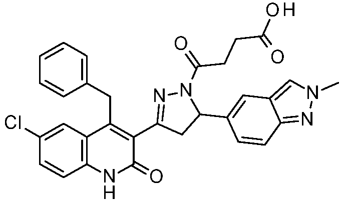

Compound d.101 was synthesized following the general procedure D1 using g.19 (83 mg, 0.18 mmol) with succinic anhydride (120 °C, 200 W). THF was evaporated and the resulted crude solid was triturated in CH₂Cl₂ (10 mL) and cold MeOH (10 mL), filtered and washed with diethyl ehter (10 mL) to obtain the title compound as an off-white solid (38 mg, yield 37 %). ¹H NMR(400 MHz, DMSO-*d*₆) δ 12.20 (s, 1H), 12.02 (s, 1H), 8.22 (s, 1H), 7.76 (d, J = 2.3 Hz, 1H), 7.56 (dd, J = 8.8, 2.3 Hz, 1H), 7.51 - 7.43 (m, 2H), 7.38 (d, J = 8.8 Hz, 1H), 7.32 - 7.23 (m, 4H), 7.21 - 7.13 (m, 1H), 7.08 (dd, J = 9.1, 1.6 Hz, 1H), 5.50 (dd, J = 11.9, 4.8 Hz, 1H), 4.49 (d, J = 15.6 Hz, 1H), 4.41 (d, J = 15.6 Hz, 1H), 4.14 (s, 3H), 3.74 (dd, J = 18.4, 11.9 Hz, 1H), 3.14 (dd, J = 18.4, 4.8 Hz, 1H), 2.73 - 2.57 (m, 2H), 2.32 (t, J = 6.7 Hz, 2H). Rt 1.81 min (Generic method). ESI-MS for C₃₁H₂₆ClN₅O₄ : calculated 567.2, found m/z 568.3/570.3 [M+H]⁺.

### Example d.102. 4-[5-(4-benzyl-6-chloro-2-oxo-1H-quinolin-3-yl)-3-(1-methylindol-3-yl)-3,4-dihydropyrazol-2-yl]-4-oxo-butanoic acid (example not falling within the scope of the present invention).

### Step 1. Synthesis of 4-benzyl-6-chloro-3-[(E)-3-(1-methylindol-3-yl)prop-2-enoyl]-1H-quinolin-2-one (f.20)

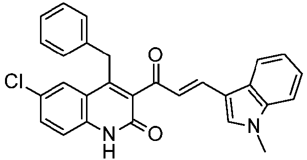

In a round-bottom flask, e.1 (0.2 g, 0.64 mmol) was suspended in a 4:3 mixture of ethanol (7 ml) and water (5 ml). The mixture was cooled to 0 °C and potassium hydroxide (1.346 g, 24 mmol) was added. After stirring for 45 minutes at 0 °C, 1-methylindole-3-carbaldehyde (183 mg, 1.15 mmol) was added. The reaction was stirred for 16 h at room temperature and then at 40 °C for 16 hours. Afterwards acetic acid (1.384 mL, 24 mmol) was added at 0 °C, resulting in a solid that was filtered and washed with water (20 mL) and ethanol (20 mL). The solid was dissolved in CH₂Cl₂ (50 mL) and washed with water (50 mL x 3), the organic solvent evaporated *in vacuo* to finally yield title compound as a brown solid (0.190 g, yield 66%). ¹H NMR(600 MHz, DMSO-*d₆*) δ 12.17 (s, 1H), 7.98 (s, 1H), 7.89 (dd, J = 8.0, 1.1 Hz, 1H), 7.73 (d, J = 16.1 Hz, 1H), 7.61 (d, J = 2.3 Hz, 1H), 7.57 - 7.51 (m, 2H), 7.38 (d, J = 8.8 Hz, 1H), 7.33 - 7.27 (m, 3H), 7.27 - 7.19 (m, 3H), 7.17 - 7.13 (m, 1H), 6.88 (d, J = 16.1 Hz, 1H), 4.16 (s, 2H), 3.81 (s, 3H). Rt 2.35 min (generic method). ESI-MS for C₂₈H₂₁ClN₂O₂: calculated 452.1, found m/z 453.1/455.2 [M+H]⁺.

### Step 2. Synthesis of 4-benzyl-6-chloro-3-[5-(1-methylindol-3-yl)-4,5-dihydro-1H-pyrazol-3-yl]-1H-quinolin-2-one (g.20).

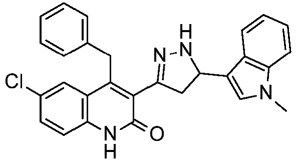

In appropriately sized microwaveable vessel, f.20 (0.117 g, 0.24 mmol) was suspended in ethanol (2.5 ml), then hydrazine hydrate (116 µL, 2.4 mmol) added, the reaction mixture microwaved at 110 °C (200 W) for 60 minutes, cooled at room temperature and resulting yellow solid filtered to yield title compound (0.98 g, yield 88%). ¹H NMR(600 MHz, DMSO-*d₆*) δ 12.07 (s, 1H), 7.66 (d, J = 2.3 Hz, 1H), 7.59 (dt, J = 8.0, 1.0 Hz, 1H), 7.49 (dd, J = 8.8, 2.3 Hz, 1H), 7.38 (dt, J = 8.2, 0.9 Hz, 1H), 7.34 (d, J = 8.8 Hz, 1H), 7.32 - 7.22 (m, 6H), 7.20 - 7.11 (m, 2H), 6.96 (ddd, J = 8.0, 7.0, 1.0 Hz, 1H), 5.03 (td, J = 10.5, 3.3 Hz, 1H), 4.51 (d, J = 15.3 Hz, 1H), 4.44 (d, J = 15.3 Hz, 1H), 3.69 (s, 3H), 3.31 - 3.28 (m, 1H), 3.08 (dd, 1H). Rt 2.38 min (generic method) .ESI-MS for C₂₈H₂₃ClN₄O: calculated 466.2, found m/z 467.3/469.1 [M+H]⁺.

### Step 3. Synthesis of 4-[5-(4-benzyl-6-chloro-2-oxo-1H-quinolin-3-yl)-3-(1-methylindol-3-yl)-3,4-dihydropyrazol-2-yl]-4-oxo-butanoic acid (d.102).

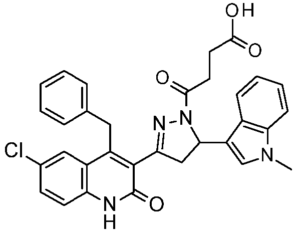

Compound d.102 was synthesized following the general procedure D1 using g.20 (50 mg, 0.11 mmol) with succinic anhydride (120 °C, 200 W). Purification by normal phase flash column chromatography (solvent A: DCM, solvent B: MeOH - gradient: 0-10% solvent B) yielded title compound (52 mg, yield 83%). ¹H NMR(400 MHz, DMSO-*d*₆) δ 12.22 (s, 1H), 12.11 (s, 1H), 7.75 (d, J = 2.3 Hz, 1H), 7.62 - 7.51 (m, 2H), 7.41 - 7.33 (m, 2H), 7.31 - 7.21 (m, 4H), 7.20 - 7.08 (m, 3H), 6.97 - 6.89 (m, 1H), 5.73 (dd, J = 11.7, 4.8 Hz, 1H), 4.51 (d, J = 15.6 Hz, 1H), 4.43 (d, J = 15.5 Hz, 1H), 3.73 (dd, J = 18.1, 11.8 Hz, 1H), 3.66 (s, 3H), 3.29 (dd, J = 18.1, 4.9 Hz, 1H), 2.61 (t, J = 6.8 Hz, 2H), 2.31 (t, J = 6.7 Hz, 2H). Rt 2.07 min (generic method). ESI-MS for C₃₂H₂₇ClN₄O₄: calculated 566.2, found m/z 567.2/569.2 [M+H]⁺.

### Example d.103. 4-benzyl-6-chloro-3-[3-(6-methoxy-3-pyridyl)-2-propanoyl-3,4-dihydropyrazol-5-yl]-1H-quinolin-2-one (example not falling within the scope of the present invention).

### Step 1. Synthesis of 4-benzyl-6-chloro-3-[(E)-3-(6-methoxy-3-pyridyl)prop-2-enoyl]-1H-quinolin-2-one (f.21)

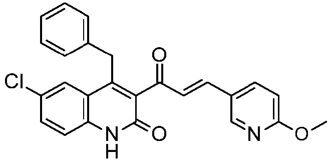

In a round-bottom flask, e.1 (0.200 g, 0.64 mmol) was suspended in a 4:3 mixture of ethanol (7 ml) and water (5 ml). The mixture was cooled to 0 °C and potassium hydroxide (0.898 g, 16 mmol) was added. After stirring for 45 minutes at 0 °C, 6-methoxy-3-pyridinecarboxaldehyde (132 mg, 0.96 mmol) was added. The reaction was stirred at room temperature for 45 minutes, afterwards acetic acid (0.923 mL, 16 mmol) was added at 0 °C, resulting in a solid that was filtered and washed with water (20 mL) and ethanol (20 mL). The solid was dissolved in CH₂Cl₂ (50 mL) and washed with water (50 mL x 3), the organic solvent evaporated *in vacuo* to finally yield title compound as an off-white-yellow solid (0.405 g, yield quant.). ¹H NMR(400 MHz, DMSO-*d₆*) δ 12.32 (s, 1H), 8.44 (d, J = 2.4 Hz, 1H), 8.14 (dd, J = 8.8, 2.4 Hz, 1H), 7.63 (d, J = 2.3 Hz, 1H), 7.59 - 7.42 (m, 3H), 7.30 - 7.20 (m, 4H), 7.18 - 7.10 (m, 1H), 7.04 (d, J = 16.3 Hz, 1H), 6.86 (d, J = 8.7 Hz, 1H), 4.12 (s, 2H), 3.88 (s, 3H). Rt 2.28 min (generic method) .ESI-MS for C₂₅H₁₉ClN₂O₃: calculated 430.1, found m/z 431.2/433.2 [M+H]⁺.

### Step 2. Synthesis of 4-benzyl-6-chloro-3-[5-(6-methoxy-3-pyridyl)-4,5-dihydro-1H-pyrazol-3-yl]-1H-quinolin-2-one (g.21).

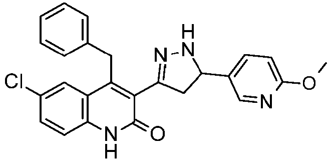

In appropriately sized microwaveable vessel, f.21 (0.400 g, 0.93 mmol) was suspended in ethanol (3 ml), then hydrazine hydrate (77 µL, 1.58 mmol) added, the reaction mixture microwaved at 110 °C (200 W) for 25 minutes, cooled at room temperature and resulting yellow solid filtered to yield title compound (0.352 g, yield 85%). ¹H NMR(400 MHz, DMSO-*d₆*) δ 12.15 (s, 1H), 8.14 (d, J = 2.4 Hz, 1H), 7.74 (dd, J = 8.6, 2.5 Hz, 1H), 7.66 (d, J = 2.2 Hz, 1H), 7.49 (dd, J = 8.7, 2.2 Hz, 1H), 7.40 (d, J = 8.8 Hz, 1H), 7.28 - 7.17 (m, 4H), 7.20 - 7.12 (m, 1H), 6.76 (d, J = 8.6 Hz, 1H), 4.77 (t, J = 10.3 Hz, 1H), 4.41 (s, 2H), 3.82 (s, 3H), 3.32 - 3.24 (m, 1H), 2.89 (dd, J = 16.4, 10.2 Hz, 1H). Rt 2.14 min (generic method) . ESI-MS for C₂₅H₂₁ClN₄O₂: calculated 444.1, found m/z 445.3/447.2 [M+H]⁺.

### Step 3. Synthesis of 4-benzyl-6-chloro-3-[3-(6-methoxy-3-pyridyl)-2-propanoyl-3,4-dihydropyrazol-5-yl]-1H-quinolin-2-one (d.103).

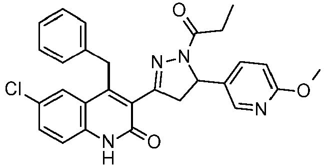

Compound d.103 was synthesized following the general procedure D2 using g.21 (111 mg, 0.25 mmol). Purification by normal phase flash column chromatography (solvent A: DCM, solvent B: MeOH - gradient: 0-10 % solvent B) yielded title compound as a yellow solid(31 mg, yield 25%) . ¹H NMR(400 MHz, DMSO-*d₆*) δ 12.20 (s, 1H), 8.10 (d, J = 2.5 Hz, 1H), 7.74 (d, J = 2.3 Hz, 1H), 7.61 - 7.53 (m, 2H), 7.39 (d, J = 8.8 Hz, 1H), 7.31 - 7.23 (m, 4H), 7.23 - 7.15 (m, 1H), 6.75 (d, J = 8.5 Hz, 1H), 5.45 (dd, J = 12.0, 4.6 Hz, 1H), 4.42 (s, 2H), 3.83 (s, 3H), 3.69 (dd, J = 18.4, 12.0 Hz, 1H), 3.19 (dd, J = 18.2, 4.7 Hz, 1H), 2.31 (qd, J = 7.7, 4.7 Hz, 2H), 0.83 (t, J = 7.5 Hz, 3H). Rt 2.27 min (generic method). ESI-MS for C₂₈H₂₅ClN₄O₃: calculated 500.2, found m/z 501.3/503.2 [M+H] ⁺.

### Example d.104. 4-[5-(4-benzyl-6-chloro-2-oxo-1H-quinolin-3-yl)-3-(6-methoxy-3-pyridyl)-3,4-dihydropyrazol-2-yl]-4-oxo-butanoic acid (example not falling within the scope of the present invention).

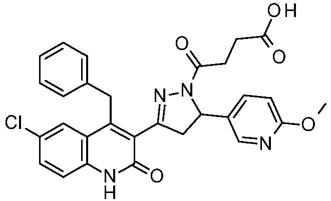

Compound d.104 was synthesized following the general procedure D1 using g.21 (116 mg, 0.26 mmol). THF was evaporated and crude dissolved in a mixture of DCM/CHCl₃ (50 mL) and washed with aq. HCl 0.1 (50 mL). Purification by normal phase flash column chromatography (solvent A: DCM, solvent B: MeOH - gradient: 0-10 % solvent B) yielded title compound as a white solid (8 mg, yield 6%). ¹H NMR(400 MHz, DMSO-*d₆*) δ 12.22 (s, 1H), 8.09 (d, J = 2.5 Hz, 1H), 7.79 (d, J = 2.3 Hz, 1H), 7.60 - 7.52 (m, 2H), 7.39 (d, J = 8.8 Hz, 1H), 7.31 - 7.21 (m, 4H), 7.21 - 7.13 (m, 1H), 6.72 (d, J = 8.7 Hz, 1H), 5.44 (dd, J = 12.0, 4.6 Hz, 1H), 4.50 - 4.36 (m, 2H), 3.82 (s, 3H), 3.65 (dd, J = 18.3, 11.9 Hz, 1H), 3.13 (dd, J = 18.4, 4.6 Hz, 1H), 2.68 - 2.53 (m, 2H), 2.29 (t, J = 6.8 Hz, 2H). Rt 1.87 min (generic method). ESI-MS for C₂₉H₂₅ClN₄O₅: calculated 544.2, found m/z 545.1/547.2 [M+H]⁺.

### Example d.105. 4-[5-(4-benzyl-6-chloro-2-oxo-1H-quinolin-3-yl)-3-(2-chlorophenyl)-3,4-dihydropyrazol-2-yl]-4-oxo-butanoic acid

### Step 1. Synthesis of 4-benzyl-6-chloro-3-[(E)-3-(2-chlorophenyl)prop-2-enoyl]-1H-quinolin-2-one (f.22)

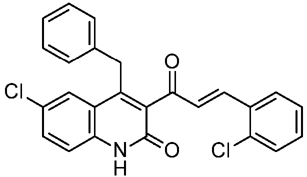

In a round-bottom flask, e.1 (300 mg, 0.96 mmol) was suspended in a 4:3 mixture of ethanol (11 ml) and water (8 ml). The mixture was cooled to 0 °C and potassium hydroxide (1.350 g, 24 mmol) was added. After stirring for 45 minutes at 0 °C, 2-chlorobenzaldehyde (243 mg, 1.73 mmol) was added. The reaction was stirred at room temperature for 3 hours, afterwards acetic acid (1.384 mL, 24 mmol) was added at 0 °C, resulting yellow solid filtered and washed with EtOH (20 mL). The solid was dissolved in CH₂Cl₂ (50 mL) and washed with water (50 mL), the organic solvent evaporated *in vacuo,* to finally yield title compound as a yellow solid (326 g, yield 78%). ¹H NMR(400 MHz, DMSO-*d₆*) δ 12.30 (s, 1H), 7.95 (dd, J = 7.8, 1.8 Hz, 1H), 7.76 (d, J = 16.2 Hz, 1H), 7.71 (d, J = 2.3 Hz, 1H), 7.58 (dd, J = 8.8, 2.3 Hz, 1H), 7.54 (dd, J = 8.0, 1.4 Hz, 1H), 7.50 - 7.38 (m, 3H), 7.29 - 7.24 (m, 4H), 7.21 (d, J = 16.2 Hz, 1H), 7.18 - 7.12 (m, 1H), 4.18 (s, 2H). Rt 2.62 min (generic method).ESI-MS for C₂₅H₁₇Cl₂NO₂: calculated 433.1, found m/z 434.1/436.1/438.2 [M+H] ⁺.

### Step 2. Synthesis of 4-benzyl-6-chloro-3-[5-(2-chlorophenyl)-4,5-dihydro-1H-pyrazol-3-yl]-1H-quinolin-2-one (g.22).

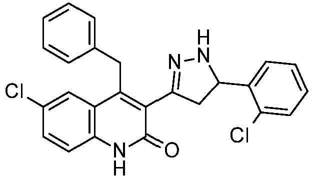

In appropriately sized microwaveable vessel, f.22 (313.0 mg, 0.72 mmol) was suspended in ethanol (1.3 ml), then hydrazine hydrate (90 µL, 1.44 mmol) added, the reaction mixture microwaved at 110 °C (200 W) for 25 minutes, cooled at room temperature and resulting solid filtered to yield title compound (300 mg, yield 93%). ¹H NMR(400 MHz, DMSO-*d₆*) δ 12.04 (s, 1H), 7.70 - 7.61 (m, 2H), 7.55 (d, J = 3.6 Hz, 1H), 7.49 (dd, J = 8.8, 2.2 Hz, 1H), 7.46 - 7.39 (m, 1H), 7.33 (d, J = 8.7 Hz, 1H), 7.31 - 7.27 (m, 2H), 7.25 (s, 3H), 7.19 - 7.12 (m, 1H), 5.07 (td, J = 10.6, 3.6 Hz, 1H), 4.45 (d, J = 15.3 Hz, 1H), 4.38 (d, J = 15.3 Hz, 1H), 3.51 (dd, J = 16.5, 10.8 Hz, 1H), 2.71 (dd, J = 16.5, 10.4 Hz, 1H). Rt 1.41 min (apolar method). ESI-MS for C₂₅H₁₉Cl₂N₃O: calculated 447.1, found m/z 448.1/ 450.1/452.1 [M+H]⁺.

### Step 3. Synthesis of 4-[5-(4-benzyl-6-chloro-2-oxo-1H-quinolin-3-yl)-3-(2-chlorophenyl)-3,4-dihydropyrazol-2-yl]-4-oxo-butanoic acid (d.105).

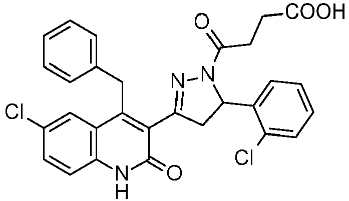

Compound d.105 was synthesized following the general procedure D1 using g.22 (100 mg, 0.22 mmol) with succinic anhydride (120 °C, 200 W). THF was evaporated and the resulted crude solid was triturated in CH₂Cl₂ (2 mL) yielding title compound (102 mg, yield 83 %). ¹H NMR(400 MHz, DMSO-*d*₆) δ 12.20 (s, 1H), 7.78 (d, J = 2.3 Hz, 1H), 7.56 (dd, J = 8.8, 2.3 Hz, 1H), 7.46 (dd, J = 7.8, 1.5 Hz, 1H), 7.37 (d, J = 8.8 Hz, 1H), 7.33 - 7.21 (m, 6H), 7.21 - 7.12 (m, 1H), 7.08 (dd, J = 7.6, 1.9 Hz, 1H), 5.66 (dd, J = 12.0, 5.4 Hz, 1H), 4.49 (d, J = 15.5 Hz, 1H), 4.39 (d, J = 15.5 Hz, 1H), 3.82 (dd, J = 18.2, 12.0 Hz, 1H), 2.99 (dd, J = 18.2, 5.3 Hz, 1H), 2.78 (dt, J = 17.0, 6.8 Hz, 1H), 2.65 - 2.54 (m, 1H), 2.45 - 2.29 (m, 2H). Rt 0.80 min (Apolar method). ESI-MS for C₂₉H₂₃Cl₂N₃O₄: calculated 547.1, found *m*/z 548.1/550.1/560.1 [M+H]⁺.

### Example d.106. 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid

### Step 1. Synthesis of 4-benzyl-6-chloro-3-[(E)-3-(3,4-dimethoxyphenyl)prop-2-enoyl]-1H-quinolin-2-one (f.23)

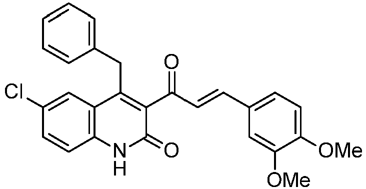

In a round-bottom flask, e.1 (300 mg, 0.96 mmol) was suspended in a 4:3 mixture of ethanol (11 ml) and water (8 ml). The mixture was cooled to 0 °C and potassium hydroxide (1.350 g, 24 mmol) was added. After stirring for 45 minutes at 0 °C, 3,4-dimethoxybenzaldehyde (287 mL, 1.73 mmol) was added. The reaction was stirred at room temperature for 16 hours, afterwards acetic acid (1.384 mL, 24 mmol) was added at 0 °C, resulting yellow solid filtered and washed with EtOH (20 mL). The solid was dissolved in CH₂Cl₂ (50 mL) and washed with water (50 mL), the organic solvent evaporated *in vacuo,* to finally yield title compound as a yellow solid (363 g, yield 82%). ¹H NMR(400 MHz, DMSO-*d₆*) δ 12.18 (s, 1H), 7.62 (d, J = 2.3 Hz, 1H), 7.55 (dd, J = 8.8, 2.3 Hz, 1H), 7.48 (d, J = 16.2 Hz, 1H), 7.38 (d, J = 8.8 Hz, 1H), 7.33 - 7.22 (m, 6H), 7.19 - 7.11 (m, 1H), 7.04 - 6.94 (m, 2H), 4.12 (s, 2H), 3.82 - 3.74 (m, 6H). Rt 2.29 min (generic method).ESI-MS for C₂₇H₂₂ClNO₄: calculated 459.1, found m/z 460.2/462.2 [M+H]⁺.

### Step 2. Synthesis of 4-benzyl-6-chloro-3-[5-(3,4-dimethoxyphenyl)-4,5-dihydro-1H-pyrazol-3-yl]-1H-quinolin-2-one (g.23).

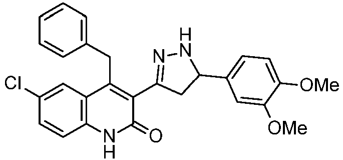

In appropriately sized microwaveable vessel, f.23 (353 mg, 0.77 mmol) was suspended in ethanol (1.4 ml), then hydrazine hydrate (96 µL, 1.54 mmol) added, the reaction mixture microwaved at 110 °C (200 W) for 25 minutes, cooled at room temperature and resulting yellow solid filtered to yield title compound (330 g, yield 91%). ¹H NMR(400 MHz, DMSO-*d₆*) δ 12.08 (s, 1H), 7.66 (d, J = 2.3 Hz, 1H), 7.50 (dd, J = 8.8, 2.2 Hz, 1H), 7.38 (d, J = 2.9 Hz, 1H), 7.34 (d, J = 8.8 Hz, 1H), 7.31 - 7.21 (m, 4H), 7.24 - 7.12 (m, 1H), 7.07 (d, J = 1.6 Hz, 1H), 6.92 - 6.80 (m, 2H), 4.74 (td, J = 10.1, 2.9 Hz, 1H), 4.42 (s, 2H), 3.73 (s, 3H), 3.72 (s, 3H), 3.33 - 3.25 (m, 1H), 2.92 (dd, J = 16.4, 9.6 Hz, 1H).Rt 0.94 min (Apolar method). ESI-MS for C₂₇H₂₄ClN₃O₃: calculated 473.2, found m/z 474.1/476.1 [M+H]⁺.

### Step 3. Synthesis of 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid (d.106).

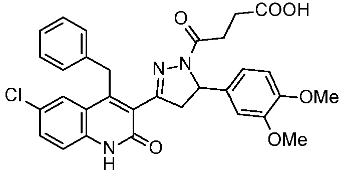

Compound d.106 was synthesized following the general procedure D1 using g.23 (100 mg, 0.21 mmol) with succinic anhydride (120 °C, 200 W). THF was evaporated and the resulted crude solid was triturated in CH₂Cl₂ (2 mL) yielding title compound (89 mg, yield 73 %). ¹H NMR(400 MHz, DMSO-*d*₆) δ 12.26 (s, 1H), 7.75 (d, J = 2.3 Hz, 1H), 7.56 (dd, J = 8.8, 2.2 Hz, 1H), 7.37 (d, J = 8.8 Hz, 1H), 7.33 - 7.22 (m, 4H), 7.22 - 7.13 (m, 1H), 6.91 (d, J = 2.0 Hz, 1H), 6.82 (d, J = 8.3 Hz, 1H), 6.76 (dd, J = 8.3, 2.0 Hz, 1H), 5.40 (dd, J = 11.8, 4.2 Hz, 1H), 4.44 (d, J = 15.5 Hz, 1H), 4.36 (d, J = 15.5 Hz, 1H), 3.71 (s, 3H), 3.69 - 3.58 (m, 4H), 3.11 (dd, J = 18.3, 4.3 Hz, 1H), 2.68 (dt, J = 17.0, 6.8 Hz, 1H), 2.58 (dt, J = 17.1, 6.5 Hz, 1H), 2.38 - 2.29 (m, 2H). Rt 0.53 min (Apolar method). ESI-MS for C₃₁H₂₈ClN₃O₆: calculated 573.2, found m/z 574.1/576.1 [M+H]⁺.

### Example d.107. 4-[5-[6-chloro-2-oxo-4-(2-phenylethyl)-1H-quinolin-3-yl]-3-(4-methoxyphenyl)-3,4-dihydropyrazol-2-yl]-4-oxo-butanoic acid (example not falling within the scope of the present invention).

### Step 1. Synthesis of 1-(2-amino-5-chloro-phenyl)-3-phenyl-propan-1-one

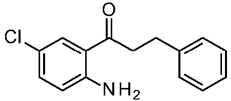

In a dried three-neck round-bottom flask, 2-amino-5-chlorobenzonitrile (0.5 g, 3.28 mmol) was suspended in anhydrous diethyl ether (1.6 ml) under nitrogen atmosphere. The mixture was cooled to 0 °C, and then Phenethylmagnesium chloride was added dropwise as solution 1.0 M in THF (9.3 ml, 9.3 mmol). After stirring for 16 hours at room temperature, the reaction was cooled to -60 °C and 6 N HCl (2.5 ml) was added while stirring vigorously. After 1 hour at room temperature, the mixture was diluted with ethyl acetate (50 ml), the aqueous layer extracted with ethyl acetate (3 x 25 ml), the combined organic phases were dried over Na₂SO₄ and evaporated to dryness. Purification by normal phase chromatography (solvent A: cyclohexane, solvent B: ethyl acetate - gradient: 0-5% solvent B) yielded title compound (623 mg, yield 73 %). ¹H NMR (400 MHz, DMSO-*d₆*) δ 7.78 (d, J = 2.5 Hz, 1H), 7.39 - 7.22 (m, 7H), 7.21 - 7.12 (m, 1H), 6.79 (d, J = 8.9 Hz, 1H), 3.29 (t, J = 7.5 Hz, 2H), 2.90 (t, J = 7.5 Hz, 2H). Rt 1.61 min (Apolar method) .ESI-MS for C₁₅H₁₄ClNO: calculated 259.1, found m/z 260.1/262.1 [M+H] ⁺.

### Step 2. Synthesis of 3-acetyl-6-chloro-4-(2-phenylethyl)-1H-quinolin-2-one (e.2)

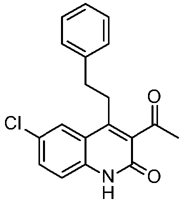

In dried microwaveable vessel, 1-(2-amino-5-chlorophenyl)-3-phenyl-propan-1-one (469 mg, 1.81 mmol) was dissolved in anhydrous tetrahydrofuran (2.8 ml) in presence of 4Å molecular sieves. Then ethyl acetoacetate (1.2 mL, 9.0 mmol) was added. The reaction mixture was microwaved at 140 °C (200 W) for 75 minutes, cooled at room temperature, resulting white-off solid filtered and rinsed with cold tetrahydrofuran to finally yield title compound (358.1 mg, yield 61 %). ¹H NMR (400 MHz, DMSO-*d₆*) δ 12.24 (s, 1H), 7.69 (d, J = 2.2 Hz, 1H), 7.55 (dd, J = 8.8, 2.2 Hz, 1H), 7.35 (d, J = 8.8 Hz, 1H), 7.27 (dt, J = 13.0, 7.4 Hz, 4H), 7.19 (t, J = 7.1 Hz, 1H), 4.14 (s, 2H), 2.42 (s, 3H). Rt 1.05 min (Apolar method) .ESI-MS for C₁₉H₁₆ClNO₂: calculated 325.1, found m/z 326.1/328.1 [M+H]⁺, 324.1/326.1 [M-H]⁻.

### Step 3. Synthesis of 6-chloro-3-[(E)-3-(4-methoxyphenyl)prop-2-enoyl]-4-(2-phenylethyl)-1H-quinolin-2-one (f.24)

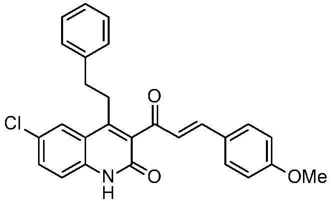

In a round-bottom flask, e.2 (268.3 mg, 0.82 mmol) was suspended in a 4:3 mixture of ethanol (9.4 ml) and water (7.1 ml). The mixture was cooled to 0 °C and potassium hydroxide (1.167 g, 20.59 mmol) was added. After stirring for 45 minutes at 0 °C, para-anisaldehyde (0.10 mL, 0.82 mmol) was added. The reaction was stirred at room temperature for 16 hours, afterwards acetic acid (2.2 mL, 37.88 mmol) was added at 0 °C, resulting yellow solid filtered, resulting solid was dissolved in CH₂Cl₂ (50 mL), washed with water (3 x 50 mL) and brine. The solvent was evaporated in vacuo to yield the title compound as an orange solid (250 mg, yield 68 %). ¹H NMR(400 MHz, DMSO-*d₆*) δ 12.13 (s, 1H), 7.92 (d, J = 2.3 Hz, 1H), 7.75 - 7.67 (m, 2H), 7.63 (dd, J = 8.8, 2.2 Hz, 1H), 7.49 (d, J = 16.2 Hz, 1H), 7.41 (d, J = 8.8 Hz, 1H), 7.28 - 7.13 (m, 5H), 7.02 - 6.96 (m, 2H), 6.93 (d, J = 16.2 Hz, 1H), 3.81 (s, 3H), 3.03 - 2.94 (m, 2H), 2.88 - 2.79 (m, 2H). Rt 1.48 min (Apolar method) .ESI-MS for C₂₇H₂₂ClNO₃: calculated 443.1, found m/z 444.2/446.2 [M+H]⁺, 442.3/444.3 [M-H]⁻.

### Step 4. Synthesis of 6-chloro-3-[5-(4-methoxyphenyl)-4,5-dihydro-1H-pyrazol-3-yl]-4-(2-phenylethyl)-1H-quinolin-2-one (g.24).

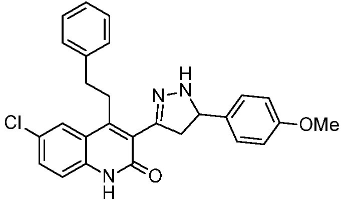

In appropriately sized microwaveable vessel, f.24 (243 mg, 0.55 mmol) was suspended in ethanol (1 ml), then hydrazine hydrate (68 µL, 1.09 mmol) added, the reaction mixture microwaved at 110 °C (200 W) for 25 minutes, cooled at room temperature and resulting yellow solid filtered to yield title compound (230 g, yield 92 %). ¹H NMR(400 MHz, DMSO-*d₆*) δ 11.97 (s, 1H), 7.87 (d, J = 2.2 Hz, 1H), 7.57 (dd, J = 8.8, 2.2 Hz, 1H), 7.47 (d, J = 3.5 Hz, 1H), 7.42 - 7.25 (m, 7H), 7.21 (tt, J = 6.0, 1.7 Hz, 1H), 6.93 - 6.84 (m, 2H), 4.75 (td, J = 10.7, 3.4 Hz, 1H), 3.74 (s, 3H), 3.30 - 3.17 (m, 2H), 3.14 (dd, J = 16.3, 10.5 Hz, 1H), 2.90 (t, J = 8.1 Hz, 2H), 2.74 (dd, J = 16.3, 10.9 Hz, 1H). Rt 1.31 min (Apolar method). ESI-MS for C₂₇H₂₄ClN₃O₂: calculated 457.2, found m/z 458.2/460.2 [M+H]⁺, 456.4/458.4 [M-H]⁻.

### Step 5. Synthesis of 4-[5-[6-chloro-2-oxo-4-(2-phenylethyl)-1H-quinolin-3-yl]-3-(4-methoxyphenyl)-3,4-dihydropyrazol-2-yl]-4-oxo-butanoic acid (d.107).

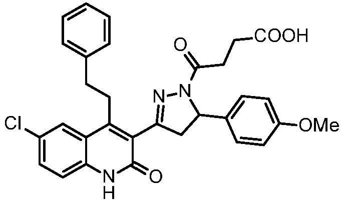

Compound d.104 was synthesized following the general procedure D1 using g.22 (105 mg, 0.23 mmol) with succinic anhydride (120 °C, 200 W). Trituration in CH₂Cl₂ (2 ml)and final purification by normal phase flash column chromatography (solvent A: CH₂Cl₂, solvent B: MeOH - gradient: 0-3 % solvent B) yielded title compound (78.1 mg, yield 61 %). ¹H NMR(400 MHz, DMSO-*d*₆) δ 12.13 (s, 1H), 7.92 (d, J = 2.3 Hz, 1H), 7.62 (dd, J = 8.8, 2.2 Hz, 1H), 7.40 (d, J = 8.8 Hz, 1H), 7.32 - 7.26 (m, 2H), 7.23 (d, J = 7.2 Hz, 3H), 7.17 (d, J = 8.7 Hz, 2H), 6.85 - 6.77 (m, 2H), 5.40 (dd, J = 11.9, 4.6 Hz, 1H), 3.72 (s, 3H), 3.48 (dd, J = 18.3, 11.9 Hz, 1H), 3.33 - 3.13 (m, 2H), 3.05 - 2.76 (m, 5H), 2.44 (t, J = 6.7 Hz, 2H). Rt 0.90 min (Apolar method). ESI-MS for C₃₁H₂₈ClN₃O₅: calculated 557,2, found m/z 558.2/560.2 [M+H]⁺.

### Example d.108. Synthesis of 6-chloro-3-[3-(4-methoxyphenyl)-2-propanoyl-3,4-dihydropyrazol-5-yl]-4-(2-phenylethyl)-1H-quinolin-2-one (example not falling within the scope of the present invention).

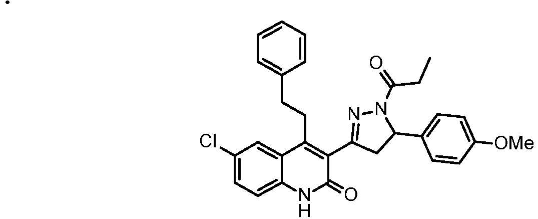

Compound d.108 was synthesized following the general procedure D2 using g.24 (100 mg, 0.22 mmol). Purification by normal phase flash column chromatography (solvent A: DCM, solvent B: MeOH - gradient: 0-4 % solvent B) yielded title compound as a white solid(80.3 mg, yield 72%). ¹H NMR(400 MHz, DMSO-*d₆*) δ 7.91 (d, J = 2.3 Hz, 1H), 7.61 (dd, J = 8.8, 2.2 Hz, 1H), 7.39 (d, J = 8.8 Hz, 1H), 7.33 - 7.25 (m, 2H), 7.25 - 7.13 (m, 5H), 6.86 - 6.77 (m, 2H), 5.40 (dd, J = 11.9, 4.6 Hz, 1H), 3.72 (s, 3H), 3.50 (dd, J = 18.3, 12.0 Hz, 1H), 3.29 - 3.18 (m, 2H), 3.03 - 2.84 (m, 3H), 2.65 - 2.50 (m, 2H), 0.96 (t, J = 7.5 Hz, 3H). Rt 1.60 min (Apolar method) . ESI-MS for C₃₀H₂₈ClN₃O₃: calculated 513.2, found m/z 514.2/516.2 [M+H]⁺. 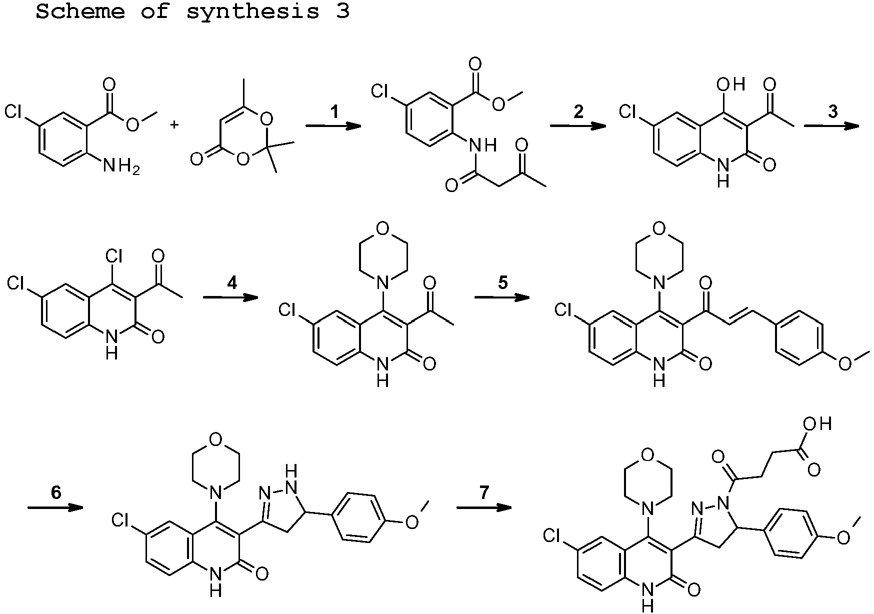
(1) Toluene, 110 °C, 16 h, yield 77 %; (2) MeONa, MeOH, 2h, yield 90 %; (3) POCl₃, reflux, 2h, yield 74 %; (4) Morpholine, Et₃N, ACN, 90 °C, 2 h, yield 22 %; (5) p-anisaldehyde, KOH, 4:3 EtOH/H₂O, 0 °C to rt, 16 h, yield 68 %; (6) hydrazine monohydrate, EtOH, 110 °C, 45 min, yield 36 %; (7) Succinic anhydride, THF (dry), 165 °C, 45 min, yield 49 %.

### Example d.109. 4-[5-(6-chloro-4-morpholino-2-oxo-1H-quinolin-3-yl)-3-(4-methoxyphenyl)-3,4-dihydropyrazol-2-yl]-4-oxo-butanoic acid.

### Step 1. Synthesis of methyl 5-chloro-2-(3-oxobutanoylamino)benzoate

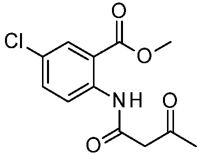

To a suspension of methyl 2-amino-5-chlorobenzoate (2.5 g, 13.5 mmol) in toluene (30 mL) was added 2,2,6-trimethyl-1,3-dioxin-4-one (2 mL, 14.8 mmol). The solution was refluxed for 16 hours (110 °C). Reaction was cooled to room temperature and solvent evaporated in vacuo. Purification by normal phase chromatography (solvent A: cyclohexane, solvent B: ethyl acetate - gradient: 0-100% solvent B) followed by trituration in MeOH yielded title compound as a white solid (2.80 g, yield 77 %). ¹H NMR (400 MHz, DMSO-*d₆*) δ 10.61 (s, 1H), 8.16 (d, J = 8.9 Hz, 1H), 7.85 (d, J = 2.6 Hz, 1H), 7.68 (dd, J = 8.9, 2.6 Hz, 1H), 3.85 (s, 3H), 3.68 (s, 2H), 2.22 (s, 3H). Rt 2.11 min (generic method). ESI-MS for C₁₂H₁₂ClNO₄: calculated 269.0, found m/z 270.0/272.0 [M+H]⁺.

### Step 2. Synthesis of 3-acetyl-6-chloro-4-hydroxy-1H-quinolin-2-one

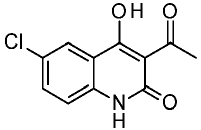

To a suspension of methyl 5-chloro-2-(3-oxobutanoylamino) benzoate (1.7 g, 6.30 mmol) in MeOH (80 mL) was added sodium methoxide (1.36 g, 25.20 mmol), the reaction mixture was refluxed for 2 hours and until formation of a white slurry, which was concentrated under vacuum and the residue suspended in H₂O (100 mL) and acidified to pH 2 with aq. HCl 0.1 M (20 mL). The formed precipitate was successively filtered, washed with H₂O (50 mL), Et₂O (50 mL) and dried to afford title product as a white solid (1.35 g, 90%). ¹H NMR (400 MHz, DMSO-*d₆*) δ 11.64 (s, 1H), 7.93 (d, J = 2.5 Hz, 1H), 7.72 (dd, J = 8.8, 2.5 Hz, 1H), 7.31 (d, J = 8.9 Hz, 1H), 2.71 (s, 3H). Rt 2.09 min (generic method). ESI-MS for C₁₁H₈ClNO₃: calculated 237.0, found m/z 236.0/238.1 [M-H]⁻.

### Step 3. Synthesis of 3-acetyl-4,6-dichloro-1H-quinolin-2-one

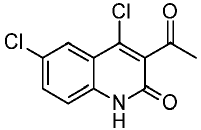

In a round-bottom flask, 3-acetyl-6-chloro-4-hydroxy-1H-quinolin-2-one (100 mg, 0.42 mmol) was suspended in POCl₃ (6 mL) and reaction was heated at 110 °C for 2 hours. Solvent was evaporated in vacuo and the resulting dark crude was suspended in acetic acid (13 mL). Sodium acetate (38 mg, 0.46 mmol) was then added and the reaction was heated at 120 °C and left stirring overnight. Reaction was cooled to room temperature, poured into water (50 mL), and the solid formed filtered and washed with water (20 mL) to obtain title compound as a brown solid (80 mg, yield 74%) that was used in the next step without further purification. ¹H NMR (400 MHz, DMSO-*d₆*) δ 12.52 (s, 1H), 7.90 (d, J = 2.3 Hz, 1H), 7.73 (dd, J = 8.8, 2.4 Hz, 1H), 7.42 (d, J = 8.8 Hz, 1H), 2.48 (s, 2H). Rt 1.97 min (generic method). ESI-MS for C₁₁H₇Cl₂NO₂: calculated 255.0, found m/z 254.0/255.9/258.3 [M-H]⁻.

### Step 4. Synthesis of 3-acetyl-6-chloro-4-morpholino-1H-quinolin-2-one.

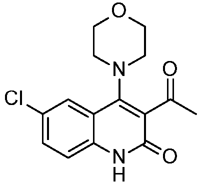

In a pressure tube, 3-acetyl-4,6-dichloro-1H-quinolin-2-one (300 mg, 1.17 mmol) was suspended in acetonitrile (3 mL). Triethylamine (327 µL, 2.34 mmol) and morpholine (113 µL, 1.29 mmol) were then added and the mixture was stirred at 90 °C for 2 hours. Solvent was evaporated in vacuo and crude purified by normal phase chromatography (solvent A: cyclohexane, solvent B: ethyl acetate - gradient: 0-50% solvent B) to yield title compound as a pale yellow solid (80 mg, yield 22 %). ¹H NMR (400 MHz, DMSO-*d₆*) δ 11.91 (s, 1H), 7.75 (d, J = 2.4 Hz, 1H), 7.59 (dd, J = 8.8, 2.3 Hz, 1H), 7.33 (d, J = 8.8 Hz, 1H), 3.77 (t, J = 4.5 Hz, 4H), 2.97 (t, J = 4.5 Hz, 4H), 2.55 (s, 3H). tR 1.82 min (generic method). ESI-MS for C₁₅H₁₅ClN₂O₃: calculated 306.1, found m/z 307.0/309.0 [M+H]⁺.

### Step 5. Synthesis of 6-chloro-3-[(E)-3-(4-methoxyphenyl)prop-2-enoyl]-4-morpholino-1H-quinolin-2-one

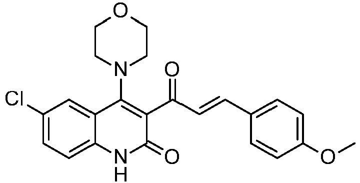

In a round-bottom flask, 3-acetyl-6-chloro-4-morpholino-1H-quinolin-2-one (250 mg, 0.82 mmol) was suspended in a 4:3 mixture of ethanol (9 mL) and water (7 mL). The mixture was cooled to 0 °C and potassium hydroxide (1.14 g, 20.38 mmol) was added. After stirring for 45 minutes at 0 °C, p-anisaldehyde (179 µL, 1.48 mmol) was added. The reaction was stirred overnight at room temperature, afterwards acetic acid (1.175 mL, 20.38 mmol) was added at 0 °C, resulting in a precipitate that was filtered and washed with ethanol (20 mL). The solid was dissolved in CH₂Cl₂ (50 mL) and washed with water (50 mL) and brine (50 mL). The solvent was evaporated in vacuo to yield the title compound as a yellow solid (238 mg, Yield 68%). ¹H NMR (400 MHz, DMSO-*d₆*) δ 11.86 (s, 1H), 7.76 (d, J = 2.4 Hz, 1H), 7.70 (d, J = 8.8 Hz, 2H), 7.60 (dd, J = 8.8, 2.4 Hz, 1H), 7.50 (d, J = 16.2 Hz, 1H), 7.35 (d, J = 8.8 Hz, 1H), 7.04 - 6.95 (m, 3H), 3.80 (s, 3H), 3.72 (t, J = 4.5 Hz, 4H), 2.99 (t, J = 4.5 Hz, 4H). Rt 2.17 min (generic method) . ESI-MS for C₂₃H₂₁ClN₂O₄: calculated 424.1, found m/z 425.0/427.0 [M+H]⁺.

### Step 6. Synthesis of 6-chloro-3-[5-(4-methoxyphenyl)-4,5-dihydro-1H-pyrazol-3-yl]-4-morpholino-1H-quinolin-2-one

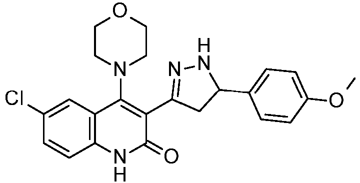

In an appropriately sized microwaveable vessel, 6-chloro-3-[(E)-3-(4-methoxyphenyl)prop-2-enoyl]-4-morpholino-1H-quinolin-2-one (230 mg, 0.54 mmol) was suspended in ethanol (around 5 mL), then hydrazine hydrate (53 µL, 1.08 mmol) added, the reaction mixture microwaved at 110 °C (200 W) for 45 minutes, cooled at room temperature and resulting solid filtered and washed with ethanol (20 mL). Purification by normal phase chromatography (solvent A: dichloromethane, solvent B: ethanol - gradient: 0-100% solvent B) yielded title compound as an off-white solid. (85 mg, Yield 36%). ¹H NMR (400 MHz, DMSO-*d₆*) δ 11.81 (s, 1H), 7.78 (d, J = 2.3 Hz, 1H), 7.55 (dd, J = 8.7, 2.3 Hz, 1H), 7.43 - 7.37 (m, 3H), 7.33 (d, J = 8.7 Hz, 1H), 6.92 (d, J = 8.6 Hz, 2H), 4.88 - 4.77 (m, 1H), 3.81 - 3.75 (m, 4H), 3.75 (s, 3H), 3.42 - 3.34 (m, 1H), 3.11 - 2.94 (m, 4H), 2.83 (dd, J = 16.4, 11.7 Hz, 1H). Rt 2.11 min (generic method). ESI-MS for C₂₃H₂₃ClN₄O₃: calculated 438.1, found m/z 439.1/441.1 [M+H]⁺.

### Step 7. Synthesis of 4-[5-(6-chloro-4-morpholino-2-oxo-1H-quinolin-3-yl)-3-(4-methoxyphenyl)-3,4-dihydropyrazol-2-yl]-4-oxo-butanoic acid

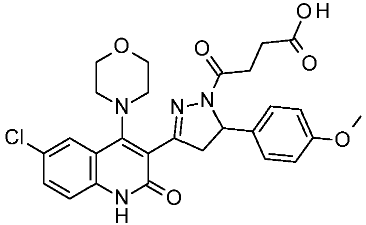

In an appropriately sized microwaveable vessel, 6-chloro-3-[5-(4-methoxyphenyl)-4,5-dihydro-1H-pyrazol-3-yl]-4-morpholino-1H-quinolin-2-one (13 mg, 0.03 mmol) was dissolved in anhydrous THF (1 mL). Succinic anhydride (6 mg, 0.06 mmol) was then added. The solution was microwaved at 165 °C (200 W) with stirring for 20 min. THF was evaporated and the resulted crude solid was triturated in CH₂Cl₂ (20 mL) and H₂O (20 mL), to obtain the title compound as a yellow solid. Purification by normal phase chromatography (solvent A: dichloromethane, solvent B: methanol - gradient: 0-10% solvent B) yielded title compound as a yellow solid (8 mg, Yield 49%). ¹H NMR (400 MHz, DMSO-*d₆*) δ 11.97 (s, 2H), 7.79 (d, J = 2.4 Hz, 1H), 7.59 (dd, J = 8.7, 2.3 Hz, 1H), 7.36 (d, J = 8.8 Hz, 1H), 7.27 (d, J = 8.7 Hz, 2H), 6.89 (d, J = 8.7 Hz, 2H), 5.49 (dd, J = 12.1, 5.3 Hz, 1H), 3.83 - 3.71 (m, 8H), 3.27 - 3.16 (m, 1H), 3.11 - 2.99 (m, 4H), 2.93 - 2.71 (m, 2H), 2.36 (t, J = 7.6 Hz, 2H). Rt 1.85 min (generic method). ESI-MS for C₂₇H₂₇ClN₄O₆: calculated 538.2, found m/z 539.1/541.0 [M+H]⁺.

### 2.Biophysical assays

### ELISA assay

A competitive ELISA screening assay using biotinylated BRC4 peptide to disrupt the BRC4/RAD51 interaction was performed by modifying the method described by Rajendra et al. (Nucleic Acids Res. 38 (2010) 82-96). BRC4-biotinylated peptide (N-term BiotinKEPTLLGFHTASGKKVKIAKESLDKVKNLFDEKEQ from Life Technologies) was used to coat 384-well plates (Nunc). After washing with PBS containing 0.05% Tween-20 (PBST), and blocking with the solution BSA 1%/PBST, overnight hybridization with human Rad51 protein (NP_002866 Creative Biomart, NY) was performed. Test compounds were added in dose-response from 0.01 to 100 mM in triplicate with constant DMSO 1%. Antibody raised against Rad51 (Millipore) and HRP-secondary antibody staining to develop the 3,30,5,50-tetramethyllbenzidine signal (Sigma) quenched with 1M HCl was used as the assay readout. Colorimetric measure was read on Victor5 (PerkinElmer) plate Reader. BRC4 and Rad51 were included in the assay as positive control. Results were analyzed using GraphPad Software.

### 3.Biological assays

### Cell culture and treatments

BxPC-3 cells were grown in RPMI 1640 containing 10% FBS, 100 U/ml penicillin/streptomycin, 2 mM glutamine. All media and supplements were from Sigma-Aldrich. Cultures were routinely tested for Mycoplasma contamination. Treatments (Olaparib and BRCA2-Rad51 disruptors) were administered in culture medium supplemented with 0.6% DMSO. The same amount of DMSO was added to the control, untreated cultures.

### Homologous Recombination assay (HR)

Homologous recombination (HR) was assessed by using a commercially available assay (Norgen). This assay is based on cell co-transfection with two plasmids that, upon cells entering, recombinate. The efficiency of HR can be assessed by real time PCR, using primer mixtures included in the assay kit. Different primer mixtures allow differentiation between the original plasmid backbones and their recombination product.

BxPC3 cells (2 × 10⁵ per well) were seeded in a 24-well plate and allowed to adhere overnight. Co-transfection with the two plasmids was performed in Lipofectamine 2000 (Invitrogen) for 5 h, according to the manufacturer's instructions. During transfection cells were exposed at different doses of BRCA2-Rad51 disruptors, dissolved in RPMI without serum in the presence of 0.6% DMSO.

After washing with PBS, cells were harvested, and DNA was isolated using QIAamp^{®} DNA Mini Kit (Qiagen). Sample concentration was measured using an ONDA Nano Genius photometer.

The thermal cycler (CFX96 TM Real Time System, BioRad) was programmed as follows: initial denaturation 95°C for 3 min then 95°C for 15 sec., 61°C for 30 sec., 72 °C for 1 min and repeated for 40 cycles. Melt curve analysis was done. Samples were run in triplicate, in 10µl reaction volume containing 25ng of template.

In each DNA sample from control and treated cultures, the ΔCt between the recombination product and the backbone plasmids was calculated. HR efficiency was evaluated from the 2^{-ΔΔCt} value obtained by comparing treated vs control samples.

### Cell viability assay

Cell viability was assessed by CellTiter Glo, Luminescent Cell Viability Assay. Briefly, BxPC-3 cells (1 × 104/well) were plated in white 96-well plates with clear bottom and treated for 72h with olaparib (10 µM) or with the BRCA2-Rad51 disruptors, given alone or in combination. After treatments, the plate was allowed to equilibrate at room temperature for 30min and the CellTiter-Glo reactive was directly added to each well. The plate was kept on a shaker for 2min to induce cell lysis and its luminescence was measured by using a Fluoroskan Ascent FL reader (Labsystems) using manufacturer's instructions.

### Cytotoxicity assay

Cell death was assessed by applying the CellToxTM Green Cytotoxicity assay (Promega). Briefly, BxPC-3 (1 × 104/well) were plated in black 96-well plates with clear bottom and treated for 72 h with olaparib (10 µM) or with the BRCA2-Rad51 disruptors, given alone or in combination. At the end of treatment, the CellToxTM dye was added to cell cultures and the green fluorescence signal, which is produced by the binding interaction with dead cell DNA, was measured following the manufacturer's instructions. The fluorescence signal was detected using the EnSpire 2300 Multilabel Reader (Perkin Elmer); for each sample, the obtained signal was normalized on the number of viable cells, detected by the CellTiterGlo reagent.

Table 1 provides a summary of ELISA and Biological assays results.

**TABLE 1**

| Compound number | Chemical structure | IUPAC name | ELISA and Biological tests results |
|---|---|---|---|
| d.1 | 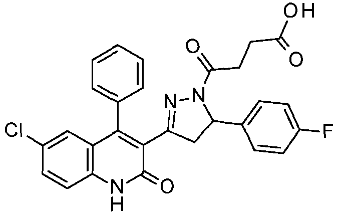 | 4-[5-(6-chloro-2-oxo-4-phenyl-1H-quinolin-3-yl)-3-(4-fluorophenyl)-3,4-dihydropyrazol-2-yl]-4-oxo-butanoic acid | ELISA assay: EC₅₀ 38 ± 4 µM |
| | | | Cell viability: EC₅₀ >50 µM |
| d.2 | 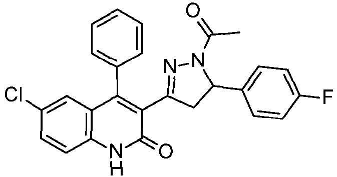 | 3-[2-acetyl-3-(4-fluorophenyl)-3,4-dihydropyrazol-5-yl]-6-chloro-4-phenyl-1H-quinolin-2-one | ELISA assay: EC₅₀ 50 ± 10 µM |
| | | | Cell viability: EC₅₀ 37 µM |
| d.3 | 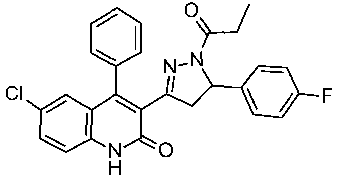 | 6-chloro-3-[3-(4-fluorophenyl)-2-propanoyl-3,4-dihydropyrazol-5-yl]-4-phenyl-1H-quinolin-2-one | ELISA assay: EC₅₀ 34 ± 3 µM |
| d.4 | 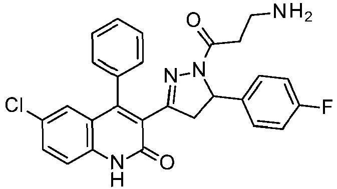 | 3-[2-(3-aminopropanoyl)-3-(4-fluorophenyl)-3,4-dihydropyrazol-5-yl]-6-chloro-4-phenyl-1H-quinolin-2-one | ELISA assay: >100 µM |
| | | | Cell viability: EC₅₀ 7.6µM |
| d.5 | 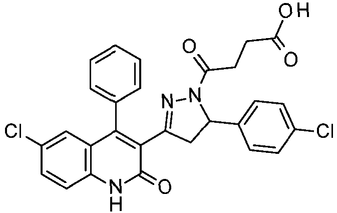 | 4-[5-(6-chloro-2-oxo-4-phenyl-1H-quinolin-3-yl)-3-(4-chlorophenyl)-3,4-dihydropyrazol-2-yl]-4-oxo-butanoic acid | ELISA assay: EC₅₀ 50 µM |
| | | | Cell viability: EC₅₀ >50 µM |
| d. 6 | 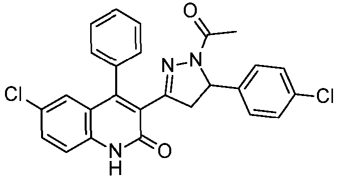 | 3-[2-acetyl-3-(4-chlorophenyl)-3,4-dihydropyrazol-5-yl]-6-chloro-4-phenyl-1H-quinolin-2-one | ELISA assay: EC₅₀ 20 µM |
| | | | Cell viability: EC₅₀ 20 µM |
| | | | HR inhibition: 24% at 10 µM |
| d.7 | 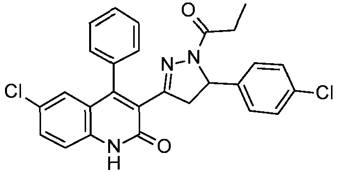 | 6-chloro-3-[3-(4-chlorophenyl)-2-propanoyl-3,4-dihydropyrazol-5-yl]-4-phenyl-1H-quinolin-2-one | ELISA assay: EC₅₀ 20 µM |
| | | | Cell viability: EC₅₀ 20 µM |
| d.8 | 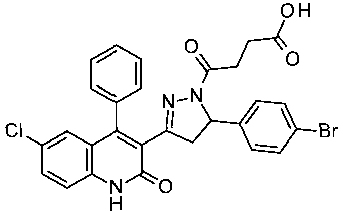 | 4-[3-(4-bromophenyl)-5-(6-chloro-2-oxo-4-phenyl-1H-quinolin-3-yl)-3,4-dihydropyrazol-2-yl]-4-oxo-butanoic acid | ELISA assay: EC₅₀ 16 ± 2 µM |
| | | | Cell viability: EC₅₀ 48 µM |
| d.9 | 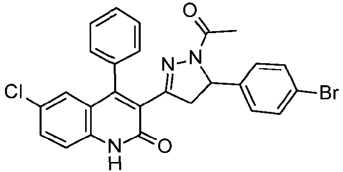 | 3-[2-acetyl-3-(4-bromophenyl)-3,4-dihydropyrazol-5-yl]-6-chloro-4-phenyl-1H-quinolin-2-one | ELISA assay: EC₅₀ 38 ± 7 µM |
| | | | Cell viability: EC₅₀ 18 µM |
| d.10 | 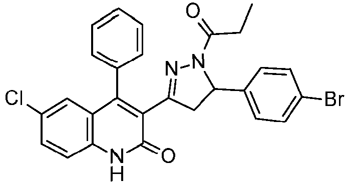 | 3-[3-(4-bromophenyl)-2-propanoyl-3,4-dihydropyrazol-5-yl]-6-chloro-4-phenyl-1H-quinolin-2-one | ELISA assay: EC₅₀ 19 ± 1 µM |
| d.11 | 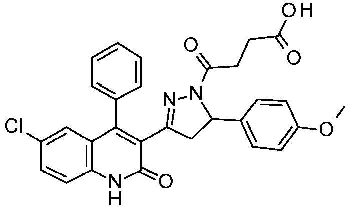 | 4-[5-(6-chloro-2-oxo-4-phenyl-1H-quinolin-3-yl)-3-(4-methoxyphenyl)-3,4-dihydropyrazol-2-yl]-4-oxo-butanoic acid | ELISA assay: >100 µM |
| | | | Cell viability: EC₅₀ > 50 µM |
| d.12 | 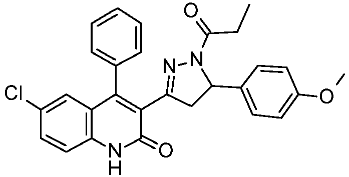 | 6-chloro-3-[3-(4-methoxyphenyl)-2-propanoyl-3,4-dihydropyrazol-5-yl]-4-phenyl-1H-quinolin-2-one | ELISA assay: EC₅₀ 19 ± 1 µM |
| | | | Cell viability: EC₅₀ 19 µM |
| | | | Olaparib Assoc.: synergy |
| | | | HR inhibition: IC₅₀ 18.39 µM |
| d.14 | 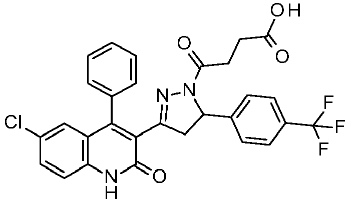 | 4-[5-(6-chloro-2-oxo-4-phenyl-1H-quinolin-3-yl)-3-[4-(trifluoromethyl)phenyl]-3,4-dihydropyrazol-2-yl]-4-oxo-butanoic acid | ELISA assay: EC₅₀ 29 ± 2 µM |
| | | | Cell viability: EC₅₀ 50 µM |
| d.15 | 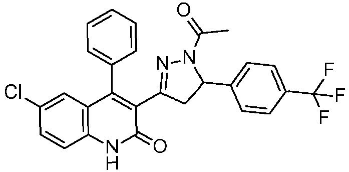 | 3-[2-acetyl-3-[4-(trifluoromethyl)phenyl]-3,4-dihydropyrazol-5-yl]-6-chloro-4-phenyl-1H-quinolin-2-one | ELISA assay: >100 µM |
| | | | Cell viability: EC₅₀ 11.4µM |
| d.16 | 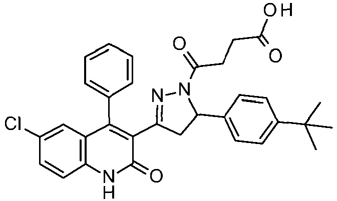 | 4-[3-(4-tert-butylphenyl)-5-(6-chloro-2-oxo-4-phenyl-1H-quinolin-3-yl)-3,4-dihydropyrazol-2-yl]-4-oxo-butanoic acid | ELISA assay: >100 µM |
| | | | Cell viability: EC₅₀ 71.2µM |
| d.17 | 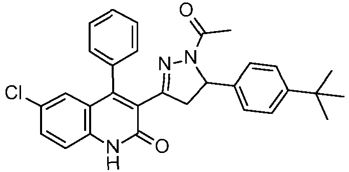 | 3-[2-acetyl-3-(4-tert-butylphenyl)-3,4-dihydropyrazol-5-yl]-6-chloro-4-phenyl-1H-quinolin-2-one | ELISA assay: EC₅₀ 15 ± 4 µM |
| | | | Cell viability: EC₅₀ 15 µM |
| d.18 | 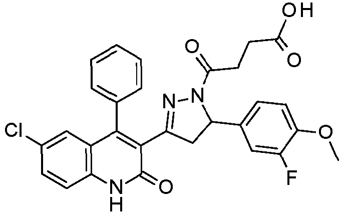 | 4-[5-(6-chloro-2-oxo-4-phenyl-1H-quinolin-3-yl)-3-(3-fluoro-4-methoxy-phenyl)-3,4-dihydropyrazol-2-yl]-4-oxo-butanoic acid | ELISA assay: >100 µM |
| | | | Cell viability: EC₅₀ 73 µM |
| d.19 | 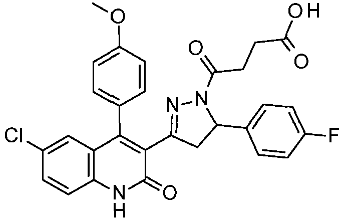 | 4-[5-[6-chloro-4-(4-methoxyphenyl)-2-oxo-1H-quinolin-3-yl]-3-(4-fluorophenyl)-3,4-dihydropyrazol-2-yl]-4-oxo-butanoic acid | ELISA assay: >100 µM |
| | | | Cell viability: EC₅₀ 76 µM |
| d.20 | 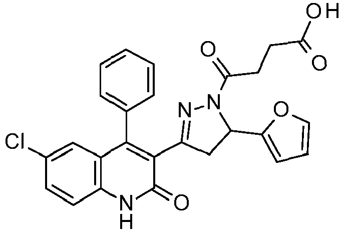 | 4-[5-(6-chloro-2-oxo-4-phenyl-1H-quinolin-3-yl)-3-(2-furyl)-3,4-dihydropyrazol-2-yl]-4-oxo-butanoic acid | ELISA assay: >100 µM |
| | | | Cell viability: EC₅₀ >100 µM |
| d.21 | 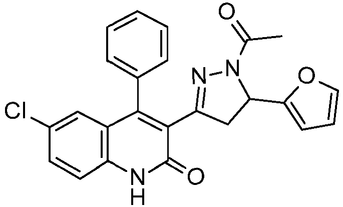 | 3-[2-acetyl-3-(2-furyl)-3,4-dihydropyrazol-5-yl]-6-chloro-4-phenyl-1H-quinolin-2-one | ELISA assay: >100 µM |
| | | | Cell viability: EC₅₀ 23 µM |
| d.22 | 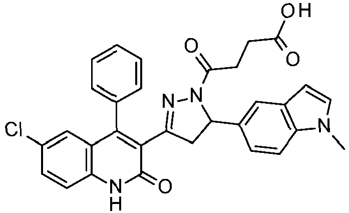 | 4-[5-(6-chloro-2-oxo-4-phenyl-1H-quinolin-3-yl)-3-(1-methylindol-5-yl)-3,4-dihydropyrazol-2-yl]-4-oxo-butanoic acid | ELISA assay: EC₅₀ 2 ± 0.5 µM |
| | | | Cell viability: EC₅₀ >20 µM |
| | | | Olaparib Assoc.: synergy |
| d.23 | 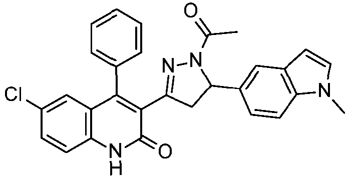 | 3-[2-acetyl-3-(1-methylindol-5-yl)-3,4-dihydropyrazol-5-yl]-6-chloro-4-phenyl-1H-quinolin-2-one | ELISA assay: EC₅₀ 18 ± 1 µM |
| | | | Cell viability: EC₅₀ 4.8 µM |
| d.24 | 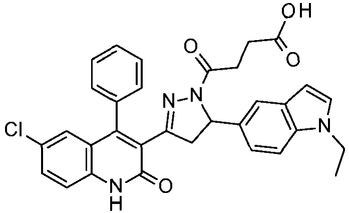 | 4-[5-(6-chloro-2-oxo-4-phenyl-1H-quinolin-3-yl)-3-(1-ethylindol-5-yl)-3,4-dihydropyrazol-2-yl]-4-oxo-butanoic acid | ELISA assay: EC₅₀ 10 ± 2 µM |
| | | | Cell viability: EC₅₀ >40 µM |
| d.25 | 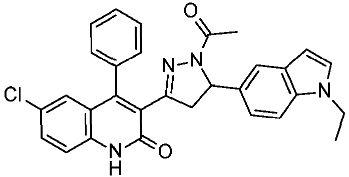 | 3-[2-acetyl-3-(1-ethylindol-5-yl)-3,4-dihydropyrazol-5-yl]-6-chloro-4-phenyl-1H-quinolin-2-one | ELISA assay: EC₅₀ 50 ± 15 µM |
| d.26 | 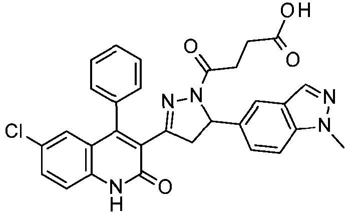 | 4-[5-(6-chloro-2-oxo-4-phenyl-1H-quinolin-3-yl)-3-(1-methylindazol-5-yl)-3,4-dihydropyrazol-2-yl]-4-oxo-butanoic acid | ELISA assay: EC₅₀ 13 ± 3 µM |
| | | | Cell viability: EC₅₀ >50 µM |
| d.27 | 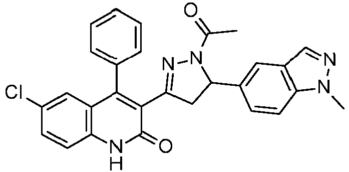 | 3-[2-acetyl-3-(1-methylindazol-5-yl)-3,4-dihydropyrazol-5-yl]-6-chloro-4-phenyl-1H-quinolin-2-one | ELISA assay: EC₅₀ 9 ± 2 µM |
| | | | Cell viability: EC₅₀ 21 µM |
| d.28 | 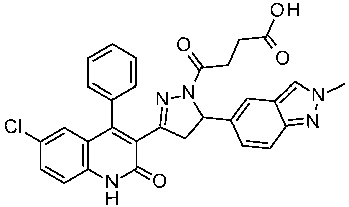 | 4-[5-(6-chloro-2-oxo-4-phenyl-1H-quinolin-3-yl)-3-(2-methylindazol-5-yl)-3,4-dihydropyrazol-2-yl]-4-oxo-butanoic acid | ELISA assay: EC₅₀ >100 µM |
| | | | Cell viability: > 100 µM |
| d.29 | 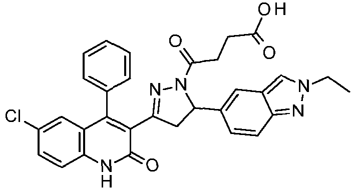 | 4-[5-(6-chloro-2-oxo-4-phenyl-1H-quinolin-3-yl)-3-(2-ethylindazol-5-yl)-3,4-dihydropyrazol-2-yl]-4-oxo-butanoic acid | ELISA assay: EC₅₀ 28 ± 4 µM |
| | | | Cell viability: EC₅₀ >50 µM |
| d.30 | 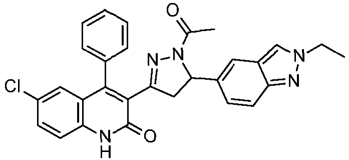 | 3-[2-acetyl-3-(2-ethylindazol-5-yl)-3,4-dihydropyrazol-5-yl]-6-chloro-4-phenyl-1H-quinolin-2-one | ELISA assay: >100 µM |
| | | | Cell viability: EC₅₀ 16.8 µM |
| d.31 | 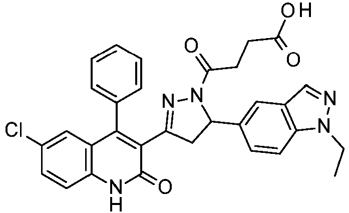 | 4-[5-(6-chloro-2-oxo-4-phenyl-1H-quinolin-3-yl)-3-(1-ethylindazol-5-yl)-3,4-dihydropyrazol-2-yl]-4-oxo-butanoic acid | ELISA assay: >100 µM |
| | | | Cell viability: EC₅₀ >50 µM |
| d.32 | 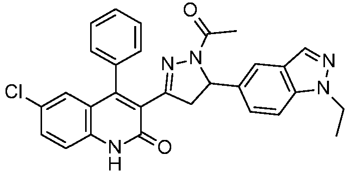 | 3-[2-acetyl-3-(1-ethylindazol-5-yl)-3,4-dihydropyrazol-5-yl]-6-chloro-4-phenyl-1H-quinolin-2-one | ELISA assay: EC₅₀ 40 ± 3 µM |
| | | | Cell viability: 27.0 µM |
| d.33 | 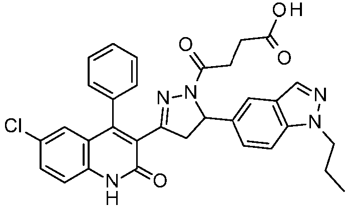 | 4-[5-(6-chloro-2-oxo-4-phenyl-1H-quinolin-3-yl)-3-(1-propylindazol-5-yl)-3,4-dihydropyrazol-2-yl]-4-oxo-butanoic acid | ELISA assay: >100 µM |
| | | | Cell viability: EC₅₀ >100 µM |
| d.34 | 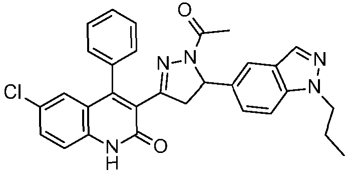 | 3-[2-acetyl-3-(1-propylindazol-5-yl)-3,4-dihydropyrazol-5-yl]-6-chloro-4-phenyl-1H-quinolin-2-one | ELISA experiment: EC₅₀ 0.95±0.05 µM |
| | | | Cell viability: EC₅₀ 11 µM |
| | | | HR inhibition: +83% at 5 µM |
| d.35 | 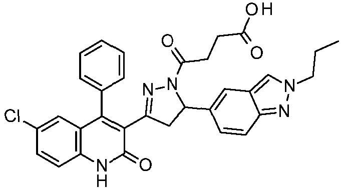 | 4-[5-(6-chloro-2-oxo-4-phenyl-1H-quinolin-3-yl)-3-(2-propylindazol-5-yl)-3,4-dihydropyrazol-2-yl]-4-oxo-butanoic acid | ELISA assay: >100 µM |
| | | | Cell viability: EC₅₀ 62.6 µM |
| d.36 | 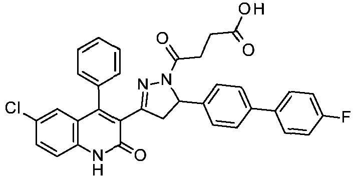 | 4-[5-(6-chloro-2-oxo-4-phenyl-1H-quinolin-3-yl)-3-[4-(4-fluorophenyl)phenyl]-3,4-dihydropyrazol-2-yl]-4-oxo-butanoic acid | ELISA assay: EC₅₀ 13 ± 1 µM |
| | | | Cell viability: EC₅₀ 31 µM |
| d.37 | 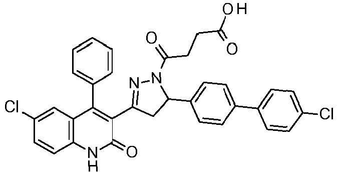 | 4-[5-(6-chloro-2-oxo-4-phenyl-1H-quinolin-3-yl)-3-[4-(4-chlorophenyl)phenyl]-3,4-dihydropyrazol-2-yl]-4-oxo-butanoic acid | ELISA assay: EC₅₀ 25 ± 5 µM |
| | | | Cell viability: 41.5 µM |
| d.38 | 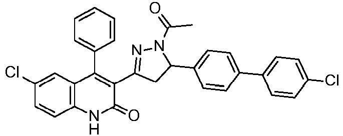 | 3-[2-acetyl-3-[4-(4-chlorophenyl)phenyl]-3,4-dihydropyrazol-5-yl]-6-chloro-4-phenyl-1H-quinolin-2-one | ELISA assay: EC₅₀ 45 ± 8 µM |
| | | | Cell viability: 18.8 µM |
| d.39 | 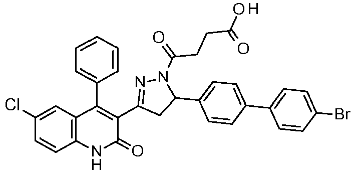 | 4-[3-[4-(4-bromophenyl)phenyl]-5-(6-chloro-2-oxo-4-phenyl-1H-quinolin-3-yl)-3,4-dihydropyrazol-2-yl]-4-oxo-butanoic acid | ELISA assay: EC₅₀ 20 ± 2 µM |
| | | | Cell viability: EC₅₀ 43 µM |
| d.40 | 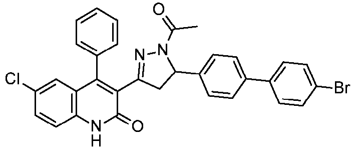 | 3-[2-acetyl-3-[4-(4-bromophenyl)phenyl]-3,4-dihydropyrazol-5-yl]-6-chloro-4-phenyl-1H-quinolin-2-one | ELISA assay: EC₅₀ 45 ± 8 µM |
| | | | Cell viability: EC₅₀ 28 µM |
| d.41 | 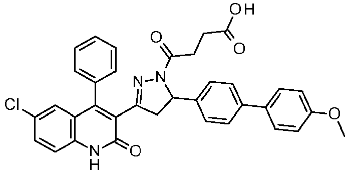 | 4-[5-(6-chloro-2-oxo-4-phenyl-1H-quinolin-3-yl)-3-[4-(4-methoxyphenyl)phenyl]-3,4-dihydropyrazol-2-yl]-4-oxo-butanoic acid | ELISA assay: EC₅₀ 20 ± 4 µM |
| | | | Cell viability: EC₅₀ 40.6 µM |
| d.42 | 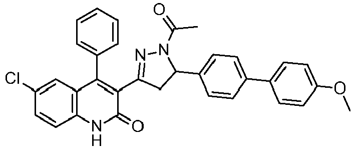 | 3-[2-acetyl-3-[4-(4-methoxyphenyl)phenyl]-3,4-dihydropyrazol-5-yl]-6-chloro-4-phenyl-1H-quinolin-2-one | ELISA assay: EC₅₀ 70 ± 6 µM |
| d.43 | 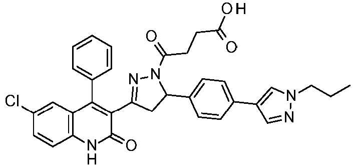 | 4-[5-(6-chloro-2-oxo-4-phenyl-1H-quinolin-3-yl)-3-[4-(1-propylpyrazol-4-yl)phenyl]-3,4-dihydropyrazol-2-yl]-4-oxo-butanoic acid | ELISA assay: EC₅₀ 65 ± 5 µM |
| | | | Cell viability: 74.2 µM |
| d.44 | 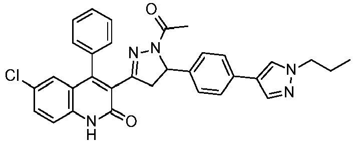 | 3-[2-acetyl-3-[4-(1-propylpyrazol-4-yl)phenyl]-3,4-dihydropyrazol-5-yl]-6-chloro-4-phenyl-1H-quinolin-2-one | ELISA assay: EC₅₀ 46 ± 9 µM |
| d.45 | 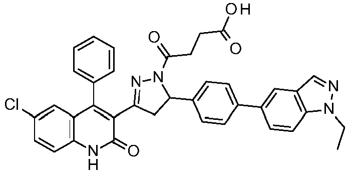 | 4-[5-(6-chloro-2-oxo-4-phenyl-1H-quinolin-3-yl)-3-[4-(1-ethylindazol-5-yl)phenyl]-3,4-dihydropyrazol-2-yl]-4-oxo-butanoic acid | ELISA assay: EC₅₀ 15 ± 3 µM Cell |
| d.46 | 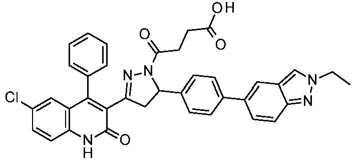 | 4-[5-(6-chloro-2-oxo-4-phenyl-1H-quinolin-3-yl)-3-[4-(2-ethylindazol-5-yl)phenyl]-3,4-dihydropyrazol-2-yl]-4-oxo-butanoic acid | ELISA assay: EC₅₀ 17 ± 4 µM |
| | | | Cell viability: EC₅₀ 44 µM |
| | | | Olaparib Assoc.: synergy |
| | | | HR inhibition: 91.7 % at 40 µM |
| d.47 | 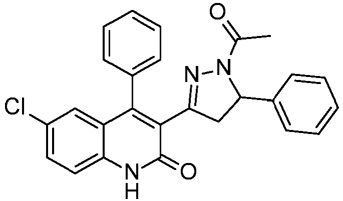 | 3-(2-acetyl-3-phenyl-3,4-dihydropyrazol-5-yl)-6-chloro-4-phenyl-1H-quinolin-2-one | ELISA assay: EC₅₀ 19±3 µM |
| | | | Cell viability: 29.7 µM |
| d.48 | 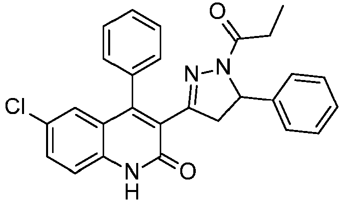 | 6-chloro-4-phenyl-3-(3-phenyl-2-propanoyl-3,4-dihydropyrazol-5-yl)-1H-quinolin-2-one | ELISA assay: EC₅₀ 24±4 µM |
| | | | Cell viability: 13.73 µM |
| d.49 | 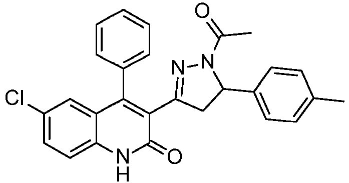 | 3-[2-acetyl-3-(p-tolyl)-3,4-dihydropyrazol-5-yl]-6-chloro-4-phenyl-1H-quinolin-2-one | ELISA assay: EC₅₀ 20±1 µM |
| | | | Cell viability: EC₅₀ 15.2 µM |
| d.50 | 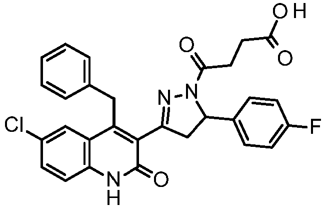 | 4-[5-(4-benzyl-6-chloro-2-oxo-1H-quinolin-3-yl)-3-(4-fluorophenyl)-3,4-dihydropyrazol-2-yl]-4-oxo-butanoic acid | ELISA assay: EC₅₀ 40±3 µM |
| | | | Cell viability: EC₅₀ 15.2 µM |
| d.51 | 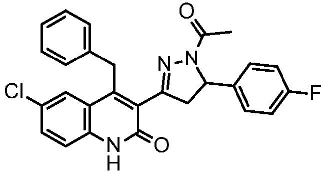 | 3-[2-acetyl-3-(4-fluorophenyl)-3,4-dihydropyrazol-5-yl]-4-benzyl-6-chloro-1H-quinolin-2-one | ELISA assay: EC₅₀ 13±4 µM |
| | | | Cell viability: EC₅₀ 15.2 µM |
| d.52 | 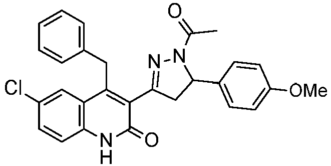 | 3-[2-acetyl-3-(4-methoxyphenyl) -3,4-dihydropyrazol-5-yl]-4-benzyl-6-chloro-1H-quinolin-2-one | ELISA assay: EC₅₀ 20±2 µM |
| | | | Cell viability: EC₅₀ 13.74 µM |
| d.53 | 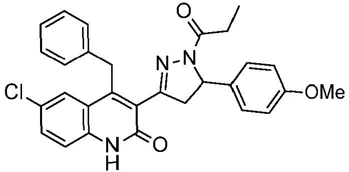 | 4-benzyl-6-chloro-3-[3-(4-methoxyphenyl)-2-propanoyl-3,4-dihydropyrazol-5-yl]-1H-quinolin-2-one | ELISA assay: EC₅₀ 20±6 µM |
| d.54 | 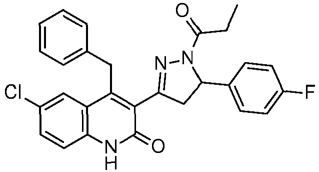 | 4-benzyl-6-chloro-3-[3-(4-fluorophenyl)-2-propanoyl-3,4-dihydropyrazol-5-yl]-1H-quinolin-2-one | ELISA assay: EC₅₀ 65±10 µM |
| d.55 | 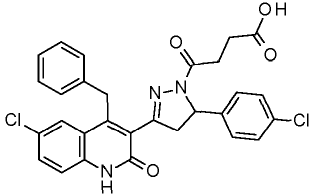 | 4-[5-(4-benzyl-6-chloro-2-oxo-1H-quinolin-3-yl)-3-(4-chlorophenyl)-3,4-dihydropyrazol-2-yl]-4-oxo-butanoic acid | ELISA assay: EC₅₀ 70±9 µM |
| | | | Cell viability: EC₅₀ 23.21 µM |
| d.58 | 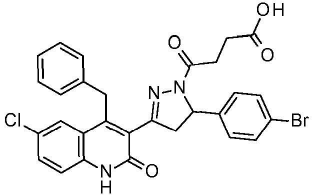 | 4-[5-(4-benzyl-6-chloro-2-oxo-1H-quinolin-3-yl)-3-(4-bromophenyl)-3,4-dihydropyrazol-2-yl]-4-oxo-butanoic acid | ELISA assay: EC₅₀ 40±5 µM |
| | | | Cell viability: EC₅₀ 23.64 µM |
| d.61 | 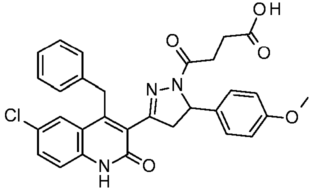 | 4-[5-(4-benzyl-6-chloro-2-oxo-1H-quinolin-3-yl)-3-(4-methoxyphenyl)-3,4-dihydropyrazol-2-yl]-4-oxo-butanoic acid | ELISA assay: EC₅₀ >100 µM |
| d.66 | 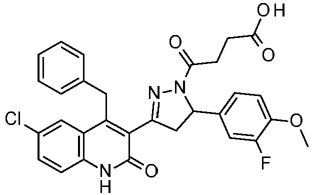 | 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid | ELISA assay: EC₅₀ 25±5 µM |
| | | | Cell viability: EC₅₀ 79.23 µM |
| | | | HR inhibition: 6.15 % at 20 µM |
| d.69 | 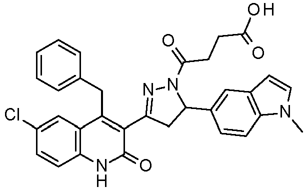 | 4-[5-(4-benzyl-6-chloro-2-oxo-1H-quinolin-3-yl)-3-(1-methylindol-5-yl)-3,4-dihydropyrazol-2-yl]-4-oxo-butanoic acid | ELISA assay: EC₅₀ 10±2 µM |
| d.73 | 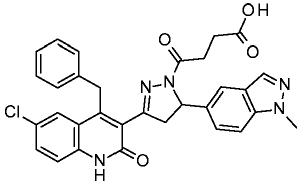 | 4-[5-(4-benzyl-6-chloro-2-oxo-1H-quinolin-3-yl)-3-(1-methylindazol-5-yl)-3,4-dihydropyrazol-2-yl]-4-oxo-butanoic acid | ELISA assay: EC₅₀ 40±5 µM |
| | | | Cell viability: EC₅₀ 81.81 µM |
| d.83 | 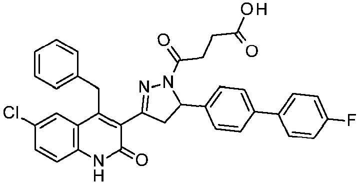 | 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid | ELISA assay: EC₅₀ 12±2 µM |
| d.84 | 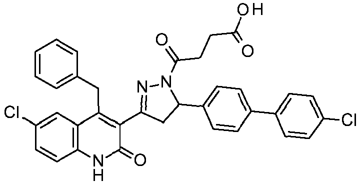 | 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid | ELISA assay: EC₅₀ 90±5 µM |
| | | | Cell viability: EC₅₀ 28.41 µM |
| | | | HR inhibition: 21.72 % at 20 µM |
| d.89 | 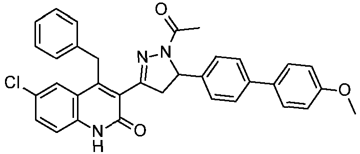 | 3-[2-acetyl-3-[4-(4-methoxyphenyl)phenyl]-3,4-dihydropyrazol-5-yl]-4-benzyl-6-chloro-1H-quinolin-2-one | ELISA assay: EC₅₀ 105±10 µM |
| d.93 | 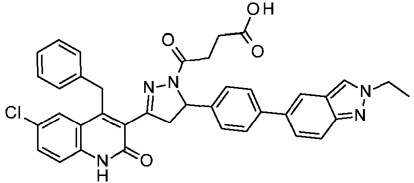 | 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid | ELISA assay: EC₅₀ 53±5 µM |
| | | | Cell viability: EC₅₀ 56.62 µM |
| d.97 | 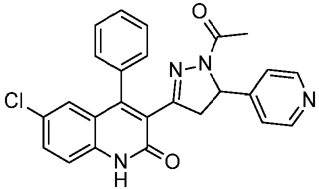 | 3-[2-acetyl-3-(4-pyridyl)-3,4-dihydropyrazol-5-yl]-6-chloro-4-phenyl-1H-quinolin-2-one | ELISA assay: EC₅₀ 60±7 µM |
| | | | Cell viability: EC₅₀ 24.33 µM |
| d.98 | 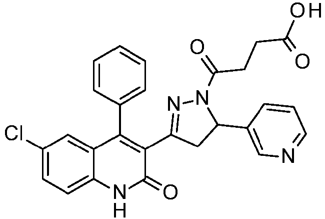 | 4-[5-(6-chloro-2-oxo-4-phenyl-1H-quinolin-3-yl)-3-(3-pyridyl)-3,4-dihydropyrazol-2-yl]-4-oxo-butanoic acid | ELISA assay: EC₅₀ 75±12 µM |
| | | | Cell viability: EC₅₀ 143.83 µM |
| | | | HR inhibition: 21.97 % at 20 µM |
| d.99 | 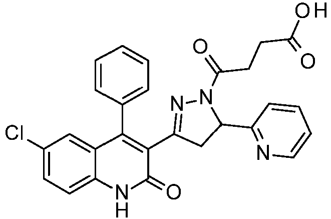 | 4-[5-(6-chloro-2-oxo-4-phenyl-1H-quinolin-3-yl)-3-(2-pyridyl)-3,4-dihydropyrazol-2-yl]-4-oxo-butanoic acid | ELISA assay: EC₅₀ 190±20 µM |
| d.100 | 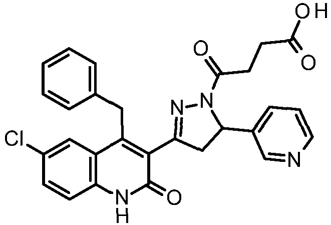 | 4-[5-(4-benzyl-6-chloro-2-oxo-1H-quinolin-3-yl)-3-(3-pyridyl)-3,4-dihydropyrazol-2-yl]-4-oxo-butanoic acid | ELISA assay: EC₅₀ 15±5 µM |
| d.104 | 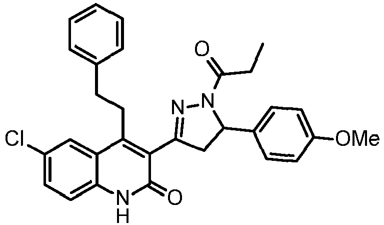 | 6-chloro-3-[3-(4-methoxyphenyl)-2-propanoyl-3,4-dihydropyrazol-5-yl]-4-(2-phenylethyl)-1H-quinolin-2-one | ELISA assay: EC₅₀ 24±6 µM |
| | | | Cell viability: EC₅₀ 53.00 µM |
| d.105 | 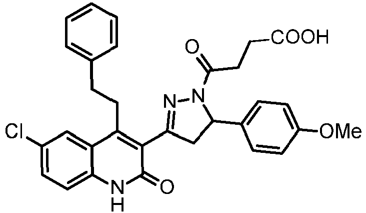 | 4-[5-[6-chloro-2-oxo-4-(2-phenylethyl)-1H-quinolin-3-yl]-3-(4-methoxyphenyl) -3,4-dihydropyrazol-2-yl]-4-oxo-butanoic acid | ELISA assay: EC₅₀ 20±3 µM |
| | | | Cell viability: EC₅₀ 29.58 µM |

### 3. Pharmacokinetic studies

### a. Animal models

Male C57B6/J mice, 8 weeks old were used (Charles River). All procedures were performed in accordance with the Ethical Guidelines of European Communities Council (Directive 2010/63/EU of 22 September 2010) and accepted by the Italian Ministry of Health. All efforts were made to minimize animal suffering and to use the minimal number of animals required to produce reliable results, according to the "3Rs concept". Animals were group-housed in ventilated cages and had free access to food and water. They were maintained under a 12-hour light/dark cycle (lights on at 8:00 am) at controlled temperature (21°C ± 1°C) and relative humidity (55% ± 10%).

### b. Experimental procedure

Compound **d.12** was administered orally (PO), intraperitoneally (IP) and intravenously (IV) to C57B6/J male mice at 2 mg/kg/10 ml, 20 mg/kg/10 ml and 2 mg/kg/5 ml, respectively. Vehicle was composed by PEG400/Tween 80/Saline solution at 10/10/80 % in volume, respectively for PO and IV administration. For IP administration, the same vehicle was used with the addition of 10 % DMSO in final volume. Three animals per dose were treated. Blood samples at 0, 15, 30, 60, 120, 240, and 480 min after administration were collected for PO and IP arms. Blood samples at 0, 5, 15, 30, 60, 120 and 240 min after administration were collected for IV arm. Plasma was separated from blood by centrifugation for 15 min at 1500 rpm a 4°C, collected in a eppendorf tube and frozen (-80°C). Control animals treated with vehicle only were also included in the experimental protocol.

### c. Pharmacokinetic measurement compound d.12

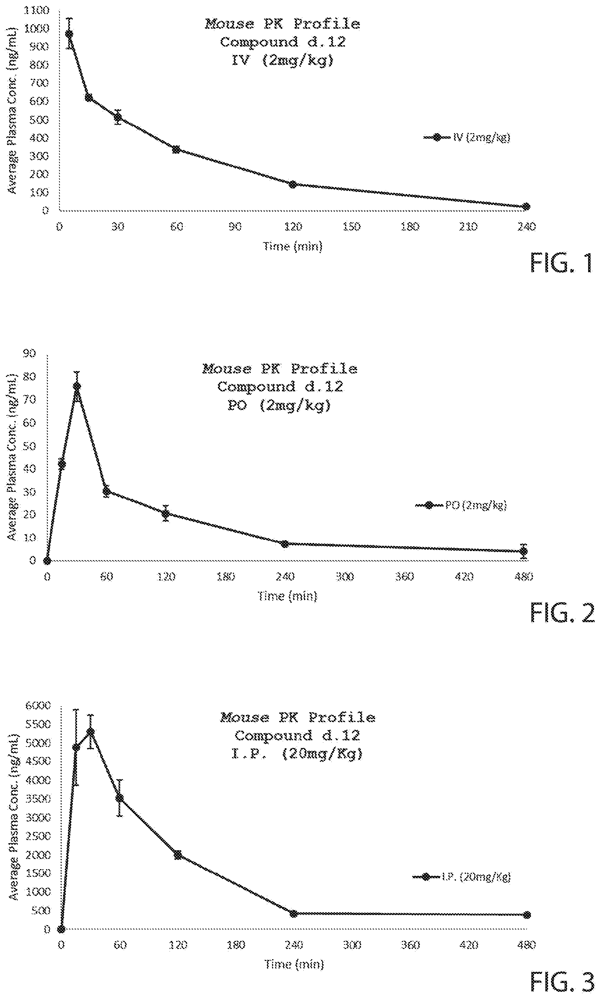

Plasma samples were centrifuged at 21.100 x g for 15 min at 4 °C. An aliquot of each plasma sample was extracted (1:3) with cold CH₃CN containing 200 nM of d.48 as internal standard being a close analogue of the parent compound. A calibration curve was prepared in naïve mouse plasma over a 1 nM - 10 µM range. Three quality control samples were prepared by spiking the parent compound in naïve mouse plasma to 20, 200 and 2000 nM as final concentrations. The calibrators and quality control samples were extracted (1:3) with the same extraction solution as the plasma samples. The plasma samples, calibrators and quality control samples were centrifuged at 3.270 x g for 15 min at 4 °C. The supernatants were further diluted (1:1) with H₂O, and analyzed by LC/MS-MS on a Waters ACQUITY UPLC/MS TQD system consisting of a Triple Quadrupole Detector (TQD) Mass Spectrometer equipped with an Electrospray Ionization interface and a Photodiode Array eλ Detector from Waters Inc. (Milford, MA, USA). Electrospray ionization was applied in positive mode. Compound-dependent parameters as MRM transitions and collision energy were developed for the parent compound and the internal standard. The analyses were run on an ACQUITY UPLC BEH C18 (50x2.1mmID, particle size 1.7µm) with a VanGuard BEH C18 pre-column (5x2.1mmID, particle size 1.7µm) at 40 °C, using H₂O + 0.1% HCOOH (A) and CH₃CN + 0.1% HCOOH (B) as mobile phase at 0.5 mL/min. A linear gradient was applied starting at 40 % B with an initial hold for 0.2 min, then 40-100 % B in 2 min, followed by a hold for 0.1 min at 100 % B. All samples (plasma samples, calibrators and quality controls) were quantified by MRM peak area response factor in order to determine the levels of the parent compound in plasma. The plasma concentrations versus time were plotted and the profiles were fitted using PK Solutions Excel Application (Summit Research Service, USA) in order to determine the pharmacokinetic parameters. The results are reported in Tables 2 and 3 and in Figures 1, 2 and 3.

**Table 2**

| | **IV (2mg/kg)** | | **PO (2mg/kg)** | |
|---|---|---|---|---|
| **Time Points** | **Average conc.** | **STD** | **Average conc.** | **STD** |
| **min** | **ng/mL** | | **ng/mL** | |
| 0 | - | - | 0 | 0 |
| 5 | 973 | 81 | - | - |
| 15 | 622 | 16 | 42 | 2 |
| 30 | 515 | 39 | 76 | 6 |
| 60 | 337 | 16 | 30 | 3 |
| 120 | 146 | 11 | 21 | 3 |
| 240 | 23 | 3 | 7 | 0 |
| 480 | - | - | 4 | 3 |

**Table 3**

| **PK Parameters** | | | | |
|---|---|---|---|---|
| **Parameter** | **Unit** | **IV (2 mg/kg)** | **PO (2 mg/kg)** | **IP (20 mg/kg)** |
| **Cmax (obs)** | ng/mL | 973 | 76 | 5304 |
| **Tmax (obs)** | min | 5 | 30 | 30 |
| **AUC (calc) ¹** | min*ng/mL | 53913 | 6000 | 557088 |
| **t_{½} (calc)** | min | 47 | 281 | 175 |
| **VD (calc)** | mL/kg | 2199 | 90009 | 6701 |
| **CL (calc)** | mL/min/Kg | 33 | 222 | 27 |
| **F²** | % | 11 | | - |

### BIBLIOGRAPHY

(1) Nijman SMB. "Synthetic lethality: General principles, utility and detection using genetic screens in human cells." FEBS Lett. Federation of European Biochemical Societies (2011), 585, 1-6.
(2) Kaelin WG. "The concept of synthetic lethality in the context of anticancer therapy." Nat Rev Cancer 2005; 5:689-98.
(3) Deeks ED. "Olaparib: first global approval." Drugs (2015), 75, 231-40.
(4) Jdey W. et al. "Drug-Driven Synthetic Lethality: Bypassing Tumor Cell Genetics with a Combination of AsiDNA and PARP Inhibitors. Clin. Cancer Res. (2017), 23, 1001-1011.
(5) Roy R, Chun J, Powell SN. "BRCA1 and BRCA2: different roles in a common pathway of genome protection". Nat Rev Cancer Nature Publishing Group (2012), 12, 68-78.
(6) Falchi F. et al, "Synthetic Lethality Triggered by Combining Olaparib with BRCA2-Rad51 Disruptors." ACS Chem Biol. (2017), 12(10), 2491-2497.
(7) Roberti M. et al, "Rad51/BRCA2 disruptors inhibit homologous recombination and synergize with olaparib in pancreatic cancer cells", European Journal of Medicinal Chemistry (2019), 165, 80-92.
(8) Klein HL. "The consequences of Rad51 overexpression for normal and tumor cells". DNA Repair (Amst) (2008), 7,686-93.

## Claims

1. Compound of formula (Ia): 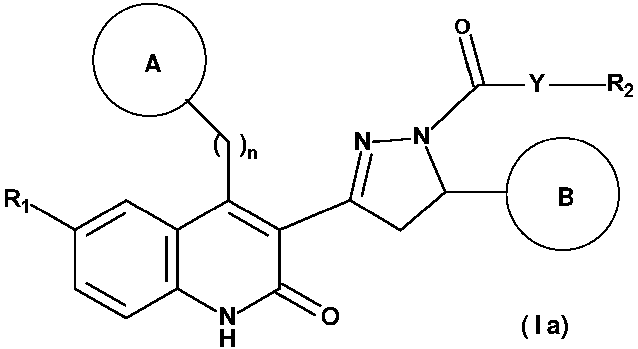 wherein
**R₁** is halogen;
**R₂** is selected from the group consisting of hydrogen and COOH;
n is 1;
Y is C₁₋₄alkyl;
A is phenyl;
B is 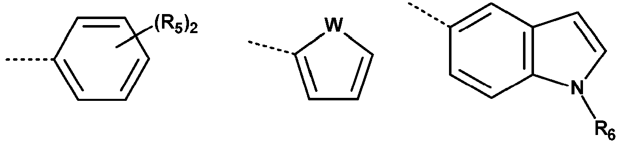 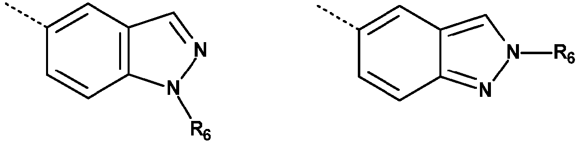 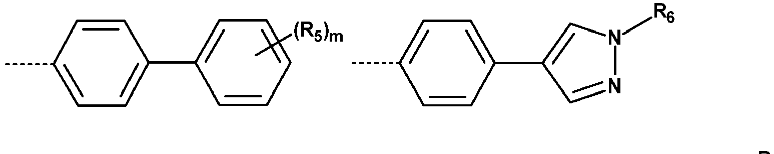 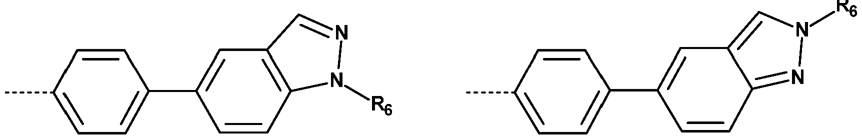
**R₅** is hydrogen, halogen, linear or branched C₁₋₄alkyl, halo-C₁₋₄alkyl, O-C₁₋₄alkyl;
**R₆** is selected from the group consisting of H and C₁₋₄alkyl;
**m** is 1 or 2; and
**W** is selected from the group consisting of O and NH;
or compounds of formula (Ia): 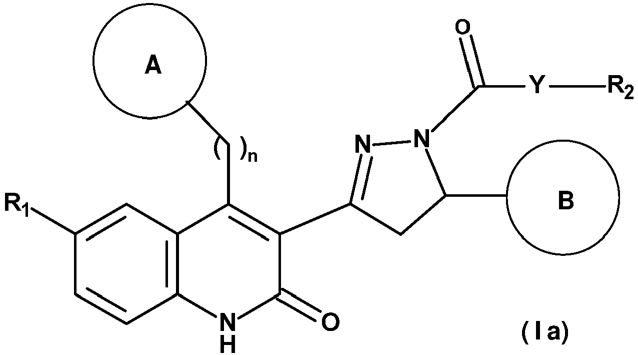
wherein
**R₁** is halogen;
R₂ is selected from the group consisting of hydrogen and COOH; n is 0;
Y is selected from the group consisting of C₁₋₄alkyl;
A is morpholino;
B is 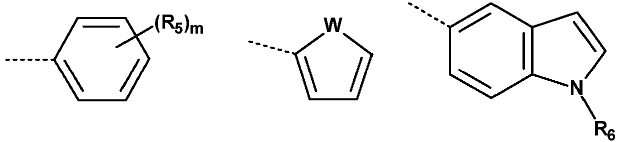 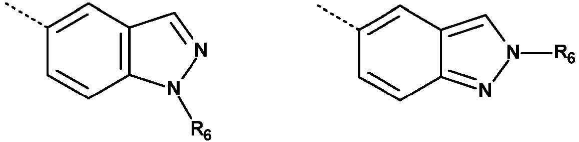 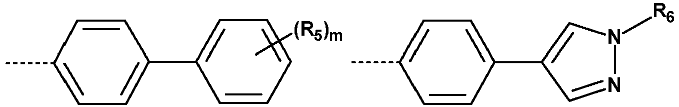 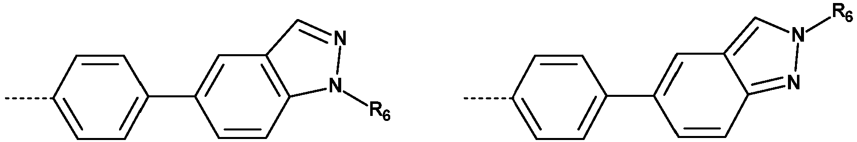
**R₅** is hydrogen, halogen, linear or branched C₁₋₄alkyl, halo-C₁₋₄alkyl, O-C₁₋₄alkyl;
**R₆** is selected from the group consisting of H and C₁₋₄alkyl;
**m** is 1 or 2; and
**W** is selected from the group consisting of O and NH,
or their pharmaceutically acceptable salts or solvates thereof.

2. Compound according to claim 1 selected from the group consisting of:
| | |
|---|---|
| d.50 | 4-[5-(4-benzyl-6-chloro-2-oxo-1H-quinolin-3-yl)-3-(4-fluorophenyl)-3,4-dihydropyrazol-2-yl]-4-oxo-butanoic acid |
| d.51 | 3-[2-acetyl-3-(4-fluorophenyl)-3,4-dihydropyrazol-5-yl]-4-benzyl-6-chloro-1H-quinolin-2-one |
| d.52 | 3-[2-acetyl-3-(4-methoxyphenyl)-3,4-dihydropyrazol-5-yl]-4-benzyl-6-chloro-1H-quinolin-2-one |
| d.53 | 4-benzyl-6-chloro-3-[3-(4-methoxyphenyl)-2-propanoyl-3,4-dihydropyrazol-5-yl]-1H-quinolin-2-one |
| d.54 | 4-benzyl-6-chloro-3-[3-(4-fluorophenyl)-2-propanoyl-3,4-dihydropyrazol-5-yl]-1H-quinolin-2-one |
| d.55 | 4-[5-(4-benzyl-6-chloro-2-oxo-1H-quinolin-3-yl)-3-(4-chlorophenyl)-3,4-dihydropyrazol-2-yl]-4-oxo-butanoic acid |
| d.56 | 3-[2-acetyl-3-(4-chlorophenyl)-3,4-dihydropyrazol-5-yl]-4-benzyl-6-chloro-1H-quinolin-2-one |
| d.57 | 4-benzyl-6-chloro-3-[3-(4-chlorophenyl)-2-propanoyl-3,4-dihydropyrazol-5-yl]-1H-quinolin-2-one |
| d.58 | 4-[5-(4-benzyl-6-chloro-2-oxo-1H-quinolin-3-yl)-3-(4-bromophenyl)-3,4-dihydropyrazol-2-yl]-4-oxo-butanoic acid |
| d.59 | 3-[2-acetyl-3-(4-bromophenyl)-3,4-dihydropyrazol-5-yl]-4-benzyl-6-chloro-1H-quinolin-2-one |
| d.60 | 4-benzyl-3-[3-(4-bromophenyl)-2-propanoyl-3,4-dihydropyrazol-5-yl]-6-chloro-1H-quinolin-2-one |
| d.61 | 4-[5-(4-benzyl-6-chloro-2-oxo-1H-quinolin-3-yl)-3-(4-methoxyphenyl)-3,4-dihydropyrazol-2-yl]-4-oxo-butanoic acid |
| d.62 | 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid |
| d.63 | 3-[2-acetyl-3-[4-(trifluoromethyl)phenyl]-3,4-dihydropyrazol-5-yl]-4-benzyl-6-chloro-1H-quinolin-2-one |
| d.64 | 4-[5-(4-benzyl-6-chloro-2-oxo-1H-quinolin-3-yl)-3-(4-tert-butylphenyl)-3,4-dihydropyrazol-2-yl]-4-oxo-butanoic acid |
| d.65 | 3-[2-acetyl-3-(4-tert-butylphenyl)-3,4-dihydropyrazol-5-yl]-4-benzyl-6-chloro-1H-quinolin-2-one |
| d.66 | 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid |
| d.67 | 4-[5-(4-benzyl-6-chloro-2-oxo-1H-quinolin-3-yl)-3-(2-furyl)-3,4-dihydropyrazol-2-yl]-4-oxo-butanoic acid |
| d.68 | 3-[2-acetyl-3-(2-furyl)-3,4-dihydropyrazol-5-yl]-4-benzyl-6-chloro-1H-quinolin-2-one |
| d.69 | 4-[5-(4-benzyl-6-chloro-2-oxo-1H-quinolin-3-yl)-3-(1-methylindol-5-yl)-3,4-dihydropyrazol-2-yl]-4-oxo-butanoic acid |
| d.70 | 3-[2-acetyl-3-(1-methylindol-5-yl)-3,4-dihydropyrazol-5-yl]-4-benzyl-6-chloro-1H-quinolin-2-one |
| d.71 | 4-[5-(4-benzyl-6-chloro-2-oxo-1H-quinolin-3-yl)-3-(1-ethylindol-5-yl)-3,4-dihydropyrazol-2-yl]-4-oxo-butanoic acid |
| d.72 | 3-[2-acetyl-3-(1-ethylindol-5-yl)-3,4-dihydropyrazol-5-yl]-4-benzyl-6-chloro-1H-quinolin-2-one |
| d.73 | 4-[5-(4-benzyl-6-chloro-2-oxo-1H-quinolin-3-yl)-3-(1-methylindazol-5-yl)-3,4-dihydropyrazol-2-yl]-4-oxo-butanoic acid |
| d.74 | 3-[2-acetyl-3-(1-methylindazol-5-yl)-3,4-dihydropyrazol-5-yl]-4-benzyl-6-chloro-1H-quinolin-2-one |
| d.75 | 4-[5-(4-benzyl-6-chloro-2-oxo-1H-quinolin-3-yl)-3-(2-methylindazol-5-yl)-3,4-dihydropyrazol-2-yl]-4-oxo-butanoic acid |
| d.76 | 4-[5-(4-benzyl-6-chloro-2-oxo-1H-quinolin-3-yl)-3-(2-ethylindazol-5-yl)-3,4-dihydropyrazol-2-yl]-4-oxo-butanoic acid |
| d.77 | 3-[2-acetyl-3-(2-ethylindazol-5-yl)-3,4-dihydropyrazol-5-yl]-4-benzyl-6-chloro-1H-quinolin-2-one |
| d.78 | 4-[5-(4-benzyl-6-chloro-2-oxo-1H-quinolin-3-yl)-3-(1-ethylindazol-5-yl)-3,4-dihydropyrazol-2-yl]-4-oxo-butanoic acid |
| d.79 | 3-[2-acetyl-3-(1-ethylindazol-5-yl)-3,4-dihydropyrazol-5-yl]-4-benzyl-6-chloro-1H-quinolin-2-one |
| d.80 | 4-[5-(4-benzyl-6-chloro-2-oxo-1H-quinolin-3-yl)-3-(1-propylindazol-5-yl)-3,4-dihydropyrazol-2-yl]-4-oxo-butanoic acid |
| d.81 | 3-[2-acetyl-3-(1-propylindazol-5-yl)-3,4-dihydropyrazol-5-yl]-4-benzyl-6-chloro-1H-quinolin-2-one |
| d.82 | 4-[5-(4-benzyl-6-chloro-2-oxo-1H-quinolin-3-yl)-3-(2-propylindazol-5-yl)-3,4-dihydropyrazol-2-yl]-4-oxo-butanoic acid |
| d.83 | 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid |
| d.84 | 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid |
| d.85 | 3-[2-acetyl-3-[4-(4-chlorophenyl)phenyl]-3,4-dihydropyrazol-5-yl]-4-benzyl-6-chloro-1H-quinolin-2-one |
| d.86 | 4-[5-(4-benzyl-6-chloro-2-oxo-1H-quinolin-3-yl)-3-[4-(4-bromophenyl)phenyl]-3,4-dihydropyrazol-2-yl]-4-oxo-butanoic acid |
| d.87 | 3-[2-acetyl-3-[4-(4-bromophenyl)phenyl]-3,4-dihydropyrazol-5-yl]-4-benzyl-6-chloro-1H-quinolin-2-one |
| d.88 | 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid |
| d.89 | 3-[2-acetyl-3-[4-(4-methoxyphenyl)phenyl]-3,4-dihydropyrazol-5-yl]-4-benzyl-6-chloro-1H-quinolin-2-one |
| d.90 | 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid |
| d.91 | 3-[2-acetyl-3-[4-(1-propylpyrazol-4-yl)phenyl]-3,4-dihydropyrazol-5-yl]-4-benzyl-6-chloro-1H-quinolin-2-one |
| d.92 | 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid |
| d.93 | 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid |
| d.94 | 3-(2-acetyl-3-phenyl-3,4-dihydropyrazol-5-yl)-4-benzyl-6-chloro-1H-quinolin-2-one |
| d.95 | 4-benzyl-6-chloro-3-(3-phenyl-2-propanoyl-3,4-dihydropyrazol-5-yl)-1H-quinolin-2-one |
| d.96 | 3-[2-acetyl-3-(p-tolyl)-3,4-dihydropyrazol-5-yl]-4-benzyl-6-chloro-1H-quinolin-2-one |
| d.101 | 4-[5-(4-benzyl-6-chloro-2-oxo-1H-quinolin-3-yl)-3-(3-pyridyl)-3,4-dihydropyrazol-2-yl]-4-oxo-butanoic acid |
| d.105 | 4-[5-(4-benzyl-6-chloro-2-oxo-1H-quinolin-3-yl)-3-(2-chlorophenyl)-3,4-dihydropyrazol-2-yl]-4-oxo-butanoic acid |
| d.106 | 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid |
| d.109 | 4-[5-(6-chloro-4-morpholino-2-oxo-1H-quinolin-3-yl)-3-(4-methoxyphenyl)-3,4-dihydropyrazol-2-yl]-4-oxo-butanoic acid |

3. Pharmaceutical composition comprising a compound according to any of claims 1 or 2 and at least one pharmaceutically acceptable carrier, stabilizer, diluent or excipient thereof.

4. Compound according to any of claims 1 or 2 for the use as a medicament.

5. Compound according to any of claims 1 or 2 for the use in the treatment of diseases or disorders selected from the group consisting of primary and metastatic neoplastic diseases, hyperplasia, metaplasia, dysplasia, cancer, cancer metastasis, benign tumors, hyperproliferative disorders.

6. Compound for the use according to claim 5 wherein the diseases or disorders are selected from the group consisting of ovarian, breast and pancreatic cancer.

7. Compound for the use according to any of claims 5 or 6, selected from the group consisting of:
| | |
|---|---|
| d.50 | 4-[5-(4-benzyl-6-chloro-2-oxo-1H-quinolin-3-yl)-3-(4-fluorophenyl)-3,4-dihydropyrazol-2-yl]-4-oxo-butanoic acid |
| d.51 | 3-[2-acetyl-3-(4-fluorophenyl)-3,4-dihydropyrazol-5-yl]-4-benzyl-6-chloro-1H-quinolin-2-one |
| d.52 | 3-[2-acetyl-3-(4-methoxyphenyl)-3,4-dihydropyrazol-5-yl]-4-benzyl-6-chloro-1H-quinolin-2-one |
| d.53 | 4-benzyl-6-chloro-3-[3-(4-methoxyphenyl)-2-propanoyl-3,4-dihydropyrazol-5-yl]-1H-quinolin-2-one |
| d.54 | 4-benzyl-6-chloro-3-[3-(4-fluorophenyl)-2-propanoyl-3,4-dihydropyrazol-5-yl]-1H-quinolin-2-one |
| d.55 | 4-[5-(4-benzyl-6-chloro-2-oxo-1H-quinolin-3-yl)-3-(4-chlorophenyl)-3,4-dihydropyrazol-2-yl]-4-oxo-butanoic acid |
| d.56 | 3-[2-acetyl-3-(4-chlorophenyl)-3,4-dihydropyrazol-5-yl]-4-benzyl-6-chloro-1H-quinolin-2-one |
| d.57 | 4-benzyl-6-chloro-3-[3-(4-chlorophenyl)-2-propanoyl-3,4-dihydropyrazol-5-yl]-1H-quinolin-2-one |
| d.58 | 4-[5-(4-benzyl-6-chloro-2-oxo-1H-quinolin-3-yl)-3-(4-bromophenyl)-3,4-dihydropyrazol-2-yl]-4-oxo-butanoic acid |
| d.59 | 3-[2-acetyl-3-(4-bromophenyl)-3,4-dihydropyrazol-5-yl]-4-benzyl-6-chloro-1H-quinolin-2-one |
| d.60 | 4-benzyl-3-[3-(4-bromophenyl)-2-propanoyl-3,4-dihydropyrazol-5-yl]-6-chloro-1H-quinolin-2-one |
| d.61 | 4-[5-(4-benzyl-6-chloro-2-oxo-1H-quinolin-3-yl)-3-(4-methoxyphenyl)-3,4-dihydropyrazol-2-yl]-4-oxo-butanoic acid |
| d.62 | 4-[5-(4-benzyl-6-chloro-2-oxo-1H-quinolin-3-yl)-3-[4-(trifluoromethyl)phenyl]-3,4-dihydropyrazol-2-yl]-4-oxo-butanoic acid |
| d.63 | 3-[2-acetyl-3-[4-(trifluoromethyl)phenyl]-3,4-dihydropyrazol-5-yl]-4-benzyl-6-chloro-1H-quinolin-2-one |
| d.64 | 4-[5-(4-benzyl-6-chloro-2-oxo-1H-quinolin-3-yl)-3-(4-tert-butylphenyl)-3,4-dihydropyrazol-2-yl]-4-oxo-butanoic acid |
| d.65 | 3-[2-acetyl-3-(4-tert-butylphenyl)-3,4-dihydropyrazol-5-yl]-4-benzyl-6-chloro-1H-quinolin-2-one |
| d.66 | 4-[5-(4-benzyl-6-chloro-2-oxo-1H-quinolin-3-yl)-3-(3-fluoro-4-methoxy-phenyl)-3,4-dihydropyrazol-2-yl]-4-oxo-butanoic acid |
| d.67 | 4-[5-(4-benzyl-6-chloro-2-oxo-1H-quinolin-3-yl)-3-(2-furyl)-3,4-dihydropyrazol-2-yl]-4-oxo-butanoic acid |
| d.68 | 3-[2-acetyl-3-(2-furyl)-3,4-dihydropyrazol-5-yl]-4-benzyl-6-chloro-1H-quinolin-2-one |
| d.69 | 4-[5-(4-benzyl-6-chloro-2-oxo-1H-quinolin-3-yl)-3-(1-methylindol-5-yl)-3,4-dihydropyrazol-2-yl]-4-oxo-butanoic acid |
| d.70 | 3-[2-acetyl-3-(1-methylindol-5-yl)-3,4-dihydropyrazol-5-yl]-4-benzyl-6-chloro-1H-quinolin-2-one |
| d.71 | 4-[5-(4-benzyl-6-chloro-2-oxo-1H-quinolin-3-yl)-3-(1-ethylindol-5-yl)-3,4-dihydropyrazol-2-yl]-4-oxo-butanoic acid |
| d.72 | 3-[2-acetyl-3-(1-ethylindol-5-yl)-3,4-dihydropyrazol-5-yl]-4-benzyl-6-chloro-1H-quinolin-2-one |
| d.73 | 4-[5-(4-benzyl-6-chloro-2-oxo-1H-quinolin-3-yl)-3-(1-methylindazol-5-yl)-3,4-dihydropyrazol-2-yl]-4-oxo-butanoic acid |
| d.74 | 3-[2-acetyl-3-(1-methylindazol-5-yl)-3,4-dihydropyrazol-5-yl]-4-benzyl-6-chloro-1H-quinolin-2-one |
| d.75 | 4-[5-(4-benzyl-6-chloro-2-oxo-1H-quinolin-3-yl)-3-(2-methylindazol-5-yl)-3,4-dihydropyrazol-2-yl]-4-oxo-butanoic acid |
| d.76 | 4-[5-(4-benzyl-6-chloro-2-oxo-1H-quinolin-3-yl)-3-(2-ethylindazol-5-yl)-3,4-dihydropyrazol-2-yl]-4-oxo-butanoic acid |
| d.77 | 3-[2-acetyl-3-(2-ethylindazol-5-yl)-3,4-dihydropyrazol-5-yl]-4-benzyl-6-chloro-1H-quinolin-2-one |
| d.78 | 4-[5-(4-benzyl-6-chloro-2-oxo-1H-quinolin-3-yl)-3-(1-ethylindazol-5-yl)-3,4-dihydropyrazol-2-yl]-4-oxo-butanoic acid |
| d.79 | 3-[2-acetyl-3-(1-ethylindazol-5-yl)-3,4-dihydropyrazol-5-yl]-4-benzyl-6-chloro-1H-quinolin-2-one |
| d.80 | 4-[5-(4-benzyl-6-chloro-2-oxo-1H-quinolin-3-yl)-3-(1-propylindazol-5-yl)-3,4-dihydropyrazol-2-yl]-4-oxo-butanoic acid |
| d.81 | 3-[2-acetyl-3-(1-propylindazol-5-yl)-3,4-dihydropyrazol-5-yl]-4-benzyl-6-chloro-1H-quinolin-2-one |
| d.82 | 4-[5-(4-benzyl-6-chloro-2-oxo-1H-quinolin-3-yl)-3-(2-propylindazol-5-yl)-3,4-dihydropyrazol-2-yl]-4-oxo-butanoic acid |
| d.83 | 4-[5-(4-benzyl-6-chloro-2-oxo-1H-quinolin-3-yl)-3-[4-(4-fluorophenyl)phenyl]-3,4-dihydropyrazol-2-yl]-4-oxo-butanoic acid |
| d.84 | 4-[5-(4-benzyl-6-chloro-2-oxo-1H-quinolin-3-yl)-3-[4-(4-chlorophenyl)phenyl]-3,4-dihydropyrazol-2-yl]-4-oxo-butanoic acid |
| d.85 | 3-[2-acetyl-3-[4-(4-chlorophenyl)phenyl]-3,4-dihydropyrazol-5-yl]-4-benzyl-6-chloro-1H-quinolin-2-one |
| d.86 | 4-[5-(4-benzyl-6-chloro-2-oxo-1H-quinolin-3-yl)-3-[4-(4-bromophenyl)phenyl]-3,4-dihydropyrazol-2-yl]-4-oxo-butanoic acid |
| d.87 | 3-[2-acetyl-3-[4-(4-bromophenyl)phenyl]-3,4-dihydropyrazol-5-yl]-4-benzyl-6-chloro-1H-quinolin-2-one |
| d.88 | 4-[5-(4-benzyl-6-chloro-2-oxo-1H-quinolin-3-yl)-3-[4-(4-methoxyphenyl)phenyl]-3,4-dihydropyrazol-2-yl]-4-oxo-butanoic acid |
| d.89 | 3-[2-acetyl-3-[4-(4-methoxyphenyl)phenyl]-3,4-dihydropyrazol-5-yl]-4-benzyl-6-chloro-1H-quinolin-2-one |
| d.90 | 4-[5-(4-benzyl-6-chloro-2-oxo-1H-quinolin-3-yl)-3-[4-(1-propylpyrazol-4-yl)phenyl]-3,4-dihydropyrazol-2-yl]-4-oxo-butanoic acid |
| d.91 | 3-[2-acetyl-3-[4-(1-propylpyrazol-4-yl)phenyl]-3,4-dihydropyrazol-5-yl]-4-benzyl-6-chloro-1H-quinolin-2-one |
| d.92 | 4-[5-(4-benzyl-6-chloro-2-oxo-1H-quinolin-3-yl)-3-[4-(1-ethylindazol-5-yl)phenyl]-3,4-dihydropyrazol-2-yl]-4-oxo-butanoic acid |
| d.93 | 4-[5-(4-benzyl-6-chloro-2-oxo-1H-quinolin-3-yl)-3-[4-(2-ethylindazol-5-yl)phenyl]-3,4-dihydropyrazol-2-yl]-4-oxo-butanoic acid |
| d.95 | 4-benzyl-6-chloro-3-(3-phenyl-2-propanoyl-3,4-dihydropyrazol-5-yl)-1H-quinolin-2-one |
| d.96 | 3-[2-acetyl-3-(p-tolyl)-3,4-dihydropyrazol-5-yl]-4-benzyl-6-chloro-1H-quinolin-2-one |
| d.98 | 4-[5-(6-chloro-2-oxo-4-phenyl-1H-quinolin-3-yl)-3-(3-pyridyl)-3,4-dihydropyrazol-2-yl]-4-oxo-butanoic acid |
| d.99 | 4-[5-(6-chloro-2-oxo-4-phenyl-1H-quinolin-3-yl)-3-(2-pyridyl)-3,4-dihydropyrazol-2-yl]-4-oxo-butanoic acid |
| | |
| d.101 | 4-[5-(4-benzyl-6-chloro-2-oxo-1H-quinolin-3-yl)-3-(3-pyridyl)-3,4-dihydropyrazol-2-yl]-4-oxo-butanoic acid |
| d.105 | 4-[5-(4-benzyl-6-chloro-2-oxo-1H-quinolin-3-yl)-3-(2-chlorophenyl)-3,4-dihydropyrazol-2-yl]-4-oxo-butanoic acid |
| d.106 | 4-[5-(4-benzyl-6-chloro-2-oxo-1H-quinolin-3-yl)-3-(3,4-dimethoxyphenyl)-3,4-dihydropyrazol-2-yl]-4-oxo-butanoic acid |
| d.109 | 4-[5-(6-chloro-4-morpholino-2-oxo-1H-quinolin-3-yl)-3-(4-methoxyphenyl)-3,4-dihydropyrazol-2-yl]-4-oxo-butanoic acid |

## Patentansprüche

1. Verbindung der Formel (Ia): 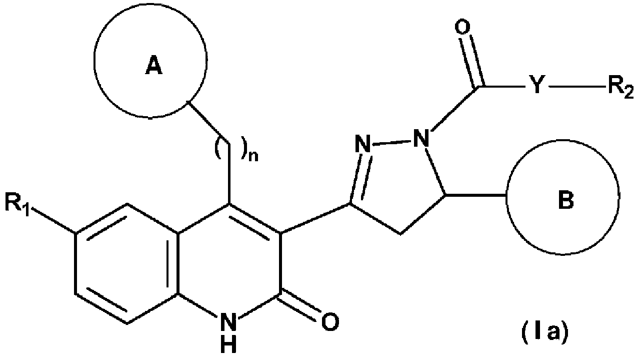
wobei
**R₁** Halogen ist;
**R₂** aus der Gruppe ausgewählt ist, die aus Wasserstoff und COOH besteht;
**n** 1 ist;
**Y** C₁₋₄-Alkyl ist;
**A** Phenyl ist;
**B** Folgendes ist: 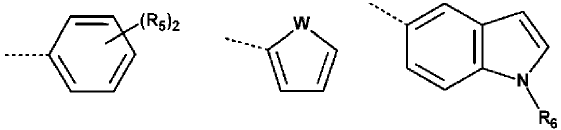 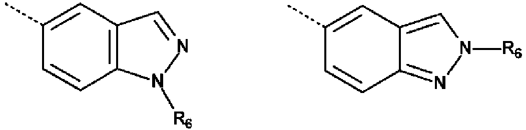 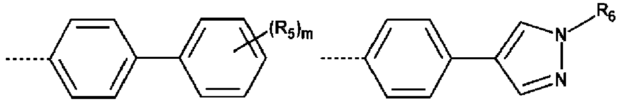 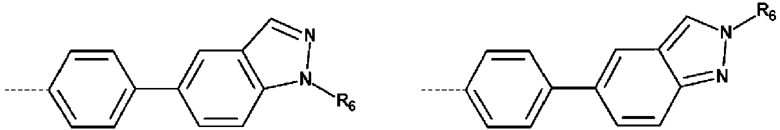
**R₅** Wasserstoff, Halogen, geradkettiges oder verzweigtes C₁₋₄-Alkyl, Halo-C₁₋₄-Alkyl, O-C₁₋₄-Alkyl ist;
**R₆** aus der Gruppe ausgewählt ist, die aus H und C₁₋₄-Alkyl besteht;
**m** gleich 1 oder 2 ist und
**W aus** der Gruppe ausgewählt ist, die aus O und NH besteht;
oder Verbindungen der Formel (Ia): 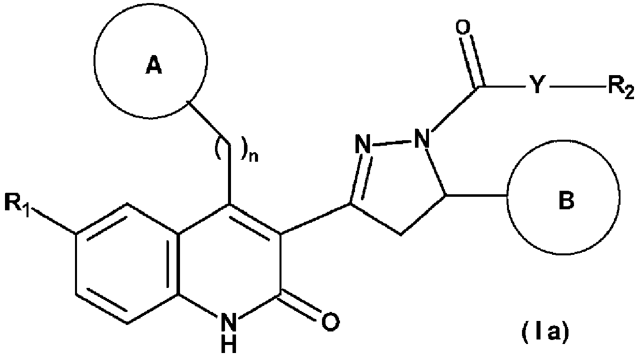
wobei
**R₁** Halogen ist;
**R₂** aus der Gruppe ausgewählt ist, die aus Wasserstoff und COOH besteht;
**n** 0 ist;
**Y** aus der Gruppe ausgewählt ist, die aus C₁₋₄-Alkyl besteht;
**A** Morpholino ist;
**B** Folgendes ist: 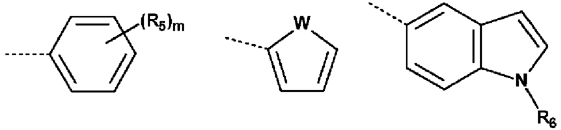 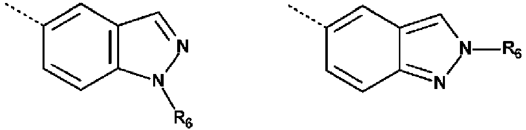 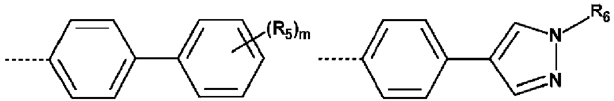 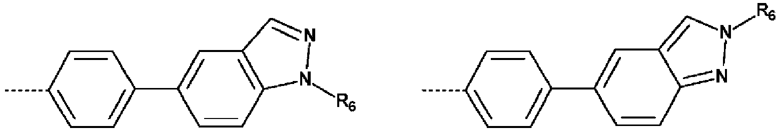
**R₅** Wasserstoff, Halogen, geradkettiges oder verzweigtes C₁₋₄-Alkyl, Halo-C₁₋₄-Alkyl, O-C₁₋₄-Alkyl ist;
**R₆** aus der Gruppe ausgewählt ist, die aus H und C₁₋₄-Alkyl besteht;
**m** gleich 1 oder 2 ist und
**W aus** der Gruppe ausgewählt ist, die aus O und NH besteht;
oder pharmazeutisch verträgliche Salze oder Solvate davon.

2. Verbindung nach Anspruch 1, ausgewählt aus der Gruppe bestehend aus:
| | |
|---|---|
| d.50 | 4-[5-(4-Benzyl-6-chlor-2-oxo-1H-chinolin-3-yl)-3-(4-fluorphenyl)-3,4-dihydropyrazol-2-yl]-4-oxobutansäure |
| d.51 | 3-[2-Acetyl-3-(4-fluorphenyl)-3,4-dihydropyrazol-5-yl]-4-benzyl-6-chlor-1H-chinolin-2-on |
| d.52 | 3-[2-Acetyl-3-(4-methoxyphenyl)-3,4-dihydropyrazol-5-yl]-4-benzyl-6-chlor-1H-chinolin-2-on |
| d.53 | 4-Benzyl-6-chlor-3-[3-(4-methoxyphenyl)-2-propanoyl-3,4-dihydropyrazol-5-yl]-1H-chinolin-2-on |
| d.54 | 4-Benzyl-6-chlor-3-[3-(4-fluorphenyl)-2-propanoyl-3,4-dihydropyrazol-5-yl]-1H-chinolin-2-on |
| d.55 | 4-[5-(4-benzyl-6-chlor-2-oxo-1H-chinolin-3-yl)-3-(4-Chlorphenyl)-3,4-dihydropyrazol-2-yl]-4-oxobutansäure |
| d.56 | 3-[2-Acetyl-3-(4-chlorphenyl)-3,4-dihydropyrazol-5-yl]-4-benzyl-6-chlor-1H-chinolin-2-on |
| d.57 | 4-Benzyl-6-chlor-3-[3-(4-chlorphenyl)-2-propanoyl-3,4-dihydropyrazol-5-yl]-1H-chinolin-2-on |
| d.58 | 4-[5-(4-benzyl-6-chlor-2-oxo-1H-chinolin-3-yl)-3-(4-bromphenyl)-3,4-dihydropyrazol-2-yl]-4-oxobutansäure |
| d.59 | 3-[2-Acetyl-3-(4-bromphenyl)-3,4-dihydropyrazol-5-yl]-4-benzyl-6-chlor-1H-chinolin-2-on |
| d.60 | 4-Benzyl-3-[3-(4-bromophenyl)-2-propanoyl-3,4-dihydropyrazol-5-yl]-6-chlor-1H-chilonin-2-on |
| d.61 | 4-[5-(4-Benzyl-6-chlor-2-oxo-1H-chinolin-3-yl)-3-(4-methoxyphenyl)-3,4-dihydropyrazol-2-yl]-4-oxobutansäure |
| d.62 | 4-[5-(4-Benzyl-6-chlor-2-oxo-1H-chinolin-3-yl)-3-[4-(trifluormethyl)phenyl]-3,4-dihydropyrazol-2-yl]-4-oxobutansäure |
| d.63 | 3-[2-Acetyl-3-[4-(trifluormethyl)phenyl]-3,4-dihydropyrazol-5-yl]-4-benzyl-6-chlor-1H-chinolin-2-on |
| d.64 | 4-[5-(4-Benzyl-6-chlor-2-oxo-1H-chinolin-3-yl)-3-(4-tert-butylphenyl)-3,4-dihydropyrazol-2-yl]-4-oxobutansäure |
| d.65 | 3-[2-Acetyl-3-(4-tert-butylphenyl)-3,4-dihydropyrazol-5-yl]-4-benzyl-6-chlor-1H-chinolin-2-on |
| d.66 | 4-[5-(4-Benzyl-6-chlor-2-oxo-1H-chinolin-3-yl)-3-(3-fluor-4-methoxyphenyl)-3,4-dihydropyrazol-2-yl]-4-oxobutansäure |
| d.67 | 4-[5-(4-Benzyl-6-chlor-2-oxo-1H-chinolin-3-yl)-3-(2-furyl)-3,4-dihydropyrazol-2-yl]-4-oxobutansäure |
| d.68 | 3-[2-Acetyl-3-(2-furyl)-3,4-dihydropyrazol-5-yl]-4-benzyl-6-chlor-1H-chinolin-2-on |
| d.69 | 4-[5-(4-Benzyl-6-chlor-2-oxo-1H-chinolin-3-yl)-3-(1-methylindol-5-yl)-3,4-dihydropyrazol-2-yl]-4-oxobutansäure |
| d.70 | 3-[2-Acetyl-3-(1-methylindol-5-yl)-3,4-dihydropyrazol-5-yl]-4-benzyl-6-chlor-1H-chinolin-2-on |
| d.71 | 4-[5-(4-Benzyl-6-chlor-2-oxo-1H-chinolin-3-yl)-3-(1-ethylindol-5-yl)-3,4-dihydropyrazol-2-yl]-4-oxobutansäure |
| d.72 | 3-[2-Acetyl-3-(1-ethylindol-5-yl)-3,4-dihydropyrazol-5-yl]-4-benzyl-6-chlor-1H-chinolin-2-on |
| d.73 | 4-[5-(4-Benzyl-6-chlor-2-oxo-1H-chinolin-3-yl)-3-(1-methylindazol-5-yl)-3,4-dihydropyrazol-2-yl]-4-oxobutansäure |
| d.74 | 3-[2-Acetyl-3-(1-methylindazol-5-yl)-3,4-dihydropyrazol-5-yl]-4-benzyl-6-chlor-1H-chinolin-2-on |
| d.75 | 4-[5-(4-Benzyl-6-chlor-2-oxo-1H-chinolin-3-yl)-3-(2-methylindazol-5-yl)-3,4-dihydropyrazol-2-yl]-4-oxobutansäure |
| d.76 | 4-[5-(4-Benzyl-6-chlor-2-oxo-1H-chinolin-3-yl)-3-(2-ethylindazol-5-yl)-3,4-dihydropyrazol-2-yl]-4-oxobutansäure |
| d.77 | 3-[2-Acetyl-3-(2-ethylindazol-5-yl)-3,4-dihydropyrazol-5-yl]-4-benzyl-6-chlor-1H-chinolin-2-on |
| d.78 | 4-[5-(4-Benzyl-6-chlor-2-oxo-1H-chinolin-3-yl)-3-(1-ethylindazol-5-yl)-3,4-dihydropyrazol-2-yl]-4-oxobutansäure |
| d.79 | 3-[2-Acetyl-3-(1-ethylindazol-5-yl)-3,4-dihydropyrazol-5-yl]-4-benzyl-6-chlor-1H-chinolin-2-on |
| d.80 | 4-[5-(4-Benzyl-6-chlor-2-oxo-1H-chinolin-3-yl)-3-(1-propylindazol-5-yl)-3,4-dihydropyrazol-2-yl]-4-oxobutansäure |
| d.81 | 3-[2-Acetyl-3-(1-propylindazol-5-yl)-3,4-dihydropyrazol-5-yl]-4-benzyl-6-chlor-1H-chinolin-2-on |
| d.82 | 4-[5-(4-Benzyl-6-chlor-2-oxo-1H-chinolin-3-yl)-3-(2-propylindazol-5-yl)-3,4-dihydropyrazol-2-yl]-4-oxobutansäure |
| d.83 | 4-[5-(4-Benzyl-6-chlor-2-oxo-1H-chinolin-3-yl)-3-[4-(4-Fluorphenyl)phenyl]-3,4-dihydropyrazol-2-yl]-4-oxobutansäure |
| d.84 | 4-[5-(4-Benzyl-6-chlor-2-oxo-1H-chinolin-3-yl)-3-[4-(4-chlorphenyl)phenyl]-3,4-dihydropyrazol-2-yl]-4-oxobutansäure |
| d.85 | 3-[2-Acetyl-3-[4-(4-chlorphenyl)phenyl]-3,4-dihydropyrazol-5-yl]-4-benzyl-6-chlor-1H-chinolin-2-on |
| d.86 | 4-[5-(4-Benzyl-6-chlor-2-oxo-1H-chinolin-3-yl)-3-[4-(4-bromphenyl)phenyl]-3,4-dihydropyrazol-2-yl]-4-oxobutansäure |
| d.87 | 3-[2-Acetyl-3-[4-(4-bromphenyl)phenyl]-3,4-dihydropyrazol-5-yl]-4-benzyl-6-chlor-1H-chinolin-2-on |
| d.88 | 4-[5-(4-Benzyl-6-chlor-2-oxo-1H-chinolin-3-yl)-3-[4-(4-methoxyphenyl)phenyl]-3,4-dihydropyrazol-2-yl]-4-oxobutansäure |
| d.89 | 3-[2-Acetyl-3-[4-(4-methoxyphenyl)phenyl]-3,4-dihydropyrazol-5-yl]-4-benzyl-6-chlor-1H-chinolin-2-on |
| d.90 | 4-[5-(4-Benzyl-6-chlor-2-oxo-1H-chinolin-3-yl)-3-[4-(1-propylpyrazol-4-yl)phenyl]-3,4-dihydropyrazol-2-yl]-4-oxobutansäure |
| d.91 | 3-[2-Acetyl-3-[4-(1-propylpyrazol-4-yl)phenyl]-3,4-dihydropyrazol-5-yl]-4-benzyl-6-chlor-1H-chinolin-2-on |
| d.92 | 4-[5-(4-Benzyl-6-chlor-2-oxo-1H-chinolin-3-yl)-3-[4-(1-ethylindazol-5-yl)phenyl]-3,4-dihydropyrazol-2-yl]-4-oxobutansäure |
| d. 93 | 4-[5-(4-Benzyl-6-chlor-2-oxo-1H-chinolin-3-yl)-3-[4-(2-ethylindazol-5-yl)phenyl]-3,4-dihydropyrazol-2-yl]-4-oxobutansäure |
| d. 94 | 3-(2-Acetyl-3-phenyl-3,4-dihydropyrazol-5-yl)-4-benzyl-6-chlor-1H-chinolin-2-on |
| d.95 | 4-Benzyl-6-chlor-3-(3-phenyl-2-propanoyl-3,4-dihydropyrazol-5-yl)-1H-chinolin-2-on |
| d.96 | 3-[2-Acetyl-3-(p-tolyl)-3,4-dihydropyrazol-5-yl]-4-benzyl-6-chlor-1H-chinolin-2-on |
| d.101 | 4-[5-(4-Benzyl-6-chlor-2-oxo-1H-chinolin-3-yl)-3-(3-pyridyl)-3,4-dihydropyrazol-2-yl]-4-oxobutansäure |
| d.105 | 4-[5-(4-Benzyl-6-chlor-2-oxo-1H-chinolin-3-yl)-3-(2-chlorphenyl)-3,4-dihydropyrazol-2-yl]-4-oxobutansäure |
| d.106 | 4-[5-(4-Benzyl-6-chlor-2-oxo-1H-chinolin-3-yl)-3-(3,4-dimethoxyphenyl)-3,4-dihydropyrazol-2-yl]-4-oxobutansäure |
| d.109 | 4-[5-(6-Chlor-4-morpholino-2-oxo-1H-chinolin-3-yl)-3-(4-methoxyphenyl)-3,4-dihydropyrazol-2-yl]-4-oxobutansäure |

3. Pharmazeutische Zusammensetzung, umfassend eine Verbindung nach einem der Ansprüche 1 oder 2 und mindestens einen pharmazeutisch verträglichen Träger, Hilfsstoff davon oder mindestens ein pharmazeutisch verträgliches Stabilisierungsmittel, Verdünnungsmittel davon.

4. Verbindung nach einem der Ansprüche 1 oder 2 zur Verwendung als Arzneimittel.

5. Verbindung nach einem der Ansprüche 1 oder 2 zur Verwendung bei der Behandlung von Krankheiten oder Störungen, ausgewählt aus der Gruppe bestehend aus primären und metastasierten neoplastischen Erkrankungen, Hyperplasie, Metaplasie, Dysplasie, Krebs, Krebsmetastasen, gutartigen Tumoren und hyperproliferativen Störungen.

6. Verbindung zur Verwendung nach Anspruch 5, wobei die Krankheiten oder Störungen aus der Gruppe, die aus Eierstock-, Brust- und Bauchspeicheldrüsenkrebs besteht, ausgewählt sind.

7. Verbindung zur Verwendung nach einem der Ansprüche 5 oder 6, ausgewählt aus der Gruppe bestehend aus:
| | |
|---|---|
| d.50 | 4-[5-(4-Benzyl-6-chlor-2-oxo-1H-chinolin-3-yl)-3-(4-fluorphenyl)-3,4-dihydropyrazol-2-yl]-4-oxobutansäure |
| d.51 | 3-[2-Acetyl-3-(4-fluorphenyl)-3,4-dihydropyrazol-5-yl]-4-benzyl-6-chlor-1H-chinolin-2-on |
| d.52 | 3-[2-Acetyl-3-(4-methoxyphenyl)-3,4-dihydropyrazol-5-yl]-4-benzyl-6-chlor-1H-chinolin-2-on |
| d.53 | 4-Benzyl-6-chlor-3-[3-(4-methoxyphenyl)-2-propanoyl-3,4-dihydropyrazol-5-yl]-1H-chinolin-2-on |
| d.54 | 4-Benzyl-6-chlor-3-[3-(4-fluorphenyl)-2-propanoyl-3,4-dihydropyrazol-5-yl]-1H-chinolin-2-on |
| d.55 | 4-[5-(4-benzyl-6-chlor-2-oxo-1H-chinolin-3-yl)-3-(4-Chlorphenyl)-3,4-dihydropyrazol-2-yl]-4-oxobutansäure |
| d.56 | 3-[2-Acetyl-3-(4-chlorphenyl)-3,4-dihydropyrazol-5-yl]-4-benzyl-6-chlor-1H-chinolin-2-on |
| d.57 | 4-Benzyl-6-chlor-3-[3-(4-chlorphenyl)-2-propanoyl-3,4-dihydropyrazol-5-yl]-1H-chinolin-2-on |
| d.58 | 4-[5-(4-benzyl-6-chlor-2-oxo-1H-chinolin-3-yl)-3-(4-bromphenyl)-3,4-dihydropyrazol-2-yl]-4-oxobutansäure |
| d.59 | 3-[2-Acetyl-3-(4-bromphenyl)-3,4-dihydropyrazol-5-yl]-4-benzyl-6-chlor-1H-chinolin-2-on |
| d.60 | 4-Benzyl-3-[3-(4-bromophenyl)-2-propanoyl-3,4-dihydropyrazol-5-yl]-6-chlor-1H-chilonin-2-on |
| d.61 | 4-[5-(4-Benzyl-6-chlor-2-oxo-1H-chinolin-3-yl)-3-(4-methoxyphenyl)-3,4-dihydropyrazol-2-yl]-4-oxobutansäure |
| d.62 | 4-[5-(4-Benzyl-6-chlor-2-oxo-1H-chinolin-3-yl)-3-[4-(trifluormethyl)phenyl]-3,4-dihydropyrazol-2-yl]-4-oxobutansäure |
| d.63 | 3-[2-Acetyl-3-[4-(trifluormethyl)phenyl]-3,4-dihydropyrazol-5-yl]-4-benzyl-6-chlor-1H-chinolin-2-on |
| d.64 | 4-[5-(4-Benzyl-6-chlor-2-oxo-1H-chinolin-3-yl)-3-(4-tert-butylphenyl)-3,4-dihydropyrazol-2-yl]-4-oxobutansäure |
| d.65 | 3-[2-Acetyl-3-(4-tert-butylphenyl)-3,4-dihydropyrazol-5-yl]-4-benzyl-6-chlor-1H-chinolin-2-on |
| d.66 | 4-[5-(4-Benzyl-6-chlor-2-oxo-1H-chinolin-3-yl)-3-(3-fluor-4-methoxyphenyl)-3,4-dihydropyrazol-2-yl]-4-oxobutansäure |
| d.67 | 4-[5-(4-Benzyl-6-chlor-2-oxo-1H-chinolin-3-yl)-3-(2-furyl)-3,4-dihydropyrazol-2-yl]-4-oxobutansäure |
| d.68 | 3-[2-Acetyl-3-(2-furyl)-3,4-dihydropyrazol-5-yl]-4-benzyl-6-chlor-1H-chinolin-2-on |
| d.69 | 4-[5-(4-Benzyl-6-chlor-2-oxo-1H-chinolin-3-yl)-3-(1-methylindol-5-yl)-3,4-dihydropyrazol-2-yl]-4-oxobutansäure |
| d.70 | 3-[2-Acetyl-3-(1-methylindol-5-yl)-3,4-dihydropyrazol-5-yl]-4-benzyl-6-chlor-1H-chinolin-2-on |
| d.71 | 4-[5-(4-Benzyl-6-chlor-2-oxo-1H-chinolin-3-yl)-3-(1-ethylindol-5-yl)-3,4-dihydropyrazol-2-yl]-4-oxobutansäure |
| d.72 | 3-[2-Acetyl-3-(1-ethylindol-5-yl)-3,4-dihydropyrazol-5-yl]-4-benzyl-6-chlor-1H-chinolin-2-on |
| d.73 | 4-[5-(4-Benzyl-6-chlor-2-oxo-1H-chinolin-3-yl)-3-(1-methylindazol-5-yl)-3,4-dihydropyrazol-2-yl]-4-oxobutansäure |
| d.74 | 3-[2-Acetyl-3-(1-methylindazol-5-yl)-3,4-dihydropyrazol-5-yl]-4-benzyl-6-chlor-1H-chinolin-2-on |
| d.75 | 4-[5-(4-Benzyl-6-chlor-2-oxo-1H-chinolin-3-yl)-3-(2-methylindazol-5-yl)-3,4-dihydropyrazol-2-yl]-4-oxobutansäure |
| d.76 | 4-[5-(4-Benzyl-6-chlor-2-oxo-1H-chinolin-3-yl)-3-(2-ethylindazol-5-yl)-3,4-dihydropyrazol-2-yl]-4-oxobutansäure |
| d.77 | 3-[2-Acetyl-3-(2-ethylindazol-5-yl)-3,4-dihydropyrazol-5-yl]-4-benzyl-6-chlor-1H-chinolin-2-on |
| d.78 | 4-[5-(4-Benzyl-6-chlor-2-oxo-1H-chinolin-3-yl)-3-(1-ethylindazol-5-yl)-3,4-dihydropyrazol-2-yl]-4-oxobutansäure |
| d.79 | 3-[2-Acetyl-3-(1-ethylindazol-5-yl)-3,4-dihydropyrazol-5-yl]-4-benzyl-6-chlor-1H-chinolin-2-on |
| d.80 | 4-[5-(4-Benzyl-6-chlor-2-oxo-1H-chinolin-3-yl)-3-(1-propylindazol-5-yl)-3,4-dihydropyrazol-2-yl]-4-oxobutansäure |
| d.81 | 3-[2-Acetyl-3-(1-propylindazol-5-yl)-3,4-dihydropyrazol-5-yl]-4-benzyl-6-chlor-1H-chinolin-2-on |
| d.82 | 4-[5-(4-Benzyl-6-chlor-2-oxo-1H-chinolin-3-yl)-3-(2-propylindazol-5-yl)-3,4-dihydropyrazol-2-yl]-4-oxobutansäure |
| d.83 | 4-[5-(4-Benzyl-6-chlor-2-oxo-1H-chinolin-3-yl)-3-[4-(4-Fluorphenyl)phenyl]-3,4-dihydropyrazol-2-yl]-4-oxobutansäure |
| d.84 | 4-[5-(4-Benzyl-6-chlor-2-oxo-1H-chinolin-3-yl)-3-[4-(4-chlorphenyl)phenyl]-3,4-dihydropyrazol-2-yl]-4-oxobutansäure |
| d.85 | 3-[2-Acetyl-3-[4-(4-chlorphenyl)phenyl]-3,4-dihydropyrazol-5-yl]-4-benzyl-6-chlor-1H-chinolin-2-on |
| d.86 | 4-[5-(4-Benzyl-6-chlor-2-oxo-1H-chinolin-3-yl)-3-[4-(4-bromphenyl)phenyl]-3,4-dihydropyrazol-2-yl]-4-oxobutansäure |
| d.87 | 3-[2-Acetyl-3-[4-(4-bromphenyl)phenyl]-3,4-dihydropyrazol-5-yl]-4-benzyl-6-chlor-1H-chinolin-2-on |
| d.88 | 4-[5-(4-Benzyl-6-chlor-2-oxo-1H-chinolin-3-yl)-3-[4-(4-methoxyphenyl)phenyl]-3,4-dihydropyrazol-2-yl]-4-oxobutansäure |
| d.89 | 3-[2-Acetyl-3-[4-(4-methoxyphenyl)phenyl]-3,4-dihydropyrazol-5-yl]-4-benzyl-6-chlor-1H-chinolin-2-on |
| d.90 | 4-[5-(4-Benzyl-6-chlor-2-oxo-1H-chinolin-3-yl)-3-[4-(1-propylpyrazol-4-yl)phenyl]-3,4-dihydropyrazol-2-yl]-4-oxobutansäure |
| d.91 | 3-[2-Acetyl-3-[4-(1-propylpyrazol-4-yl)phenyl]-3,4-dihydropyrazol-5-yl]-4-benzyl-6-chlor-1H-chinolin-2-on |
| d.92 | 4-[5-(4-Benzyl-6-chlor-2-oxo-1H-chinolin-3-yl)-3-[4-(1-ethylindazol-5-yl)phenyl]-3,4-dihydropyrazol-2-yl]-4-oxobutansäure |
| d.93 | 4-[5-(4-Benzyl-6-chlor-2-oxo-1H-chinolin-3-yl)-3-[4-(2-ethylindazol-5-yl)phenyl]-3,4-dihydropyrazol-2-yl]-4-oxobutansäure |
| d.95 | 4-Benzyl-6-chlor-3-(3-phenyl-2-propanoyl-3,4-dihydropyrazol-5-yl)-1H-chinolin-2-on |
| d.96 | 3-[2-Acetyl-3-(p-tolyl)-3,4-dihydropyrazol-5-yl]-4-benzyl-6-chlor-1H-chinolin-2-on |
| d.98 | 4-[5-(6-Chlor-2-oxo-4-phenyl-1H-chinolin-3-yl)-3-(3-pyridyl)-3,4-dihydropyrazol-2-yl]-4-oxobutansäure |
| d.99 | 4-[5-(6-Chlor-2-oxo-4-phenyl-1H-chinolin-3-yl)-3-(2-pyridyl)-3,4-dihydropyrazol-2-yl]-4-oxobutansäure |
| | |
| d.101 | 4-[5-(4-Benzyl-6-chlor-2-oxo-1H-chinolin-3-yl)-3-(3-pyridyl)-3,4-dihydropyrazol-2-yl]-4-oxobutansäure |
| d.105 | 4-[5-(4-Benzyl-6-chlor-2-oxo-1H-chinolin-3-yl)-3-(2-chlorphenyl)-3,4-dihydropyrazol-2-yl]-4-oxobutansäure |
| d.106 | 4-[5-(4-Benzyl-6-chlor-2-oxo-1H-chinolin-3-yl)-3-(3,4-dimethoxyphenyl)-3,4-dihydropyrazol-2-yl]-4-oxobutansäure |
| d.109 | 4-[5-(6-Chlor-4-morpholino-2-oxo-1H-chinolin-3-yl)-3-(4-methoxyphenyl)-3,4-dihydropyrazol-2-yl]-4-oxobutansäure |

## Revendications

1. Composé de formule (Ia) : 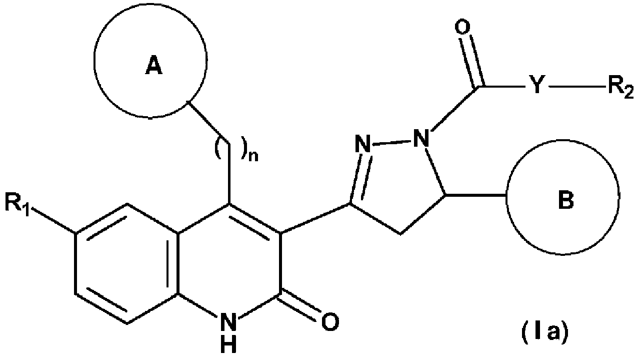 où
**R₁** est un halogène ;
**R₂** est choisi dans le groupe constitué par l'hydrogène et COOH ;
**n** vaut 1 ;
**Y** est un alkyle en C₁₋₄ ;
**A** est un phényle ;
**B** est 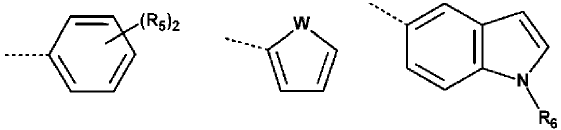 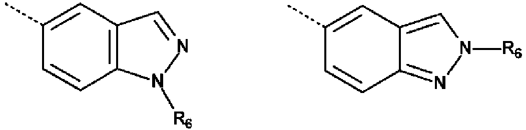 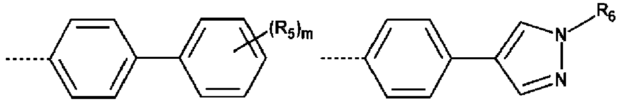 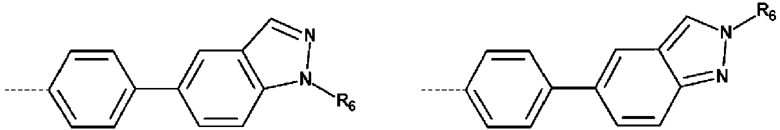
**R₅** est un hydrogène, un halogène, un alkyle en C₁₋₄, un haloalkyle en C₁₋₄, un O-alkyle en C₁₋₄ linéaires ou ramifiés ;
**R₆** est choisi dans le groupe constitué par H et un alkyle en C₁₋₄ ;
m est 1 ou 2 ; et
W est choisi dans le groupe constitué par O et NH ;
ou des composés de formule (Ia) : 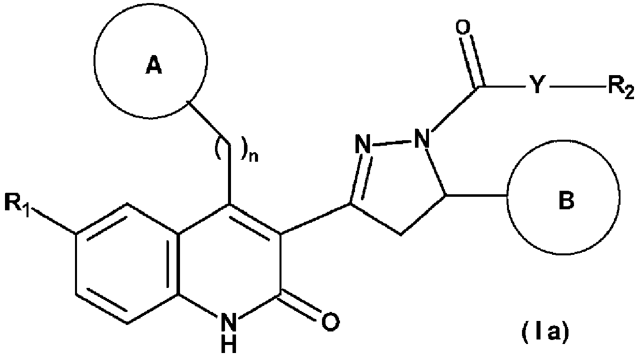
où
**R₁** est un halogène ;
**R₂** est choisi dans le groupe constitué par l'hydrogène et COOH ;
**n** vaut 0 ;
**Y** est choisi dans le groupe constitué par un alkyle en C₁₋₄ ;
**A** est de la morpholine ;
**B** est 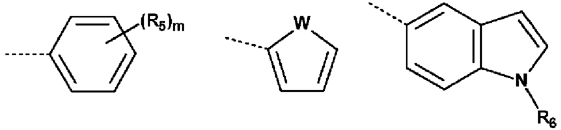 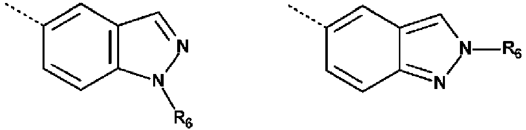 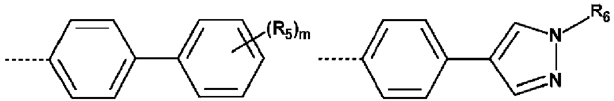 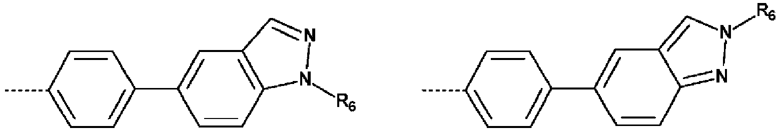
**R₅** est un hydrogène, un halogène, un alkyle en C₁₋₄, un haloalkyle en C₁₋₄, un O-alkyle en C₁₋₄ linéaires ou ramifiés ;
**R₆** est choisi dans le groupe constitué par H et un alkyle en C₁₋₄ ;
**m** est 1 ou 2 ; et
**W** est choisi dans le groupe constitué par O et NH,
ou leurs sels ou solvates pharmaceutiquement acceptables.

2. Composé selon la revendication 1, choisi dans le groupe constitué par :
| | |
|---|---|
| d.50 | Acide 4-[5-(4-benzyl-6-chloro-2-oxo-1H-quinoléine-3-yl)-3-(4-fluorophényle)-3,4-dihydropyrazol-2-yl]-4-oxo-butanoïque |
| d.51 | 3-[2-acétyl-3-(4-fluorophényle)-3,4-dihydropyrazol-5-yl]-4-benzyl-6-chloro-1H-quinoléine-2-one |
| d.52 | 3-[2-acétyl-3-(4-méthoxyphényle)-3,4-dihydropyrazol-5-yl]-4-benzyl-6-chloro-1H-quinoléine-2-one |
| d.53 | 4-benzyl-6-chloro-3-[3-(4-méthoxyphényle)-2-propanoyl-3,4-dihydropyrazol-5-yl]-1H-quinoléine-2-one |
| d.54 | 4-benzyl-6-chloro-3-[3-(4-fluorophényle)-2-propanoyl-3,4-dihydropyrazol-5-yl]-1H-quinoléine-2-one |
| d.55 | Acide 4-[5-(4-benzyl-6-chloro-2-oxo-1H-quinoléine-3-yl)-3-(4-chlorophényle)-3,4-dihydropyrazol-2-yl]-4-oxo-butanoïque |
| d.56 | 3-[2-acétyl-3-(4-chlorophényle)-3,4-dihydropyrazol-5-yl]-4-benzyl-6-chloro-1H-quinoléine-2-one |
| d.57 | 4-benzyl-6-chloro-3-[3-(4-chlorophényle)-2-propanoyl-3,4-dihydropyrazol-5-yl]-1H-quinoléine-2-one |
| d.58 | Acide 4-[5-(4-benzyl-6-chloro-2-oxo-1H-quinoléine-3-yl)-3-(4-bromophényle)-3,4-dihydropyrazol-2-yl]-4-oxo-butanoïque |
| d.59 | 3-[2-acétyl-3-(4-bromophényle)-3,4-dihydropyrazol-5-yl]-4-benzyl-6-chloro-1H-quinoléine-2-one |
| d.60 | 4-benzyl-3-[3-(4-bromophényle)-2-propanoyl-3,4-dihydropyrazol-5-yl]-6-chloro-1H-quinoléine-2-one |
| d.61 | Acide 4-[5-(4-benzyl-6-chloro-2-oxo-1H-quinoléine-3-yl)-3-(4-méthoxyphényle)-3,4-dihydropyrazol-2-yl]-4-oxo-butanoïque |
| d.62 | Acide 4-[5-(4-benzyl-6-chloro-2-oxo-1H-quinoléine-3-yl)-3-[4-(trifluorométhyl)phényle]-3,4-dihydropyrazol-2-yl]-4-oxo-butanoïque |
| d.63 | 3-[2-acétyl-3-[4-(trifluorométhyl)phényle]-3,4-dihydropyrazol-5-yl]-4-benzyl-6-chloro-1H-quinoléine-2-one |
| d.64 | Acide 4-[5-(4-benzyl-6-chloro-2-oxo-1H-quinoléine-3-yl)-3-(4-tert-butylphényle)-3,4-dihydropyrazol-2-yl]-4-oxo-butanoïque |
| d.65 | 3-[2-acétyl-3-(4-tert-butylphényle)-3,4-dihydropyrazol-5-yl]-4-benzyl-6-chloro-1H-quinoléine-2-one |
| d.66 | 4-[5-(4-benzyl-6-chloro-2-oxo-1H-quinoléine-3-yl)-3-(3-fluoro-4-méthoxy-phényle)-3,4-dihydropyrazol-2-yl]-4-oxo-butanoïque |
| d.67 | Acide 4-[5-(4-benzyl-6-chloro-2-oxo-1H-quinoléine-3-yl)-3-(2-furyl)-3,4-dihydropyrazol-2-yl]-4-oxo-butanoïque |
| d.68 | 3-[2-acétyl-3-(2-furyl)-3,4-dihydropyrazol-5-yl]-4-benzyl-6-chloro-1H-quinoléine-2-one |
| d.69 | Acide 4-[5-(4-benzyl-6-chloro-2-oxo-1H-quinoléine-3-yl)-3-(1-méthylindol-5-yl)-3,4-dihydropyrazol-2-yl]-4-oxo-butanoïque |
| d.70 | 3-[2-acétyl-3-(1-méthylindol-5-yl)-3,4-dihydropyrazol-5-yl]-4-benzyl-6-chloro-1H-quinoléine-2-one |
| d.71 | Acide 4-[5-(4-benzyl-6-chloro-2-oxo-1H-quinoléine-3-yl)-3-(1-éthylindol-5-yl)-3,4-dihydropyrazol-2-yl]-4-oxo-butanoïque |
| d.72 | 3-[2-acétyl-3-(1-éthylindol-5-yl)-3,4-dihydropyrazol-5-yl]-4-benzyl-6-chloro-1H-quinoléine-2-one |
| d.73 | Acide 4-[5-(4-benzyl-6-chloro-2-oxo-1H-quinoléine-3-yl)-3-(1-méthylindazol-5-yl)-3,4-dihydropyrazol-2-yl]-4-oxo-butanoïque |
| d.74 | 3-[2-acétyl-3-(1-méthylindazol-5-yl)-3,4-dihydropyrazol-5-yl]-4-benzyl-6-chloro-1H-quinoléine-2-one |
| d.75 | Acide 4-[5-(4-benzyl-6-chloro-2-oxo-1H-quinoléine-3-yl)-3-(2-méthylindazol-5-yl)-3,4-dihydropyrazol-2-yl]-4-oxo-butanoïque |
| d.76 | Acide 4-[5-(4-benzyl-6-chloro-2-oxo-1H-quinoléine-3-yl)-3-(2-éthylindazol-5-yl)-3,4-dihydropyrazol-2-yl]-4-oxo-butanoïque |
| d.77 | 3-[2-acétyl-3-(2-éthylindazol-5-yl)-3,4-dihydropyrazol-5-yl]-4-benzyl-6-chloro-1H-quinoléine-2-one |
| d.78 | Acide 4-[5-(4-benzyl-6-chloro-2-oxo-1H-quinoléine-3-yl)-3-(1-éthylindazol-5-yl)-3,4-dihydropyrazol-2-yl]-4-oxo-butanoïque |
| d.79 | 3-[2-acétyl-3-(1-éthylindazol-5-yl)-3,4-dihydropyrazol-5-yl]-4-benzyl-6-chloro-1H-quinoléine-2-one |
| d.80 | Acide 4-[5-(4-benzyl-6-chloro-2-oxo-1H-quinoléine-3-yl)-3-(1-propylindazol-5-yl)-3,4-dihydropyrazol-2-yl]-4-oxo-butanoïque |
| d.81 | 3-[2-acétyl-3-(1-propylindazol-5-yl)-3,4-dihydropyrazol-5-yl]-4-benzyl-6-chloro-1H-quinoléine-2-one |
| d.82 | Acide 4-[5-(4-benzyl-6-chloro-2-oxo-1H-quinoléine-3-yl)-3-(2-propylindazol-5-yl)-3,4-dihydropyrazol-2-yl]-4-oxo-butanoïque |
| d.83 | 4-[5-(4-benzyl-6-chloro-2-oxo-1H-quinoléine-3-yl)-3-[4-(4-fluorophényle)phényle]-3,4-dihydropyrazol-2-yl]-4-oxo-butanoïque |
| d.84 | 4-[5-(4-benzyl-6-chloro-2-oxo-1H-quinoléine-3-yl)-3-[4-(4-chlorophényle)phényle]-3,4-dihydropyrazol-2-yl]-4-oxo-butanoïque |
| d.85 | 3-[2-acétyl-3-[4-(4-chlorophényle)phényle]-3,4-dihydropyrazol-5-yl]-4-benzyl-6-chloro-1H-quinoléine-2-one |
| d.86 | 4-[5-(4-benzyl-6-chloro-2-oxo-1H-quinoléine-3-yl)-3-[4-(4-bromophényle)phényle]-3,4-dihydropyrazol-2-yl]-4-oxo-butanoïque |
| d.87 | 3-[2-acétyl-3-[4-(4-bromophényle)phényle]-3,4-dihydropyrazol-5-yl]-4-benzyl-6-chloro-1H-quinoléine-2-one |
| d.88 | 4-[5-(4-benzyl-6-chloro-2-oxo-1H-quinoléine-3-yl)-3-[4-(4-méthoxyphényle)phényle]-3,4-dihydropyrazol-2-yl]-4-oxo-butanoïque |
| d.89 | 3-[2-acétyl-3-[4-(4-méthoxyphényle)phényle]-3,4-dihydropyrazol-5-yl]-4-benzyl-6-chloro-1H-quinoléine-2-one |
| d.90 | 4-[5-(4-benzyl-6-chloro-2-oxo-1H-quinoléine-3-yl)-3-[4-(1-propylpyrazol-4-yl)phényle]-3,4-dihydropyrazol-2-yl]-4-oxo-butanoïque |
| d.91 | 3-[2-acétyl-3-[4-(1-propylpyrazol-4-yl)phényle]-3,4-dihydropyrazol-5-yl]-4-benzyl-6-chloro-1H-quinoléine-2-one |
| d.92 | 4-[5-(4-benzyl-6-chloro-2-oxo-1H-quinoléine-3-yl)-3-[4-(1-éthylindazol-5-yl)phényle]-3,4-dihydropyrazol-2-yl]-4-oxo-butanoïque |
| d.93 | 4-[5-(4-benzyl-6-chloro-2-oxo-1H-quinoléine-3-yl)-3-[4-(2-éthylindazol-5-yl)phényle]-3,4-dihydropyrazol-2-yl]-4-oxo-butanoïque |
| d.94 | 3-(2-acétyl-3-phényle-3,4-dihydropyrazol-5-yl)-4-benzyl-6-chloro-1H-quinoléine-2-one |
| d.95 | 4-benzyl-6-chloro-3-(3-phényle-2-propanoyl-3,4-dihydropyrazol-5-yl)-1H-quinoléine-2-one |
| d.96 | 3-[2-acétyl-3-(p-tolyl)-3,4-dihydropyrazol-5-yl]-4-benzyl-6-chloro-1H-quinoléine-2-one |
| d.101 | Acide 4-[5-(4-benzyl-6-chloro-2-oxo-1H-quinoléine-3-yl)-3-(3-pyridyl)-3,4-dihydropyrazol-2-yl]-4-oxo-butanoïque |
| d.105 | Acide 4-[5-(4-benzyl-6-chloro-2-oxo-1H-quinoléine-3-yl)-3-(2-chlorophényle)-3,4-dihydropyrazol-2-yl]-4-oxo-butanoïque |
| d.106 | Acide 4-[5-(4-benzyl-6-chloro-2-oxo-1H-quinoléine-3-yl)-3-(3,4-diméthoxyphényle)-3,4-dihydropyrazol-2-yl]-4-oxo-butanoïque |
| d.109 | Acide 4-[5-(6-chloro-4-morpholino-2-oxo-1H-quinoléine-3-yl)-3-(4-méthoxyphényle)-3,4-dihydropyrazol-2-yl]-4-oxo-butanoïque |

3. Composition pharmaceutique comprenant un composé selon l'une quelconque des revendications 1 ou 2 et au moins un vecteur, stabilisant, diluant ou excipient pharmaceutiquement acceptable de celui-ci.

4. Composé selon l'une quelconque des revendications 1 ou 2, destiné à être utilisé comme médicament.

5. Composé selon l'une quelconque des revendications 1 ou 2, destiné à être utilisé dans le traitement de maladies ou de troubles choisis dans le groupe constitué par les maladies néoplasiques primaires et métastatiques, l'hyperplasie, la métaplasie, la dysplasie, le cancer, les métastases cancéreuses, les tumeurs bénignes et les troubles hyperprolifératifs.

6. Composé destiné à être utilisé selon la revendication 5, dans lequel les maladies ou troubles sont choisis dans le groupe constitué par le cancer de l'ovaire, le cancer du sein et le cancer du pancréas.

7. Composé destiné à être utilisé selon l'une quelconque des revendications 5 ou 6, choisi dans le groupe constitué par :
| | |
|---|---|
| d.50 | Acide 4-[5-(4-benzyl-6-chloro-2-oxo-1H-quinoléine-3-yl)-3-(4-fluorophényle)-3,4-dihydropyrazol-2-yl]-4-oxo-butanoïque |
| d.51 | 3-[2-acétyl-3-(4-fluorophényle)-3,4-dihydropyrazol-5-yl]-4-benzyl-6-chloro-1H-quinoléine-2-one |
| d.52 | 3-[2-acétyl-3-(4-méthoxyphényle)-3,4-dihydropyrazol-5-yl]-4-benzyl-6-chloro-1H-quinoléine-2-one |
| d.53 | 4-benzyl-6-chloro-3-[3-(4-méthoxyphényle)-2-propanoyl-3,4-dihydropyrazol-5-yl]-1H-quinoléine-2-one |
| d.54 | 4-benzyl-6-chloro-3-[3-(4-fluorophényle)-2-propanoyl-3,4-dihydropyrazol-5-yl]-1H-quinoléine-2-one |
| d.55 | Acide 4-[5-(4-benzyl-6-chloro-2-oxo-1H-quinoléine-3-yl)-3-(4-chlorophényle)-3,4-dihydropyrazol-2-yl]-4-oxo-butanoïque |
| d.56 | 3-[2-acétyl-3-(4-chlorophényle)-3,4-dihydropyrazol-5-yl]-4-benzyl-6-chloro-1H-quinoléine-2-one |
| d.57 | 4-benzyl-6-chloro-3-[3-(4-chlorophényle)-2-propanoyl-3,4-dihydropyrazol-5-yl]-1H-quinoléine-2-one |
| d.58 | Acide 4-[5-(4-benzyl-6-chloro-2-oxo-1H-quinoléine-3-yl)-3-(4-bromophényle)-3,4-dihydropyrazol-2-yl]-4-oxo-butanoïque |
| d.59 | 3-[2-acétyl-3-(4-bromophényle)-3,4-dihydropyrazol-5-yl]-4-benzyl-6-chloro-1H-quinoléine-2-one |
| d.60 | 4-benzyl-3-[3-(4-bromophényle)-2-propanoyl-3,4-dihydropyrazol-5-yl]-6-chloro-1H-quinoléine-2-one |
| d.61 | Acide 4-[5-(4-benzyl-6-chloro-2-oxo-1H-quinoléine-3-yl)-3-(4-méthoxyphényle)-3,4-dihydropyrazol-2-yl]-4-oxo-butanoïque |
| d.62 | Acide 4-[5-(4-benzyl-6-chloro-2-oxo-1H-quinoléine-3-yl)-3-[4-(trifluorométhyl)phényle]-3,4-dihydropyrazol-2-yl]-4-oxo-butanoïque |
| d.63 | 3-[2-acétyl-3-[4-(trifluorométhyl)phényle]-3,4-dihydropyrazol-5-yl]-4-benzyl-6-chloro-1H-quinoléine-2-one |
| d.64 | Acide 4-[5-(4-benzyl-6-chloro-2-oxo-1H-quinoléine-3-yl)-3-(4-tert-butylphényle)-3,4-dihydropyrazol-2-yl]-4-oxo-butanoïque |
| d.65 | 3-[2-acétyl-3-(4-tert-butylphényle)-3,4-dihydropyrazol-5-yl]-4-benzyl-6-chloro-1H-quinoléine-2-one |
| d.66 | 4-[5-(4-benzyl-6-chloro-2-oxo-1H-quinoléine-3-yl)-3-(3-fluoro-4-méthoxy-phényle)-3,4-dihydropyrazol-2-yl]-4-oxo-butanoïque |
| d.67 | Acide 4-[5-(4-benzyl-6-chloro-2-oxo-1H-quinoléine-3-yl)-3-(2-furyl)-3,4-dihydropyrazol-2-yl]-4-oxo-butanoïque |
| d.68 | 3-[2-acétyl-3-(2-furyl)-3,4-dihydropyrazol-5-yl]-4-benzyl-6-chloro-1H-quinoléine-2-one |
| d.69 | Acide 4-[5-(4-benzyl-6-chloro-2-oxo-1H-quinoléine-3-yl)-3-(1-méthylindol-5-yl)-3,4-dihydropyrazol-2-yl]-4-oxo-butanoïque |
| d.70 | 3-[2-acétyl-3-(1-méthylindol-5-yl)-3,4-dihydropyrazol-5-yl]-4-benzyl-6-chloro-1H-quinoléine-2-one |
| d.71 | Acide 4-[5-(4-benzyl-6-chloro-2-oxo-1H-quinoléine-3-yl)-3-(1-éthylindol-5-yl)-3,4-dihydropyrazol-2-yl]-4-oxo-butanoïque |
| d.72 | 3-[2-acétyl-3-(1-éthylindol-5-yl)-3,4-dihydropyrazol-5-yl]-4-benzyl-6-chloro-1H-quinoléine-2-one |
| d.73 | Acide 4-[5-(4-benzyl-6-chloro-2-oxo-1H-quinoléine-3-yl)-3-(1-méthylindazol-5-yl)-3,4-dihydropyrazol-2-yl]-4-oxo-butanoïque |
| d.74 | 3-[2-acétyl-3-(1-méthylindazol-5-yl)-3,4-dihydropyrazol-5-yl]-4-benzyl-6-chloro-1H-quinoléine-2-one |
| d.75 | Acide 4-[5-(4-benzyl-6-chloro-2-oxo-1H-quinoléine-3-yl)-3-(2-méthylindazol-5-yl)-3,4-dihydropyrazol-2-yl]-4-oxo-butanoïque |
| d.76 | Acide 4-[5-(4-benzyl-6-chloro-2-oxo-1H-quinoléine-3-yl)-3-(2-éthylindazol-5-yl)-3,4-dihydropyrazol-2-yl]-4-oxo-butanoïque |
| d.77 | 3-[2-acétyl-3-(2-éthylindazol-5-yl)-3,4-dihydropyrazol-5-yl]-4-benzyl-6-chloro-1H-quinoléine-2-one |
| d.78 | Acide 4-[5-(4-benzyl-6-chloro-2-oxo-1H-quinoléine-3-yl)-3-(1-éthylindazol-5-yl)-3,4-dihydropyrazol-2-yl]-4-oxo-butanoïque |
| d.79 | 3-[2-acétyl-3-(1-éthylindazol-5-yl)-3,4-dihydropyrazol-5-yl]-4-benzyl-6-chloro-1H-quinoléine-2-one |
| d.80 | Acide 4-[5-(4-benzyl-6-chloro-2-oxo-1H-quinoléine-3-yl)-3-(1-propylindazol-5-yl)-3,4-dihydropyrazol-2-yl]-4-oxo-butanoïque |
| d.81 | 3-[2-acétyl-3-(1-propylindazol-5-yl)-3,4-dihydropyrazol-5-yl]-4-benzyl-6-chloro-1H-quinoléine-2-one |
| d.82 | Acide 4-[5-(4-benzyl-6-chloro-2-oxo-1H-quinoléine-3-yl)-3-(2-propylindazol-5-yl)-3,4-dihydropyrazol-2-yl]-4-oxo-butanoïque |
| d.83 | 4-[5-(4-benzyl-6-chloro-2-oxo-1H-quinoléine-3-yl)-3-[4-(4-fluorophényle)phényle]-3,4-dihydropyrazol-2-yl]-4-oxo-butanoïque |
| d.84 | 4-[5-(4-benzyl-6-chloro-2-oxo-1H-quinoléine-3-yl)-3-[4-(4-chlorophényle)phényle]-3,4-dihydropyrazol-2-yl]-4-oxo-butanoïque |
| d.85 | 3-[2-acétyl-3-[4-(4-chlorophényle)phényle]-3,4-dihydropyrazol-5-yl]-4-benzyl-6-chloro-1H-quinoléine-2-one |
| d.86 | 4-[5-(4-benzyl-6-chloro-2-oxo-1H-quinoléine-3-yl)-3-[4-(4-bromophényle)phényle]-3,4-dihydropyrazol-2-yl]-4-oxo-butanoïque |
| d.87 | 3-[2-acétyl-3-[4-(4-bromophényle)phényle]-3,4-dihydropyrazol-5-yl]-4-benzyl-6-chloro-1H-quinoléine-2-one |
| d.88 | 4-[5-(4-benzyl-6-chloro-2-oxo-1H-quinoléine-3-yl)-3-[4-(4-méthoxyphényle)phényle]-3,4-dihydropyrazol-2-yl]-4-oxo-butanoïque |
| d.89 | 3-[2-acétyl-3-[4-(4-méthoxyphényle)phényle]-3,4-dihydropyrazol-5-yl]-4-benzyl-6-chloro-1H-quinoléine-2-one |
| d.90 | 4-[5-(4-benzyl-6-chloro-2-oxo-1H-quinoléine-3-yl)-3-[4-(1-propylpyrazol-4-yl)phényle]-3,4-dihydropyrazol-2-yl]-4-oxo-butanoïque |
| d.91 | 3-[2-acétyl-3-[4-(1-propylpyrazol-4-yl)phényle]-3,4-dihydropyrazol-5-yl]-4-benzyl-6-chloro-1H-quinoléine-2-one |
| d.92 | 4-[5-(4-benzyl-6-chloro-2-oxo-1H-quinoléine-3-yl)-3-[4-(1-éthylindazol-5-yl)phényle]-3,4-dihydropyrazol-2-yl]-4-oxo-butanoïque |
| d.93 | 4-[5-(4-benzyl-6-chloro-2-oxo-1H-quinoléine-3-yl)-3-[4-(2-éthylindazol-5-yl)phényle]-3,4-dihydropyrazol-2-yl]-4-oxo-butanoïque |
| d.95 | 4-benzyl-6-chloro-3-(3-phényle-2-propanoyl-3,4-dihydropyrazol-5-yl)-1H-quinoléine-2-one |
| d.96 | 3-[2-acétyl-3-(p-tolyl)-3,4-dihydropyrazol-5-yl]-4-benzyl-6-chloro-1H-quinoléine-2-one |
| d.98 | Acide 4-[5-(6-chloro-2-oxo-4-phényle-1H-quinoléin-3-yl)-3-(3-pyridyl)-3,4-dihydropyrazol-2-yl]-4-oxo-butanoïque |
| d.99 | Acide 4-[5-(6-chloro-2-oxo-4-phényle-1H-quinoléin-3-yl)-3-(2-pyridyl)-3,4-dihydropyrazol-2-yl]-4-oxo-butanoïque |
| | |
| d.101 | Acide 4-[5-(4-benzyl-6-chloro-2-oxo-1H-quinoléine-3-yl)-3-(3-pyridyl)-3,4-dihydropyrazol-2-yl]-4-oxo-butanoïque |
| d.105 | Acide 4-[5-(4-benzyl-6-chloro-2-oxo-1H-quinoléine-3-yl)-3-(2-chlorophényle)-3,4-dihydropyrazol-2-yl]-4-oxo-butanoïque |
| d.106 | Acide 4-[5-(4-benzyl-6-chloro-2-oxo-1H-quinoléine-3-yl)-3-(3,4-diméthoxyphényle)-3,4-dihydropyrazol-2-yl]-4-oxo-butanoïque |
| d.109 | Acide 4-[5-(6-chloro-4-morpholino-2-oxo-1H-quinoléine-3-yl)-3-(4-méthoxyphényle)-3,4-dihydropyrazol-2-yl]-4-oxo-butanoïque |
